# EUROPEAN PATENT SPECIFICATION

(11) **EP 1 888 565 B1**
(45) Date of publication and mention of the grant of the patent: **23.03.2011**
(21) Application number: 06750082.7
(22) Date of filing: 11.04.2006
(51) Int. Cl.: C07D 407/14, C07D 417/14, C07D 401/14, C07D 403/04

(54) **2, 6-DI (HETERO) ARYL -4-AMIDO-PYRIMIDINES AS ADENOSINE RECEPTOR ANTAGONISTS**
2, 6-DI-(HETERO-)ARYL-4-AMIDO-PYRIMIDINE ALS ADENOSIN-REZEPTOR-ANTAGONISTEN
2,6-DI-(HÉTÉRO)ARYL-4-AMIDO-PYRIMIDINES UTILES COMME ANTAGONISTES DES RÉCEPTEURS DE L'ADÉNOSINE

(30) Priority: 11.04.2005 US 670482 P
(43) Date of publication of application: 20.02.2008
(73) Proprietor: Almirall, S.A., 08022 Barcelona (ES)
(72) Inventor: SLEE, Deborah, Cardiff, CA 92007 (US); LANIER, Marion, San Diego, CA 92124 (US); VONG, Binh, G., San Diego, CA 92126 (US); CHEN, Yongsheng, San Diego, California 92127 (US); ZHANG, Xiaohu, San Diego, CA 92131; (US); LIN, Emily, San Diego, CA 92103 (US); MOORJANI, Manisha, San Diego, CA 92130-4650 (US); CASTRO PALOMINO LARIA, Julio, Cesar, E-08330 Premia del Mar (ES)
(74) Representative: Viering, Jentschura & Partner
(86) International application number: PCT/US2006/013940
(87) International publication number: WO 2006/110884

(56) References cited:
- EP-A- 1 283 056
- EP-A- 1 439 175
- WO-A-02/47690
- WO-A-2005/033084
- WO-A-2005/033085
- WO-A1-2005/058883
- US-A1- 2001 027 196
- A.P.IJZERMAN ET AL: "2,4,6-trisubstituted pyrimidines as a new class of selective adenosine A1 receptor antagonists" J.MED.CHEM., vol. 47, no. 26, 13 November 2004 (2004-11-13), pages 6529-6540, XP002392392

## Description

### Field of the invention

The present invention relates to new antagonists of adenosine receptors, in particular antagonist of the A_{2A} adenosine receptor subtype, the use of said compounds in the treatment of diseases, and disorders susceptible of being ameliorated by antagonism of adenosine receptors, in particular in the treatment of disorders of the central nervous system which are known to be improved by the use of antagonists of the A_{2A} adenosine receptors, more specifically movement disorders such as Parkinson's disease, restless leg syndrome and dyskinesia and to pharmaceutical compositions comprising said compounds.

### Description of the Related Art

The effects of adenosine are mediated through at least four specific cell membrane receptors so far identified and classified as receptors A₁,-A_{2A}, A_{2B} and A₃ belonging to the G protein-coupled receptor family. The A₁ and A₃ receptors down-regulate cellular cAMP levels through their coupling to G proteins, which inhibit adenylate cyclase. In contrast, A_{2A} and A_{2B} receptors couple to G proteins that activate adenylate cyclase and increase intracellular levels of cAMP. Through these receptors, adenosine regulates a wide range of physiological functions.

Thus, in the cardiovascular system the activation of the A₁ receptor protects cardiac tissue from the effects of ischemia and hypoxia. A similar protective effect is also produced by antagonism of the A_{2A} receptor, which enhances A₁-receptor-induced antiadrenergic responses and may also be useful in the treatment of acute myocardial ischemia and supraventricular arrhythmias (Norton GR et al. Am J Physiol. 1999; 276(2 Pt 2):H341-9; Auchampach JA, Bolli R. Am J Physiol. 1999; 276(3 Pt 2):H1113-6). In addition, the A_{2B} adenosine receptor subtype (Feoktistov, I. et al., Pharmacol. Rev. 1997. 49, 381-402) appears to be involved in the control of vascular tone and the regulation of vascular smooth muscle growth.

In the kidney, adenosine exerts a biphasic action, inducing vasodilation at high concentrations and vasoconstriction at low concentrations. Thus, adenosine plays a role in the pathogenesis of some forms of acute renal failure that may be ameliorated by A₁ receptor antagonists (Costello-Boerrigter LC, et al. Med Clin North Am. 2003 Mar; 87(2): 475-91; Gottlieb SS., Drugs. 2001; 61(10): 1387-93).

Adenosine is also involved in the physiopathology of the immune system. It can induce degranulation of activated human mast cells through the A_{2B} and /or A₃ receptor. Thus A_{2B} and /or A₃ antagonists prevent mast cell degranulation and are, therefore, useful in the treatment, prevention or suppression of disease states induced by activation of the A_{2B} and/or A₃ receptor and mast cell degranulation. These disease states include but are not limited to asthma, myocardial reperfusion injury, allergic reactions including but not limited to rhinitis, urticaria, scleroderm arthritis, other autoimmune diseases and inflammatory bowel diseases.

Furthermore, in the respiratory system adenosine induces bronchoconstriction, modulates airway inflammation and promotes neutrophil chemotaxis. Therefore, an adenosine antagonist would be particularly useful in the treatment of asthma.

In the gastrointestinal and metabolic system, the A_{2B} adenosine receptor subtype (Feoktistov, I. et al., Pharmacol. Rev. 1997, 49, 381-402) seems to be involved in the regulation of hepatic glucose production, the modulation of intestinal tone, as well as intestinal secretion. Thus, A_{2B} antagonists may also be useful in the treatment of diabetes mellitus and obesity.

In the central nervous system adenosine is a potent endogenous neuromodulator, which controls the presynaptic release of many neurotransmitters and is thus involved in motor function, sleep, anxiety, pain and psychomotor activity. All adenosine receptor subtypes are present in the brain, with A₁ and A_{2A} subtypes being differentially distributed. The former are found predominantly in the hippocampus and cortex, whilst the latter are found mainly in the striatum. Adenosine A_{2A} receptors modulate the release of GABA in the striatum, which possibly regulates the activity of medium spiny neurons.

Thus, A_{2A} receptor antagonists may be a useful treatment for neurodegenerative movement disorders such as Parkinson and Huntington's disease (Tuite P, et al., J. Expert Opin Investig Drugs. 2003; 12: 1335-52; Popoli P. et al. J Neurosci. 2002; 22:1967-75), dystonias such as restless leg syndrome (Happe S, et al., Neuropsychobiology. 2003; 48: 82-6), and dyskinesias such as those caused by prolonged use of neuroleptic and dopaminergic drugs (Jenner P. J Neuron. 2000; 247 Suppl2: II43-50).

In the treatment of Parkinson's disease an A_{2A} antagonist may be useful not only as monotherapy, but also when administered in combination with L-DOPA and/or one or more of the following drugs: dopamine agonists, inhibitors of dopamine decarboxylase, catechol-O-methyltransferase inhibitors and inhibitors of monoamine oxidase.

In addition, A_{2A} antagonists may have therapeutic potential as neuroprotectants (Stone TW. et al., Drug. Dev. Res. 2001; 52: 323-330), and in the treatment of sleep disorders (Dunwiddie TV et al., Ann. Rev. Neurosci. 2001; 24: 31-55).

2,6-diphenyl-4-amidopyrimidines missing a terminal hydrogen containing heterocycle are described, e.g., by A.P. Ijzerman et al. in "2,4,6-trisubstituted pyrimidines as a new class of selective adenosine A1 receptor antagonists", J.MED.CHEM., vol. 47, no. 26, 13 November 2004, pages 6529-6540. EP 1 283 056 A1 describes 2,4,5,6-substituted pyrimidine compounds having an A₂ receptor antagonism and its use as defecation-promoting agents.

US 2001/027196 A1 describes 2,4,6-trisubstituted pyrimidines having, e.g., a 2-amino and a 4-acetamide substituent.

It has now been found that certain 4-aminopyrimidine derivatives are novel potent antagonists of the A_{2A} adenosine receptor and can therefore be used in the treatment or prevention of diseases susceptible to amelioration by antagonism of the adenosine receptor.

Further objectives of the present invention are to provide a method for preparing said compounds; pharmaceutical compositions comprising an effective amount of said compounds; the use of the compounds in the manufacture of a medicament for the treatment of pathological conditions or diseases susceptible of being improved by antagonism of an adenosine receptor, in particular by antagonism of the A_{2A} adenosine receptor; use of the compounds in the manufacture of a medicament for treating pathological conditions or diseases susceptible to amelioration by antagonism of an adenosine receptor, in particular by antagonism of the A_{2A} adenosine receptor wherein the compounds of the invention is to be administrated to a subject in need of treatment and combinations of said compounds with one or more of the following drugs: L-DOPA, dopamine agonists, inhibitors of dopamine decarboxylase, catechol-O-methyltransferase inhibitors and inhibitors of monoamine oxidase.

### BRIEF SUMMARY OF THE INVENTION

In brief, this invention is generally directed to adenosine receptor antagonists, as well as to methods for their preparation and use, and to pharmaceutical compositions containing the same. More specifically, the adenosine receptor antagonists of this invention are compounds having the following general structure (I): 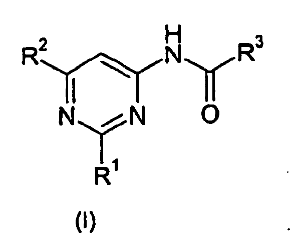 and pharmaceutically acceptable salts, esters, solvates or stereoisomers thereof, wherein R¹, R² and R³ are as defined below. Specific embodiments are described in the dependent claims.

The compounds of this invention may generally be used to treat a variety of disorders or conditions, particularly those which benefit from inhibition of adenosine (particularly A_{2A}) receptors. Accordingly, in another embodiment, they are used for treating one or more of a variety of diseases or conditions, including (but not limited to) ischemia, supraventricular arrhythmias, acute renal failure, myocardial reperfusion injury, autoimmune disease, inflammatory bowel diseases, asthma, diabetes mellitus, obesity, Parkinson disease, Huntington's disease, dystonia or dyskinesia.

The methods generally involve administering an effective amount of one or more compounds of this invention, typically in the form of a pharmaceutical composition, to an animal (also referred to here as a "patient", including a human) in need thereof. Accordingly, in still another embodiment, compositions are disclosed containing one or more compounds of this invention and a pharmaceutically acceptable carrier and/or diluent.

These and other aspects of the invention will be apparent upon reference to the following detailed description. To that end, various references are set forth herein which describe in more detail certain procedures, compounds and/or compositions.

### DETAILED DESCRIPTION OF THE INVENTION

As mentioned above, the present invention is directed generally to compounds useful as adenosine receptor antagonists. The compounds of this invention have the following structure (I): 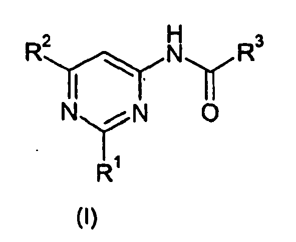 and pharmaceutically acceptable salts, esters, solvates, or stereoisomers thereof, wherein:
each of R¹ and R² independently is an aryl or heteroaryl group optionally substituted by one or more substituents selected from the group of lower alkyl, halogen, cycloalkyl, hydroxy, lower alkoxy, -SH, NO₂, lower alkylthio, lower alkylamino, cyano, and amino, wherein the lower alkyl, cycloalkyl, lower alkoxy, lower alkylthio and lower alkylamino groups are optionally substituted;
R³ is -(CR⁴R⁵)ₙ-R⁶, -(CR⁴R⁵)ₙ-NR⁷R⁸, -O-(CR⁴R⁵)ₙ-R⁶ or is -(CR⁴R⁵)ₙ-R⁸;
each of R⁴ and R⁵ independently is at each occurrence selected from the group of hydrogen, lower alkyl, halogen, cycloalkyl, hydroxy, lower alkoxy, -SH, NO₂, lower alkylthio, lower alkylamino, cyano, and amino, wherein the lower alkyl, cycloalkyl, lower alkoxy, lower alkylthio and lower alkylamino groups are optionally substituted;
R⁸ is a heterocycle having at least one nitrogen atom, wherein the heterocycle is optionally substituted by one or more members selected from the group of lower alkyl, alkoxy, cycloalkyl, aminoalkyl, alkylaminoalkyl, dialkylaminoalkyl, alkoxyalkyl, aryl, arylalkyl, heteroaryl heteroarylalkyl, heterocycle, heterocycylalkyl, amino, alkylamino, dialkylamino, cycloalkylamino, halogen, haloalkyl, ester, amide, acyl, carbamoyl, carbamoylalkyl, oxo, isoquinolinyl and imidoylamino, wherein said lower alkyl, alkoxy, cycloalkyl, aminoalkyl, alkylaminoalkyl, dialkylaminoalkyl, alkoxyalkyl, aryl, arylalkyl, heteroaryl heteroarylalkyl, heterocycle, heterocycylalkyl, amino, alkylamino, dialkylamino, cycloalkylamino, haloalkyl, ester, amide, acyl, carbamoyl, carbamoylalkyl, isoquinolinyl and imidoylamino groups are optionally substituted with lower alkyl, alkoxy, hydroxy, oxo or halogen;
R⁷ is hydrogen or optionally substituted lower alkyl;
R⁸ is -(CR⁴R⁵)ₙ-R⁶; or
R⁷ and R⁸ together with the nitrogen atom to which they are attached form an optionally substituted heterocyclic ring; and
n is independently at each occurrence 0, 1 , 2, 3 or 4, for example n is 1 or 2;
with the proviso that when R¹ and R² are both heteroaryl, R⁶ is a non-aromatic heterocycle.

Other aspects of the present invention are: a) pharmaceutical compositions containing a pharmaceutically effective amount of said compounds, b) the use of said compounds in the manufacture of a medicament for the treatment of diseases susceptible of being improved by antagonism of an adenosine receptor, in particular by antagonism of the A_{2A} adenosine receptor; c) use of the compounds in the manufacture of a medicament for the treatment of diseases susceptible to amelioration by antagonism of an adenosine receptor, in particular by antagonism of the A_{2A} adenosine receptor, which use comprises the administration of the compounds of the invention to a subject in need of treatment and administration of combinations of said compounds with one or more of the following drugs: L-DOPA1 dopamine agonists, inhibitors of dopamine decarboxylase, catechol-O-methyltransferase inhibitors and inhibitors of monoamine oxidase.

As used herein the term lower alkyl embraces optionally substituted, linear or branched alkyl radicals having 1 to 8 carbon atoms. Typically lower alkyl groups have 1 to 6 or 1 to 4 carbon atoms. Typical examples of substituents in said alkyl groups are halogen, hydroxy and amino.

Examples of lower alkyl groups include methyl, ethyl, n-propyl, i-propyl, n-butyl, sec-butyl and tert-butyl, n-pentyl, 1-methylbutyl, 2-methylbutyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1 ,3-dimethylbutyl, 2,2-dimethylbutyl, 2,3-dimethylbutyl, 2- methylpentyl, 3-methylpentyl and iso-hexyl radicals.

As used herein, the term lower alkoxy embraces optionally substituted, linear or brached oxy-containing radicals each having alkyl portions of 1 to 8, typically 1 to 6 and more typically 1 to 4 carbon atoms. Typical examples of substituents in said alkoxy groups are halogen, hydroxy and amino.

Examples of lower alkoxy groups include methoxy, ethoxy, n-propoxy, I-propoxy, n-butoxy, sec-butoxy, t-butoxy, trifluoromethoxy, difluoromethoxy, hydroxymethoxy, 2-hydroxyethoxy or 2-hydroxypropoxy.

As used herein, the term lower alkylthio embraces radicals containing an optionally substituted, linear or brached alkyl radicals of 1 to 8, typically 1 to 6 and more typically 1 to 4 carbon atoms. Typical examples of substituents in said alkylthio groups are halogen, hydroxy and amino.

Examples of optionally substituted lower alkylthio radicals include methylthio, ethylthio, n-propylthio, i-propylthia, n-butylthio, sec-butylthio, t-butylthio, trifluoromethylthio, difluoromethylthio, hydroxymethylthio, 2-hydroxyethylthio or 2-hydroxypropylthio.

As used herein the term "acyl" refers to groups represented by the formula alkyl-C(=O)-, where the alkyl group may be substituted or unsubstituted.

As used herein, the term cyclic group embraces, unless otherwise specified, carbocyclic and heterocyclic radicals. The cyclic radicals can contain one or more rings. Carbocyclic radicals may be aromatic or alicyclic, for example cycloalkyl radicals. Heterocyclic radicals also include heteroaryl radicals.

As used herein, the term aromatic group embraces typically a 5- to 14- membered aromatic ring system, such as a 5- or 6- membered ring which may contain one or more heteroatoms selected from O, S and N. When no heteroatoms are present the radical is named aryl radical and when at least one heteroatom is present it is named heteroaryl radical. The aromatic radical can be monocyclic or polycyclic, such as phenyl or naphthyl. When an aromatic radical or moiety carries 2 or more substituents, the substituents may be the same or different.

As used herein, the term aryl radical embraces typically a C₅-C₁₄ monocyclic or polycyclic aryl radical such as phenyl, naphthyl, anthranyl or phenanthryl. When an aryl radical carries 2 or more substituents, the substituents may be the same or different.

As used herein, the term heteroaryl radical embraces typically a 5- to 14- membered ring system comprising at least one heteroaromatic ring and containing at least one heteroatom selected from U, S and N. A heteroaryl radical may be a single ring or two or more fused rings wherein at least one ring contains a heteroatom.

Examples of heteroaryls include pyridyl, pyrazinyl, pyrimidinyl, pyridazinyl, furyl, oxadiazolyl, oxazolyl, isoxazolyl, imidazolyl, thiazolyl, thiadiazolyl, thienyl, pyrrolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl and pyrazolyl. When a heteroaryl radical carries 2 or more substituents, the substituents may be the same or different.

As used herein, the term heterocycle radical embraces typically a 5- to 14- membered ring system comprising at least one heterocyclic ring and containing at least one heteroatom selected from O, S and N. A heteocycle radical may be a single ring or two or more fused rings wherein at least one ring contains a heteroatom. A heterocycle radical may be aromatic, in which case it is a heteroaryl radical, or it may be non-aromatic.

Examples of aromatic heterocycles (i.e., heteroaryls) are provided above. Examples of non-aromatic heterocycles include piperidinyl, piperazinyl, morpholinyl, pyrrolidinyl, thiomorpholinyl, oxazolidinyl, imidazolidinyl, thiazolidinyl, azepanyl, [1,4]diazepanyl, [1,4]oxazepanyl and thiazepanyl.

As used herein, the term cycloalkyl embraces saturated optionally substituted carbocyclic radicals and, unless otherwise specified, a cycloalkyl radical typically has from 3 to 7 carbon atoms. The preferred substituents in said cycloalkyl groups are selected from halogen atoms, hydroxy groups, alkyl groups and amino groups.

Examples include cyclopropyl, cyclobutyl, cyclopentyl, cyclohexyl and cycloheptyl. It is preferably cyclopropyl, cyclopentyl or cyclohexyl. When a cycloalkyl radical carries 2 or more substituents, the substituents may be the same or different.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

The substituents of an *"optionally substituted"* structure may include, without limitation, one or more, typically one to four, and more typically one to two of the following substituents: alkyl, alkenyl, alkynyl, aryl, heteroaryl, alkoxy, aryloxy, alkylthio, arylthio, cycloalkyl, arylalkyl, amino, alkylamino, dialkylamino, amido (e.g. CONH2, CONHalkyl and CONHdialkyl and reverse NCOH or NCOalkyl), F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, and CF₃.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

According to one embodiment of the present invention in the compounds of formula (I), R¹ represents a monocyclic aryl or heteroaryl group selected from the group of phenyl, pyridinyl, furanyl, thiophenyl, thiazolyl, pyrazolyl, imidiazolyl, oxazolyl, isoxazolyl and oxadiazolyl groups which are optionally substituted by one or more substituents selected from the group of halogen, hydroxyl, amino, alkylamino, optionally substituted lower alkoxy and optionally substituted lower alkyl.

According to another embodiment of the present invention in the compounds of formula (I), R² represents a monocyclic aryl or heteroaryl group selected from the group of phenyl, pyridinyl, furanyl, thiophenyl, thiazolyl, pyrazolyl, imidiazolyl, oxazolyl, isoxazolyl and oxadiazolyl groups which are optionally substituted by one or more substituents selected from the group of halogen, hydroxyl, amino, alkylamino, optionally substituted lower alkoxy and optionally substituted lower alkyl.

According to still another embodiment of the present invention in the compounds of formula (I), R³ represents a heterocycle having at least one nitrogen atom, wherein the heterocycle is optionally substituted by one or more lower alkyl groups. Such hetereocycles include, for example, optionally substituted piperidinyl, piperazinyl, morpholinyl, thiomorpholinyl, pyrrolidinyl, isoquinolinyl, diazepanyl, dihydropyrrolyl, azepanyl, oxazepanyl, and pyrrolopyrazinyl.

In one embodiment of the present invention n may be 1 or 2 and R¹ may be selected from the group of pyridinyl, furanyl, thiophenyl, thiazolyl, pyrazolyl, imidazolyl, oxazolyl and isoxazolyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂,_SH, SCH₃, OH, OCH₃, OCF₃, CH₃, and CF₃. In a further embodiment R² may then be selected from the group of pyridinyl, thiazolyl and pyrazolyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino amido, F, Cl, Br, I, CN. NO₂, NH₂, NHCH₃, NHCH₂CH₃, N(CH₃)₂, N(CH₂CH₃)₂, SH. SCH₃,OH, OCH₃,_OCF₃, CH₃. and CF₃ In a still further embodiment R¹ may be selected selected from the group of pyridinyl furanyl 5-methyl-furanyl, thiophenyl, thiazolyl, pyrazoly, imidazolyl, oxazolyl, 4-methyl-oxazolyl and isoxazolyl; and R² may be selected from the group of pyridinyl, thiazolyl, pyrazolyl and 3,5-dimethylpyrazolyl.

In a still further embodiment R³ may then be selected from -CR⁴R⁵)ₙ-R⁶ or -(CR⁴R⁵)ₙ-NR⁷R⁸: wherein each of R⁴ and R⁵ independently is at each occurrence hydrogen or lower alkyl having 1 to 8 carbon atoms: R⁶ is a heterocyclic ring selected from the group of piperidinyl, piperazinyl, morpholinyl, thiomorpholinyl, pyrrolidinyl, isoquinolinyl, diazepanyl, dihydropyrroiyl, azepanyl, oxazepanyl, and pyrrolopyrazinyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃; and R⁷ and R⁸ together with the nitrogen atom to which they are attached form a heterocyclic ring selected from the group of piperidinyl, piperazinyl, morpholinyl, thiomorpholinyl, pyrrolidinyl, isoquinolinyl, diazeoanyl, dihydropyrrolyl, azepanyl, oxazepanyl, and pyrrolopyrazinyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃. For example, R⁶ may be substituted with one or more members selected from the group of lower alkyl having 1 to 8 carbon atoms, alkoxy, amino, alkylamino, dialkylamino, piperidinyl, piperazinyl, pyrrolidinyl, morpholinyl, phenyl and benzyl. In this embodiment, R¹ may be furanyl, 5-methyl-furanyl, oxazolyl, 4-methyl-oxazolyl, pyridinyl, pyrazolyl, or isoxazolyl and R² is pyrazolyl, for example R¹ may be furanyl, 5-methyl-furanyl, pyridinyl, thiophenyl or thiazolyl and R² is thiazolyl. In a further embodiment R¹ may be 5-methyl-furanyl, thiazolyl or pyridinyl and R² may be 3,5- dimethylpyrazolyl, for example R¹ may be 5-methyl-furanyl or thiophenyl and R² may be pyridinyl.

In a still further embodiment. R¹ may be furanyl or 5-methyl-furanyl; and R² may be pyrazolyl, 3,5-dimethylpyrazolyl or thiazolyl. In this embodiment. R³ may be -(CR⁴R⁵VR⁶; each of R⁴ and R⁵ may be hydrogen; and n may be 1 or 2. Still, R⁶ may be piperazinyl substituted with lower alkyl having 1 to 8 carbon atoms, pyrolidinyl substsituted with alkylamino or dialkylamino, [1,4]-diazepanyl substituted with lower alkyl having 1 to 8 carbon atoms or [1.4]-oxazepanyl.

Particular individual compounds of the invention include:
N-(2-Furan-2-yl-6-pyrazol-1-yl-pyrimidin-4-yl)-2-piperidin-1-yl-acetamide (Compound 1-1);
N-(2-Furan-2-yl-6-pyrazol-1-yl-pyrimidin-4-yl)-2-morpholin-4-yl-acetamide (Compound 1-2);
N-(2-Furan-2-yl-6-pyrazol-1-yl-pyrimidin-4-yl)-2-(4-methyl-piperazin-1-yl)-acetamide (Compound 1-3);
N-(2-Furan-2-yl-6-pyrazol-1-yl-pyrimidin-4-yl)-2-piperazin-1-yl-acetamide (Compound 1-4);
N-[2-(5-Methyl-furan-2-yl)-6-thiazol-2-yl-pyrimidin-4-yl]-2-morpholin-4-yl-acetamide (Compound 2-1);
N-[2-(5-Methyl-furan-2-yl)-6-thiazol-2-yl-pyrimidin-4-yl]-2-pipendin-1-yl-acetamide (Compound 2-2);
N-[2-(5-Methyl-furan-2-yl)-6-thiazol-2-yl-pyrimidin-4-yl]-2-pyrrolidin-I -yl-acetamide (Compound 2-3);
N-[2-(5-Methyl-furan-2yl)-6thiazol-2-yl-pyrimidin-4-yl]-2-(4-methyl-piperazin-1-yl)-acetamide (Compound 2-4);
2-(2,5-Dimethyl-piperazin-1-yl)-N-[2-(5-methyl-furan-2-yl)-6-thiazol-2-yl-pyrimidin-4-yl]-acetamide (Compound 2-5);
N-[2-(5-Methyl-furan-2-yl)-6-thiazol-2-yl-pyrimidin-4-yl]-2-piperazin-1-yl-acetamide (Compound 2-6);
N-[2-(5-Methyl-furan-2-yl)-6-thiazol-2-yl-pyrimidin-4-yl]-2-(4-phenyl-piperazin-1-yl)-acetamide (Compound 2-7);
2-[1,4]Diazepan-1-yl-N-[2-(5-methyl-furan-2-yl)-6-thiazol-2-yl-pyrimidin-4-yl]-acetamide (Compound 2-8);
N-[2-(5-Methyl-furan-2-yl)-6-thiazol-2-yl-pyrimidin-4-yl]-2-(2-phenyl-piperidin-1-yl)-acetamide (Compound 2-9);
N-[2-(5-Methyl-furan-2-yl)-6-thiazol-2-yl-pyrimidin-4-yl]-2-(3-phenyl-piperidin-1-yl)-acetamide (Compound 2-10);
2-(4-Benzyl-piperazin-1-yl)-N-[2-(5-methyl-furan-2-yl)-6-thiazol-2-yl-pyrimidin-4-yl]-acetamide (Compound 2-11);
N-[2-(5-Methyl-furan-2-yl)-6-thiazol-2-yl-pyrimidin-4-yl]-2-(2-methyl-piperidin-1-yl)-acetamide (Compound 2-12);
2-(2,5-Dihydro-pyrrol-1-yl)-N-[2-(5-methyl-furan-2-yl)-6-thiazol-2-yl-pyrimidin-4-yl]-acetamide (Compound 2-13);
2-(2,5-Dimethyl-2,5-dihydro-pyrrol-1-yl)-N-[2-(5-methyl-furan-2-yl)-6-thiazol-2-yl-pyrimidin-4-yl]-acetamide (Compound 2-14);
2-(2,5-Dimethyl-pyrrolidin-1-yl)-N-[2-(5-ethyl-furan-2-yl)-6-thiazol-2-yl-pyrimidin-4-yl]-acetamide (Compound 2-15);
2-(3,5-Dimethyl-piperazin-1-yl)-N-[2-(5-methyl-furan-2-yl)-6-thiazol-2-yl-pyrimidin-4-yl]-acetamide (Compound 2-16);
2-[4-(2-Methoxy-phenyl)-piperazin-1-yl]-N-[2-(5-methyl-furan-2-yl)-6-thiazol-2-yl-pyrimidin-4-yl]-acetamide (Compound 2-17);
N-[2-(5-Methyl-furan-2-yl)-6-thiazol-2-yl-pyrimidin-4-yl]-2-(3-methyl-piperidin-1-yl)-acetamide (Compound 2-18);
2-Azepan-1-yl-N-[2-(5-methyl-furan-2-yl)-6-thiazol-2-yl-pyrimidin-4-yl]-acetamide (Compound 2-19);
2-((S)-2-Methoxymethyl-pyrrolidin-1-yl)-N-[2-(5-methyl-furan-2-yl)-6-thiazol-2-yl-pyrimidin-4-yl]-acetamide (Compound 2-20);
2-(3,3-Dimethyl-piperidin-1-yl)-N-[2-(5-methyl-furan-2-yl)-6-thiazol-2-yl-pyrimidin-4-yl]-acetamide (Compound 2-21);
2-(3,5-Dimethyl-piperidin-1yl)-N-[2-(5-methy-furan-2-yl)-6-thiazol-2-yl-pyrimidin-4-yl]-acetamide (Compound 2-22);
N-[2-(5-Methyl-furan-2-yl)-6-thiazol-2-yl-pyrimidin-4-yl]-2-(4-phenyl-piperidin-1-yl)-acetamide (Compound 2-23);
N-[2-(5-Methyl-furan-2-yl)-6-thiazol-2-yl-pyrimidin-4-yl]-2-(4-methyl-piperidin-1-yl)-acetamide (Compound 2-24);
2-(4-Benzyl-piperidin-1-yl)-N-[2-(5-methyl-furan-2-yl)-6-thiazol-2-yl-pyrimidin-4-yl]-acetamide (Compound 2-25);
2-[1,4']Bipiperidinyl-1'-yl-N-[2-(5-methyl-furan-2-yl)-6-thiazol-2-yl-pyrimidin-4-yl]-acetamide (Compound 2-26);
2-(3,4-Dihydro-1H-isoquinolin-2-yl)-N-[2-(5-methyl-furan-2-yl)-6-thiazol-2-yl-pyrimidin-4-yl]-acetamide (Compound 2-27);
N-[2-(5-Methyl-furan-2-yl)-6-thiazol-2-yl-pyrimidin-4-yl]-2-(octahydro-isoquinolin-2-yl)-acetamide (Compound 2-28);
N-[2-(5-Methyl-furan-2-yl)-6-thiazol-2-yl-pyrimidin-4-yl]-2-((S)-pyrrolidin-1-ylmethyl-pyrrolidin-1-yl)-acetamide (Compound 2-29);
2-[4-(3,4-Dimethyl-phenyl)piperazin-1-yl]-N-[2-(5-methyl-furan-2-yl)-6-thiazol-2-yl-pyrimidin-4-yl]-acetamide (Compound 2-30);
N-[2-(5-Methyl-furan-2-yl)-6-thiazol-2-yl-pyrimidin-4-yl]-2-(4-pyrrolidin-1-yl-piperidin-1-yl)-acetamide (Compound 2-31);
2-[4-(3-Chloro-phenyl)-piperazin-1-yl]-N-[2-(5-methyl-furan-2-yl)-6-thiazol-2-yl-pyrimidin-4-yl]-acetamide (Compound 2-32);
2-(2,6-Dimethyl-morpholin-4-yl)-N-[2-(5-methyl-furan-2-yl)-6-thiazol-2-yl-pyrimidin-4-yl]-acetamide (Compound 2-33);
N-[2-(5-Methyl-furan-2-yl)-6-thiazol-2-yl-pyrimidin-4-yl]-2-(3-methyl-4-m-tolyl-piperazin-1-yl)-acetamide (Compound 2-34);
2-(4-Methyl-[1,4]diazepan-1-yl)-N-[2-(5-methyl-furan-2-yl)-6-thiazol-2-yl-pyrimidin-4-yl]-acetamide (Compound 2-35);
2-[4-(3-Methoxy-phenyl)-piperazin-1-yl]-N-[2-(5-methyl-furan-2-yl)-6-thiazol-2-yl-pyrimidin-4-yl]-acetamide (Compound 2-36);
N-[2-(5-Methyl-furan-2-yl)-6-thiazol-2-yl-pyrimidin-4-yl]-2-[2-(2-piperidin-1-yl-ethyl)-piperidin-1-yl]-acetamide (Compound 2-37);
N-[2-(5-Methyl-furan-2-yl)-6-thiazol-2-yl-pyrimidin-4-yl]-2-[1,4]oxazepan-4-yl-acetamide (Compound 2-38);
2-(4,4-Difluoro-piperidin-1-yl)-N-[2-(5-methyl-furan-2-yl)-6-thiazol-2-yl-pyrimidin-4-yl]-acetamide (Compound 2-39);
2-(4-Acetyl-piperazin-1-yl)-N-[2-(5-methyl-furan-2-yl)-6-thiazol-2-yl-pyrimidin-4-yl]-acetamide (Compound 2-40);
2-[(1-Benzyl-pyrrolidin-3-yl)-methyl-amino]-N-[2-(5-methyl-furan-2-yl)-6-thiazol-2-yl-pyrimidin-4-yl]-acetamide (Compound 2-41);
2-(4-Acetyl-[1,4]diazepan-1-yl)-N-[2-(5-methyl-furan-2-yl)-6-thiazol-2-yl-pyrimidin-4-yl]-acetamide (Compound 2-42);
2-(4-Benzyl-[1,4]diazepan-1-yl)-N-[2-(5-methyl-furan-2-yl)-6-thiazol-2-yl-pyrimidin-4-yl]-acetamide (Compound 2-43);
2-(3-Dimethylamino-pyrrolidin-1-yl)-N-[2-(5-methyl-furan-2-yl)-6-thiazol-2-yl-pyrimidin-4-yl]-acetamide (Compound 2-44);
N-[2-(5-Methyl-furan-2-yl)-6-thiazol-2-yl-pyrimidin-4-yl]-2-(3-trifluoromethyl-piperidin-1-yl)-acetamide (Compound 2-45);
2-(3-Diethylamino-pyrrolidin-1-yl)-N-[2-(5-methyl-furan-2-yl)-6-thiazol-2-yl-pyrimidin-4-yl]-acetamide (Compound 2-46);
2-((R)-3-Dimethylamino-pyrrolidin-1-yl)-N-[2-(5-methyl-furan-2-yl)-6-thiazol-2-yl-pyrimidin-4-yl]-acetamide (Compound 2-47);
2-((S)-3-Dimethylamino-pyrrolidin-1-yl)-N-[2-(5-methyl-furan-2-yl)-6-thiazol-2-yl-pyrimidin-4-yl]-acetamide (Compound 2-48);
N-[2-(5-Methyl-furan-2-yl)-6-thiazol-2-yl-pyrimidin-4-yl]-2-(2-pyrrolidin-1-yl-ethylamino)-acetamide (Compound 2-49);
1-{[2-(5-Methyl-furan-2-yl)-6-thiazol-2-yl-pyrimidin-4-ylcarbamoyl]-methyl}-piperidine-3-carboxylic acid amide (Compound 2-50);
1-{[2-(5-Methyl-furan-2-yl)-6-thiazol-2-yl-pyrimidin-4-ylcarbamoyl]-methyl}-piperidine-4-carboxylic acid amide (Compound 2-51);
N-[2-(5-Methyl-furan-2-yl)-6-thiazol-2-yl-pyrimidin-4-yl]-2-((R)-3-methyl-piperazin-1-yl)-acetamide (Compound 2-52);
2-[4-(Isopropylcarbamoyl-methyl)-piperazin-1-yl]-N-[2-(5-methyl-furan-2-yl)-6-thiazol-2-yl-pyrimidin-4-yl]-acetamide (Compound 2-53);
2-(4-Amino-piperidin-1-yl)-N-[2-(5-methyl-furan-2-yl)-6-thiazol-2-yl-pyrimidin-4-yl]-acetamide (Compound 2-54);
2-(4-Dimethylamino-piperidin-1-yl)-N-[2-(5-methyl-furan-2-yl)-6-thiazol-2-yl-pyrimidin-4-yl]-acetamide (Compound 2-55);
2-(4-Diethylamino-piperidin-1-yl)-N-[2-(5-methyl-furan-2-yl)-6-thiazol-2-yl-pyrimidin-4-yl]-acetamide (Compound 2-56);
N-[2-(5-Methyl-furan-2-yl)-6-thiazol-2-yl-pyrimidin-4-yl]-2-(4-morpholin-4-yl-piperidin-1-yl)-acetamide (Compound 2-57);
2-(4-Isopropylamino-piperidin-1-yl)-N-[2-(5-methyl-furan-2-yl)-6-thiazol-2-yl-pyrimidin-4-yl]-acetamide (Compound 2-58);
2-(4-Cyclopentylamino-piperidin-1-yl)-N-[2-(5-methyl-furan-2-yl)-6-thiazol-2-yl-pyrimidin-4-yl]-acetamide (Compound 2-59);
2-(4-Dipropylamino-piperidin-1-yl)-N-[2-(5-methyl-furan-2-yl)-6-thiazol-2-yl-pyrimidin-4-yl]-acetamide (Compound 2-60);
2-(3-Amino-pyrrolidin-1-yl)-N-[2-(5-methyl-furan-2-yl)-6-thiazol-2-yl-pyrimidin-4-yl]-acetamide (Compound 2-61);
2-(3-Isopropylamino-pyrrolidin-1-yl)-N-[2-(5-methyl-furan-2-yl)-6-thiazol-2-yl-pyrimidin-4-yl]-acetamide (Compound 2-62);
2-(3-Cyclopentylamino-pyrrolidin-1-yl)-N-[2-(5-methyl-furan-2-yl)-6-thiazol-2-yl-pyrimidin-4-yl]-acetamide (Compound 2-63);
2-(4-Acetylamino-piperidin-1-yl)-N-[2-(5-methyl-furan-2-yl)-6-thiazol-2-yl-pyrimidin-4-yl]-acetamide (Compound 2-64);
N-(1-{[2-(5-Methyl-furan-2-yl)-6-thiazol-2-yl-pyrimidin-4-ylcarbamoyl]-methyl}-piperidin-4-yl)-propionamide (Compound 2-65);
2-(3-Acetylamino-pyrrolidin-1-yl)-N-[2-(5-methyl-furan-2-yl)-6-thiazol-2-yl-pyrimidin-4-yl]-acetamide (Compound 2-66);
N-(1-{[2-(5-Methyl-furan-2-yl)-6-thiazol-2-yl-pyrimidin-4-ylcarbamoyl]-methyl}-pyrrolidin-3-yl)-propionamide (Compound 2-67);
N-[2-(5-Methyl-furan-2-yl)-6-thiazol-2-yl-pyrimidin-4-yl]-2-(4-thiazol-2-yl-piperazin-1-yl)-acetamide (Compound 2-68);
2-(Hexahydro-pyrrolo[1,2-a]pyrazin-2-yl)-N-[2-(5-methyl-furan-2-yl)-6-thiazol-2-yl-pyrimidin-4-yl]-acetamide (Compound 2-69);
N-[2-(5-Methyl-furan-2-yl)-6-thiazol-2-yl-pyrimidin-4-yl]-2-(3-piperidin-1-yl-pyrrolidin-1-yl)-acetamide (Compound 2-70);
N-[2-(5-Methyl-furan-2-yl)-6-thiazol-2-yl-pyrimidin-4-yl]-2-(3-morpholin-4-yl-pyrrolidin-1-yl)-acetamide (Compound 2-71);
N-[2-(5-Methyl-furan-2-yl)-6-thiazol-2-yl-pyrimidin-4-yl]-2-(2-methyl-3-oxo-piperazin-1-yl)-acetamide (Compound 2-72);
1-{[2-(5-Methyl-furan-2-yl)-6-thiazol-2-yl-pyrimidin-4-ylcarbamoyl]-methyl}-piperidine-3-carboxylic acid ethyl ester (Compound 2-73);
1-{[2-(5-Methyl-furan-2-yl)-6-thiazol-2-yl-pyrimidin-4-ylcarbamoyl]-methyl}-piperidine-3-carboxylic acid (2-hydroxy-ethyl)-amide (Compound 2-74);
2-((S)-3-Ethylamino-pyrrolidin-1-yl)-N-[2-(5-methyl-furan-2-yl)-6-thiazol-2-yl-pyrimidin-4-yl]-acetamide (Compound 2-75);

2-((R)-3-Ethylamino-pyrrolidin-1-yl)-N-[2-(5-methyl-furan-2-yl)-6-thiazol-2-yl-pyrimidin-4-yl]-acetamide (Compound 2-76);
2-(4-Ethyl-piperazin-1-yl)-N-[2-(5-methyl-furan-2-yl)-6-thiazol-2-yl-pyrimidin-4-yl]-acetamide (Compound 2-77);
N-[2-(5-Methyl-furan-2-yl)-6-thiazol-2-yl-pyrimidin-4-yl]-2-(2-piperidin-1-yl-ethylamino)-acetamide (Compound 2-78);
N-[2-(5-Methyl-furan-2-yl)-6-thiazol-2-yl-pyrimidin-4-yl]-2-[methyl-(1-methyl-piperidin-4-yl)-amino]-acetamide (Compound 2-79);
N-[2-(5-Methyl-furan-2-yl)-6-thiazol-2-yl-pyrimidin-4-yl]-2-[2-(1-methyl-pyrrolidin-2-yl)-ethylamino]-acetamide (Compound 2-80);
N-[2-(5-Methyl-furan-2-yl)-6-thiazol-2-yl-pyrimidin-4-yl]-2-(pyrrolidin-3-ylamino)-acetamide (Compound 2-81);
N-[2-(5-Methyl-furan-2-yl)-6-thiazol-2-yl-pyrimidin-4-yl]-2-(3-pyrrolidin-1-yl-propylamino)-acetamide (Compound 2-82);
N-[2-(5-Methyl-furan-2-yl)-6-thiazol-2-yl-pyrimidin-4-yl]-2-(3-piperidin-1-yl-propylamino)-acetamide (Compound 2-83);
N-[2-(5-Methyl-furan-2-yl)-6-thiazol-2-yl-pyrimidin-4-yl]-2-(3-morpholin-4-yl-propylamino)-acetamide (Compound 2-84);
2-(4-Hydroxy-piperidin-1-yl)-N-[2-(5-methyl-furan-2-yl)-6-thiazol-2-yl-pyrimidin-4-yl]-acetamide (Compound 2-85);
2-(3-Hydroxy-piperidin-1-yl)-N-[2-(5-methyl-furan-2-yl)-6-thiazol-2-yl-pyrimidin-4-yl]-acetamide (Compound 2-86);
2-((S)-3-Hydroxy-pyrrolidin-1-yl)-N-[2-(5-methyl-furan-2-yl)-6-thiazol-2-yl-pyrimidin-4-yl]-acetamide (Compound 2-87);
2-((S)-3-Acetylamino-pyrrolidin-1-yl)-N-[2-(5-methyl-furan-2-yl)-6-thiazol-2-yl-pyrimidin-4-yl]-acetamide (Compound 2-88);
2-((R)-3-Acetylamino-pyrrolidin-1-yl)-N-[2-(5-methyl-furan-2-yl)-6-thiazol-2-yl-pyrimidin-4-yl]-acetamide (Compound 2-89);
2-(3-Hydroxy-pyrrolidin-1-yl)-N-[2-(5-methyl-furan-2-yl)-6-thiazol-2-yl-pyrimidin-4-yl]-acetamide (Compound 2-90);
2-(4-Isopropyl-piperazin-1-yl)-N-[2-(5-methyl-furan-2-yl)-6-thiazol-2-yl-pyrimidin-4-yl]-acetamide (Compound 2-91);
2-(4-Cyclopentyl-piperazin-1-yl)-N-[2-(5-methyl-furan-2-yl)-6-thiazol-2-yl-pyrimidin-4-yl]-acetamide (Compound 2-92);
2-(4-Cyclohexyl-piperazin-1-yl)-N-[2-(5-methyl-furan-2-yl)-6-thiazol-2-yl-pyrimidin-4-yl]-acetamide (Compound 2-93);
N-[2-(5-Methyl-furan-2-yl)-6-thiazol-2-yl-pyrimidin-4-yl]-2-(4-propionyl-piperazin-1-yl)-acetamide (Compound 2-94);
2-(4-Isobutyryl-piperazin-1-yl)-N-[2-(5-methyl-furan-2-yl)-6-thiazol-2-yl-pyrimidin-4-yl]-acetamide (Compound 2-95);
2-(4-Aminomethyl-piperidin-1-yl)-N-[2-(5-methyl-furan-2-yl)-6-thiazol-2-yl-pyrimidin-4-yl]-acetamide (Compound 2-96);
N-[2-(5-Methyl-furan-2-yl)-6-thiazol-2-yl-pyrimidin-4-yl]-2-[(pyrrolidin-3-ylmethyl)-amino]-acetamide (Compound 2-97);
2-(3-Aminomethyl-piperidin-1-yl)-N-[2-(5-methyl-furan-2-yl)-6-thiazol-2-yl-pyrimidin-4-yl]-acetamide (Compound 2-98);
N-[2-(5-Methyl-furan-2-yl)-6-thiazol-2-yl-pyrimidin-4-yl]-2-[(1-methyl-pyrrolidin-3-ylmethyl)-amino]-acetamide (Compound 2-99);
2-(3-Aminomethyl-pyrrolidin-1-yl)-N-[2-(5-methyl-furan-2-yl)-6-thiazol-2-yl-pyrimidin-4-yl]-acetamide (Compound 2-100);
2-(4-Methoxy-piperidin-1-yl)-N-[2-(5-methyl-furan-2-yl)-6-thiazol-2-yl-pyrimidin-4-yl]-acetamide (Compound 2-101);
2-(3-Dimethylaminomethyl-piperidin-1-yl)-N-[2-(5-methyl-furan-2-yl)-6-thiazol-2-yl-pyrimidin-4-yl]-acetamide (Compound 2-102);
2-(4-Dimethylaminomethyl-piperidin-1-yl)-N-[2-(5-methyl-furan-2-yl)-6-thiazol-2-yl-pyrimidin-4-yl]-acetamide (Compound 2-103);
N-[2-(5-Methyl-furan-2-yl)-6-thiazol-2-yl-pyrimidin-4-yl]-2-[methyl-(1-methyl-pyrrolidin-3-ylmethyl)-amino]-acetamide (Compound 2-104);
2-(3-Dimethylaminomethyl-pyrrolidin-1-yl)-N-[2-(5-methyl-furan-2-yl)-6-thiazol-2-yl-pyrimidin-4-yl]-acetamide (Compound 2-105);
N-[2-(5-Methyl-furan-2-yl)-6-thiazol-2-yl-pyrimidin-4-yl]-2-morpholin-4-yl-acetamide (Compound 2-106);
N-[2-(5-Methyl-furan-2-yl)-6-thiazol-2-yl-pyrimidin-4-yl]-2-(4-phenyl-piperidin-1-yl)-acetamide (Compound 2-107);
2-(2,5-Dimethyl-piperazin-1-yl)-N-[2-(5-methyl-furan-2-yl)-6-thiazol-2-yl-pyrimidin-4-yl]-acetamide (Compound 2-108);
N-[2-(5-Methyl-furan-2-yl)-6-thiazol-2-yl-pyrimidin-4-yl]-2-piperazin-1-yl-acetamide (Compound 2-109);
2-[1,4]Diazepan-1-yl-N-[2-(5-methyl-furan-2-yl)-6-thiazol-2-yl-pyrimidin-4-yl]-acetamide (Compound 2-110);
3-((S)-3-Dimethylamino-pyrrolidin-1-yl)-N-[2-(5-methyl-furan-2-yl)-6-thiazol-2-yl-pyrimidin-4-yl]-propionamide (Compound 2-111);
3-(4-Acetyl-piperazin-1-yl)-N-[2-(5-methyl-furan-2-yl)-6-thiazol-2-yl-pyrimidin-4-yl]-propionamide (Compound 2-112);
N-[2-(5-Methyl-furan-2-yl)-6-thiazol-2-yl-pyrimidin-4-yl]-3-(4-methyl-piperazin-1-yl)-propionamide (Compound 2-113);
2-(5-Methyl-furan-2-yl)-6-(3-piperazin-1-yl-propionylamino)-N-vinyl-pyrimidine-4-carboximidothioic acid methyl ester (Compound 2-114);
N-[2-(5-Methyl-furan-2-yl)-6-thiazol-2-yl-pyrimidin-4-yl]-3-(4-pyrrolidin-1-yl-piperidin-1-yl)-propionamide (Compound 2-115);
N-[2-(5-Methyl-furan-2-yl)-6-thiazol-2-yl-pyrimidin-4-yl]-3-morpholin-4-yl-propionamide (Compound 2-116);
3-((R)-3-Dimethylamino-pyrrolidin-1-yl)-N-[2-(5-methyl-furan-2-yl)-6-thiazol-2-yl-pyrimidin-4-yl]-propionamide (Compound 2-117);
3-(4-Acetyl-[1,4]diazepan-1-yl)-N-[2-(5-methyl-furan-2-yl)-6-thiazol-2-yl-pyrimidin-4-yl]-propionamide (Compound 2-118);
N-[2-(5-Methyl-furan-2-yl)-6-thiazol-2-yl-pyrimidin-4-yl]-3-[1,4]oxazepan-4-yl-propionamide (Compound 2-119);
3-(4-Methyl-[1,4]diazepan-1-yl)-N-[2-(5-methyl-furan-2-yl)-6-thiazol-2-yl-pyrimidin-4-yl]-propionamide (Compound 2-120);
3-((R)-3-Ethylamino-pyrrolidin-1-yl)-N-[2-(5-methyl-furan-2-yl)-6-thiazol-2-yl-pyrimidin-4-yl]-propionamide (Compound 2-121);
3-(3,5-Dimethyl-piperazin-1-yl)-N-[2-(5-methyl-furan-2-yl)-6-thiazol-2-yl-pyrimidin-4-yl]-propionamide (Compound 2-122);
3-[1,4]Diazepan-1-yl-N-[2-(5-methyl-furan-2-yl)-6-thiazol-2-yl-pyrimidin-4-yl]-propionamide (Compound 2-123);
N-[2-(5-Methyl-furan-2-yl)-6-thiazol-2-yl-pyrimidin-4-yl]-2-(1-methyl-piperidin-4-yl)-acetamide (Compound 2-124);
N-[2-(5-Methyl-furan-2-yl)-6-thiazol-2-yl-pyrimidin-4-yl]-2-piperidin-4-yl-acetamide (Compound 2-125);
N-[2-(5-Methyl-furan-2-yl)-6-thiazol-2-yl-pyrimidin-4-yl]-2-(2-pyrrolidin-1-yl-ethoxy)-acetamide (Compound 2-126);
N-[6-(3,5-Dimethyl-pyrazol-1-yl)-2-(4-methoxy-pyridin-3-yl)-pyrimidin-4-yl]-2-(4-methyl-piperazin-1-yl)-acetamide (Compound 2-127);
N-[6-(3,5-Dimethyl-pyrazol-1-yl)-2-(6-methoxy-pyridin-3-yl)-pyrimidin-4-yl]-2-(4-methyl-piperazin-1-yl)-acetamide (Compound 2-128);
N-[2-(3,4-Dimethoxy-phenyl)-6-(3,5-dimethyl-pyrazol-1-yl)-pyrimidin-4-yl]-2-(4-methyl-piperazin-1-yl)-acetamide (Compound 2-129);
N-[2-(4-Trifuoromethoxy-phenyl)-6-(3,5-dimethyl-pyrazol-1-yl)-pyrimidin-4-yl]-2-(4-methyl-piperazin-1-yl)-acetamide (Compound 2-130);
N-[2-(3-Fluoro-phenyl)-6-(3,5-dimethyl-pyrazol-1-yl)-pyrimidin-4-yl]-2-(4-methyl-piperazin-1-yl)-acetamide (Compound 2-131);
N-[2-(2-Fluoro-phenyl)-6-(3,5-dimethyl-pyrazol-1-yl)-pyrimidin-4-yl]-2-(4-methyl-piperazin-1-yl)-acetamide (Compound 2-132);
N-[2-(2-Fluoro-3-methoxy-phenyl)-6-(3,5-dimethyl-pyrazol-1-yl)-pyrimidin-4-yl]-2-(4-methyl-piperazin-1-yl)-acetamide (Compound 2-133);
N-[2-(2,4-Dimethoxy-phenyl)-6-(3,5-dimethyl-pyrazol-1-yl)-pyrimidin-4-yl]-2-(4-methyl-piperazin-1-yl)-acetamide (Compound 2-134);
N-[2-(2-Cyano-phenyl)-6-(3,5-dimethyl-pyrazol-1-yl)-pyrimidin-4-yl]-2-(4-methyl-piperazin-1-yl)-acetamide (Compound 2-135);
N-[2-(2-Methoxy-phenyl)-6-(3,5-dimethyl-pyrazol-1-yl)-pyrimidin-4-yl]-2-(4-methyl-piperazin-1-yl)-acetamide (Compound 2-136);
N-[2-(4-Fluoro-2-methyl-phenyl)-6-(3,5-dimethyl-pyrazol-1-yl)-pyrimidin-4-yl]-2-(4-methyl-piperazin-1-yl)-acetamide (Compound 2-137);
N-[2-(3-Methoxy-phenyl)-6-(3,5-dimethyl-pyrazol-1-yl)-pyrimidin-4-yl]-2-(4-methyl-piperazin-1-yl)-acetamide (Compound 2-138);
N-[2-(3,5-Dimethoxy-phenyl)-6-(3,5-dimethyl-pyrazol-1-yl)-pyrimidin-4-yl]-2-(4-methyl-piperazin-1-yl)-acetamide (Compound 2-139);
N-[2-(3,5-Difluoro-phenyl)-6-(3,5-dimethyl-pyrazol-1-yl)-pyrimidin-4-yl]-2-(4-methyl-piperazin-1-yl)-acetamide (Compound 2-140);
N-[2-(3-Fluoro-4.-methyl-phenyl)-6-(3,5-dimethyl-pyrazol-1-yl)-pyrimidin-4-yl]-2-(4-methyl-piperazin-1-yl)-acetamide (Compound 2-141);
N-[2-(3-Fluoro-2-methoxy-phenyl)-6-(3,5-dimethyl-pyrazol-1-yl)-pyrimidin-4-yl]-2-(4-methyl-piperazin-1-yl)-acetamide (Compound 2-142);
N-[2-(3-Fluoro-4-methoxy-phenyl)-6-(3,5-dimethyl-pyrazol-1-yl)-pyrimidin-4-yl]-2-(4-methyl-piperazin-1-yl)-acetamide (Compound 2-143);
N-[2-(2,3-Difluoro-phenyl)-6-(3,5-dimethyl-pyrazol-1-yl)-pyrimidin-4-yl]-2-(4-methyl-piperazin-1-yl)-acetamide (Compound 2-144);
N-[2-(5-Methoxy-pyridin-3-yl)-6-(3,5-dimethyl-pyrazol-1-yl)-pyrimidi n-4-yl]-2-(4-methyl-piperazin-1-yl)-acetamide (Compound 2-145);
N-[2-(3-Methoxy-phenyl)-6-pyrazol-1-yl-pyrimidin-4-yl]-2-(4-methyl-piperazin-1-yl)-acetamide (Compound 2-146);
N-[2-(3,4-Dimethoxy-phenyl)-6-pyrazol-1-yl-pyrimidin-4-yl]-2-(4-methyl-piperazin-1-yl)-acetamide (Compound 2-147);
N-[2-(3-Cyano-phenyl)-6-pyrazol-1-yl-pyrimidin-4-yl]-2-(4-methyl-piperazin-1-yl)-acetamide (Compound 2-148);
N-[2-(3-Flouro-4-Methoxy-phenyl)-6-pyrazol-1-yl-pyrimidin-4-yl]-2-(4-methyl-piperazin-1-yl)-acetamide (Compound 2-149);
N-[2-(2-Methoxy-phenyl)-6-pyrazol-1-yl-pyrimidin-4-yl]-2-(4-methyl-piperazin-1-yl)-acetamide (Compound 2-150);
N-[2-(4-Methoxy-phenyl)-6-pyrazol-1-yl-pyrimidin-4-yl]-2-(4-methyl-piperazin-1-yl)-acetamide (Compound 2-151);
N-[2-(3-Trifluoromethyl-phenyl)-6-pyrazol-1-yl-pyrimidin-4-yl]-2-(4-methyl-piperazin-1-yl)-acetamide (Compound 2-152);
N-[2-(2,3-Difluoro-phenyl)-6-pyrazol-1-yl-pyrimidin-4-yl]-2-(4-methyl-piperazin-1-yl)-acetamide (Compound 2-153);
N-[2-(3-Trifluoromethoxy-phenyl)-6-pyrazol-1-yl-pyrimidin-4-yl]-2-(4-methyl-piperazin-1-yl)-acetamide (Compound 2-154);
N-[2-(2-Fluoro-3-methoxy-phenyl)-6-pyrazol-1-yl-pyrimidin-4-yl]-2-(4-methyl-piperazin-1-yl)-acetamide (Compound 2-155);
N-[2-(5-Methyl-furan-2-yl)-6-pyridin-2-yl-pyrimidin-4-yl]-2-morpholin-4-yl-acetamide (Compound 3-1);
N-[2-(5-Methyl-furan-2-yl)-6-pyridin-2-yl-pyrimidin-4-yl]-2-piperidin-1-yl-acetamide (Compound 3-2);
2-[4-(Isopropylcarbamoyl-methyl)-piperazin-1-yl]-N-[2-(5-methyl-furan-2-yl)-6-pyridin-2-yl-pyrimidin-4-yl]-acetamide (Compound 3-3);
N-[2-(5-Methyl-furan-2-yl)-6-pyridin-2-yl-pyrimidin-4-yl]-2-(4-phenyl-piperidin-1-yl)-acetamide (Compound 3-4);
2-[1,4']Bipiperidinyl-1'-yl-N-[2-(5-methyl-furan-2-yl)-6-pyridin-2-yl-pyrimidin-4-yl]-acetamide (Compound 3-5);
2-(3,4-Dihydro-1H-isoquinolin-2-yl)-N-[2-(5-methyl-furan-2-yl)-6-pyridin-2-yl-pyrimidin-4-yl]-acetamide (Compound 3-6);
2-[4-(3-Methoxy-phenyl)-piperazin-1-yl]-N-[2-(5-methyl-furan-2-yl)-6-pyridin-2-yl-pyrimidin-4-yl]-acetamide (Compound 3-7);
N-[2-(5-Methyl-furan-2-yl)-6-pyridin-2-yl-pyrimidin-4-yl]-2-(4-methyl-piperazin-1-yl)-acetamide (Compound 3-8);
2-((R)-3-Dimethylamino-pyrrolidin-1-yl)-N-(2-pyridin-2-yl-6-thiazol-2-yl-pyrimidin-4-yl)-acetamide (Compound 4-1);
2-((S)-3-Dimethylamino-pyrrolidin-1-yl)-N-(2-pyridin-2-yl-6-thiazol-2-yl-pyrimidin-4-yl)-acetamide (Compound 4-2);
2-(2,5-Dimethyl-piperazin-1-yl)-N-(2-pyridin-2-yl-6-thiazol-2-yl-pyrimidin-4-yl)-acetamide (Compound 4-3);
2-(3,5-Dimethyl-piperazin-1-yl)-N-(2-pyridin-2-yl-6-thiazol-2-yl-pyrimidin-4-yl)-acetamide (Compound 4-4);
2-(4-Phenyl-piperazin-1-yl)-N-(2-pyridin-2-yl-6-thiazol-2-yl-pyrimidin-4-yl)-acetamide (Compound 4-5);
2-[4-(2-Methoxy-phenyl)-piperazin-1-yl]-N-(2-pyridin-2-yl-6-thiazol-2-yl-pyrimidin-4-yl)-acetamide (Compound 4-6);
2-(4-Methyl-piperazin-1-yl)-N-(2-pyridin-2-yl-6-thiazol-2-yl-pyrimidin-4-yl)-acetamide (Compound 4-7);
2-(4-Benzyl-piperazin-1-yl)-N-(2-pyridin-2-yl-6-thiazol-2-yl-pyrimidin-4-yl)-acetamide (Compound 4-8);
2-Morpholin-4-yl-N-(2-pyridin-2-yl-6-thiazol-2-yl-pyrimidin-4-yl)-acetamide (Compound 4-9);
2-(2,6-Dimethyl-morpholin-4-yl)-N-(2-pyridin-2-yl-6-thiazol-2-yl-pyrimidin-4-yl)-acetamide (Compound 4-10);
2-Piperidin-1-yl-N-(2-pyridin-2-yl-6-thiazol-2-yl-pyrimidin-4-yl)-acetamide (Compound 4-11);
2-(2-Methyl-piperidin-1-yl)-N-(2-pyridin-2-yl-6-thiazol-2-yl-pyrimidin-4-yl)-acetamide (Compound 4-12);
2-(3-Methyl-piperidin-1-yl)-N-(2-pyridin-2-yl-6-thiazol-2-yl-pyrimidin-4-yl)-acetamide (Compound 4-13);
2-(3,3-Dimethyl-piperidin-1-yl)-N-(2-pyridin-2-yl-6-thiazol-2-yl-pyrimidin-4-yl)-acetamide (Compound 4-14);
2-(3,5-Dimethyl-piperidin-1-yl)-N-(2-pyridin-2-yl-6-thiazol-2-yl-pyrimidin-4-yl)-acetamide (Compound 4-15);
2-(4-Methyl-pipeddin-1-yl)-N-(2-pyridin-2-yl-6-thiazol-2-yl-pyrimidin-4-yl)-acetamide (Compound 4-16);
2-(4-Benzyl-piperidin-1-yl)-N-(2-pyridin-2-yl-6-thiazol-2-yl-pyrimidin-4-yl)-acetamide (Compound 4-17);
2-[1,4']Bipiperidinyl-1'-yl-N-(2-pyridin-2-yl-6-thiazol-2-yl-pyrimidin-4-yl)-acetamide (Compound 4-18);
2-((S)-2-Methoxymethyl-pyrrolidin-1-yl)-N-(2-pyridin-2-yl-6-thiazol-2-yl-pyrimidin-4-yl)-acetamide (Compound 4-19);
2-(Octahydro-isoquinolin-2-yl)-N-(2-pyridin-2-yl-6-thiazol-2-yl-pyrimidin-4-yl)-acetamide (Compound 4-20);

N-(2-Pyridin-2-yl-6-thiazol-2-yl-pyrimidin-4-yl)-2-((S)-2-pyrrolidin-1-ylmethyl-pyrrolidin-1-yl)-acetamide (Compound 4-21);
2-[4-(3,4-Dimethyl-phenyl)-piperazin-1-yl]-N-(2-pyridin-2-yl-6-thiazol-2-yl-pyrimidin-4-yl)-acetamide (Compound 4-22);
N-(2-Pyridin-2-yl-6-thiazol-2-yl-pyrimidin-4-yl)-2-(4-pyrrolidin-1-yl-piperidin-1-yl)-acetamide (Compound 4-23);
2-[4-(3-Chloro-phenyl)-piperazin-1-yl]-N-(2-pyridin-2-yl-6-thiazol-2-yl-pyrimidin-4-yl)-acetamide (Compound 4-24);
2-[4-(3-Methoxy-phenyl)-piperazin-1-yl]-N-(2-pyridin-2-yl-6-thiazol-2-yl-pyrimidin-4-yl)-acetamide (Compound 4-25);
2-[4-(4-Methoxy-phenyl)-piperazin-1-yl]-N-(2-pyridin-2-yl-6-thiazol-2-yl-pyrimidin-4-yl)-acetamide (Compound 4-26);
2-(4-Acetyl-piperazin-1-yl)-N-(2-pyridin-2-yl-6-thiazol-2-yl-pyrimidin-4-yl)-acetamide (Compound 4-27);
2-(4-Methyl-[1,4]diazepan-1-yl)-N-(2-pyridin-2-yl-6-thiazol-2-yl-pyrimidin-4-yl)-acetamide (Compound 4-28);
2-[2-((S)-1-Methyl-pyrrolidin-2-ylmethyl)-piperidin-1-yl]-N-(2-pyridin-2-yl-6-thiazol-2-yl-pyrimidin-4-yl)-acetamide (Compound 4-29);
2-[2-(2-Piperidin-1-yl-ethyl)-piperidin-1-yl]-N-(2-pyridin-2-yl-6-thiazol-2-yl-pyrimidin-4-yl)-acetamide (Compound 4-30);
2-((R)-2-Methyl-piperazin-1-yl)-N-(2-pyridin-2-yl-6-thiazol-2-yl-pyrimidin-4-yl)-acetamide (Compound 4-31);
2-(2,5-Dihydro-pyrrol-1-yl)-N-(2-pyridin-2-yl-6-thiazol-2-yl-pyrimidin-4-yl)-acetamide (Compound 4-32);
2-(4-Acetyl-[1,4]diazepan-1-yl)-N-(2-pyridin-2-yl-6-thiazol-2-yl-pyrimidin-4-yl)-acetamide (Compound 4-33);
2-(3-Dimethylamino-pyrrolidin-1-yl)-N-(2-pyridin-2-yl-6-thiazol-2-yl-pyrimidin-4-yl)-acetamide (Compound 4-34);
N-(2-Pyridin-2-yl-6-thiazol-2-yl-pyrimidin-4-yl)-2-(3-trifluoromethyl-piperidin-1-yl)-acetamide (Compound 4-35);
2-(3-Diethylamino-pyrrolidin-1-yl)-N-(2-pyridin-2-yl-6-thiazol-2-yl-pyrimidin-4-yl)-acetamide (Compound 4-36);
2-((R)-2-Methoxymethyl-pyrrolidin-1-yl)-N-(2-pyridin-2-yl-6-thiazol-2-yl-pyrimidin-4-yl)-acetamide (Compound 4-37);
2-(2,5-Dimethyl-2,5-dihydro-pyrrol-1-yl)-N-(2-pyridin-2-yl-6-thiazol-2-yl-pyrimidin-4-yl)-acetamide (Compound 4-38);
N-(2-Pyridin-2-yl-6-thiazol-2-yl-pyrimidin-4-yl)-2-pyrrolidin-1-yl-acetamide (Compound 4-39);
2-(2,5-Dimethyl-pyrrolidin-1-yl)-N-(2-pyridin-2-yl-6-thiazol-2-yl-pyrimidin-4-yl)-acetamide (Compound 4-40);
2-(3-Phenyl-piperidin-1-yl)-N-(2-pyridin-2-yl-6-thiazol-2-yl-pyrimidin-4-yl)-acetamide (Compound 4-41);
2-(2-Phenyl-piperidin-1-yl)-N-(2-pyridin-2-yl-6-thiazol-2-yl-pyrimidin-4-yl)-acetamide (Compound 4-42);
2-[1,4]Oxazepan-4-yl-N-(2-pyridin-2-yl-6-thiazol-2-yl-pyrimidin-4-yl)-acetamide (Compound 4-43);
2-(4,4-Difluoro-piperidin-1-yl)-N-(2-pyridin-2-yl-6-thiazol-2-yl-pyrimidin-4-yl)-acetamide (Compound 4-44);
2-(4-Phenyl-piperidin-1-yl)-N-(2-pyridin-2-yl-6-thiazol-2-yl-pyrimidin-4-yl)-acetamide (Compound 4-45);
2-Piperazin-1-yl-N-(2-pyridin-2-yl-6-thiazol-2-yl-pyrimidin-4-yl)-acetamide (Compound 4-46);
2-[4-(isopropylcarbamoyl-methyl)-piperazin-1-yl]-N-(2-pyridin-2-yl-6-thiazol-2-yl-pyrimidin-4-yl)-acetamide (Compound 4-47);
1-[(2-Pyridin-2-yl-6-thiazol-2-yl-pyrimidin-4-ylcarbamoyl)-methyl]-piperidine-3-carboxylic acid amide (Compound 4-48);
2-[1,4]Diazepan-1-yl-N-(2-pyridin-2-yl-6-thiazol-2-yl-pyrimidin-4-yl)-acetamide (Compound 4-49);
2-[Methyl-(2-pyrrolidin-1-yl-ethyl)-amino]-N-(2-pyridin-2-yl-6-thiazol-2-yl-pyrimidin-4-yl)-acetamide (Compound 4-50);
2-(3,5-Dimethyl-piperazin-1-yl)-N-(2-pyridin-2-yl-6-thiazol-2-yl-pyrimidin-4-yl)-acetamide (Compound 4-51);
2-(4-Acetyl-piperazin-1-yl)-N-(2-pyridin-2-yl-6-thiazol-2-yl-pyrimidin-4-yl)-acetamide (Compound 4-52);
2-[4-(3-Chloro-phenyl)-piperazin-1-yl]-N-(2-pyridin-2-yl-6-thiazol-2-yl-pyrimidin-4-yl)-acetamide (Compound 4-53);
2-Morpholin-4-yl-N-(2-pyridin-2-yl-6-thiazol-2-yl-pyrimidin-4-yl)-acetamide (Compound 4-54);
2-Piperidin-1-yl-N-(2-pyridin-2-yl-6-thiazol-2-yl-pyrimidin-4-yl)-acetamide (Compound 4-55);
2-(4-Methyl-piperazin-1-yl)-N-(6-pyridin-2-yl-2-thiophen-2-yl-pyrimidin-4-yl)-acetamide (Compound 5-1);
2-Morpholin-4-yl-N-(6-pyridin-2-yl-2-thiophen-2-yl-pyrimidin-4-yl)-acetamide (Compound 5-2);
N-(6-Pyridin-2-yl-2-thiophen-2-yl-pyrimidin-4-yl)-2-pyrrolidin-1-yl-acetamide (Compound 5-3);
2-[Methyl-(1-methyl-piperidin-4-yl)-amino]-N-(6-pyridin-2-yl-2-thiophen-2-yl-pyrimidin-4-yl)-acetamide (Compound 5-4);
2-[1,4]Diazepan-1-yl-N-(6-pyridin-2-yl-2-thiophen-2-yl-pyrimidin-4-yl)-acetamide (Compound 5-5);
2-(4-Methyl-[1,4]diazepan-1-yl)-N-(6-pyridin-2-yl-2-thiophen-2-yl-pyrimidin-4-yl)-acetamide (Compound 5-6);
2-(4-Ethyl-[1,4]diazepan-1-yl)-N-(6-pyridin-2-yl-2-thiophen-2-yl-pyrimidin-4-yl)-acetamide (Compound 5-7);
2-(4-Propyl-[1,4]diazepan-1-yl)-N-(6-pyridin-2-yl-2-thiophen-2-yl-pyrimidin-4-yl)-acetamide (Compound 5-8);
2-(4-Amino-piperidin-1-yl)-N-(6-pyridin-2-yl-2-thiophen-2-yl-pyrimidin-4-yl)-acetamide (Compound 5-9);
2-(4-Dimethylamino-piperidin-1-yl)-N-(6-pyridin-2-yl-2-thiophen-2-yl-pyrimidin-4-yl)-acetamide (Compound 5-10);
2-(4-Diethylamino-piperidin-1-yl)-N-(6-pyridin-2-yl-2-thiophen-2-yl-pyrimidin-4-yl)-acetamide (Compound 5-11);
2-(4-Dipropylamino-piperidin-1-yl)-N-(6-pyridin-2-yl-2-thiophen-2-yl-pyrimidin-4-yl)-acetamide (Compound 5-12);
2-(4-Acetylamino-piperidin-1-yl)-N-(6-pyridin-2-yl-2-thiophen-2-yl-pyrimidin-4-yl)-acetamide (Compound 5-13);
N-{1-[(6-Pyridin-2-yl-2-thiophen-2-yl-pyrimidin-4-ylcarbamoyl)-methyl]-piperidin-4-yl}-propionamide (Compound 5-14);
N-(6-Pyridin-2-yl-2-thiophen-2-yl-pyrimidin-4-yl)-2-(4-pyrrolidin-1-yl-piperidin-1-yl)-acetamide (Compound 5-15);
2-(4-Morpholin-4-yl-piperidin-1-yl)-N-(6-pyridin-2-yl-2-thiophen-2-yl-pyrimidin-4-yl)-acetamide (Compound 5-16);
2-[1,4']Bipiperidinyl-1'-yl-N-(6-pyridin-2-yl-2-thiophen-2-yl-pyrimidin-4-yl)-acetamide (Compound 5-17);
2-[4-(2-Oxo-imidazolidin-1-yl)-piperidin-1-yl]-N-(6-pyridin-2-yl-2-thiophen-2-yl-pyrimidin-4-yl)-acetamide (Compound 5-18);
2-(4-Acetimidoylamino-piperidin-1-yl)-N-(6-pyridin-2-yl-2-thiophen-2-yl-pyrimidin-4-yl)-acetamide (Compound 5-19);
2-(4-Azetidin-1-yl-piperidin-1-yl)-N-(6-pyridin-2-yl-2-thiophen-2-yl-pyrimidin-4-yl)-acetamide (Compound 5-20);
N-(2-Furan-2-yl-6-thiazol-2-yl-pyrimidin-4-yl)-2-(4-methyl-piperazin-1-yl)acetamide (Compound 6-1);
2-Piperidin-1-yl-N-(6-thiazol-2-yl-2-thiophen-2-yl-pyrimidin-4-yl)-acetamide (Compound 7-1);
2-(2-Pyrrolidin-1-ylmethyl-piperidin-1-yl)-N-(6-thiazol-2-yl-2-thiophen-2-yl-pyrimidin-4-yl)-acetamide (Compound 7-2);
2-[2-(2-Piperidin-1-yl-ethyl)-piperidin-1-yl]-N-(6-thiazol-2-yl-2-thiophen-2-yl-pyrimidin-4-yl)-acetamide (Compound 7-3);
2-(3-Methyl-piperidin-1-yl)-N-(6-thiazol-2-yl-2-thiophen-2-yl-pyrimidin-4-yl)-acetamide (Compound 7-4);
N-(6-Thiazol-2-yl-2-thiophen-2-yl-pyrimidin-4-yl)-2-(3-trifluoromethyl-piperidin-1-yl)-acetamide (Compound 7-5);
1-[(6-Thiazol-2-yl-2-thiophen-2-yl-pyrimidin-4-ylcarbamoyl)-methyl]-piperidine-3-carboxylic acid amide (Compound 7-6);
2-[1,4']Bipiperidinyl-1'-yl-N-(6-thiazol-2-yl-2-thiophen-2-yl-pyrimidin-4-yl)-acetamide (Compound 7-7);
2-[1,4]Diazepan-1-yl-N-(6-thiazol-2-yl-2-thiophen-2-yl-pyrimidin-4-yl)-acetamide (Compound 7-8);
2-(4-Acetyl-[1,4]diazepan-1-yl)-N-(6-thiazol-2-yl-2-thiophen-2-yl-pyrimidin-4-yl)-acetamide (Compound 7-9);
2-(4-Pyrrolidin-1-yl-piperidin-1-yl)-N-(6-thiazol-2-yl-2-thiophen-2-yl-pyrimidin-4-yl)-acetamide (Compound 7-10);
2-Piperazin-1-yl-N-(6-thiazol-2-yl-2-thiophen-2-yl-pyrimidin-4-yl)-acetamide (Compound 7-111);
2-(2,5-Dimethyl-piperazin-1-yl)-N-(6-thiazol-2-yl-2-thiophen-2-yl-pyrimidin-4-yl)-acetamide (Compound 7-12);
2-(3,5-Dimethyl-piperazin-1-yl)-N-(6-thiazol-2-yl-2-thiophen-2-yl-pyrimidin-4-yl)-acetamide (Compound 7-13);
2-(4-Acetyl-piperazin-1-yl)-N-(6-{1-[(E)-methylimino]-ethyl}-2-thiophen-2-yl-pyrimidin-4-yl)-acetamide (Compound 7-14);
2-(4-Phenyl-piperazin-1-yl)-N-(6-thiazol-2-yl-2-thiophen-2-yl-pyrimidin-4-yl)-acetamide (Compound 7-15);
N-Isopropyl-2-{4-[(6-thiazol-2-yl-2-thiophen-2-yl-pyrimidin-4-ylcarbamoyl)-methyl]-piperazin-1-yl}-acetamide (Compound 7-16);
2-Morpholin-4-yl-N-(6-thiazol-2-yl-2-thiophen-2-yl-pyrimidin-4-yl)-acetamide (Compound 7-17);
2-(2,6-Dimethyl-morpholin-4-yl)-N-(6-thiazol-2-yl-2-thiophen-2-yl-pyrimidin-4-yl)-acetamide (Compound 7-18);
N-(6-Thiazol-2-yl-2-thiophen-2-yl-pyrimidin-4-yl)-2-thiomorpholin-4-yl-acetamide (Compound 7-19);
2-Pyrrolidin-1-yl-N-(6-thiazol-2-yl-2-thiophen-2-yl-pyrimidin-4-yl)acetamide (Compound 7-20);
2-(3-Dimethylamino-pyrrolidin-1-yl) -N-(6-thiazol-2-yl-2-thiophen-2-yl-pyrimidin-4-yl)-acetamide (Compound 7-21);
2-(3-Diethylamino-pyrrolidin-1-yl)-N-(6-thiazol-2-yl-2-thiophen-2-yl-pyrimidin-4-yl)-acetamide (Compound 7-22);
2-[2-(1-Methyl-pyrrolidin-2-yl)-ethylamino]-N-(6-thiazol-2-yl-2-thiophen-2-yl-pyrimidin-4-yl)-acetamide (Compound 7-23);
2-(3,5-Dimethyl-piperidin-1-yl)-N-(6-thiazol-2-yl-2-thiophen-2-yl-pyrimidin-4-yl)-acetamide (Compound 7-24);
2-(4,4-Difluoro-piperidin-1-yl)-N-(6-thiazol-2-yl-2-thiophen-2-yl-pyrimidin-4-yl)-acetamide (Compound 7-25);
2-(4-Phenyl-piperidin-1-yl)-N-(6-thiazol-2-yl-2-thiophen-2-yl-pyrimidin-4-yl)-acetamide (Compound 7-26);
2-Azepan-1-yl-N-(6-thiazol-2-yl-2-thiophen-2-yl-pyrimidin-4-yl)-acetamide (Compound 7-27);
1-[(6-Thiazol-2-yl-2-thiophen-2-yl-pyrimidin-4-ylcarbamoyl)-methyl]-piperidine-4-carboxylic acid amide (Compound 7-28);
2-[4-(3-Methoxy-phenyl)-piperazin-1-yl]-N-(6-thiazol-2-yl-2-thiophen-2-yl-pyrimidin-4-yl)-acetamide (Compound 7-29);
2-((R)-3-Dimethylamino-pyrrolidin-1-yl)-N-(6-thiazol-2-yl-2-thiophen-2-yl-pyrimidin-4-yl)-acetamide (Compound 7-30);
2-((S)-3-Dimethylamino-pyrrolidin-1-yl)-N-(6-thiazol-2-yl-2-thiophen-2-yl-pyrimidin-4-yl)-acetamide (Compound 7-31);
2-[(1-Ethyl-pyrrolidin-2-ylmethyl)-amino]-N-(6-thiazol-2-yl-2-thiophen-2-yl-pyrimidin-4-yl)-acetamide (Compound 7-32);
2-((R)-3-Ethylamino-pyrrolidin-1-yl)-N-(6-thiazol-2-yl-2-thiophen-2-yl-pyrimidin-4-yl)-acetamide (Compound 7-33);
2-((S)-3-Ethylamino-pyrrolidin-1-yl)-N-(6-thiazol-2-yl-2-thiophen-2-yl-pyrimidin-4-yl)-acetamide (Compound 7-34);
2-(4-Methyl-piperazin-1-yl)-N-(6-thiazol-2-yl-2-thiophen-2-yl-pyrimidin-4-yl)-acetamide (Compound 7-35);
1-[(6-Thiazol-2-yl-2-thiophen-2-yl-pyrimidin-4-ylcarbamoyl)-methyl]-piperidine-3-carboxylic acid amide (Compound 7-36);
2-(4-Pyrrolidin-1-yl-piperidin-1-yl)-N-(6-thiazol-2-yl-2-thiophen-2-yl-pyrimidin-4-yl)-acetamide (Compound 7-37);
2-(3,5-Dimethyl-piperazin-1-yl)-N-(6-thiazol-2-yl-2-thiophen-2-yl-pyrimidin-4-yl)-acetamide (Compound 7-38);
2-[2-(1-Methyl-pyrrolidin-2-yl)-ethylamino]-N-(6-thiazol-2-yl-2-thiophen-2-yl-pyrimidin-4-yl)-acetamide (Compound 7-39);
N-[2-(5-Methyl-furan-2-yl)-6-pyrazol-1-yl-pyrimidin-4-yl]-2-(4-pyrrolidin-1-yl-piperidin-1-yl)-acetamide (Compound 8-1);
N-[2-(5-Methyl-furan-2-yl)-6-pyrazol-1-yl-pyrimidin-4-yl]-2-(4-methyl-piperazin-1-yl)-acetamide (Compound 8-2);
2-(4-Acetyl-piperazin-1-yl)-N-[2-(5-methyl-furan-2-yl)-6-pyrazol-1-yl-pyrimidin-4-yl]-acetamide (Compound 8-3);
2-((S)-3-Ethylamino-pyrrolidin-1-yl)-N-[2-(5-methyl-furan-2-yl)-6-pyrazol-1-yl-pyrimidin-4-yl]-acetamide (Compound 8-4)
2-(3,5-Dimethyl-piperazin-1-yl)-N-[6-(3,5-dimethyl-pyrazol-1-yl)-2-(5-methyl-furan-2-yl)-pyrimidin-4-yl]-acetamide (Compound 9-1);
N-[6-(3,5-Dimethyl-pyrazol-1-yl)-2-(5-methyl-furan-2-yl)-pyrimidin-4-yl]-2-((R)-3-ethylamino-pyrrolidin-1-yl)-acetamide (Compound 9-2);
N-[6-(3,5-Dimethyl-pyrazol-1-yl)-2-(5-methyl-furan-2-yl)-pyrimidin-4-yl]-2-((S)-3-ethylamino-pyrrolidin-1-yl)-acetamide (Compound 9-3);
2-(3-Diethylamino-pyrrolidin-1-yl)-N-[6-(3,5-dimethyl-pyrazol-1-yl)-2-(5-methyl-furan-2-yl)-pyrimidin-4-yl]-acetamide (Compound 9-4);
N-[6-(3,5-Dimethy)-pyrazol-1-yl)-2-(5-methyl-furan-2-yl)-pyrimidin-4-yl]-2-(pyrrolidin-3-ylamino)-acetamide (Compound 9-5);
N-[6-(3,5-Dimethyl-pyrazol-1-yl)-2-(5-methyl-furan-2-yl)-pyrimidin-4-yl]-2-(2-pyrrolidin-1-yl-ethylamino)-acetamide (Compound 9-6);
N-[6-(3,5-Dimethyl-pyrazol-1-yl)-2-(5-methyl-furan-2-yl)-pyrimidin-4-yl]-2-(3-pyrrolidin-1-yl-propylamino)-acetamide (Compound 9-7);
N-[6-(3,5-Dimethyl-pyrazol-1-yl)-2-(5-methyl-furan-2-yl)-pyrimidin-4-yl]-2-[2-(1-methylpyrrolidin-2-yl)-ethylamino]-acetamide (Compound 9-8);
N-[6-(3,5-Dimethyl-pyrazol-1-yl)-2-(5-methyl-furan-2-yl)-pyrimidin-4-yl]-2-(2-piperidin-1-yl-ethylamino)-acetamide (Compound 9-9);
N-[6-(3,5-Dimethyl-pyrazol-1-yl)-2-(5-methyl-furan-2-yl)-pyrimidin-4-yl]-2-(3-piperidin-1-yl-propylamino)-acetamide (Compound 9-10);
N-[6-(3,5-Dimethyl-pyrazol-1-yl)-2-(5-methyl-furan-2-yl)-pyrimidin-4-yl]-2-(3-morpholin-4-yl-propylamino)-acetamide (Compound 9-11);
N-[6-(3,5-Dimethyl-pyrazol-1-yl)-2-(5-methyl-furan-2-yl)-pyrimidin-4-yl]-2-(4-methyl-piperazin-1-yl)-acetamide (Compound 9-12);
N-[6-(3,5-Dimethyl-pyrazol-1-yl)-2-(5-methyl-furan-2-yl)-pyrimidin-4-yl]-2-morpholin-4-yl-acetamide (Compound 9-13);
2-[1,4]Diazepan-1-yl-N-[6-(3,5-dimethyl-pyrazol-1-yl)-2-(5-methyl-furan-2-yl)-pyrimidin-4-yl]-acetamide (Compound 9-14);
N-[6-(3,5-Dimethyl-pyrazol-1-yl)-2-(5-methyl-furan-2-yl)-pyfimidin-4-yi]-2-(4-pyrrolidin-1 - yl-piperidin-1-yl)-acetamide (Compound 9-15);
N-[6-(3,5-Dimethyl-pyrazol-1-yl)-2-(5-methyl-furan-2-yl)-pyrimidin-4-yl]-2-(4-methyl-[1,4]diazepan-1yl)acetamide (Compound 9-16);
N-[6-(3,5-Dimethyl-pyrazol-1-yl)-2-(5-methyl-furan-2-yl)-pyrimidin-4-yl]-2-[1,4]oxazepan-4-yl-acetamide (Compound 9-17);
2-((R)-3-Dimethylamino-pyrrolidin-1-yl)-N-[6-(3,5-dimethyl-pyrazol-1-yl)-2-(5-methylfuran-2-yl)-pyrimidin-4-yl]-acetamide (Compound 9-18);
2-((S)-3-Dimethylamino-pyrrolidin-1-yl)-N-[6-(3,5-dimethyl-pyrazol-1-yl)-2-(5-methylfuran-2-yl)-pyrimidin-4-yl]-acetamide (Compound 9-19);
N-[6-(3,5-Dimethyl-pyrazol-1-yl)-2-(5-methyl-furan-2-yl)-pyrimidin-4-yl]-2-pyrrolidin-1-yl-acetamide (Compound 9-20);
2-(4,4-Difluoro-piperidin-1-yl)-N-[6-(3,5-dimethyl-pyrazol-1-yl)-2-(5-methyl-furan-2-yl)-pyrimidin-4-yl]-acetamide (Compound 9-21);
N-[6-(3,5-Dimethyl-pyrazol-1-yl)-2-(5-methyl-furan-2-yl)-pyrimidin-4-yl]-2-(4-thiazol-2-yl-piperazin-1-yl)-acetamide (Compound 9-22);
N-[6-(3,5-Dimethyl-pyrazol-1-yl)-2-(5-methyl-furan-2-yl)-pyrimidin-4-yl]-2-(4-ethyl-piperazin-1-yl)-acetamide (Compound 9-23);
2-((R)-3-Acetylamino-pyrrolidin-1-yl)-N-[6-(3,5-dimethyl-pyrazol-1-yl)-2-(5-methyl-furan-2-yl)-pyrimidin-4-yl]-acetamide (Compound 9-24);
2-((S)-3-Acetylamino-pyrrolidin-1-yl)-N-[6-(3,5-dimethyl-pyrazol-1-yl)-2-(5-methyl-furan-2-yl)-pyrimidin-4-yl]-acetamide (Compound 9-25);
N-[6-(3,5-Dimethyl-pyrazol-1-yl)-2-(5-methyl-furan-2-yl)-pyrimidin-4-yl]-2-piperazin-1-yl-acetamide (Compound 9-26);
2-(2,6-Dimethyl-morpholin-4-yl)-N-[6-(3,5-dimethyl-pyrazol-1-yl)-2-(5-methyl-furan-2-yl)-pyrimidin-4-yl]-acetamide (Compound 9-27);
N-[6-(3,5-Dimethyl-pyrazol-1-yl)-2-(5-methyl-furan-2-yl)-pyrimidin-4-yl]-2-(4-hydroxy-piperidin-1-yl)-acetamide (Compound 9-28);
2-[1,4']Bipiperidinyl-1'-yl-N-[6-(3,5-dimethyl-pyrazol-1-yl)-2-(5-methyl-furan-2-yl)-pyrimidin-4-yl]-acetamide (Compound 9-29);
N-[6-(3,5-Dimethyl-pyrazol-1-yl)-2-(5-methyl-furan-2-yl)-pyrimidin-4-yl]-2-(4-methoxy-piperidin-1-yl)-acetamide (Compound 9-30);
2-(4-Acetyl-piperazin-1-yl)-N-[6-(3,5-dimethyl-pyrazol-1-yl)-2-(5-methyl-furan-2-yl)-pyrimidin-4-yl]-acetamide (Compound 9-31);
2-((R)-3-Diethylamino-pyrrolidin-1-yl)-N-[6-(3,5-dimethyl-pyrazol-1-yl)-2-(5-methyl-furan-2-yl)-pyrimidin-4-yl]-acetamide (Compound 9-32);
2-((S)-3-Diethylamino-pyrrolidin-1-yl)-N-[6-(3,5-dimethyl-pyrazol-1-yl)-2-(5-methyl-furan-2-yl)-pyrimidin-4-yl]-acetamide (Compound 9-33);
N-[6-(3,5-Dimethyl-pyrazol-1-yl)-2-(5-methyl-furan-2-yl)-pyrimidin-4-yl]-2-[(S)-3-(ethylmethyl-amino)-pyrrolidin-1-yl]-acetamide (Compound 9-34);
N-[6-(3,5-Dimethyl-pyrazol-1-yl)-2-(5-methyl-furan-2-yl)-pyrimidin-4-yl]-2-[(RF3-(ethylmethyl-amino)-pyrrolidin-1-yl]-acetamide (Compound 9-35);
2-((S)-3-Amino-pyrrolidin-1-yl)-N-[6-(3,5-dimethyl-pyrazol-1-yl)-2-(5-methyl-furan-2-yl)-pyrimidin-4-yl]-acetamide (Compound 9-36)
2-((R)-3-Amino-pyrrolidin-1-yl)-N-[6-(3,5-dimethyl-pyrazol-1-yl)-2-(5-methyl-furan-2-yl)-pyrimidin-4-yl]-acetamide (Compound 9-37)
N-[6-(3,5-Dimethyl-pyrazol-1-yl)-2-(5-methyl-furan-2-yl)-pyrimidin-4-yl]-2-((R)-3-morpholin-4-yl-pyrrolidin-1-yl)-acetamide (Compound 9-38);
N-[6-(3,5-Dimethyl-pyrazol-1-yl)-2-(5-methyl-furan-2-yl)-pyrimidin-4-yl]-2-((R)-3-piperidin-1-yl-pyrrolidin-1-yl)-acetamide (Compound 9-39);
2-(R)-[1,3']Bipyrrolidinyl-1'-yl-N-[6-(3,5-dimethyl-pyrazol-1-yl)-2-(5-methyl-furan-2-yl)-pyrimidin-4-yl]-acetamide (Compound 9-40);
N-[6-(3,5-Dimethyl-pyrazol-1-yl)-2-(5-methyl-furan-2-yl)-pyrimidin-4-yl]-2-((S)-3-morpholin-4-yl-pyrrolidin-1-yl)-acetamide (Compound 9-41);
2-(S)-[1,3']Bipyrrolidinyl-1'-yl-N-[6-(3,5-dimethyl-pyrazol-1-yl)-2-(5-methyl-furan-2-yl)-pyrimidin-4-yl]-acetamide (Compound 9-42);
N-[6-(3,5-Dimethyl-pyrazol-1-yl)-2-(5-methyl-furan-2-yl)-pyrimidin-4-yl]-2,((S)-3-piperidin-1-yl-pyrrolidin-1-yl)-acetamide (Compound 9-43);
N-(1-{[6-(3,5-Dimethyl-pyrazol-1-yl)-2-(5-methyl-furan-2-yl)-pyrimidin-4-ylcarbamoyl]-methyl}-pyrrolidin-3-yl)-2,2,2-trifluoro-acetamide (Compound 9-44);
N-[6-(3,5-Dimethyl-pyrazol-1-yl)-2-(5-methyl-furan-2-yl)-pyrimidin-4-yl]-2-((R)-3-hydroxy-pyrrolidin-1-yl)-acetamide (Compound 9-45);

N-[6-(3,5-Dimethyl-pyrazol-1-yl)-2-(5-methyl-furan-2-yl)-pyrimidin-4-yl]-2-((S)-3-hydroxy-pyrrolidin-1-yl)-acetamide (Compound 9-46);
N-[6-(3,5-Dimethyl-pyrazol-1-yl)-2-(5-methyl-furan-2-yl)-pyrimidin-4-yl]-2-((3R,3'R)-3-fluoro-[1,3']bipyrrolidinyl-1'-yl)-acetamide (Compound 9-47);
N-[6-(3,5-Dimethyl-pyrazol-1-yl)-2-(5-methyl-furan-2-yl)-pyrimidin-4-yl]-2-{(R)-3-[(2-methoxy-ethyl)-methyl-amino]-pyrrolidin-1-yl}-acetamide (Compound 9-48);
N-[6-(3,5-Dimethyl-pyrazol-1-yl)-2-(5-methyl-furan-2-yl)-pyrimidin-4-yl]-2-((3S,3'S)-3-fluoro-[1,3']bipyrrolidinyl-1'-yl)-acetamide (Compound 9-49);
N-[6-(3,5-Dimethyl-pyrazol-1-yl)-2-(5-methyl-furan-2-yl)-pyrimidin-4-yl]-2-((3R,3'S)-3-fluoro-[1,3']bipyrrolidinyl-1'-yl)-acetamide (Compound 9-50);
N-[6-(3,5-Dimethyl-pyrazol-1-yl)-2-(5-methyl-furan-2-yl)-pyrimidin-4-yl]-2-(2,5,2',3',4',5'-hexahydro-[1,3']bipyrrolyl-1'-yl)-acetamide (Compound 9-51);
N-[6-(3,5-Dimethyl-pyrazol-1-yl)-2-(5-methyl-furan-2-yl)-pyrimidin-4-yl]-2-{(S)-3-[(2-methoxy-ethyl)-methyl-amino]-pyrrolidin-1-yl}-acetamide (Compound 9-52);
N-[6-(3,5-Dimethyl-pyrazol-1-yl)-2-(5-methyl-furan-2-yl)-pyrimidin-4-yl]-2-((S)-3-fluoro-pyrrolidin-1-yl)-acetamide (Compound 9-53);
N-[6-(3,5-Dimethyl-pyrazol-1-yl)-2-(5-methyl-furan-2-yl)-pyrimidin-4-yl]-2-((R)-3-fluoro-pyrrolidin-1-yl)-acetamide (Compound 9-54);
N-[6-(3,5-Dimethyl-pyrazol-1-yl)-2-(5-methyl-furan-2-yl)-pyrimidin-4-yl]-2-piperidin-1-yl-acetamide (Compound 9-55);
N-[6-(3,5-Dimethyl-pyrazol-1-yl)-2-(5-methyl-furan-2-yl)-pyrimidin-4-yl]-2-[(R)-3-(2-methoxy-ethylamino)-pyrrolidin-1-yl]-acetamide (Compound 9-56);
N-[6-(3,5-Dimethyl-pyrazol-1-yl)-2-(5-methyl-furan-2-yl)-pyrimidin-4-yl]-2-(3-trifluoromethyl-piperidin-1-yl)-acetamide (Compound 9-57);
N-[6-(3,5-Dimethyl-pyrazol-1-yl)-2-(5-methyl-furan-2-yl)-pyrimidin-4-yl]-2-(3-trifluoromethyl-5,6-dihydro-8H-[1,2,4]triazolo[4,3-a]pyrazin-7-yl)-acetamide (Compound 9-58);
N-[6-(3,5-Dimethyl-pyrazol-1-yl)-2-(5-methyl-furan-2-yl)-pyrimidin-4-yl]-2-[4-(2-oxopyrrolidin-1-yl)-piperidin-1-yl]-acetamide (Compound 9-59);
2-(4-Acetylamino-piperidin-1-yl)-N-[6-(3,5-dimethyl-pyrazol-1-yl)-2-(5-methyl-furan-2-yl)-pyrimidin-4-yl]-acetamide (Compound 9-60);
4-{[6-(3,5-Dimethyl-pyrazol-1-yl)-2-(5-methyl-furan-2-yl)-pyrimidin-4-ylcarbamoyl]-methyl}-piperazine-1-carboxylic acid phenyl ester (Compound 9-61);
4-{[6-(3,5-Dimethyl-pyrazol-1-yl)-2-(5-methyl-furan-2-yl)-pyrimidin-4-ylcarbamoyl]-methyl}-piperazine-1-carboxylic acid benzylamide (Compound 9-62);
N-[6-(3,5-Dimethyl-pyrazol-1-yl)-2-(5-methyl-furan-2-yl)-pyrimidin-4-yl]-2-[4-(3-methyl-benzoyl)-piperazin-1-yl]-acetamide (Compound 9-63);
4-{[6-(3,5-Dimethyl-pyrazol-1-yl)-2-(5-methyl-furan-2-yl)-pyridin-4-ylcarbamoyl]-methyl}-piperazine-1-carboxylic acid dimethylamide (Compound 9-64);
N-[6-(3,5-Dimethyl-pyrazol-1-yl)-2-(5-methyl-furan-2-yl)-pyrimidin-4-yl]-2-{(S)-3-[ethyl(2-methoxy-ethyl)-amino]-pyrrolidin-1-yl}-acetamide (Compound 9-65);
N-[6-(3,5-Dimethyl-pyrazol-1-yl)-2-(5-methyl-furan-2-yl)-pyrimidin-4-yl]-2-((S)-3-isopropoxy-pyrrolidin-1-yl)-acetamide (Compound 9-66);
2-(3,9-Diaza-bicyclo[4.2.1]non-3-yl)-N-[6-(3,5-dimethyl-pyrazol-1-yl)-2-(5-methyl-furan-2-yl)-pyrimidin-4-yl]-acetamide (Compound 9-67);
3-{[6-(3,5-Dimethyl-pyrazol-1-yl)-2-(5-methyl-furan-2-yl)-pyrimidin-4-ylcarbamoyl]-methyl}-3,9-diaza-bicyclo[4.2.1]nonane-9-carboxylic acid tert-butyl ester (Compound 9-68);
N-[6-(3,5-Dimethyl-pyrazol-1-yl)-2-(5-methyl-furan-2-yl)-pyrimidin-4-yl]-2-(9-methyl-3,9-diaza-bicyclo[4.2.1]non-3-yl)-acetamide (Compound 9-69);
N-[6-(3,5-Dimethyl-pyrazol-1-yl)-2-(5-methyl-furan-2-yl)-pyrimidin-4-yl]-2-(4-{[(2-methoxy-ethyl)-methyl-amino]-methyl}-piperidin-1-yl)-acetamide (Compound 9-70);
N-[6-(3,5-Dimethyl-pyrazol-1-yl)-2-(5-methyl-furan-2-yl)-pyrimidin-4-yl]-2-[9-(2-methoxyethyl)-3,9-diaza-bicyclo[4.2.1]non-3-yl]-acetamide (Compound 9-71);
(1-{[6-(3,5-Dimethyl-pyrazol-1-yl)-2-(5-methyl-furan-2-yl)-pyrimidin-4-ylcarbamoyl]-methyl}-piperidin-4-yl)-methyl-carbamic acid tert-butyl ester (Compound 9-72);
N-[6-(3,5-Dimethyl-pyrazol-1-yl)-2-(5-methyl-furan-2-yl)-pyrimidin-4-yl]-2-{4-[(2-methoxyethyl)-methyl-amino]-piperidin-1-yl}-acetamide (Compound 9-73);
2-(2-Dimethylamino-morpholin-4-yl)-N-[6-(3,5-dimethyl-pyrazol-1-yl)-2-(5-methyl-furan-2-yl)-pyrimidin-4-yl]-acetamide (Compound 9-74);
2-((S)-3-Dimethylamino-piperidin-1-yl)-N-[6-(3,5-dimethyl-pyrazol-1-yl)-2-(5-methylfuran-2-yl)-pyrimidin-4-yl]-acetamide (Compound 9-75);
2-((R)-3-Dimethylamino-piperidin-1-yl)-N-[6-(3,5-dimethyl-pyrazol-1-yl)-2-(5-methylfuran-2-yl)-pyrimidin-4-yl]-acetamide (Compound 9-76);
2-((R)-3-Dimethylamino-pyrrolidin-1-yl)-N-[6-(3,5-dimethyl-pyrazol-1-yl)-2-(5-methylfuran-2-yl)-pyrimidin-4-yl]-acetamide (Compound 9-77);
2-((S)-3-Dimethylaminomethyl-pyrrolidin-1-yl)-N-[6-(3,5-dimethyl-pyrazol-1-yl)-2-(5-methyl-furan-2-yl)-pyrimidin-4-yl]-acetamide (Compound 9-78);
2-(4-Dimethylaminomethyl-piperidin-1-yl)-N-[6-(3,5-dimethyl-pyrazol-1-yl)-2-(5-methylfuran-2-yl)-pyrimidin-4-yl]-acetamide (Compound 9-79);
N-[6-(3,5-Dimethyl-pyrazol-1-yl)-2-(5-methyl-furan-2-yl)-pyrimidin-4-yl]-2-(1-methyl-piperidin-4-ylamino)-acetamide (Compound 9-80);
N-[6-(3,5-Dimethyl-pyrazol-1-yl)-2-(5-methyl-furan-2-yl)-pyrimidin-4-yl]-2-((R)-pyrrolidin-3-ylamino)-acetamide (Compound 9-81);
N-[6-(3,5-Dimethyl-pyrazol-1-yl)-2-(5-methyl-furan-2-yl)-pyrimidin-4-yl]-2-{[(R)-1-(tetrahydro-furan-2-yl)methyl]-amino}-acetamide (Compound 9-82);
N-[6-(3,5-Dimethyl-pyrazol-1-yl)-2-(5-methyl-furan-2-yl)-pyrimidin-4-yl]-2-[(tetrahydropyran-4-ylmethyl)-amino]-acetamide (Compound 9-83);
N-[6-(3,5-Dimethyl-pyrazol-1-yl)-2-(5-methyl-furan-2-yl)-pyrimidin-4-yl]-2-{[(S)-1-(tetrahydro-furan-2-yl)methyl]-amino}-acetamide (Compound 9-84);
N-[6-(3,5-Dimethyl-pyrazol-1-yl)-2-(5-methyl-furan-2-yl)-pyrimidin-4-yl]-2-(piperidin-4-ylamino)-acetamide (Compound 9-85);
N-[6-(3,5-Dimethyl-pyrazol-1-yl)-2-(5-methyl-furan-2-yl)-pyrimidin-4-yl]-2-((S)-piperidin-3-ylamino)-acetamide (Compound 9-86); .
2-(Azetidin-3-ylamino)-N-[6-(3,5-dimethyl-pyrazol-1-yl)-2-(5-methyl-furan-2-yl)-pyrimidin-4-yl]-acetamide (Compound 9-87);
N-[6-(3,5-Dimethyl-pyrazol-1-yl)-2-(5-methyl-furan-2-yl)-pyrimidin-4-yl]-2-(2-morpholin-4-yl-ethylamino)-acetamide (Compound 9-88);
N-[6-(3,5-Dimethyl-pyrazol-1-yl)-2-(5-methyl-furan-2-yl)-pyrimidin-4-yl]-2-((R)-piperidin-3-ylamino)-acetamide (Compound 9-89);
N-[6-(3,5-Dimethyl-pyrazol-1-yl)-2-(5-methyl-furan-2-yl)-pyrimidin-4-yl]-2-[((R)-1-pyrrolidin-2-ylmethyl)-amino]-acetamide (Compound 9-90);
N-[6-(3,5-Dimethyl-pyrazol-1-yl)-2-(5-methyl-furan-2-yl)-pyrimidin-4-yl]-2-((S)-pyrrolidin-3-ylamino)-acetamide (Compound 9-91);
N-[6-(3,5-Dimethyl-pyrazol-1-yl)-2-(5-methyl-furan-2-yl)-pyrimidin-4-yl]-2-((R)-pyrrolidin-3-ylamino)-acetamide (Compound 9-92);
N-[6-(3,5-Dimethyl-pyrazol-1-yl)-2-(5-methyl-furan-2-yl)-pyrimidin-4-yl]-2-[((R)-1-pyrrolidin-3-ylmethyl)-amino]-acetamide (Compound 9-93);
N-[6-(3,5-Dimethyl-pyrazol-1-yl)-2-(5-methyl-furan-2-yl)-pyrimidin-4-yl]-3-((R)-3-ethylamino-pyrrolidin-1-yl)-propionamide (Compound 9-94);
3-((S)-3-Dimethylamino-pyrrolidin-1-yl)-N-[6-(3,5-dimethyl-pyrazol-1-yl)-2-(5-methylfuran-2-yl)-pyrimidin-4-yl]-propionamide (Compound 9-95);
N-[6-(3,5-Dimethyl-pyrazol-1-yl)-2-(5-methyl-furan-2-yl)-pyrimidin-4-yl]-3-(4-methyl-piperazin-1-yl)-propionamide (Compound 9-96);
N-[6-(3,5-Dimethyl-pyrazol-1-yl)-2-(5-methyl-furan-2-yl)-pyrimidin-4-yl]-3-morpholin-4-yl-propionamide (Compound 9-97);
3-(2,6-Dimethyl-morpholin-4-yl)-N-[6-(3,5-dimethyl-pyrazol-1-yl)-2-(5-methyl-furan-2-yl)-pyrimidin-4-yl]-propionamid (Compound 9-98);
N-[6-(3,5-Dimethyl-pyrazol-1-yl)-2-(5-methyl-furan-2-yl)-pyrimidin-4-yl]-3-(4-pyrrolidin-1-yl-piperidin-1-yl)-propionamid (Compound 9-99);
3-[1,4]Diazepan-1-yl-N-[6-(3,5-dimethyl-pyrazol-1-yl)-2-(5-methyl-furan-2-yl)-pyrimidin-4-yl]-propionamide (Compound 9-100);
N-[6-(3,5-Dimethyl-pyrazol-1-yl)-2-(5-methyl-furan-2-yl)pyrimidin-4-yl]-3-(4-methyl-[1,4]diazepan-1-yl)-propionamide (Compound 9-101);
N-[6-(3,5-Dimethyl-pyrazol-1-yl)-2-(5-methyl-furan-2-yl)-pyrimidin-4-yl]-3-(4-methyl-[1,4]diazepan-1-yl)-propionamide (Compound 9-102);
3-((R)-3-Dimethylamino-pyrrolidin-1-yl)-N-[6-(3,5-dimethyl-pyrazol-1-yl)-2-(5-methylfuran-2-yl)-pyrimidin-4-yl]-propionamide (Compound 9-103);
N-[6-(3,5-Dimethyl-pyrazol-1-yl)-2-(5-methyl-furan-2-yl)-pyrimidin-4-yl]-3-((S)-3-ethylamino-pyrrolidin-1-yl)-propionamide (Compound 9-104);
N-[6-(3,5-Dimethyl-pyrazol-1-yl)-2-(5-methyl-furan-2-yl)-pyrimidin-4-yl]-3-[1,4]oxazepan-4-yl-propionamide (Compound 9-105);
3-[1,4']Bipiperidinyl-1'-yl-N-[6-(3,5-dimethyl-pyrazol-1-yl)-2-(5-methyl-furan-2-yl)-pyrimidin-4-yl]-propionamide (Compound 9-106);
N-[6-(3,5-Dimethyl-pyrazol-1-yl)-2-(5-methyl-furan-2-yl)-pyrimidin-4-yl]-3-(4-methoxy-piperidin-1-yl)-propionamide (Compound 9-107);
N-[6-(3,5-Dimethyl-pyrazol-1-yl)-2-(5-methyl-furan-2-yl)-pyrimidin-4-yl]-3-(4-thiazol-2-yl-piperazin-1-yl)-propionamide (Compound 9-108);
N-[6-(3,5-Dimethyl-pyrazol-1-yl)-2-(5-methyl-furan-2-yl)-pyrimidin-4-yl]-3-(4-ethyl-piperazin-1-yl)-propionamide (Compound 9-109);
3-(3-Diethylamino-pyrrolidin-1-yl)-N-[6-(3,5-dimethyl-pyrazol-1-yl)-2-(5-methyl-furan-2-yl)-pyrimidin-4-yl]-propionamide (Compound 9-110);
3-((3R,5S)-3,5-Dimethyl-piperazin-1-yl)-N-[6-(3,5-dimethyl-pyrazol-1-yl)-2-(5-methylfuran-2-yl)-pyrimidin-4-yl]-propionamide (Compound 9-111);
N-[6-(3,5-Dimethyl-pyrazol-1-yl)-2-(5-methyl-furan-2-yl)-pyrimidin-4-yl]-3-piperazin-1-yl-propionamide (Compound 9-112);
N-[6-(3,5-Dimethyl-pyrazol-1-yl)-2-(5-methyl-furan-2-yl)-pyrimidin-4-yl]-4-morpholin-4-yl-butyramide (Compound 9-113);
N-[6-(3,5-Dimethyl-pyrazol-1-yl)-2-(5-methyl-furan-2-yl)-pyrimidin-4-yl]-4-pyrrolidin-1-yl-butyramide (Compound 9-114);
N-[6-(3,5-Dimethyl-pyrazol-1-yl)-2-(5-methyl-furan-2-yl)-pyrimidin-4-yl]-4-((S)-3-fluoro-pyrrolidin-1-yl)-butyramide (Compound 9-115);
N-[6-(3,5-Dimethyl-pyrazol-1-yl)-2-(5-methyl-furan-2-yl)-pyrimidin-4-yl]-4-((R)-3-fluoro-pyrrolidin-1-yl)-butyramide (Compound 9-116);
4-{[6-(3,5-Dimethyl-pyrazol-1-yl)-2-(5-methyl-furan-2-yl)-pyrimidin-4-ylcarbamoyl]-methyl}-piperidine-1-carboxylic acid tert-butyl ester (Compound 9-117);
N-[6-(3,5-Dimethyl-pyrazol-1-yl)-2-(5-methyl-furan-2-yl)-pyrimidin-4-yl]-2-piperidin-4-yl-acetamide (Compound 9-118);
N-[6-(3,5-Dimethyl-pyrazol-1-yl)-2-(5-methyl-furan-2-yl)-pyrimidin-4-yl]-2-(1-methyl-piperidin-4-yl)-acetamide (Compound 9-119);
N-[6-(3,5-Dimethyl-pyrazol-1-yl)-2-(5-methyl-furan-2-yl)-pyrimidin-4-yl]-2-[1-(2-methoxyethyl)-piperidin-4-yl]-acetamide (Compound 9-120);
N-[6-(3,5-Dimethyl-pyrazol-1-yl)-2-(5-methyl-furan-2-yl)-pyrimidin-4-yl]-2-(2-pyrrolidin-1-yl-ethoxy)-acetamide (Compound 9-121);
2-(4-Methyl-piperazin-1-yl)-N-(2-oxazol-2-yl-6-pyrazol-1-yl-pyrimidin-4-yl)-acetamide (Compound 10-1);
2-((R)-3-Dimethylamino-pyrrolidin-1-yl)-N-(6-pyrazol-1-yl-2-pyridin-2-yl-pyrimidin-4-yl)-acetamide (Compound 11-1);
2-((S)-3-Dimethylamino-pyrrolidin-1-yl)-N-(6-pyrazol-1-yl-2-pyridin-2-yl-pyrimidin-4-yl)-acetamide (Compound 11-2);
2-(2,6-Dimethyl-morpholin-4-yl)-N-(6-pyrazol-1-yl-2-pyridin-2-yl-pyrimidin-4-yl)-acetamide (Compound 11-3);
N-(6-Pyrazol-1-yl-2-pyridin-2-yl-pyrimidin-4-yl)-2-(4-pyrrolidin-1-yl-piperidin-1-yl)-acetamide (Compound 11-4);
2-(4-Acetyl-piperazin-1-yl)-N-(6-pyrazol-1-yl-2-pyridin-2-yl-pyrimidin-4-yl)-acetamide (Compound 11-5);
2-(3,5-Dimethyl-piperazin-1-yl)-N-(6-pyrazol-1-yl-2-pyridin-2-yl-pyrimidin-4-yl)-acetamide (Compound 11-6);
2-(4-Methyl-piperazin-1-yl)-N-(6-pyrazol-1-yl-2-pyridin-2-yl-pyrimidin-4-yl)-acetamide (Compound 11-7);
2-Morpholin-4-yl-N-(6-pyrazol-1-yl-2-pyridin-2-yl-pyrimidin-4-yl)-acetamide (Compound 11-8);
2-(4-Dimethylamino-piperidin-1-yl)-N-(6-pyrazol-1-yl-2-pyridin-2-yl-pyrimidin-4-yl)-acetamide (Compound 11-9);
2-(4-Methyl-[1,4]diazepan-1-yl)-N-(6-pyrazol-1-yl-2-pyridin-2-yl-pyrimidin-4-yl)-acetamide (Compound 11-10);
2-(3,5-Dimethyl-piperazin-1-yl)-N-[6-(3,5-dimethyl-pyrazol-1-yl)-2-pyridin-2-yl-pyrimidin-4-yl]-acetamide (Compound 12-1);
N-[6-(3,5-Dimethyl-pyrazol-1-yl)-2-pyridin-2-yl-pyrimidin-4-yl]-2-(4-pyrrolidin-1-yl-piperidin-1-yl)-acetamide (Compound 12-2);
N-[6-(3,5-Dimethyl-pyrazol-1-yl)-2-pyridin-2-yl-pyrimidin-4-yl]-2-[(S)-3-(ethyl-methyl-amino)-pyrrolidin-1-yl]-acetamide (Compound 12-3);
2-((R)-3-Amino-pyrrolidin-1-yl)-N-[6-(3,5-dimethyl-pyrazol-1-yl)-2-pyridin-2-yl-pyrimidin-4-yl]-acetamide (Compound 12-4);
2-((S)-3-Amino-pyrrolidin-1-yl)-N-[6-(3,5-dimethyl-pyrazol-1-yl)-2-pyridin-2-yl-pyrimidin-4-yl]-acetamide (Compound 12-5);
N-((S)-1-{[6-(3,5-Dimethyl-pyrazol-1-yl)-2-pyridin-2-yl-pyrimidin-4-ylcarbamoyl]-methyl}-pyrrolidin-3-yl)-2,2,2-trifluoro-acetamide (Compound 12-6);
N-[6-(3,5-Dimethyl-pyrazol-1-yl)-2-pyridin-2-yl-pyrimidin-4-yl]-2-(3-trifluoromethyl-5,6-dihydro-8H-[1,2,4]triazolo[4,3-a]pyrazin-7-yl)-acetamide (Compound 12-7);
N-[6-(3,5-Dimethyl-pyrazol-1-yl)-2-pyridin-2-yl-pyrimidin-4-yl]-2-morpholin-4-yl-acetamide (Compound 12-8);
N-[6-(3,5-Dimethyl-pyrazol-1-yl)-2-pyridin-2-yl-pyrimidin-4-yl]-2-[1,4]oxazepan-4-yl-acetamide (Compound 12-9);
N-[6-(3,5-Dimethyl-pyrazol-1-yl)-2-pyridin-2-yl-pyrimidin-4-yl]-2-(4-methyl-piperazin-1-yl)-acetamide (Compound 12-10);
N-[6-(3,5-Dimethyl-pyrazol-1-yl)-2-pyridin-2-yl-pyrimidin-4-yl]-2-(4-methyl-[1,4]diazepan-1-yl)-acetamide (Compound 12-11);
2-((R)-3-Dimethylamino-pyrrolidin-1-yl)-N-[6-(3,5-dimethyl-pyrazol-1-yl)-2-pyridin-2-yl-pyrimidin-4-yl]-acetamide (Compound 12-12);
2-((S)-3-Dimethylamino-pyrrolidin-1-yl)-N-[6-(3,5-dimethyl-pyrazol-1-yl)-2-pyridin-2-yl-pyrimidin-4-yl]-acetamide (Compound 12-13);
2-((R)-3-Dimethylaminomethyl-piperidin-1-yl)-N-[6-(3,5-dimethyl-pyrazol-1-yl)-2-pyridin-2-yl-pyrimidin-4-yl]-acetamide (Compound 12-14);
2-((S)-3-Dimethylaminomethyl-piperidin-1-yl)-N-[6-(3,5-dimethyl-pyrazol-1-yl)-2-pyridin-2-yl-pyrimidin-4-yl]-acetamide (Compound 12-15);
N-(2,6-Di-pyrazol-1-yl-pyrimidin-4-yl)-2-(4-methyl-piperazin-1-yl)-acetamide (Compound 13-1);
N-(2,6-Di-pyrazol-1-yl-pyrimidin-4-yl)-2-(4-pyrrolidin-1-yl-piperidin-1-yl)-acetamide (Compound 13-2);
2-(3-Dimethylamino-pyrrolidin-1-yl)-N-(2,6-di-pyrazol-1-yl-pyrimidin-4-yl)-acetamide (Compound 13-3);
N-(2,6-Bis-thiazol-2-yl-pyrimidin-4-yl)-2-(4-methyl-piperazin-1-yl)-acetamide (Compound 14-1);
2-(4-Methyl-piperazirl-1-yl)-N-(2-oxazol-5-yl-6-pyrazol-1-yl-pyrimidin-4-yl)-acetamide (Compound 15-1);
2-(3-Dimethylamino-pyrrolidin-1-yl)-N-(2-oxazol-5-yl-6-pyrazol-1-yl-pyrimidin-4-yl)-acetamide (Compound 15-2);
N-[2-(4-Methyl-oxazol-5-yl)-6-pyrazol-1-yl-pyrimidin-4-yl]-2-(4-methyl-piperazin-1-yl)-acetamide (Compound 16-1);
N-(2-Isoxazol-3-yl-6-pyrazol-1-yl-pyrimidin-4-yl)-2-(4-methyl-piperazin-1-yl)-acetamide (Compound 17-1);
2-(3-Dimethylamino-pyrrolidin-1-yl)-N-(2-isoxazol-3-yl-6-pyrazol-1-yl-pyrimidin-4-yl)-acetamide (Compound 17-2);
2-(4-Methyl-piperazin-1-yl)-N-(6-pyrazol-1-yl-2-thiazol-2-yl-pyrimidin-4-yl)-acetamide (Compound 18-1);
N-(6-Pyrazol-1-yl-2-thiazol-2-yl-pyrimidin-4-yl)-2-(4-pyrrolidin-1-yl-piperidin-1-yl)-acetamide (Compound 18-2);
2-((S)-3-Dimethylamino-pyrrolidin-1-yi)-N-(6-pyrazol-1-yi-2-thiazol-2-yl-pyrimidin-4-yi)-acetamide (Compound 18-3);
2-((S)-3-Ethylamino-pyrrolidin-1-yl)-N-(6-pyrazol-1-yl-2-thiazol-2-yl-pyrimidin-4-yl)-acetamide (Compound 18-4);
3-((R)-3-Ethylamino-pyrrolidin-1-yl)-N-(6-pyrazol-1-yl-2-thiazol-2-yl-pyrimidin-4-yl)-propionamide (Compound 18-5);
N-[6-(3,5-Dimethyl-pyrazol-1-yl)-2-thiazol-2-yl-pyrimidin-4-yl]-2-(4-methyl-piperazin-1-yl)-acetamide (Compound 19-1);
N-[6-(3,5-Dimethyl-pyrazol-1-yl)-2-thiazol-2-yl-pyrimidin-4-yl]-2-(4-pyrrolidin-1-yl-piperidin-1-yl)-acetamide (Compound 19-2);
2-((S)-3-Dimethylamino-pyrrolidin-1-yl)-N-[6-(3,5-dimethyl-pyrazol-1-yl)-2-thiazol-2-yl-pyrimidin-4-yl]-acetamide (Compound 19-3);
N-[6-(3,5-Dimethyl-pyrazol-1-yl)-2-thiazol-2-yl-pyrimidin-4-yl]-2-((S)-3-ethylamino-pyrrolidin-1-yl)-acetamide (Compound 19-4);
N-[6-(3,5-Dimethyl-pyrazol-1-yl)-2-thiazol-2-yl-pyrimidin-4-yl]-2-(3-trifluoromethyl-5,6-dihydro-8H-[1,2,4]triazolo[4,3-a]pyrazin-7-yl)-acetamide (Compound 19-5);
N-[6-(3,5-Dimethyl-pyrazol-1-yl)-2-furan-2-yl-pyrimidin-4-yl]-2-(4-methyl-piperazin-1-yl)-acetamide (Compound 20-1);
N-[6-(3,5-Dimethyl-pyrazol-1-yl)-2-furan-2-yl-pyrimidin-4-yl]-2-(4-methyl-piperazin-1-yl)-propionamide (Compound 21-1);
N-[2-(5-Methyl-furan-2-yl)-6-(5-methyl-3-trifluoromethyl-pyrazol-1-yl)-pyrimidin-4-yl]-2-(4-methyl-piperazin-1-yl)-acetamide (Compound 22-1);
(S)-Pyrrolidine-3-carboxylic acid [6-(3,5-dimethyl-pyrazol-1-yl)-2-(5-methyl-furan-2-yl)-pyrimidin-4-yl]-amide (Compound 23-1);
(S)-1-Methyl-pyrrolidine-3-carboxylic acid [6-(3,5-dimethyl-pyrazol-1-yl)-2-(5-methylfuran-2-yl)-pyrimidin-4-yl]-amide (Compound 23-2);
(S)-1-(2-Methoxy-ethyl)-pyrrolidine-3-carboxylic acid [6-(3,5-dimethyl-pyrazol-1-yl)-2-(5-methyl-furan-2-yl)-pyrimidin-4-yl]-amide (Compound 23-3);
(R)-Pyrrolidine-3-carboxylic acid [6-(3,5-dimethyl-pyrazol-1-yl)-2-(5-methyl-furan-2-yl)-pyrimidin-4-yl]-amide (Compound 24-1);
(R)-1-Methyl-pyrrolidine-3-carboxylic acid [6-(3,5-dimethyl-pyrazol-1-yl)-2-(5-methylfuran-2-yl)-pyrimidin-4-yl]-amide (Compound 24-2);
(R)-1-(2-Methoxy-ethyl)-pyrrolidine-3-carboxylic acid [6-(3,5-dimethyl-pyrazol-1-yl)-2-(5-methyl-furan-2-yl)-pyrimidin-4-yl]-amide (Compound 24-3);
(R)-Pyrrolidine-2-carboxylic acid [6-(3,5-dimethyl-pyrazol-1-yl)-2-(5-methyl-furan-2-yl)-pyrimidin-4-yl]-amide (Compound 25-1);
(R)-1-(2-Methoxy-ethyl)-pyrrolidine-2-carboxylic acid [6-(3,5-dimethyl-pyrazol-1-yl)-2-(5-methyl-furan-2-yl)-pyrimidin-4-yl]-amide (Compound 25-2);
2-[6-(3,5-Dimethyl-pyrazol-1-yl)-2-(5-methyl-furan-2-yl)-pyrimidin-4-ylcarbamoyl]-pyrrolidine-1-carboxylic acid benzyl ester (Compound 26-1);
(S)-Pyrrolidine-2-carboxylic acid [6-(3,5-dimethyl-pyrazol-1-yl)-2-(5-methyl-furan-2-yl)-pyrimidin-4-yl]-amide (Compound 26-2);
Pyrrolidine-2-carboxylic acid [6-(3,5-dimethyl-pyrazol-1-yl)-2-(5-methyl-furan-2-yl)-pyrimidin-4-yl]-amide (Compound 26-3);
N-[6-(3,5-Dimethyl-pyrazol-1-yl)-2-thiazol-2-yl-pyrimidin-4-yl]-3-(4-methyl-piperazin-1-yl)-propionamide (Compound 27-1);
N-[6-(3,5-Dimethyl-pyrazol-1-yl)-2-thiazol-2-yl-pyrimidin-4-yl]-2-(4-pyrrolidin-1-yl-piperidin-1-yl)-propionamide (Compound 27-2);
2-((R)-3-Dimethylamino-pyrrolidin-1-yl)-N-[6-(3,5-dimethyl-pyrazol-1-yl)-2-thiazol-2-yl-pyrimidin-4-yl]-propionamide (Compound 27-3);
N-[6-(3,5-Dimethyl-pyrazol-1-yl)-2-thiazol-2-yl-pyrimidin-4-yl]-3-((R)-3-ethylamino-pyrrolidin-1-yl)-propionamide (Compound 27-4);
N-[6-(3,5-Dimethyl-pyrazol-1-yl)-2-thiazol-2-yl-pyrimidin-4-yl]-3-[1,4]oxazepan-4-yl-propionamide (Compound 27-5);
3-((R)-3-Ethylamino-pyrrolidin-1-yl)-N-(6-pyrazol-1-yl-2-thiazol-2-yl-pyrimidin-4-yl)-propionamide (Compound 28-1);
Morpholine-2-carboxylic acid [6-(3,5-dimethyl-pyrazol-1-yl)-2-(5-methyl-furan-2-yl)-pyrimidin-4-yl]-amide (Compound 29-1);
4-Methyl-morpholine-2-carboxylic acid [6-(3,5-dimethyl-pyrazol-1-yl)-2-(5-methyl-furan-2-yl)-pyrimidin-4-yl]-amide (Compound 29-2);
4-(Pyrrolidine-1-carbonyl)-morpholine-2-carboxylic acid [6-(3,5-dimethyl-pyrazol-1-yl)-2-(5-methyl-furan-2-yl)-pyrimidin-4-yl]-amide (Compound 29-3);
Pyrrolidine-1-carboxylic acid [6-(3,5-dimethyl-pyrazol-1-yl)-2-(5-methyl-furan-2-yl)-pyrimidin-4-yl]-amide (Compound 30-1);
Morpholine-4-carboxylic acid [6-(3,5-dimethyl-pyrazol-1-yl)-2-(5-methyl-furan-2-yl)-pyrimidin-4-yl]-amide (Compound 30-2);
4-Methyl-piperazine-1-carboxylic acid [6-(3,5-dimethyl-pyrazol-1-yl)-2-(5-methyl-furan-2-yl)-pyrimidin-4-yl]-amide (Compound 30-3);
1-[6-(3,5-Dimethyl-pyrazol-1-yl)-2-(5-methyl-furan-2-yl)-pyrimidin-4-yl]-3-(2-morpholin-4-yl-ethyl)-urea (Compound 30-4);
1-[6-(3,5-Dimethyl-pyrazol-1-yl)-2-(5-methyl-furan-2-yl)-pyrimidin-4-yl]-3-(3-piperidin-1-yl-propyl)-urea (Compound 30-5);
N-[6-(3,5-Dimethyl-pyrazol-1-yl)-2-(5-methyl-furan-2-yl)-pyrimidin-4-yl]-2-(S)-pyrrolidin-3-yl-acetamide (Compound 31-1);
N-[6-(3,5-Dimethyl-pyrazol-1-yl)-2-(5-methyl-furan-2-yl)-pyrimidin-4-yl]-2-((S)-1-methylpyrrolidin-3-yl)-acetamide (Compound 31-2);
(S)-3-{[6-(3,5-Dimethyl-pyrazol-1-yl)-2-(5-methyl-furan-2-yl)-pyrimidin-4-ylcarbamoyl]-methyl}-1,1-dimethyl-pyrrolidinium (Compound 31-3);
N-[6-(3,5-Dimethyl-pyrazol-1-yl)-2-(5-methyl-furan-2-yl)-pyrimidin-4-yl]-2-[(S)-1-(2-methoxy-ethyl)-pyrrolidin-3-yl]-acetamide (Compound 31-4);
N-[6-(3,5-Dimethyl-pyrazol-1-yl)-2-(5-methyl-furan-2-yl)-pyrimidin-4-yl]-2-(R)-pyrrolidin-3-yl-acetamide (Compound 31-5);
N-[6-(3,5-Dimethyl-pyrazol-1-yl)-2-(5-methyl-furan-2-yl)-pyrimidin-4-yl]-2-((R)-1-methylpyrrolidin-3-yl)-acetamide (Compound 31-6);
N-[6-(3,5-Dimethyl-pyrazol-1-yl)-2-(5-methyl-furan-2-yl)-pyrimidin-4-yl]-2-[(R)-1-(2-methoxy-ethyl)-pyrrolidin-3-yl]-acetamide (Compound 31-7);
1-Methyl-piperidine-4-carboxylic acid [6-(3,5-dimethyl-pyrazol-1-yl)-2-(5-methyl-furan-2-yl)-pyrimidin-4-yl]-amide (Compound 32-1);
N-[6-(3,5-Dimethyl-pyrazol-1-yl)-2-(5-methyl-furan-2-yl)-pyrimidin-4-yl]-2-[(R)-1-(2-methoxy-ethyl)-piperidin-3-yl]-acetamide (Compound 33-1);
1-[6-(3,5-Dimethyl-pyrazol-1-yl)-2-(5-methyl-furan-2-yl)-pyrimidin-4-yl]-3-(3-pyrrolidin-1-yl-propyl)-urea (Compound 34-1);
1-[6-(3,5-Dimethyl-pyrazol-1-yl)-2-(5-methyl-furan-2-yl)-pyrimidin-4-yl]-3-[3-(4-methyl-piperazin-1-yl)-propyl]-urea (Compound 34-2);
1-[6-(3,5-Dimethyl-pyrazol-1-yl)-2-(5-methyl-furan-2-yl)-pyrimidin-4-yl]-3-[2-(1-methylpyrrolidin-2-yl)-ethyl]-urea (Compound 34-3);
1-[6-(3,5-Dimethyl-pyrazol-1-yl)-2-(5-methyl-furan-2-yl)-pyrimidin-4-yl]-3-[2-(1-methyl-piperidin-2-yl)-ethyl]-urea (Compound 34-4);
1-[6-(3,5-Dimethyl-pyrazol-1-yl)-2-(5-methyl-furan-2-yl)-pyrimidin-4-yl]-3-(2-piperidin-2-yl-ethyl)-urea (Compound 34-5);
1-[6-(3,5-Dimethyl-pyrazol-1-yl)-2-(5-methyl-furan-2-yl)-pyrimidin-4-yl]-3-(3-morpholin-4-yl-propyl)-urea (Compound 34-6);
N-[6-(3,5-Dimethyl-pyrazol-1-yl)-2-(5-methyl-furan-2-yl)-pyrimidin-4-yl]-2-(2-methoxy-ethylamino)-acetamide (Compound 35-1);
[6-(3,5-Dimethyl-pyrazol-1-yl)-2-(5-methyl furan-2-yl)-pyrimidin-4-yl]-carbamic acid 2-azepan-1-yl-ethyl ester (Compound 35-2);
[6-(3,5-Dimethyl-pyrazol-1-yl)-2-(5-methyl-furan-2-yl)pyrimidin-4-yl]-carbamic acid 3-piperidin-1-yl-propyl ester (Compound 35-3);
[6-(3,5-Dimethyl-pyrazol-1-yl)-2-(5-methyl-furan-2-yl)-pyrimidin-4-yl]-carbamic acid 3-(2-oxo-pyrrolidin-1-yl)-propyl ester (Compound 35-4);
[6-(3,5-Dimethyl-pyrazol-1-yl)-2-(5-methyl-furan-2-yl)-pyrimidin-4-yl]-carbamic acid 2-morpholin-4-yl-ethyl ester (Compound 35-5);
[6-(3,5-Dimethyl-pyrazol-1-yl)-2-(5-methyl-furan-2-yl)-pyrimidin-4-yl]-carbamic acid 2-piperidin-1-yl-ethyl ester (Compound 35-6);
[6-(3,5-Dimethyl-pyrazol-1-yl)-2-(5-methyl-furan-2-yl)-pyrimidin-4-yl]-carbamic acid 2-(2-oxo-pyrrolidin-1-yl)-ethyl ester (Compound 35-7); and
[6-(3,5-Dimethyl-pyrazol-1-yl)-2-(5-methyl-furan-2-yl)-pyrimidin-4-yl]-carbamic acid 2-pyrrolidin-1-yl-ethyl ester (Compound 35-8).

The compounds of the present invention may be prepared by one of the processes described below.

Compounds of formula (I) and in particular those of formulas (VIIIa) or (IXa) where R¹ is a monocyclic or polycyclic heteroaryl group linked to the pyrimidine ring through a carbon atom and R² is a monocyclic or polycyclic heteroaryl group linked to the pyrimidine ring through a nitrogen atom can be obtained as shown is Scheme 1.

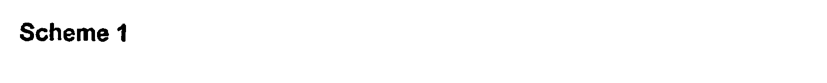

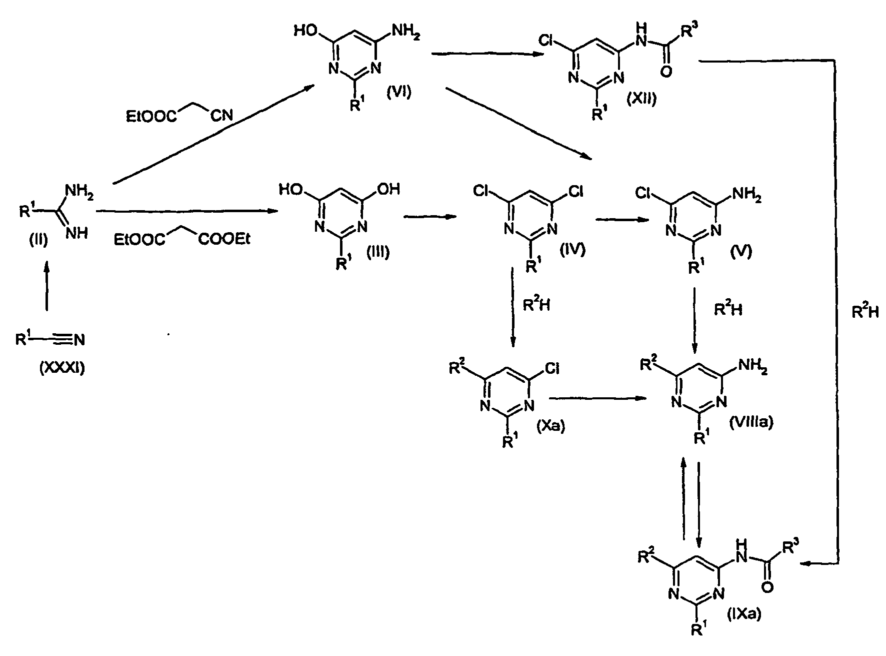

The carboxyamidines of formula (II), wherein R¹ is a monocyclic or polycyclic heteroaryl group linked to the carboxyamidine group through a carbon atom can be obtained by reacting a nitrile of formula (XXXI) with trimethylaluminum and ammonium chloride, in a solvent such as benzene, toluene or xylene, at a temperature from 80° to 120°C. It also can be obtained by reaction of a nitrile of formula (XXXI) with sodium methoxide in methanol at room temperature, followed by reaction with ammonium chloride at the same temperature.

The carboxyamidines of formula (II) can be reacted with diethyl malonate in a solvent such as methanol, ethanol, isopropyl alcohol, butyl alcohol or tetrahydrofuran, in the presence of a base, such as sodium methoxide, sodium ethoxide or potassium *tert*butoxide and at a temperature from room temperature to the boiling point of the solvent to yield the pyrimidine-4,6-diols of formula (III).

The resulting pyrimidine-4,6-diols of formula (III) can be reacted with a chlorinated agent such a phosphorus oxychloride, phosphorus pentachloride or a mixture of them, in a solvent such as phosphorus oxychloride, benzene or toluene, at a temperature from room temperature to the boiling point of the solvent to yield the 4,6-dichloropyrimidine compounds of formula (IV). Optionally, the presence of a base such as dimethylaminoaniline, triethylamine or diisopropyl-ethylamine may be needed in this reaction step.

The reaction of the 4,6-dichloropyrimidine compounds of formula (IV) with ammonium hydroxide in a solvent such as methanol, ethanol, isopropyl alcohol or tetrahydrofuran, at a temperature from 80° to 140°C produces the 6-chloropyrimidin-4-amines of formula (V).

The resulting the 6-chloropyrimidin-4-amines of formula (V) are reacted with a compound of formula R²-H wherein R² is a monocyclic or polycyclic heteroaryl group linked to the pyrimidine ring through a nitrogen atom to yield the compounds of formula (VIIIa) which is a particular case of the compounds of formula (I) according to the invention. The reaction is carried out in a solvent such as dimethylformamide, dimethylacetamide or dimethylsulfoxide, in the presence of a base, such as sodium hydride, potassium carbonate or cesium carbonate, at a temperature from 60° to 140°C.

The compounds of formula (VIIIa) can be acylated by an acid chloride and a base, such as pyridine, triethylamine or diisopropylethylamine, in a solvent such as tetrahydrofuran, methylene chloride, chloroform or pyridine, at a temperature from room temperature to the boiling point of the solvent to yield the compounds of formula (IXa) which is a particular case of the compounds of formula (I) according to the invention. Compounds of formula (IXa) can also be prepared by reaction of amine (VIIIa) with an anhydride, at a temperature from 80° to 160°C.

The 4,6-dichloropyrimidine compounds of formula (IV) can also be converted into the 4-chloropyrimidines of formula (Xa) by reaction with a compound of formula R²-H wherein R² is a monocyclic or polycyclic heteroaryl group linked to the pyrimidine ring through a nitrogen atom. The reaction is carried out in a solvent such as dimethylformamide, dimethylacetamide or dimethylsulfoxide, in the presence of a base, such as sodium hydride, potassium carbonate or cesium carbonate, at a temperature from 60° to 140°C.

The resulting 4-chloropyrimidines of formula (Xa) can then be converted to the compounds of formula (VIIIa) according to the invention by reaction with ammonium hydroxide in a solvent such as methanol, ethanol, isopropyl alcohol or tetrahydrofuran, at a temperature from 80°C to 140°C.

Alternatively, the compounus of formula (VIIIa) according to the invention can also be obtained from the compounds of formula (IXa) by reaction with a mineral acid, such as hydrochloric acid or sulphuric acid, in a solvent such as water, methanol, ethanol or isopropyl alcohol, at a temperature from room temperature to the boiling point of the solvent.

The compounds of formula (IXa) according to the invention can be obtained by reaction of the compounds of formula (XII) with compounds of formula R²H wherein R² is as hereinabove-defined. The reaction is carried out in a solvent such as dimethylformamide, dimethylacetamide or dimethylsulfoxide, in the presence of a base, such as sodium hydride, potassium carbonate or cesium carbonate, at a temperature from 60° to 140°C.

The compounds of formula (XII) can be obtained from the 6-aminopyrimidin-4-ol compounds of formula (VI) by reaction with a carboxylic acid of formula R³COOH, wherein R³ is as hereinabove-defined in the presence of a chlorinated agent such as phosphorus oxychloride, phosphorus pentachloride or thionyl chloride, at a temperature from 60° to 120°C.

The 6-aminopyrimidin-4-ol compounds of formula (VI) are in turn obtained by reaction of the carboxyamidines of formula (II) with ethylcyanoacetate. The reaction is carried out in a solvent such as methanol, ethanol, isopropyl alcohol, butyl alcohol or tetrahydrofuran, in the presence of a base, such as sodium methoxide, sodium ethoxide or potassium *tert*butoxide and at a temperature from room temperature to the boiling point of the solvent.

Compounds of formula (I) and in particular those of formulas (Vlllb) or (IXb) where R¹ is a monocyclic or polycyclic heteroaryl group linked to the pyrimidine ring through a nitrogen atom and R² is a monocyclic or polycyclic heteroaryl group linked to the pyrimidine ring through a carbon atom can be obtained as shown is Scheme 2.

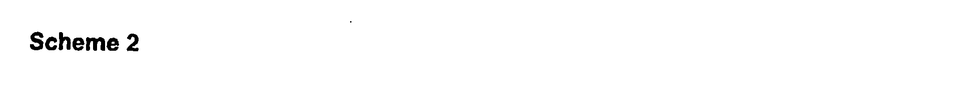

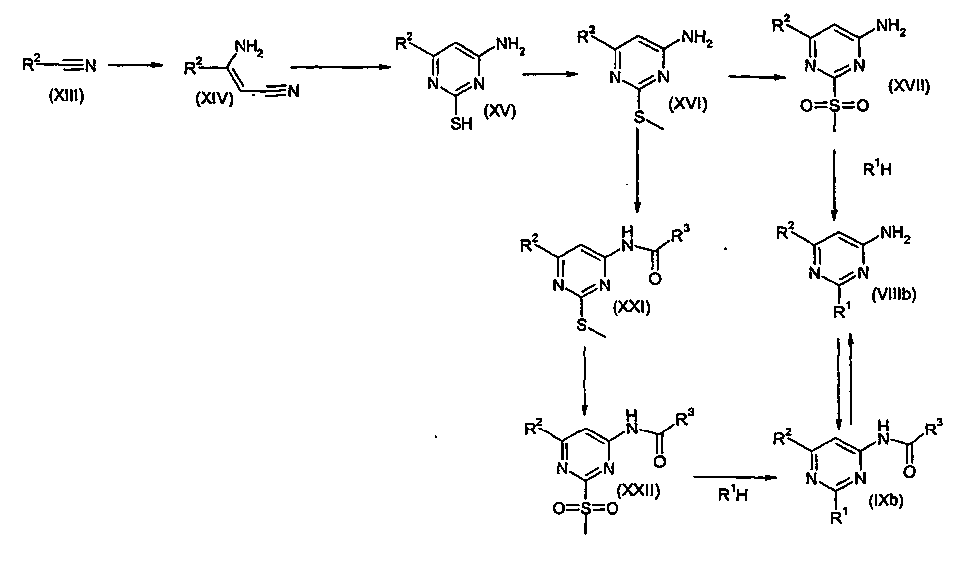

The aminonitriles of formula (XIV) can be obtained by reacting the nitriles of formula (XIII) wherein R² is as hereinabove-defined and acetonitrile, in the presence of a base, preferably as lithium diisopropylamide or potassium *tert*butoxide, in a solvent such as benzene, toluene or xylene, at a temperature from room temperature to the boiling point of the solvent.

The resulting aminonitriles (XIV) are reacted with thiourea, in a solvent such as methanol, ethanol, isopropyl alcohol, butyl alcohol or tetrahydrofuran, in the presence of a base such as sodium methoxide, sodium ethoxide or potassium *tert*butoxide, at a temperature from 60° to 140°C to yield 4-aminopyrimidine-2-thiols of formula (XV).

The 4-aminopyrimidine-2-thiols of formula (XV) can be reacted in a solvent such as water, methanol, ethanol, dimethylformamide or dimethylsulfoxide, with methyl iodide or dimethylsulfate, in the presence of a base such as sodium hydroxide, sodium carbonate, potassium carbonate or sodium hydride, and a temperature from room temperature to 80°C to yield the 2-(methylthio)pyrimidin-4-amines of formula (XVI).

The 2-(methylthio)pyrimidin-4-amines of formula (XVI) can either be reacted with an oxidizing agent, preferably m-chloroperbenzoic acid, oxone or magnesium monoperoxyphthalate, in a solvent such as methanol, ethanol, acetone, methylene chloride or chloroform, and at a temperature from 0° to 70°C to yield 2-(methylsulfonyl)pyrimidin-4-amines of formula (XVII) or in the alternative they can be acylated by an acid chloride and a base, such as pyridine, triethylamine or diisopropylethylamine, in a solvent such as tetrahydrofuran, methylene chloride, chloroform or pyridine, at a temperature from room temperature to the boiling point of the solvent to yield the 2-(methylthio)pyrimidin-4-amides of formula (XXI).

The 2-(methylsulfonyl)pyrimidin-4-amines of formula (XVII) can be converted to the compounds (VIIIb) according to the present invention by reaction with compounds of formula R'-H, wherein R¹ is a monocyclic or polycyclic heteroaryl group linked to the pyrimidine ring through a nitrogen atom. The reaction is carried out in a solvent such as dimethylformamide, dimethylacetamide or dimethylsulfoxide, in the presence of a base, preferably sodium hydride, potassium carbonate or cesium carbonate, and at a temperature from 60° to 160°C. Similarly the 2-(methylsulfonyl)pyrimidin-4-amides of formula (XXII) can be converted to the compounds (IXb) according to the present invention following the same procedure.

The 2-(methylthio)pyrimidin-4-amides of formula (XXI) can be reacted with an oxidizing agent, preferably m-chloroperbenzoic acid, oxone or magnesium monoperoxyphthalate, in a solvent such as methanol, ethanol, acetone, methylene chloride or chloroform, and at a temperature from 0° to 70°C to yield the 2-(methylsulfonyl)pyrimidin-4-amides of formula (XXII).

Finally the compounds (VIIIb) according to the invention can be converted to the compounds (IXb) also according to the invention by reaction with an acid chloride and a base, such as pyridine, triethylamine or diisopropylethylamine, in a solvent such as tetrahydrofuran, methylene chloride, chloroform or pyridine, at a temperature from room temperature to the boiling point of the solvent. Compounds of formula (IXb) can also be prepared by reaction of amine (VIIIb) with an anhydride, at a temperature from 80° to 160°C.

The reverse operation through which compounds of formula (IXb) are converted into compounds of formula (VIIIb) is also possible and can be carried out by reaction with a mineral acid, such as hydrochloric acid or sulphuric acid, in a solvent such as water, methanol, ethanol or isopropyl alcohol, at a temperature from room temperature to the boiling point of the solvent.

Compounds of formula (I) and in particular those of formulas (VIIIc) or (IXc) where R¹ is a monocyclic or polycyclic heteroaryl group linked to the pyrimidine ring through a carbon atom and R² is a monocyclic or polycyclic heteroaryl group linked to the pyrimidine ring through a carbon atom can be obtained as shown is Scheme 3.

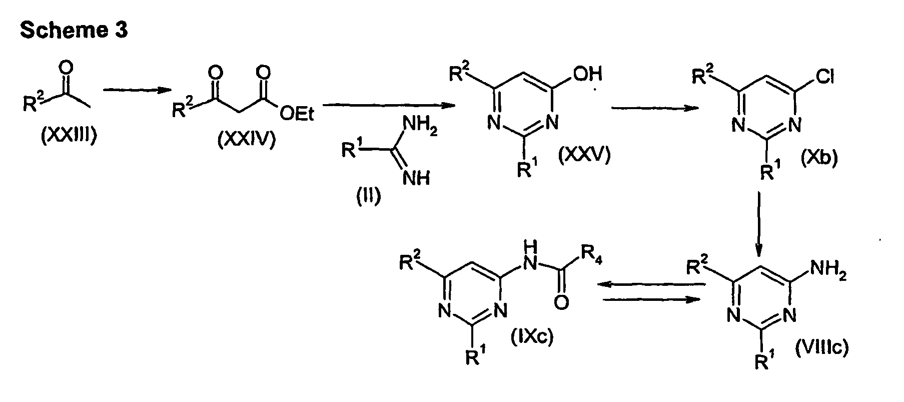

The reaction between methyl ketones of formula (XXIII), wherein R² is a monocyclic or polycyclic heteroaryl group linked to the pyrimidine ring through a carbon atom and diethyl carbonate can be carried out in the presence of a base, preferably sodium hydride, in a solvent such as benzene, toluene, ethyl ether, tetrahydrofuran or dioxane, and at a temperature from 40° to 120°C to yield the substituted ethyl 3-oxo-propanoates of formula (XXIV).

The pyrimidin-4-ol compounds of formula (XXV) can be obtained from the substituted ethyl 3-oxo-propanoates of formula (XXIV) by reaction with carboxyamidines of formula (II) in a solvent such as methanol, ethanol, isopropyl alcohol, butyl alcohol or tetrahydrofuran, in the presence of a base, such as sodium methoxide, sodium ethoxide or potassium *tert*butoxide and at a temperature from room temperature to the boiling point of the solvent.

The pyrimidin-4-ol compounds of formula (XXV) can be reacted with a chlorinated agent such a phosphorus oxychloride, phosphorus pentachoride or a mixture of them, in a solvent such as phosphorus oxychloride, benzene or toluene, at a temperature from room temperature to the boiling point of the solvent to yield the 4-chloropyrimidines of formula (Xb). Optionally, the presence of a base such as dimethylaminoaniline, triethylamine or diisopropyl-ethylamine may be needed in this reaction step.

The compounds of formula (VIIIc) according to the present invention can be prepared from 4-chloropyrimidines of formula (Xb) by reaction with ammonium hydroxide in a solvent such as methanol, ethanol, isopropyl alcohol or tetrahydrofuran, at a temperature from 80°C to 140°C.

Finally the compounds of formula (IXc) according to the present invention can be prepared from the compounds of formula (VIIIc) by acylation with an acid chloride and a base, such as pyridine, triethylamine or diisopropylethylamine, in a solvent such as tetrahydrofuran, methylene chloride, chloroform or pyridine, at a temperature from room temperature to the boiling point of the solvent. Compounds of formula (IXc) can also be prepared by reaction of amine (VIIIc) with an anhydride, at a temperature from 80° to 160°C.

Compounds of formula (VIIIc) can also be obtained from compounds of formula (IXc) by reaction with a mineral acid, such as hydrochloric acid or sulphuric acid, in a solvent such as water, methanol, ethanol or isopropyl alcohol, at a temperature from room temperature to the boiling point of the solvent.

Compounds of formulae (VIIIc) and (IXc) where R¹ is a monocyclic or polycyclic heteroaryl group linked to the pyrimidine ring through a carbon atom and R² is a monocyclic or polycyclic heteroaryl group linked to the pyrimidine ring through a carbon atom can also be obtained as shown is Scheme 4.

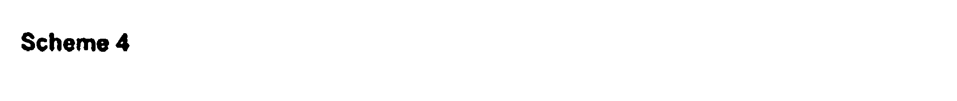

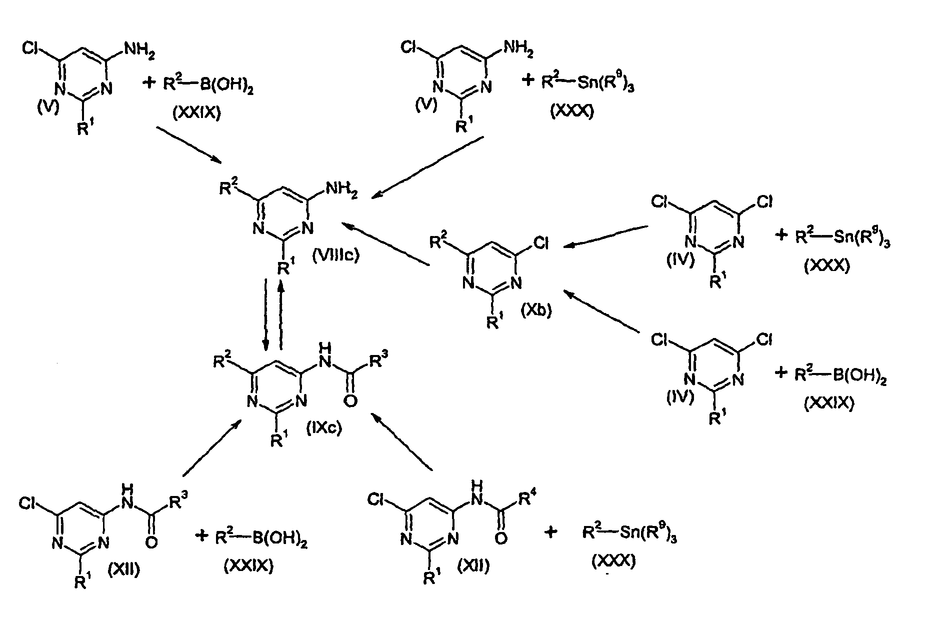

The Suzuki reaction between the 4-aminopirimidines of formulae (IV), (V) or (XII) and the boronic acid of formula (XXIX), wherein R² is a monocyclic or polycyclic heteroaryl group linked to the pyrimidine ring through a carbon atom, is preferably carried out in an organic solvent such as methanol, ethanol, acetonitrile, dioxane, tetrahydrofuran, dimethoxyethane, benzene or toluene, optionally in the presence of water, at a temperature between 60° and 120°C, with a base such as sodium or potassium carbonate and a palladium(0) catalyst such as tetrakis(triphenylphosphine)palladium(0).

The Stille reaction between the 4-aminopirimidines of formulae (IV), (V) or (XII) and the organotin derivative of formula (XXX), wherein R² is a monocyclic or polycyclic heteroaryl group linked to the pyrimidine ring through a carbon atom, is preferably carried out in an organic solvent such as methanol, ethanol, acetonitrile, dioxane, tetrahydrofuran, dimethoxyethane, benzene or toluene, optionally in the presence of water, at a temperature between 60° and 120°C, with a base such as sodium or potassium carbonate and a catalyst such as tetrakis(triphenylphosphine)palladium(0) or bis(triphenylphosphine)palladium(II) chloride.

The 4-chloropyrimidine compounds of formula (Xb) can be converted to the compounds of formula (VIIIc) by reaction with ammonium hydroxide in a solvent such as methanol, ethanol, isopropyl alcohol or tetrahydrofuran, at a temperature from 80° to 140°C. Finally the compounds of formula (IXc) according to the present invention can be prepared from the compounds of formula (VIIIc) by acylation with an acid chloride and a base, such as pyridine, triethylamine or diisopropylethylamine, in a solvent such as tetrahydrofuran, methylene chloride, chloroform or pyridine, at a temperature from room temperature to the boiling point of the solvent. Compounds of formula (IXc) can also be prepared by reaction of amine (VIIIc) with an anhydride, at a temperature from 80° to 160°C.

Compounds of formula (VIIIc) can also be obtained from compounds of formula (IXc) by reaction with a mineral acid, such as hydrochloric acid or sulphuric acid, in a solvent such as water, methanol, ethanol or isopropyl alcohol, at a temperature from room temperature to the boiling point of the solvent.

Compounds of formulae (VIIId) and (IXd) where R¹ is a monocyclic or polycyclic heteroaryl group linked to the pyrimidine ring through a carbon atom and R² is a substituted heterocyclic group can be obtained as shown is Scheme 5.

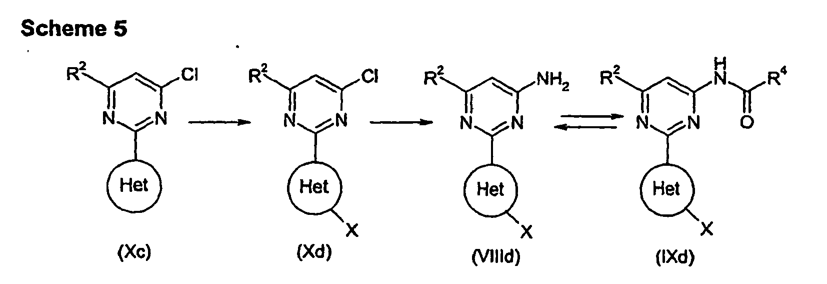

The substituted 4-chloro-2-(2-heteroaryl)pyrimidines of formula (Xd) can be obtained by reaction of the corresponding unsubstituted 4-chloro-2-(2-heteroaryl)pyrimidines of formula (X*c*). When the heteroaryl group is a furyl group the reaction is preferably carried out with *N*-chlorosuccinimide (X = chloro) or *N*-bromosuccinimide (X = bromo), with a solvent such as dimethylformamide or dimethylsulfoxide, at a temperature from 40° to 100°C. Alternatively halogenating agent can be selected from the group consisting of Cl₂, Br₂, SOCl₂ and SOBr₂.

The 4-chloropyrimidine compounds of formula (Xd) can then be converted to the compounds of formula (VIIId) by reaction with ammonium hydroxide in a solvent such as methanol, ethanol, isopropyl alcohol or tetrahydrofuran, at a temperature from 80° to 140°C.

Finally the compounds of formula (IXd) according to the present invention can be prepared from the compounds of formula (VIIId) by acylation with an acid chloride and a base, such as pyridine, triethylamine or diisopropylethylamine, in a solvent such as tetrahydrofuran, methylene chloride, chloroform or pyridine, at a temperature from room temperature to the boiling point of the solvent. Compounds of formula (IXd) can also be prepared by reaction of amine (VIIId) with an anhydride, at a temperature from 80° to 160°C.

Compounds of formula (VIIId) can also be obtained from compounds of formula (IXd) by reaction with a mineral acid, such as hydrochloric acid or sulphuric acid, in a solvent such as water, methanol, ethanol or isopropyl alcohol, at a temperature from room temperature to the boiling point of the solvent.

Carbamates of formula (XXVI), and ureas of formula (XX) can be synthesised as it is summarised on Scheme 6

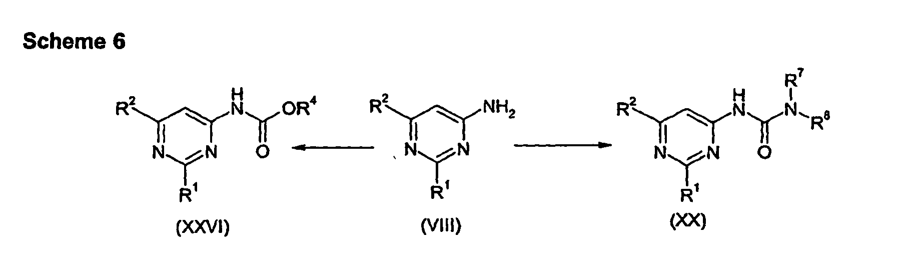

The carbamates of formula (XXVI) are obtained by reaction of a compound of formula (VIII) with a compound of formula Z-COOR³, wherein Z represents a leaving group such as halogen atom, preferably chlorine or a group selected from ethoxy, methoxy, p-nitrophenoxy and imidazolyl. The reaction is carried out in a solvent, such as tetrahydrofuran, chloroform, methylene chloride or dimethylformamide, in the presence of a base, preferably triethylamine, diisopropylethylamine, potassium carbonate or sodium hydroxide, at a temperature from -70° to 100°C.

The compounds of formula (VIII) can also be converted to the ureas of formula (XX) wherein R⁸ is a hydrogen atom by reaction with an isocyanate of formula R⁷-N=C=O in a solvent such as benzene, toluene or xylene, at a temperature from room temperature to 140°C.

The synthesis of amides of formulae (XXXII) and (XXXIII) can be prepared following Scheme 7

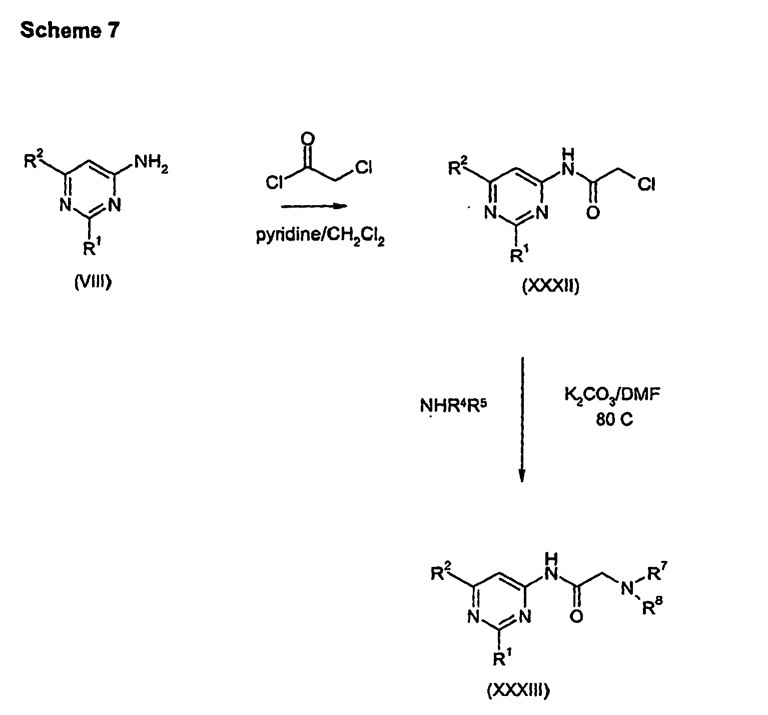

The amides of formula (XXXII) are obtained by reaction of a compound of formula (VIII) with chloroacetyl chloride in a solvent such as dichloromethane and base (e.g., pyridine). The resultant compound of formula (XXXII) is reacted with the desired amine (e.g., NHR⁷R⁸) in the presence of potassium carbonate and DMF to yield the desired amide of formula (XXXIII).

The compounds of formulae (XIII), (XXIII), (XXIX), (XXX) and (XXXI) are known compounds or can be prepared by analogy with known methods. In particular compounds of formulae (XXIX) and (XXX) can be prepared by the methods described in Tyrrell, E.; Brookes, P; Synthesis, 2003, 4, 469-483; Condret, C. Synthetic Communications 1996,26(19), 3543-3547 and Handbook of Organopalladium Chemistry for Organic Synthesis, Two Volume Set Edited by Ei-ichi Negishi. John Wiley and Sons, 2002.

When the defined groups R¹ to R⁸ are susceptible to chemical reaction under the conditions of the hereinbefore described processes or are incompatible with said processes, conventional protecting groups may be used in accordance with standard practice, for example see T. W. Greene and P. G. M. Wuts in 'Protective Groups in Organic Chemistry', 3rd Edition, John Wiley & Sons (1999). It may be that deprotection will form the last step in the synthesis of compounds of formula (I).

### PHARMACOLOGICAL ACTIVITY

### Adenosine A_{2A} receptor binding assays

### Receptor cloning

The coding sequence of the human A_{2A} receptor was amplified from a human brain cDNA library by the polymerase chain reaction. The amplicon was cloned into the pcDNA5/FRT/V5-His-TOPO expression vector (Invitrogen) and sequence confirmed using an ABI 3100 automated sequencer (Applied Biosystems). The expression construct was transfected into Flp-ln HEK cells (Invitrogen) using Lipofectamine 2000 (Invitrogen). Cells stably expressing the human A_{2A} receptor were selected using 1 mg/ml hygromycin in complete DMEM.

### Membrane preparation

Crude membranes were prepared from Flp-ln HEK cells transfected with the human A_{2A} receptor by resuspending cells in lysis buffer (50 mM Tris-HCl pH 7.4, 5mM EDTA, 10 mM MgCl₂) and disrupting under N₂ at a pressure of 900 psi (Parr Cell disruption bomb, cat.4639) for 30 min on ice followed by differential centrifugation. The resulting crude membrane pellet was resuspended in assay buffer (50 mM Tris HCl pH 7.4, 1 mM EDTA, 10 mM MgCl₂). Membrane protein concentration was determined by Bradford assay and aliquots were stored at -80°C.

### Binding assay

An aliquot of membranes (5-10 µg of protein) was pre-incubated for 30 min at RT in the presence of 10 µg/ml Adenosine Deaminase (Type IV Calf Spleen, Sigma). Membranes were then incubated for 90 min with 1.0 nM [³H]-ZM 241385 (27.40 Ci/mmol Tocris R1036) in the presence of varying concentrations of competing ligand. Non-specific binding was determined in the presence of excess (1 µM) of CGS15943. Bound and free ligand were separated by rapid vacuum filtration using a Packard 96-well cell harvester onto UniFilter GF/C filter plates (PerkinElmer) that had been pretreated with 0.5% polyethyleneimine. The filter plates were than washed 3 x 200 µl with 50mM Tris HCl, 50mM NaCl pH 7.4. Bound radioligand was determined by scintillation counting using a TopCount-NXT (Packard). Binding data was analyzed by nonlinear, least-squares curve fitting algorithms using GraphPad Prism (GraphPad Software, Inc. San Diego, CA) or ActivityBase (IDBS, Guildford, Surrey, UK). K, values were calculated from IC₅₀ values using the Cheng-Prusoff equation (Cheng, Y, Prusoff, W.H. Biochem. Pharm. 22:3099-3108, 1973.).

### For A_{2A} membrane assay:

ZM241385 measured Kd = 0.3 ± 0.2 nM; Bₘₐₓ = 33 ± 8 pmol/mg by Scatchard Analysis Binding Ki = 0.25 ± 0.04 nM.

With reference to A_{2A} receptor binding affinities, A_{2A} receptor antagonists of this invention may have a IC₅₀ of less than 10 µM. In one embodiment of this invention, a A_{2A} receptor antagonist has a IC₅₀ of less than 1µM. In another embodiment the IC₅₀ is less than 0.25 µM (*i.e.*, 250 nM).

The pyrimidin-4-amine derivatives of the invention are useful in the treatment or prevention of diseases known to be susceptible to improvement by treatment with an antagonist of an adenosine receptor, in particular those susceptible to improvement by treatement with and antagonist of the A_{2A} adenosine receptor. Such diseases are, for example ischemia, supraventricular arrhythmias, acute renal failure, myocardial reperfusion injury, allergic reactions including but not limited to rhinitis, urticaria, scleroderm arthritis, other autoimmune diseases, inflammatory bowel diseases, asthma, diabetes mellitus, obesity, Parkinson disease, Huntington's disease, dystonias such as restless leg syndrome, dyskinesias such as those caused by prolonged use of neuroleptic and dopaminergic drugs or sleep disorders.

Accordingly, the pyrimidin-4-amine derivatives of the invention and pharmaceutically acceptable salts thereof, and pharmaceutical compositions comprising such compound and/or salts thereof, may be used in a method of treatment of disorders of the human body which comprises administering to a subject requiring such treatment an effective amount of pyrimidin-4-amine derivative of the invention or a pharmaceutically acceptable salt thereof.

The present invention also provides pharmaceutical compositions which comprise, as an active ingredient, at least a pyrimidin-4-amine derivative of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight of the composition depending upon the nature of the formulation and whether further dilution is to be made prior to application. Preferably the compositions are made up in a form suitable for oral, topical, nasal, rectal, percutaneous or injectable administration.

The pharmaceutically acceptable excipients which are admixed with the active compound, or salts of such compound, to form the compositions of this invention are well-known per se and the actual excipients used depend *inter alia* on the intended method of administering the compositions.

Compositions of this invention are preferably adapted for injectable and *per os* administration. In this case, the compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 2 and 500 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Effective doses are normally in the range of 2-2000 mg of active ingredient per day. Daily dosage may be administered in one or more treatments, preferably from 1 to 4 treatments, per day.

Reagents, starting materials, and solvents were purchased from commercial suppliers and used as received. Concentration refers to evaporation under vacuum using a Büchl rotatory evaporator. Reaction products were purified, when necessary, by flash chromatography on silica gel (40-63 µm) with the solvent system indicated. Spectroscopic data were recorded on a Varian Mercury 300 MHz Spectrometer and a Bruker Avance 500 MHz spectrometer. Melting points were recorded on a Büchi 535 apparatus.

### Analytical HPLC-MS Method 1

Platform: Agilent 1100 series: equipped with an auto-sampler, an UV detector (220 nM and 254 nM), a MS detector (APCI);
HPLC column: YMC ODS AQ, S-5, 5µ, 2.0 x50 mm cartridge;
HPLC gradient: 1.0 mL/minute, from 10 % acetonitrile in water to 90 % acetonitrile in water in 2.5 minutes, maintaining 90 % for 1 minute. Both acetonitrile and water have 0.025% TFA.

### Analytical HPLC-MS Method 2

Platform: Agilent 1100 series: equipped with an auto-sampler, an UV detector (220 nM and 254 nM), a MS detector (APCI);
HPLC column: Phenomenex Synergi-Max RP, 2.0 x 50 mm column;
HPLC gradient: 1.0 mL/minute, from 5 % acetonitrile in water to 95 % acetonitrile In water in 13.5 minutes, maintaining 95 % for 2 minute. Both acetonitrile and water have 0.025% TFA.

### Analytical HPLC-MS Method 3

Platform: Agilent 1100 series: equipped with an auto-sampler, an UV detector (220 nM and 254 nM), a MS detector (electrospray);
HPLC column: XTerra MS, C₁₈, 5µ, 3.0 x 250 mm column;
HPLC gradient: 1.0 mL/minute, from 10 % acetonitrile in water to 90 % acetonitrile in water in 46 minutes, jump to 99% acetonitrile and maintain 99 % acetonitrile for 8.04 minutes. Both acetonitrile and water have 0.025% TFA.

### Analytical HPLC-MS Method 4

Platform: Agilent 1100 series: equipped with an auto-sampler, an UV detector (220 nM and 254 nM), a MS detector (APCI) and Berger FCM 1200 CO₂ pump module;
HPLC column: Berger Pyridine, PYR 60A, 6µ, 4.6 x 150 mm column;
HPLC gradient: 4.0 mL/minute, 120 bar, from 10 % methanol in supercritical CO₂ to 60% methanol in supercritical CO₂ in 1.67 minutes, maintaining 60 % for 1 minute. Methanol has 1.5% water. Backpressure regulated at 140 bar.

### Analytical HPLC-MS Method 5

Platform: Dionex: equipped with an autosampler, an UV detector (220nM and 254 nM), a MS detector (APCI);
HPLC column: Phenomenex CX18 4.6x150mm;
HPLC gradient: 95% 0.04%NH4OH/H2O to 90% 0.04%NH4OH/ACN over 9.86 min, 12.30 min run

### Analytical HPLC-MS Method 6

Platform: Agilent: equipped with an autosampler, an UV detector (220nM and 254 nM), a MS detector (APCI);
HPLC column: Waters XTerraMS C18 5microM 125A 3mml.D, x 250mm S/N. 90% 0.025%TFA/H2O to 90% CAN/0.025TFA over 46 min, 60 min run.

### Preparative HPLC-MS

Platform: Shimadzu HPLC equipped with a Gilson 215 auto-sampler/fraction collector, UV detector and a PE Sciez API150EX mass detector,
HPLC column: BHK ODS-O/B, 5 µ, 30x75 mm
HPLC gradient: 35 mL/minute, 10% acetonitrile in water to 100 % acetonitrile in 7 minutes, maintaining 100 % acetonitrile for 3 minutes, with 0.025% TFA.

### Intermediate 1. 2-Furancarboxamidine (HCl)

To a solution of sodium methoxide (5.55 mmol) in methanol (50 mL) was added 2-furonitrile (5.0 g, 53.2 mmol). The mixture was stirred at room temperature for 3 hours. To the resulting solution was slowly added ammonium chloride (3.14 g, 58.7 mmol) and the mixture was stirred at room temperature for 68 hours. The resulting suspension was filtered and the solvent removed under reduced pressure. The solid obtained was washed with ethyl ether (3x25 mL) to give 7.5 g (96%) of 2-furancarboxamidine (HCl). δ (200 MHz, DMSO-d₆): 6.88-6.86 (m, 1H); 7.89 (d, *J*=3.8 Hz, 1H); 8.19 (s, 1H); 9.22 (s, 3H).

### Intermediate 2. 2-(2-Furyl)pyrimidine-4,6-diol

To a solution of sodium ethoxide (0.191 mol) in ethanol (90 mL) was slowly added Intermediate 1 (5.6 g, 38.2 mmol). The mixture was stirred at room temperature for 30 minutes and then, diethyl malonate (4.87 g, 30.4 mmol) was added. The suspension was refluxed for 32 hours. The solvent was removed under reduced pressure, the residue was suspended in water (100 mL) and acidified to pH=6 with 5N hydrochloric acid. The resulting solid was filtered and washed with water (50 mL), ethanol/ethyl ether (4:1, 25 mL), ethyl ether (2x25 mL). 2-(2-Furyl)pyrimidine-4,6-diol was obtained (4.2 g, 78%) as a pale yellow solid.
δ (300 MHz, DMSO-d₆): 5.00 (s, 1H); 6.60-6.70 (m, 1H); 7.40 (d, *J*=3.4 Hz, 1H); 7.80 (s, 1H).

### Intermediate 3.4,6-Dichioro-2-(2-furyl)pyrimidine

A suspension of Intermediate 2 (3.0 g, 16.8 mmol) and *N,N*-diisopropylethylamine (3.85 g, 29.8 mmol) in phosphorous oxychloride (17 mL) was refluxed for 3 hours. The solvent was removed under pressure and methylene chloride (50 mL) and ice were slowly added. The organic layer was washed with water (2x25 mL), saturated solution of sodium bicarbonate (2x25 mL), brine, and dried (Na₂SO₄). The solvent was removed under reduced pressure to give 4,6-dichloro-2-(2-furyl)pyrimidine (3.15 g, 87%) as a grey solid.
δ (300 MHz, CDCl₃): 6.63-6.61 (m, 1H); 7.22 (s, 1H); 7.46 (d, *J*=3.4 Hz, 1H); 7.68 (s, 1H).

### Intermediate 4. 6-Chloro-2-(2-furyl)pyrimidin-4-amine

A suspension of Intermediate 3 (2.0 g, 9.3 mmol) in methanol (14 mL) and 30% ammonium hydroxide (27 mL) was heated in a pressure reactor for 20 hours. The solvent was partially removed under reduced pressure. The resulting solid was filtered, washed with water (25 mL), ethyl ether (25 mL), and dried. 6-Chloro-2-(2-furyl)pyrimidin-4-amine was obtained (1.48 g, 76%) as an off-white solid.
δ (400 MHz, CDCl₃): 5.21 (bs, 2H); 6.31 (s, 1H); 6.54 (m, 1H); 7.28 (d, *J*1=3.7 Hz, 1H); 7.58 (s, 1H).

### Intermediate 5. 2-(2-Furyl)-6-(1H-pyrazol-1-yl)pyrimidin-4-amine

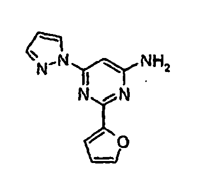

10 To a solution of Intermediate 4 (1.0 g, 5.1 mmol) in anhydrous DMF. (20 mL) was added pyrazol (0.7 g, 10.2 mmol) and cesium carbonate (3.34 g, 10.2 mmol). The mixture was heated at 85°C for 21 hours. The solution was poured into water (50 mL) and extracted with ethyl acetate (2x25 mL). The organic layer was washed with water (2x25 mL) and brine (25 mL), dried (Na₂SO₄), and the solvent removed under reduced pressure. The resulting solid was purified by column chromatography with silica gel, eluting with methylene chloride/methanol (3%), to give (2-furyl)-6-(1*H*-pyrazol-1-yl)pyrimidin-4-amine (0.64 g, 55%) as an off-white solid.
δ (250 MHz, CDCl₃): 5.12 (bs, 2H); 6.48-6.46 (m, 1H); 6.57-6.55 (m, 1H); 6.90 (s, 1H); 7.31 (d, *J*=3.6 Hz, 1H): 7.61 (s, 1H); 7.75 (d, *J*=1.2 Hz, 1H); 8.63 (d, *J*=3.0 Hz, 1H).

### Intermediate 6. 2-Chloro-N-(2-furan-2-yl-6-pyrazol-1-yl-pyrimidin-4-yl)-acetamide

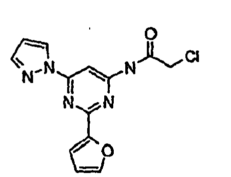

To 5 mL dichloromethane were added 0.3g (1.3mmol) of the compound of Intermediate 5, 0.22g chloroacetyl chloride (0.20mmol, 1.5eq) and 0.16g pyridine. The reaction mixture was stirred at r.t. for 2 hours. The reaction was quenched with 5 mL saturated sodium bicarbonate and extracted; the aqueous solution was washed with an additional 5 mL dichloromethane. The organic layers combined and dried under sodium sulfate, concentrated to a yellow solid (0.4g, 100% crude yield).

The compounds of Table 1 were prepared by reacting Intermediate 6 with the appropriate amine.

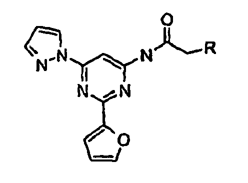

**Table 1**

| **No.** | **R** | **MW** | **MS lON** | **Reten time (min)** | **HPLC GRADIENT** |
|---|---|---|---|---|---|
| 1-1 | piperidin-1-yl | 352.4 | 353 | 4.14 | Method 2 |
| 1-2 | morpholin-4-yl | 354.4 | 355 | 1.77 | Method 4 |
| 1-3 | 4-Methyl-piperazine | 367.4 | 368 | 13.25 | Method 3 |
| 1-4 | Piperazin-1-yl | 353.4 | 354.1 | 12.904 | Method 3 |

### Intermediate 7. Ethyl 3-oxo-3-(1,3-thiazol-2-yl)propanoate

To a solution of 60% sodium hydride (95.4 mmol) in diethyl carbonate (90 ml) was slowly added 2-acetylthiazole (5.0 g). The resulting solution was stirred at room temperature for 1 hour and at 90°C for 2 hours. The reaction mixture was poured into ice/water and acetic acid (5 mL) was added. The mixture was extracted with ethyl acetate (2x75 mL). The organic layer was washed with water (2x50 mL), brine (50 mL), dried (Na₂SO₄), and the solvent removed under reduced pressure. The title compound was obtained (4.4 g, 56%) as an oil by distillation under reduced pressure.
δ (250 MHz, CDCl₃): 1.23 (t, 3H); 4.15 (m, 4H); 7.71 (d, *J*=5.3 Hz, 1H); 7.99 (d, *J*=5.3 Hz, 1H).

### Intermediate 8. 5-Methyl-2-furancarboxamidine (HCl)

The title compound (3.71g, 87%) was obtained as a pale yellow solid starting from 5-methyl-2-furonitrile (2.85 g) by the procedure described in Intermediate 1.
δ (300 MHz, DMSO-d₆): 2.27 (s, 3H); 6.36 (d, *J*=3.6 Hz, 1H); 7.64 (d, *J*=3.6 Hz, 1H); 8.49 (bs, 4 H).

### Intermediate 9. 2-(5-Methyl-2-furyl)-6-(1,3-thiazol-2-yl)pyrimidin-4-ol

To a solution of potassium tertbutoxide (0.57 g, 6.03 mmol) in butanol (2 mL) were added Intermediate 7 (0.85 g, 4.26 mmol) and Intermediate 8 (0.75 g, 4.69 mmol). The mixture was heated at 135°C for 3 hours. The crude reaction was poured into water (20 mL) and acidified with 10% hydrochloric acid (25 mL). The resulting solid was filtered, washed with water (2x25 mL) and dried. The title compound was obtained (0.64 g, 50%) as an off-white solid.
δ (250 MHz, CDCl₃): 2.45 (s, 3H); 6.38 (d, *J*=2.8 Hz, 1H); 6.77 (s, 1H); 7.44 (d, *J*=2.8 Hz, 1H); 7.98 (d, *J*=2.8 Hz, 1H); 8.03 (d, *J*=2.8 Hz, 1H).

### Intermediate 10. 4-Chloro-2-(5-methyl-2-furyl)-6-(1,3-thiazol-2-yl)pyrimidine

A suspension of Intermediate 9 (0.63 g) in phosphorous oxychloride (20 mL) was refluxed for 24 hours. The solvent was removed under pressure and ice and water were slowly added. The resulting solid was filtered, washed with 2N sodium hydroxide, and dried. Purification by column chromatography with silica gel and methylene chloride as eluent gave 4-chloro-2-(5-methyl-2-furyl)-6-(1,3-thiazol-2-yl)pyrimidine (0.44 g, 66%) as an off-white solid.
δ (250 MHz, CDCl₃): 2.41 (s, 3H); 6.15 (d, *J*=4.8 Hz, 1H); 7.31 (d, *J*=3.2 Hz, 1H); 7.53 (d, *J*=3.2 Hz, 1H); 7.81 (s, 1H); 8.03 (d, *J*=4.8 Hz, 1H).

### Intermediate 11. 2-(5-Methyl-2-furyl)-6-(1,3-thiazol-2-yl)pyrimidin-4-amine

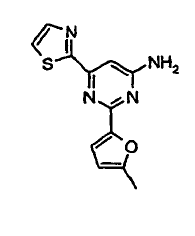

A suspension of Intermediate 10 (0.25 g) in ethanol (22 mL) and 30% ammonium hydroxide (22 mL) was heated at 120°C in a pressure reactor for 2 hours 30 minutes. The solvent was removed under reduced pressure and the residue was dissolved in ethyl acetate (50 mL). The resulting solution was washed with water (2x25 mL), brine (25 mL), dried (Na₂SO₄), and the solvent removed under reduced pressure. Purification by trituration with ethyl ether gave 2-(5-methyl-2-furyl)-6-(1,3-thiazol-2-yl)pyrimidin-4-amine (0.12 g, 53%) as an off-white solid.
δ (250 MHz, DMSO-d₆): 2.38 (s, 3H); 6.29 (dd, *J*1=3.0 Hz, *J*2=1.0 Hz, 1H); 6.99 (m, 1H); 7.08 (d, *J*=3.4 Hz, 1H); 7.28 (bs, 2H); 7.93 (dd, *J*1=3.0 Hz, *J*2=1.0 Hz, 1H); 8.03 (dd, *J*1=3.0 Hz, *J*2=1.0 Hz, 1H).

### Intermediate 12A. 2-Chloro-N-[2-(5-methyl-furan-2-yl)-6-thiazol-2-yl-pyrimidin-4-yl]-acetamide

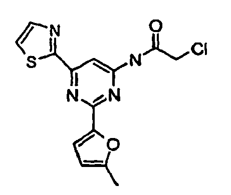

To 5 mL dichloromethane were added 0.34g (1.3mmol) of the compound of Intermediate 11, 0.22g chloroacetyl chloride (2.0mmol, 1.5eq) and 0.16g pyridine. The reaction mixture was stirred at r.t. for 2 hours. The reaction was quenched with 5 mL saturated sodium bicarbonate and extracted; the aqueous solution was washed with an additional 5 mL dichloromethane. The organic layers combined and dried under sodium sulfate, concentrated to a off white solid (0.37g, 85% yield).

The compounds of Table 2A were prepared by reacting Intermediate 12A with the appropriate amine.

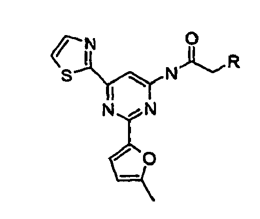

**Table 2A**

| **No.** | **R** | **MW** | **MS ION** | **Reten time (min)** | **HPLC GRADIENT** |
|---|---|---|---|---|---|
| 2-1 | morpholin-4-yl | 385.4 | 386 | 4.3 | Method 2 |
| 2-2 | piperidin-1-yl | 383.5 | 384.0 | 4.422 | Method 2 |
| 2-3 | pyrrolidin-1-yl | 369.4 | 370.0 | 4.429 | Method 2 |
| 2-4 | 4-Methyl-piperazin-1-yl | 398.5 | 399.0 | 4.372 | Method 2 |
| 2-5 | 2,5-Dimethyl-piperazin-1-yl | 412.5 | 413.0 | 4.91 | Method 2 |
| 2-6 | piperazin-1-yl | 384.5 | 385.0 | 4.499 | Method 2 |
| 2-7 | 4-phenyl-piperazin-1-yl | 460.6 | 461.1 | 5.895 | Method 2 |
| 2-8 | [7,4]Diazepan-1-yl | 398.5 | 399.0 | 4.253 | Method 2 |
| 2-9 | 2-phenyl-piperidin-1-yl | 459.6 | 460.0 | 6.002 | Method 2 |
| 2-10 | 3-phenyl-piperidin-1-yl | 459.6 | 460.1 | 6.088 | Method 2 |
| 2-11 | 4-benzyl-piperazin-1-yl | 474.6 | 475.1 | 5.554 | Method 2 |
| 2-12 | 2-methyl-piperidin-1-yl | 397.5 | 398.0 | 4.868 | Method 2 |
| 2-13 | 2,5-Dihydro-pyrrol-1-yl | 367.4 | 368.0 | 4.506 | Method 2 |
| 2-14 | 2,5-Dimethyl-2,5-dihydro-pyrrol-1-yl | 395.5 | 396.0 | 4.907 | Method 2 |
| 2-15 | 2,5-Dimethyl-pyrrolidin-1-yl | 397.5 | 398.0 | 4.917 | Method 2 |
| 2-16 | 3,5-Dimethyl-piperazin-1-y | 412.5 | 413.0 | 4.753 | Method 2 |
| 2-17 | 4-(2-Methoxy-phenyl)-piperazin-1-yl | 490.6 | 491.1 | 5.712 | Method 2 |
| 2-18 | 3-methyl-piperidin-1-yl | 397.5 | 398.0 | 4.964 | Method 2 |
| 2-19 | Azepan-1-yl | 397.5 | 398.0 | 4.974 | Method 2 |
| 2-20 | (S)-2-Methoxymethyl-pyrrolidin-1-yl | 413.5 | 414.0 | 4.836 | Method 2 |
| 2-21 | 3,3-Dimethyl-piperidin-1-yl | 411.5 | 412.1 | 5.409 | Method 2 |
| 2-22 | 3.5-Dimethyl-piperidin-1-yl | 411.5 | 412.1 | 5.351 | Method 2 |
| 2-23 | 4-phenyl-piperidin-1-yl | 459.6 | 460.1 | 5.979 | Method 2 |
| 2-24 | 4-methyl-piperidin-1-yl | 397.5 | 398.0 | 4.994 | Method 2 |
| 2-25 | 4-benzyl-piperidin-1-yl | 473.6 | 474.1 | 6.292 | Method 2 |
| 2-26 | [1,4']Bipiperidinyl-1'-yl | 466.6 | 467.1 | 4.107 | Method 2 |
| 2-27 | 3,4-Dihydro-1H-isoquinolin-2-yl | 431.5 | 432.0 | 5.509 | Method 2 |
| 2-28 | Octahydro-isoquinolin-2-yl | 437.6 | 438.1 | 5.893 | Method 2 |
| 2-29 | (S)-2-pyrrolidin-1-ylmethyl-pyrrolidin-1-yl | 452.6 | 453.1 | 4.437 | Method 2 |
| 2-30 | 4-(3,4-Dimethyl-phenyl)-piperazin-1-yl | 488.6 | 489.1 | 6.476 | Method 2 |
| 2-31 | 4-pyrrolidin-1-yl-piperidin-1-yl | 452.6 | 453.1 | 3.936 | Method 2 |
| 2-32 | 4-(3-Chloro-phenyl)-piperazine1-yl | 495.0 | 495.0 | 6.374 | Method 2 |
| 2-33 | 2,6-Dimethyl-morpholin-4-yl | 413.5 | 414.0 | 5.06 | Method 2 |
| 2-34 | 3-methyl-4-m-tolyl-piperazin-1-yl | 488.6 | 489.1 | 6.206 | Method 2 |
| 2-35 | 4-Methyl-[1,4]diazepan-1-yl | 412.5 | 413.0 | 4.31 | Method 2 |
| 2-36 | piperazin-1-yl | 490.6 | 491.1 | 5.911 | Method 2 |
| 2-37 | 2-(2-piperidin)n-1-yl-ethyl)-piperidin-1-yl | 494.7 | 495.1 | 4.398 | Method 2 |
| 2-38 | [1,4]oxazepan-4-yl | 399.5 | 400.0 | 1.799 | Method 4 |
| 2-39 | 4,4-Difluoro-piperidin-1-yl | 419.5 | 420.0 | 1.681 | Method 4 |
| 2-40 | 4-Acetyl-piperazin-1-yl | 426.5 | 427.0 | 4.462 | Method 2 |
| 2-41 | 1-Benzyl-pyrrolidin-3-yl | 488.6 | 489.1 | 5.478 | Method 2 |
| 2-42 | 4-Acetyl-[1,4]diazepan-1-yl | 440.5 | 441.1 | 4.365 | Method 2 |
| 2-43 | 4-Benzyl-[1,4]diazepan-1-yl | 488.6 | 489.1 | 5.385 | Method 2 |
| 2-44 | 3-Dimethylamino-Pymolidin-1-yl | 412.5 | 413.0 | 3.979 | Method 2 |
| 2-45 | 3-trifluoromethyl-piperidin-1-yl | 451.5 | 452.0 | 5.83 | Method 2 |
| 2-46 | 3-Diethylamino-pyrrolidin-1-yl | 440.6 | 441.1 | 4.311 | Method 2 |
| 2-47 | (R)-3-Dimethylamino-pyrrolidin-1-yl | 412.5 | 413.0 | 4.223 | Method 2 |
| 2-48 | (S)-3-Dimethylamino-pyrrofidin-1-yl | 412.5 | 413.0 | 4.226 | Method 2 |
| 2-49 | 2-pyrrolidin-1-yl ethylamino | 412.5 | 413.1 | 1.872 | Method 4 |
| 2-50 | 3-carboxamido-piperidin-1-yl | 426.5 | 427.1 | 1.905 | Method 4 |
| 2-51 | 4-carboxamido-piperidin-1-yl | 426.5 | 427.1 | 1.933 | Method 4 |
| 2-52 | (R)-3-methyl-piperazin-1-yl | 398.5 | 399.1 | 1.782 | Method 4 |
| 2-53 | 4-(Isopropylcarbamoyl-methyl)-piperazin-1-yl | 483.6 | 484.1 | 1.668 | Method 4 |
| 2-54 | 4-Amino-piperidin-1-yl | 398.5 | 399.0 | 3.739 | Method 2 |
| 2-55 | 4-Dimethylamino-piperidin-1-yl | 426.5 | 427.0 | 3.794 | Method 2 |
| 2-56 | 4-Diethylamino-piperidin-1-yl | 454.6 | 455.1 | 3.944 | Method 2 |
| 2-57 | 4-morpholin-4-yl-piperidin-1-yl | 468.6 | 469.0 | 3.909 | Method 2 |
| 2-58 | 4-Isopropylamino-pipendin-1-yl | 440.6 | 441.1 | 3.946 | Method 2 |
| 2-59 | 4-Cyclopentylamino-piperidin-1-yl | 466.6 | 467.1 | 4.059 | Method 2 |
| 2-60 | 4-Dipmpylamino-piperidin-1-yl | 482.7 | 483.0 | 4.212 | Method 2 |
| 2-61 | 3-Amino-pyrrolidin-1-yl | 384.5 | 385.0 | 3.886 | Method 2 |
| 2-62 | 3-Isopropylamino-pyrrolidin-1-yl | 426.5 | 427.0 | 4.178 | Method 2 |
| 2-63 | 3-Cyclopentylamino-pyrrolidin-1-yl | 452.6 | 453.1 | 4.485 | Method 2 |
| 2-64 | 4-Acetylamino-piperidin-1-yl | 440.5 | 441.0 | 4.443 | Method 2 |
| 2-65 | 4-propionamido-piperidin-1-yl | 454.6 | 455.0 | 4.595 | Method 2 |
| 2-66 | 3-Acetylamino-pyrrolidin-1-yl | 426.5 | 427.0 | 4.507 | Method 2 |
| 2-67 | 3-propionamido-pyrrolidin-1-yl | 440.5 | 441.0 | 4.73 | Method 2 |
| 2-68 | 4-thiazol-2-yl-piperazin-1-yl | 467.6 | 468.0 | 5.118 | Method 2 |
| 2-69 | Hexahydro-pyrrolo[1,2-a]-pyrazin-2-yl | 424.5 | 425.0 | 4.861 | Method 2 |
| 2-70 | 3-piperidin-1-yl-pyrrolidin-1-yl | 452.6 | 453.0 | 4.342 | Method 2 |
| 2-71 | 3-morpholin-4-yl-pyrrolidin-l-yl | 454.6 | 455.0 | 4.293 | Method 2 |
| 2-72 | 2-methyl-3-oxo-piperazin-1-yl | 412.5 | 413 | 5.15 | Method 2 |
| 2-73 | 3-carboxylic acid ethyl ester-piperidine-1-yl | 455.5 | 456 | 5.36 | Method 2 |
| 2-74 | 3-carboxylic acid (2-hydroxy-ethyl)-amide-piperidine-1-yl | 470.6 | 471 | 4.47 | Method 2 |
| 2-75 | (S)-3-Ethylamino-pyrrolidin-1-yl | 412.5 | 413.0 | 4.024 | Method 2 |
| 2-76 | (R)-3-Ethylamino-pyrrolidin-1-yl -yl | 412.5 | 413.0 | 4.028 | Method 2 |
| 2-77 | 4-Ethyl-piperazin-1-yl | 412.5 | 413.0 | 4.759 | Method 2 |
| 2-78 | 2-piperidin-1-yl-ethylamino | 426.5 | 427.0 | 4.056 | Method 2 |
| 2-79 | methyl-(1-methyl-piperidin-4-yl)-amino | 426.5 | 427.0 | 3.863 | Method 2 |
| 2-80 | 2-(1-methyl-pyrrolidin-2-yl)-ethylamino | 426.5 | 427.0 | 3.917 | Method 2 |
| 2-81 | pyrrolidin-3-ylamino | 384.5 | 385.0 | 3.858 | Method 2 |
| 2-82 | 3-pyrrolidin-1-yl-propylamino | 426.5 | 427.0 | 3.896 | Method 2 |
| 2-83 | 3-piperidin-1-yl-propylamino | 440.6 | 441.1 | 3.935 | Method 2 |
| 2-84 | 3-morpholin-4-yl-propylamino | 442.5 | 443.0 | 3.763 | Method 2 |
| 2-85 | 4-Hydroxy-piperin-1-yl | 399.5 | 400.0 | 4.505 | Method 2 |
| 2-86 | 3-Hydroxy-piperidin-1-yl | 399.5 | 400.0 | 4.525 | Method 2 |
| 2-87 | (S)-3-Hydroxy-pyrrolidin-1-yl | 385.4 | 386.0 | 4.522 | Method 2 |
| 2-88 | (S)-3-Acetylamino-pyrrolidin-1-yl | 426.5 | 427.0 | 4.587 | Method 2 |
| 2-89 | (R)-3-Acetylamino-pyrrolidin-1-yl | 426.5 | 427.0 | 4.574 | Method 2 |
| 2-90 | 3-Hydroxy-pyrrolidin-1-yl | 385.4 | 386.0 | 4.536 | Method 2 |
| 2-91 | 4-Isopropyl-piperazin-1-yl | 426.5 | 427.1 | 2.229 | Method 4 |
| 2-92 | 4-Cyclopentyl-piperazin-1-yl | 452.6 | 453.1 | 2.177 | Method 4 |
| 2-93 | piperazin-1-yl | 466.6 | 467.1 | 2.127 | Method 4 |
| 2-94 | 4-propionyl- piperazin-1-yl | 440.5 | 441.1 | 2.345 | Method 4 |
| 2-95 | piperazin-1-yl | 454.6 | 455.1 | 2.254 | Method 4 |
| 2-96 | 4-Aminomethyl-piperidin-1-yl | 412.5 | 413 | 3.77 | Method 2 |
| 2-97 | (pyrrolidin-3-yl-methyl)-amino | 398.5 | 399 | 3.79 | Method 2 |
| 2-98 | 3-Aminomethyl-piperidin-1-yl | 412.5 | 413 | 3.72 | Method 2 |
| 2-99 | (1-methyl-pyrrolidin- 3-ylmethyl)-amino | 412.5 | 413 | 3.89 | Method 2 |
| 2-100 | 3-Aminomethyl-pyrrolidin-1-yl | 398.5 | 399 | 3.79 | Method 2 |
| 2-101 | 4-Methoxy-piperidin-1-yl | 413.5 | 414.0 | 4.829 | Method 2 |
| 2-102 | 3-Dimethylamino-methyl-piperidin-1-yl | 440.6 | 441 | 3.62 | Method 2 |
| 2-103 | 4-Dimethylamino-methyl-piperidin-1-yl | 440.6 | 441 | 3.84 | Method 2 |
| 2-104 | methyl-(1-methyl-pyrrolidin-3-yl-methyl)-amino | 426.5 | 427 | 3.88 | Method 2 |
| 2-105 | 3-Dimethylamino-methyl-pyrrolidin-1-yl | 426.5 | 427 | 3.85 | Method 2 |
| 2-106 | morpholin-4-yl | 385.4 | 386.0 | 4.493 | Method 2 |
| 2-107 | 4-phenyl-piperidin-1-yl | 459.6 | 460.1 | 5.834 | Method 2 |
| 2-108 | 2,5-Dimethyl-piperazin-1-yl | 412.5 | 413.0 | 4.904 | Method 2 |
| 2-109 | piperazin-1-yl | 384.5 | 385.0 | 4.514 | Method 2 |
| 2-110 | [1,4]Diazepan-1-yl | 398.5 | 399.0 | 4.219 | Method 2 |

### Intermediate 12B. N-[2-(5-Methyl-furan-2-yl)-6-thiazol-2-yl-pyrimidin-4-yl]-acrylamide

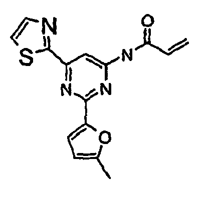

To 20 mL dichloromethane were added 1.4g (5.4 mM, M.W. 258) Intermediate 11 and 0.56 mL (1.3eq) pyridine. Acryloyl chloride (0.6 mL, 0.64g, 1.3eq) was added drop-wise and the reaction mixture was stirred at room temperature for 1 hour. The reaction was quenched with 50 mL saturated sodium bicarbonate and extracted with 50 mL dichloromethane. The product stayed in aqueous solution. The aqueous solution was concentrated to dryness and resuspended in 15 mL DMF and 15 mL EtOH. The mixture was filtered, and the filtrate was concentrated. The solid thus obtained was used for library synthesis without further purification. The solid was dissolved in 1/1 DMF/EtOH, added 2-eq amine and stirred at room temperature overnight. Purified by either TLC plates or Prep LC-MS.

The compounds of Table 2B were prepared by reacting intermediate 12B with the appropriate amine.

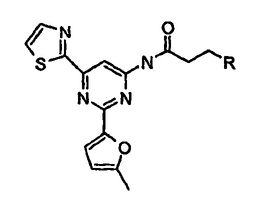

**Table 2B**

| **No.** | **R** | **MW** | **MS ION** | **Reten time (min)** | **HPLC GRADIENT** |
|---|---|---|---|---|---|
| 2-111 | (S)-3-dimethylamino-pyrrolidin-1-yl | 426.5 | 427.0 | 3.923 | Method 2 |
| 2-112 | 4-Acetyl-piperazin-1-yl | 440.5 | 441 | 4.43 | Method 2 |
| 2-113 | 4-methyl-piperazin-1-yl | 412.5 | 413 | 4.15 | Method 2 |
| 2-114 | 3-piperazin-1-yl | 398.5 | 399 | 4.04 | Method 2 |
| 2-115 | 4-pyrrolidin-1-yl-piperidin-1-yl | 466.6 | 467 | 4.05 | Method 2 |
| 2-116 | Morpholin-4-yl | 399.5 | 400 | 4.47 | Method 2 |
| 2-117 | (R)-3-Dimethylamino-pyrrolidin-1-yl | 426.5 | 427.0 | 3.929 | Method 2 |
| 2-118 | 4-Acetyl-[1,4]diazepam-1-yl | 454.6 | 455.0 | 4.738 | Method 2 |
| 2-119 | [1,4]oxazepan-4-yl | 413.5 | 414.0 | 4.824 | Method 2 |
| 2-120 | 4-Methyl-[1,4]diazepam-1-yl | 426.5 | 427.0 | 4.141 | Method 2 |
| 2-121 | (R)-Ethylamino- prrolidin-1-yl | 426.5 | 427.0 | 4.212 | Method 2 |
| 2-122 | 3,5-Dimethyl-piperazin-1-yl | 426.5 | 427.0 | 4.273 | Method 2 |
| 2-123 | [1,4]Diazepan-1-yl | 412.5 | 413.0 | 4.142 | Method 2 |

The compounds of Table 2C were prepared as described below.

### Compound 2-124. N-[2-(5-Methyl-furan-2-yl)-6-thiazol-2-yl-pyrimidin-4-yl]2-(1-methyl-piperidin-4-yl)-acetamide

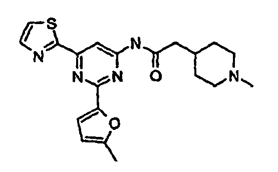

To a 20 mL reaction vial were added 1-Boc-piperidin-4-yl acetic acid (300mg, 2eq) and 2 mL dry THF followed by 0.12 mL oxalyl chloride (2.2eq) and one drop of DMF. The reaction mixture was stirred at room temperature for 30 mins. To a 20 mL reaction vial was added 2 mL dry THF and Intermediate 11 (160 mg, 0.62mmol, 1eq), followed by 100mg sodium hydride (4eq, 60% W in mineral oil). The reaction mixture was stirred at room temperature for 5 mins and was added drop wise to the acid-chloride mixture above. Upon addition the reaction mixture turned cloudy. The reaction mixture was stirred at room temperature for an hour. The reaction mixture was added to ice drop dried under sodium sulfate, concentrated and purified by prep LC-MS. LCMS (APCl) m/z 484.0 (MH⁺), Tr=3.16min.

To the boc-protected compound obtained above were added 3 mL dichloromethane and 3 mL TFA. The reaction mixture was stirred at room temperature for 30 mins and concentrated, dissolved in 2 mL ethanol, followed by 2 mL formaldehyde (30% W in water). Under stirring, 5 drops of borane-pyridine complex was added followed by 1 drop of acetic acid. The mixture was stirred at 60°C for 12 hours. The reaction mixture was concentrated and purified by prep LC-MS to give N-[2-(5-Methyl-furan-2-yl)-6-thiazol-2-yl-pyrimidin-4-yl]-2-(1-methyl-piperidin-4-yl)-acetamide. LCMS (APCI) m/z 398.0 (MH⁺), Tr=2.33min.

### Compound 2-125. N-[2-(5-Methyt-furan-2-yl)-6-thiazol-2-yl-pyrimidin-4-yl]-2-piperidin-4-yl-acetamide

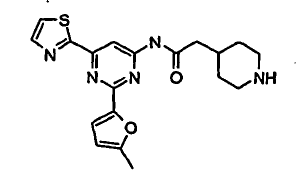

Compound 2-125 was prepared according to the procedures described above for the preparation of Compound 2-124, except that deprotection of the boc group was the final synthetic step.

**Table 2C**

| **No.** | **Structure** | **MW** | **MS ION** | **Reten time (min)** | **HPLC GRADIENT** |
|---|---|---|---|---|---|
| 2-124 | 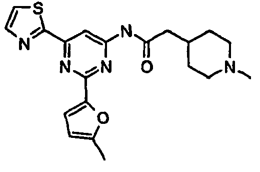 | 397.5 | 398 | 4.93 | Method 2 |
| 2-125 | 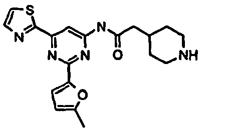 | 383.5 | 384 | 4.82 | Method 2 |

### Compound 2-126. N-[2-(5-Methyl-furan-2-yl)-6-thiazol-2-yl-pyrimidin-4-yl]-2-(2-pyrrolidin-1-yl-ethoxy)-acetamide

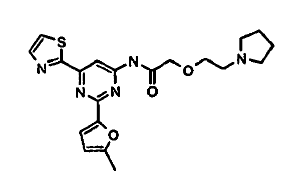

To 2 mL THF were added 100mg (0.9mmol, 3eq) 2-Pyrrolidin-1-yl-ethanol and 35mg (0.9mmol, 3eq) sodium hydride. The mixture was stirred at r.t. for 10 mins. To 2 mL DMF were added 100mg (0.3mmol, 1eq) Intermediate 12A and 30 mg TBAI, under nitrogen with stirring the hydroxyl amine solution was added drop wise. The reaction mixture was stirred at r.t. for 30mins, then heated to 60°C for 2 hours. The reaction mixture was purified by prep LC-MS using prep3. Obtained 10 mg product.

**Table 2D**

| **No.** | **Structure** | **MW** | **MS ION** | **Reten time (min)** | **HPLC GRADIENT** |
|---|---|---|---|---|---|
| 2-126 | 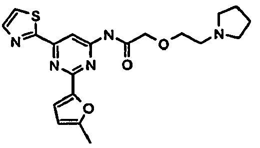 | 413.5 | 414.0 | 4.95 | Method 2 |

### Intermediate 12C. 2-Chloro-6-(3,5-dimethyl-pyrazol-1-yl)-pyrimidin-4-ylamine

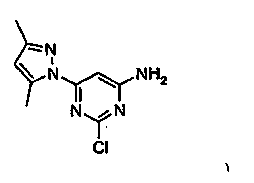

To a solution of N-[2-Chloro-6-(3,5-dimethyl-pyrazol-1-yl)-pyrimidin-4-yl]-acetamide (2.3 g, 8.7 mmol, 1eq.) in MeOH (100 mL) was added catalytic K₂CO₃ (119 mg, 0.87 mmol, 0.1 eq). The mixture was stirred overnight at room temperature. After the completion of the reaction, the solution was concentrated in vacuo. The residue solid was used in the next reaction without further purification. 2-Chloro-6-(3,5-dimethyl-pyrazol-1-yl)-pyrimidin-4-ylamine was obtained as a biege solid in 97% yield. LCMS (APCI) m/z 224.0 [MH+], Tr = 2.38 min.

### Intermediate 12D. 2-Chloro-N-[2-chloro-6-(3,5-dimethyl-pyrazol-1-yl)-pyrimidin-4-yl]-acetamide

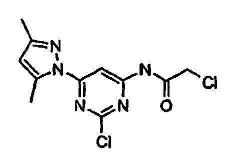

To a crude solution of Intermediate 12C (8.7 mmol) in DCM (100 mL) was added pyridine (0.95 mL, 11.7 mmol, 1.3 eq) followed by dropwise addition of chloroacetyl chloride (1.1 mL, 13.5 mmol, 1.5 eq) at 0°C. The mixture was allowed to warm up to room temperature overnight with stirring. After the completion of the reaction, the solution was cooled to 0°C with a ice bath and carefully quenched with 25 mL of half saturated solution of NaHCO₃ (aq). The reaction was extracted with DCM (3 x 25 mL). The combined organic layer was washed with brine, dried with magnesium sulfate, filtered, and concentrated. The residue was purified by silica gel chromatography (10% methanol/DCM) to afford 2-Chloro-N-[2-chloro-6-(3,5-dimethyl-pyrazol-1-yl)-pyrimidin-4-yl]-acetamide in 92% as a yellow solid. LCMS (APCI) m/z 300.0 [MH+], Tr = 2.69 min.

### Intermediate 12E. N-[6-(3,5-Dimethyl-pyrazol-1-yl)-2-mercapto-pyrimidin-4-yl]-2-(4-methyl-piperazin-1-yl)-acetamide

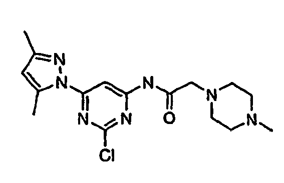

Intermediate 12D (2.6 g, 8.7 mmol, 1eq.) was dissolved in DCM (25 mL) and N-4-methyl-piperazine (1.3 mL, 11.3 mmol, 1.3 eq.) was added dropwise at room temperature. After stirring overnight, the solution was partitioned with H₂O and extracted with DCM (3 x 25 mL). The combined organic layers was washed with brine, dried, filtered and concentrated under reduced pressure. Column chromagraphy (10% methanol in DCM) yielded N-[6-(3,5-Dimethylpyrazol-1-yl)-2-mercapto-pyrimidin-4-yl]-2-(4-methyl-piperazin-1-yl)-acetamide as a yellow solid in 64% along with some disubsituted product. LCMS (APCI) m/z 363.6 [MH+], Tr = 7.34 min.

Compounds of Table 2E were prepared by Suzuki coupling, according to the following scheme:

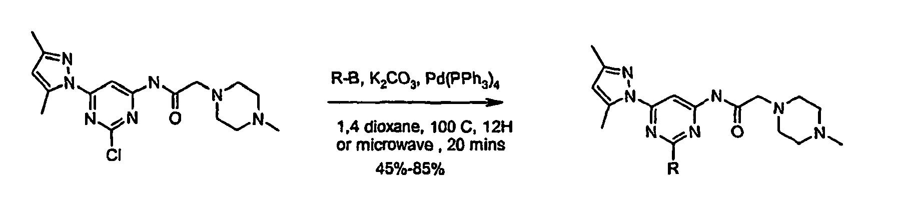

**Table 2E**

| **No.** | **R** | **MW** | **MS ION** | **Reten time (min)** | **HPLC GRADIENT** |
|---|---|---|---|---|---|
| 2-127 | 4-Methoxy-pyridin-3-yl | 436.5 | 436.5 | 6.41 | Method 5 |
| 2-128 | 6-Methoxy-pyridin-3-yl | 436.5 | 437.1 | 7.59 | Method 5 |
| 2-129 | 3,4-Dimethoxy-phenyl | 465.6 | 466.8 | 7.63 | Method 5 |
| 2-130 | 4-Trifluoromethoxy-phenyl | 489.5 | 490.5 | 9.64 | Method 5 |
| 2-131 | 3-Fluoro-phenyl | 423.5 | 424.1 | 8.16 | Method 5 |
| 2-132 | 2-Fluoro-phenyl | 423.5 | 424.8 | 8.21 | Method 5 |
| 2-133 | 2-Fluoro-3-methoxy-phenyl | 453.5 | 454.2 | 8.16 | Method 5 |
| 2-134 | 2,4-Dimethoxy-phenyl | 465.6 | 466.3 | 8.23 | Method 5 |
| 2-135 | 2-Cyano-phenyl | 430.5 | 431.0 | 7.511 | Method 5 |
| 2-136 | 2-Methoxy-phenyl | 435.5 | 437.0 | 8.11 | Method 5 |
| 2-137 | 4-Fluoro-2-methyl-phenyl | 437.5 | 438.1 | 8.74 | Method 5 |
| 2-138 | 3-Methoxy-phenyl | 435.5 | 435.8 | 8.22 | Method 5 |
| 2-139 | 3,5-Dimethoxy-phenyl | 465.6 | 466.0 | 8.38 | Method 5 |
| 2-140 | 3,5-Difluoro-phenyl | 441.5 | 442.4 | 8.65 | Method 5 |
| 2-141 | 3-Fluoro-4-methyl-phenyl | 437.5 | 437.5 | 9.20 | Method 5 |
| 2-142 | 3-Fluoro-2-methoxy-phenyl | 453.5 | 454.7 | 8.39 | Method 5 |
| 2-143 | 2-143 3-Fluoro-4-methoxy-phenyl | 453.5 | 454.1 | 8.27 | Method 5 |
| 2-144 | 2,3-Difluoro-phenyl | 441.5 | 441.9 | 8.37 | Method 5 |
| 2-145 | 5-Methoxy-pyridin-3-yl | 436.517 | 437.1 | 3.765 | Method 2 |

### Intermediate 12F.2-Chloro-6-pyrazol-1-yl-pyrimidin-4-ylamine

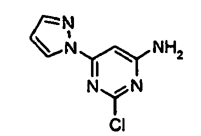

Intermediate 12F was obtained according to the same procedure as described above using pyrazole instead of 3,5-dimethylpyrazole and obtained in in similar yield. LCMS (APCI) m/z 195.9 [MH+], Tr = 2.15 min.

### Intermediate 12G. 2-Chloro-N-(2-chloro-6-pyrazol-1yl-pyrimidin-4-yl)-acetamide

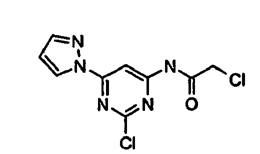

Intermediate 12G was prepared in similar manner as described above using pyrazole instead of 3,5-dimethylpyrazole and obtained in similar yield. LCMS (APCI) m/z 271.9 [MH+], Tr = 2.51 min.

### Intermediate 12H. N-(2-Chloro-6-pyrazol-1-yl-pyrimidin-4-yl)-2-(4-methyl-piperazin-1-yl)-acetamide

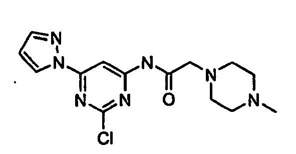

Intermediate 12H was produced in 45% yield using the same procedure as decribed above using pyrazole instead of 3,5-dimethylpyrazole. LCMS (APCI) m/z 335.9 [MH+], Tr = 6.13 min.

The compounds of Table 2F were prepared from Intermediate 12H via Suzuki coupling.

**Table 2F**

| **No.** | **R** | **MW** | **MS ION** | **Reten time (min)** | **HPLC RADIENT** |
|---|---|---|---|---|---|
| 2-146 | 3-Methoxy-phenyl | 407.5 | 408.5 | 7.49 | Method 5 |
| 2-147 | 3,4-Dimethoxy-phenyl | 437.5 | 439.0 | 7.07 | Method 5 |
| 2-148 | 3-Cyano-phenyl | 402.5 | 402.7 | 7.05 | Method 5 |
| 2-149 | 3-Fluoro-4-methoxy-phenyl | 425.5 | 426.3 | 7.57 | Method 5 |
| 2-150 | 2-Methoxy-phenyl | 407.5 | 408.2 | 7.57 | Method 5 |
| 2-151 | 4-Methoxy-phenyl | 407.5 | 407.9 | 7.5 | Method 5 |
| 2-152 | 3-Trifluoromethyl-phe nyl | 445.5 | 446.0 | 8.44 | Method 5 |
| 2-153 | 2,3-Difluoro-phenyl | 413.4 | 414.0 | 7.74 | Method 5 |
| 2-154 | 3-Trifluoromethoxy- phenyl | 461.5 | 462.0 | 8.75 | Method 5 |
| 2-155 | 2-Fluoro-3-methoxy-phenyl | 425.5 | 426.5 | 7.54 | Method 5 |

### Intermediate 13.2-Chloro-N-[2-(5-methyl-furan-2-yl)-6-pyridin-2-yl-pyrimidin-4-yl]-acetamide

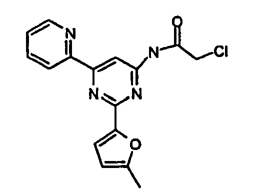

Intermediate 13 was prepared according to the procedures described in Intermediate 12A, except that Ethyl 3-oxo-3-(pyridin-2-yl)propanoate was used instead of Intermediate 7.

The compounds of Table 3 were prepared by reacting Intermediate 13 with the appropriate amine.

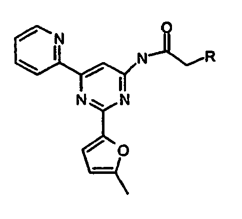

**Table 3**

| **No.** | **R** | **MW** | **MS ION** | **Reten time (min)** | **HPLC GRADIENT** |
|---|---|---|---|---|---|
| 3-1 | morpholin-4-yl | 379.4 | 380.0 | 4.011 | Method 2 |
| 3-2 | piperidin-1-yl | 377.4 | 378.1 | 4.297 | Method 2 |
| 3-3 | 4-(Isopropyl-carbamoyl-methyl)-piperazin-1-yl | 477.6 | 478.1 | 4.577 | Method 2 |
| 3-4 | 4-phenyl-piperidin-1-yl | 453.5 | 454.1 | 5.675 | Method 2 |
| | 3-5 [1,4']Bipiperidinyl-1'-yl | 460.6 | 461.1 | 3.895 | Method 2 |
| 3-6 | 3,4-Dihydro-1H-isoquinolin-2-yl | 425.5 | 426.1 | 5.131 | Method 2 |
| | 3-7 4-(3-Methoxy-phenyl)-piperazin-1-yl | 484.6 | 485.1 | 5.591 | Method 2 |
| 3-8 | 4-methyl-piperazin-1-yl | 392.5 | 393.1 | 4.095 | Method 2 |

### Intermediate 14A. 2-Chloro-N-(2-pyridin-2-yl-6-thiazol-2-yl-pyrimidin-4-yl)-acetamide

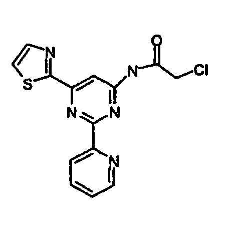

Intermediate 14A was prepared according to the procedures described in Intermediate 12A, except that Pyridine-2-carboxamidine HCl was used instead of Intermediate 8.

The compounds of Table 4A were prepared by reacting Intermediate 14A with the appropriate amine.

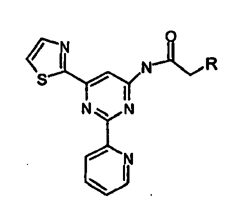

**Table 4A**

| **No.** | **R** | **MW** | **MS ION** | **Reten time (min)** | **HPLC GRADIENT** |
|---|---|---|---|---|---|
| 4-1 | (R)-3-Dimethylamino-pyrrolidin-1-yl | 409.5 | 410.1 | 2.725 | Method 2 |
| 4-2 | (S)-3-Dimethylamino-pyrrolidin-1-yl | 409.5 | 410.1 | 2.756 | Method 2 |
| 4-3 | 2,5-Dimethyl-piperazin-1-yl | 409.5 | 410 | 1.802 | Method 4 |
| 4-4 | 3,5-Dimethyl-piperazin-1-yl | 409.5 | 410 | 1.804 | Method 4 |
| 4-5 | 4-Phenyl-piperazin-1-yl | 457.6 | 458 | 1.788 | Method 4 |
| 4-6 | 4-(2-Methoxy-phenyl)-piperazin-1-yl | 487.6 | 488 | 1.726 | Method 4 |
| 4-7 | 4-Methyl-piperazin-1-yl | 395.5 | 396 | 1.877 | Method 4 |
| 4-8 | 4-Benzyl-piperazin-1-yl | 471.6 | 472.1 | 1.771 | Method 4 |
| 4-9 | Morpholin-4-yl | 382.4 | 383 | 1.774 | Method 4 |
| 4-10 | 2.6-Dimethyl-morpholin-4-yl | 410.5 | 411 | 1.675 | Method 4 |
| 4-11 | Piperidin-1-yl | 380.5 | 381 | 1.733 | Method 4 |
| 4-12 | 2-Methyl-piperidin-1-yl | 394.5 | 395 | 3.338 | Method 2 |
| 4-13 | 3-Methyl-piperidin-1-yl | 394.5 | 395 | 1.671 | Method 4 |
| 4-14 | 3,3-Dimethyl-piperidin-1-yl | 408.5 | 409 | 1.655 | Method 4 |
| 4-15 | 3,5-Dimethyl-piperidin-1-yl | 408.5 | 409.1 | 1.63 | Method 4 |
| 4-16 | 4-Methyl-piperidin-1-yl | 394.5 | 395.1 | 1.675 | Method 4 |
| 4-17 | 4-Benzyl-piperidin-1-yl | 470.6 | 471 | 1.719 | Method 4 |
| 4-18 | [1,4']Bipiperidinyl-1'-yl | 463.6 | 464.1 | 1.928 | Method 4 |
| 4-19 | (S)-2-Methoxymethyl-pyrrolidin-1-yl | 410.5 | 411 | 1.67 | Method 4 |
| 4-20 | Octahydro-isoquinolin-2-yl | 434.6 | 435 | 1.671 | Method 4 |
| 4-21 | (S)2-pyrrolidin-1 -ylmethyl-pyrrolidin-1-yl | 449.6 | 450.1 | 1.886 | Method 4 |
| 4-22 | 4-(3,4-Dimethyl-phenyl)-piperazin-1-yl | 485.6 | 486 | 1.749 | Method 4 |
| 4-23 | 4-pyrrolidin-1-yl-piperidin-1-yl | 449.6 | 450.1 | 1.968 | Method 4 |
| 4-24 | 4-(3-Chloro-phenyl)-piperazin-1-yl | 492.0 | 492 | 1.803 | Method 4 |
| 4-25 | 4-(3-Methoxy-phenyl)-piperazin-1-yl | 487.6 | 488 | 1.793 | Method 4 |
| 4-26 | 4-(4-Methoxy-phenyl)-piperazin-1-yl | 487.6 | 488.1 | 1.784 | Method 4 |
| 4-27 | 4-Acetyl-piperazin-1-yl | 423.5 | 424 | 1.847 | Method 4 |
| 4-28 | 4-Methyl-[1,4]diazepan-1-yl | 409.5 | 410.1 | 1.972 | Method 4 |
| 4-29 | 2-((S)-1-Methyl-pyrrolidin-2-ylmethyl)-piperidin-1-yl | 477.6 | 478.1 | 1.917 | Method 4 |
| 4-30 | 2-(2-Piperidin-1-yl-ethyl)-piperidin-1-yl | 491.7 | 492.1 | 1.852 | Method 4 |
| 4-31 | (R)-2-Methyl-piperazin-1-yl | 395.5 | 396.1 | 1.866 | Method 4 |
| 4-32 | 2,5-Dihydro-pyrrol-1-yl | 364.4 | 365.0 | 1.766 | Method 4 |
| 4-33 | 4-Acetyl-[1,4]diazepan-1-yl | 437.5 | 438 | 1.851 | Method 4 |
| 4-34 | 3-Dimethylamino-pyrrolidin-1-yl | 409.5 | 410.1 | 1.948 | Method 4 |
| 4-35 | 3-trifluoromethyl-piperidin-1-yl | 448.5 | 449 | 1.705 | Method 4 |
| 4-36 | 3-Diethylamino-pyrrolidin-1-yl | 437.6 | 438.1 | 1.897 | Method 4 |
| 4-37 | (R)-2-Methoxymethyl-pyrrolidin-1-yl | 410.5 | 411 | 1.684 | Method 4 |
| 4-38 | 2,5-Dimethyl-2,5-dihydro-pyrrol-1-yl | 392.5 | 393.0 | 1.659 | Method 4 |
| 4-39 | pyrrolidin-1-yl | 366.4 | 367.0 | 1.776 | Method 4 |
| 4-40 | 2,5-Dimethyl-pyrrolidin-1-yl | 394.5 | 395.1 | 1.631 | Method 4 |
| 4-41 | 3-Phenyl-piperidin-1-yl | 456.6 | 457.0 | 1.688 | Method 4 |
| 4-42 | 2-Phenyl-piperidin-1-yl | 456.6 | 457.0 | 1.602 | Method 4 |
| 4-43 | [1,4]Oxazepan-4-yl | 396.5 | 397.0 | 1.725 | Method 4 |
| 4-44 | 4,4-Difluoro-piperidin-1-yl | 416.5 | 417.0 | 1.769 | Method 4 |
| 4-45 | 4-Phenyl-piperidin-1-yl | 456.6 | 457.1 | 4.437 | Method 2 |
| 4-46 | Piperazin-1-yl | 381.5 | 382 | 2.96 | Method 2 |
| 4-47 | 4-(Isopropyl-carbamoyl-methyl)-piperazin-1-yl | 480.6 | 481 | 3.49 | Method 2 |
| 4-48 | 3-carboxamido-piperidin-1yl | 423.5 | 424 | 2.98 | Method 2 |
| 4-49 | [1,4]Diazepan-1-yl | 395.5 | 396 | 2.77 | Method 2 |
| 4-50 | Methyl-(2-pyrrolidin-1-yl-ethyl)-amino | 423.5 | 424 | 2.54 | Method 2 |
| 4-51 | 3,5-Dimethyl-piperazin-1-yl | 409.5 | 410.0 | 3.129 | Method 2 |

### Intermediate 14B. N-(2-Pyridin-2 yl-6-thiazol-2-yl-pyrimidin-4-yl)-acrylamide

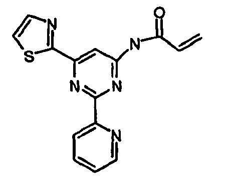

Intermediate 14B was prepared according to the procedures decribed in Intermediate 12B, except that 2-Pyridin-2-yl-6-thiazol-2-yl-pyrimidin-4-ylamine was used instead of Intermediate 11.

The compounds of Table 4B were prepared by reacting Intermediate 14B with the appropriate amine.

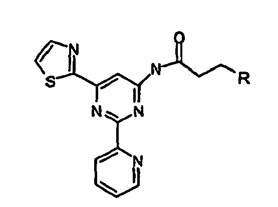

**Table 4B**

| **No.** | **R** | **MW** | **MS ION** | **Reten time (min)** | **HPLC GRADIENT** |
|---|---|---|---|---|---|
| 4-52 | 4-Acetyl-piperazin-1-yl | 437.5 | 438 | 3.08 | Method 2 |
| 4-53 | 4-(3-Chloro-phenyl)-piperazin-1-yl | 506.0 | 506 | 4.84 | Method 2 |
| 4-54 | Morpholin-4-yl | 396.5 | 397 | 3.15 | Method 2 |
| 4-55 | Piperidin-1-yl | 394.5 | 395.0 | 3.386 | Method 2 |

### Intermediate 15. 2-Chloro-N-(6-pyridin-2-yl-2-thiophen-2-yl-pyrimidin-4-yl)-acetamide

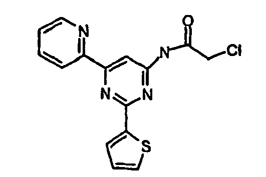

Intermediate 15 was prepared according to the procedures described in Intermediate 13, except that Thiophene-2-carboxamidine was used instead of Intermediate 8. The compounds of Table 5 were prepared by reacting Intermediate 15 with the appropriate amine.

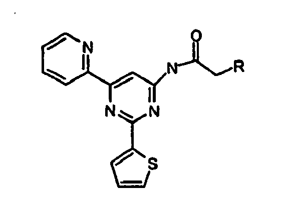

**Table 5**

| **No.** | **R** | **MW** | **MS ION** | **Reten time (min)** | **HPLC GRADIENT** |
|---|---|---|---|---|---|
| 5-1 | 4-Methyl-piperazin-1-yl | 394.5 | 395.0 | 4.301 | Method 2 |
| 5-2 | Morpholin-4-yl | 381.5 | 382.0 | 4.459 | Method 2 |
| 5-3 | pyrrolidin-1-yl | 365.5 | 366.0 | 4.393 | Method 2 |
| 5-4 | Methyl-(1-methyl-piperidin-4-yl) -amino | 422.6 | 423.0 | 3.843 | Method 2 |
| 5-5 | [1,4]Diazepan-1-yl | 394.5 | 395.0 | 4.036 | Method 2 |
| 5-6 | 4-Methyl-[1,4]diazepan-1-yl | 408.5 | 409.0 | 4.181 | Method 2 |
| 5-7 | 4-Ethyl-[1,4]diazepan-1-yl | 422.6 | 423.0 | 4.259 | Method 2 |
| 5-8 | 4-Propyl-[1,4]diazepan-1-yl | 436.6 | 437.0 | 4.49 | Method 2 |
| 5-9 | 4-Amino-piperidin-1-yl | 394.5 | 395.0 | 3.832 | Method 2 |
| 5-10 | 4-Dimethylamino-piperidin-1-yl | 422.6 | 423.0 | 3.976 | Method 2 |
| 5-11 | 4-Diethylamino-piperidin-1-yl | 450.6 | 451.1 | 4.041 | Method 2 |
| 5-12 | 4-Dipropylamino-piperidin-1-yl | 478.7 | 479.1 | 4.347 | Method 2 |
| 5-13 | 4-Acetylamino-piperidin-1-yl | 436.5 | 437.0 | 4.437 | Method 2 |
| 5-14 | 4-propionamido-piperidin-1-yl | 450.6 | 451.0 | 4.601 | Method 2 |
| 5-15 | 4-pyrrolidin-1-yl -piperidin-1-yl | 448.6 | 449.0 | 3.97 | Method 2 |
| 5-16 | 4-Morpholin-4-yl-piperidin-1-yl | 464.6 | 465.0 | 4.039 | Method 2 |
| 5-17 | [1,4']Bipiperidinyl-1'-yl | 462.6 | 463.1 | 3.998 | Method 2 |
| 5-18 | 4-(2-Oxo-imidazolidin-1-yl)-piperidin-1-yl | 463.6 | 464.0 | 4.559 | Method 2 |
| 5-19 | 4-Acetimidoylamino-piperidin-1-yl | 435.6 | 436.0 | 3.966 | Method 2 |
| 5-20 | 4-Azetidin-1-yl-piperidin-1-yl | 434.6 | 435.0 | 3.905 | Method 2 |

### Intermediate 16. 2-Chloro-N-(2-furan-2-yl-6-thiazol-2-yl-pyrimidin-4-yl)-acetamide

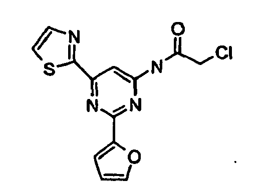

Intermediate 16 was prepared according to the procedures described in Intermediate 12A, except that Furan-2-carboxamidine was used instead of Intermediate 8.

The compound of Table 6 was prepared by reacting Intermediate 16 with the appropriate amine.

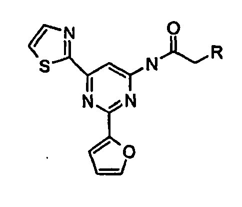

**Table 6**

| **No.** | **R** | **MW** | **MS ION** | **Reten time (min)** | **HPLC GRADIENT** |
|---|---|---|---|---|---|
| 6-1 | 4-methyl-piperazin-1-yl | 384.5 | 385 | 14.2 | Method 3 |

### Intermediate 17. 2-Chloro-N-(6-thiazol-2-yl-2-thiophen-2-yl-pyrimidin-4-yl)-acetamide

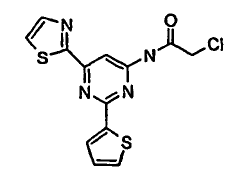

Intermediate 17 was prepared according to the procedures described in Intermediate 12A, except that Thiophene-2-carboxamidine was used instead of Intermediate 8. The compounds of Table 7 were prepared by reacting Intermediate 17 with the appropriate amine.

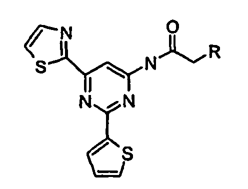

**Table 7**

| **No.** | **R** | **MW** | **MS ION** | **Reten time (min)** | **HPLC GRADIENT** |
|---|---|---|---|---|---|
| 7-1 | Piperidin-1-yl | 385.5 | 385.9 | 5.124 | Method 2 |
| 7-2 | 2-Pyrrolidin-1-yl-methyl-piperidin-1-yl | 468.6 | 469.0 | 5.242 | Method 2 |
| 7-3 | 2-(2-Piperidin-1-yl-ethyl)-piperidin-1-yl | 496.7 | 497.1 | 4.519 | Method 2 |
| 7-4 | 3-Methyl-piperidin-1-yl | 399.5 | 400.0 | 5.435 | Method 2 |
| 7-5 | 3-trifluoromethyl -piperidin-1-yl | 453.5 | 453.9 | 6.336 | Method 2 |
| 7-6 | 3-carboxamido-piperidine-1-ylpiperidine-1-yl | 428.5 | 429.0 | 4.928 | Method 2 |
| 7-7 | [1,4']Bipiperidinyl-1'-yl | 468.6 | 469.0 | 4.336 | Method 2 |
| 7-8 | [1,4]Diazepan-1-yl | 400.5 | 401.0 | 4.333 | Method 2 |
| 7-9 | 4-Acetyl-[1,4]diazepan-1-yl | 442.6 | 443.0 | 4.72 | Method 2 |
| 7-10 | 4-Pyrrolidin-1-yl-piperidin-1-yl | 454.6 | 455.0 | 4.242 | Method 2 |
| 7-11 | Piperazin-1-yl | 386.5 | 386.9 | 4.842 | Method 2 |
| 7-12 | 2,5-Dimethyl-piperazin-1-yl | 414.6 | 415.0 | 5.256 | Method 2 |
| 7-13 | 3,5-Dimethyl-piperazin-1-yl | 414.6 | 415.0 | 5.137 | Method 2 |
| 7-14 | 4-Acetyl-piperazin-1-yl | 428.5 | 429.0 | 4.771 | Method 2 |
| 7-15 | 4-Phenyl-piperazin-1-yl | 462.6 | 463.0 | 6.157 | Method 2 |
| 7-16 | 4-(N-isopropyl-acetamido)-piperazin-1-yl | 485.6 | 486.1 | 5.225 | Method 2 |
| 7-17 | Morpholin-4-yl | 387.5 | 387.9 | 4.84 | Method 2 |
| 7-18 | 2,6-Dimethyl-morpholin-4-yl | 415.5 | 416.0 | 5.505 | Method 2 |
| 7-19 | thiomorpholin-4-yl | 403.6 | 404.0 | 5.2 | Method 2 |
| 7-20 | Pyrrolidin-1-yl | 371.5 | 371.9 | 5.035 | Method 2 |
| 7-21 | 3-Dimethylamino-pyrrolidin-1-yl | 414.6 | 415.0 | 4.43 | Method 2 |
| 7-22 | 3-Diethylamino-pyrrolidin-1-yl | 442.6 | 443.0 | 4.573 | Method 2 |
| 7-23 | 2-(1-Methyl-pyrrolidin-2-yl)-ethylamino | 428.6 | 429.0 | 4.291 | Method 2 |
| 7-24 | 3,5-Dimethyl-piperidin-1-yl | 413.6 | 414.1 | 5.837 | Method 2 |
| 7-25 | 4,4-Difluoro-piperidin-1-yl | 421.5 | 422.0 | 5.804 | Method 2 |
| 7-26 | 4-Phenyl-piperidin-1-yl | 461.6 | 462.1 | 6.394 | Method 2 |
| 7-27 | Azepan-1-yl | 399.5 | 400.1 | 5.512 | Method 2 |
| 7-28 | 4-carboxamido-piperidine-1-yl | 428.5 | 429.1 | 4.864 | Method 2 |
| 7-29 | 4-(3-Methoxy-phenyly piperazin-1-yl | 492.6 | 493.0 | 6.36 | Method 2 |
| 7-30 | (R)-3-Dimethylamino-pyrrolidin-1-yl | 414.6 | 415.0 | 4.486 | Method 2 |
| 7-31 | (S)-3-Dimethylamino-pyrrolidin-1-yl | 414.6 | 415.0 | 4.493 | Method 2 |
| 7-32 | (1-Ethyl-pyrrolidin-2-ylmethyl)-amino | 428.6 | 429.0 | 4.592 | Method 2 |
| 7-33 | (R)-3-Ethylamino-pyrrolidin-1-yl | 414.6 | 415.0 | 4.438 | Method 2 |
| 7-34 | (S)-3-Ethylamino-pyrrolidin-1-yl | 414.6 | 415.0 | 4.443 | Method 2 |
| 7-35 | 4-Methyl-piperazin-1-yl | 400.5 | 401.0 | 5.025 | Method 2 |
| 7-36 | 3-carboxamido-piperidine-1-yl | 428.5 | 429.0 | 4.901 | Method 2 |
| 7-37 | 4-Pyrrolidin-1-yl)-piperidin-1-yl | 454.6 | 455.0 | 4.398 | Method 2 |
| 7-38 | 3,5-Dimethyl-piperazin-1-yl | 414.6 | 415.0 | 5.19 | Method 2 |
| 7-39 | 2-(1-Methyl-pyrrolidin-2-yl)-ethylamino | 428.6 | 429.0 | 4.369 | Method 2 |

### Intermediate 18. 2-Chloro-N-[2-(5-methyl-furan-2-yl)-6-pyrazol-1-yl-pyrimidin-4-yl]-acetamide

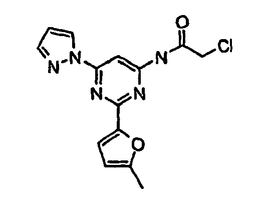

Intermediate 18 was prepared according to the procedures described in Intermediate 6, except that 5-Methyl-furan-2-carboxamidine was used instead of Intermediate 1.

The compounds of Table 8 were prepared by reacting Intermediate 18 with the appropriate amine.

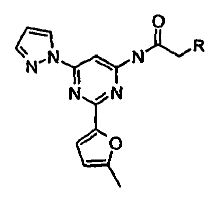

**Table 8**

| **No.** | **R** | **MW** | **MS ION** | **Reten time (min)** | **HPLC GRADIENT** |
|---|---|---|---|---|---|
| 8-1 | 4-pyrrolidin-1-yl-piperidin-1-yl | 435.5 | 436 | 3.84 | Method 2 |
| 8-2 | 4-methyl-piperazin-1-yl | 381.4 | 382 | 4.56 | Method 2 |
| 8-3 | 4-Acetyl-piperazin-1-yl | 409.4 | 410 | 4.43 | Method 2 |
| 8-4 | (S)-3-Ethylamino-pyrrolidin-1-yl) | 395.5 | 396.1 | 3.973 | Method 2 |

### Intermediate 19A. 2-Chloro-N-[6-(3,5-dimethyl-pyrazol-1-yl)-2-(5-methyl-furan-2-yl)-pyrimidin-4-yl]-acetamide

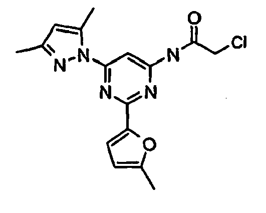

Intermediate 19A was prepared according to the procedures described in Intermediate 6, except that (i) 5-Methyl-furan-2-carboxamidine was used instead of Intermediate 1 and (ii) 3,5-dimethylpyrazole was used instead of pyrazole.

The compounds of Table 9A were prepared by reacting Intermediate 19A with the appropriate amine.

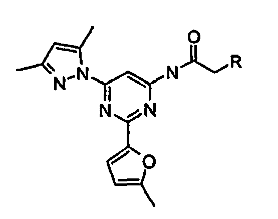

**Table 9A**

| **No.** | **R** | **MW** | **MS ION** | **Reten time (min)** | **HPLC GRADIENT** |
|---|---|---|---|---|---|
| 9-1 | 3,5-Dimethyl-piperazin-1-yl | 423.5 | 424.1 | 5.506 | Method 2 |
| 9-2 | (R)-3-ethylamino-pyrrolidin-1-yl | 423.5 | 424.1 | 2.334 | Method 4 |
| 9-3 | (S)-3-ethylamino-pyrrolidin-1-yl | 423.5 | 424.1 | 2.185 | Method 4 |
| 9-4 | 3-Diethylamino-pyrrolidin-1-yl | 451.6 | 452.2 | 2.133 | Method 4 |
| 9-5 | pyrrolidin-3-ylamino | 395.5 | 396.1 | 2.351 | Method 4 |
| 9-6 | 2-pyrrolidin-1-yl-ethylamino | 423.5 | 424.1 | 2.259 | Method 4 |
| 9-7 | 3-pyrrolidin-1-yl-propylamino | 437.5 | 438.1 | 2.269 | Method 4 |
| 9-8 | 2-(1-methyl-pyrrolidin-2-yl)-ethylamino | 437.5 | 438.1 | 4.606 | Method 2 |
| 9-9 | 2-piperidin-1-yl-ethylamino | 437.5 | 438.2 | 2.201 | Method 4 |
| 9-10 | 3-piperidin-1-yl-propylamino | 451.6 | 452.2 | 2.185 | Method 4 |
| 9-11 | 3-morpholin-4-yl-propylamino | 453.5 | 454.1 | 2.22 | Method 2 |
| 9-12 | 4-methyl-piperazin-1-yl | 409.5 | 410.1 | 5.286 | Method 2 |
| 9-13 | morpholin-4-yl | 396.4 | 397.1 | 5.235 | Method 2 |
| 9-14 | [1,4]Diazepan-1-yl | 409.5 | 410.1 | 4.901 | Method 2 |
| 9-15 | 4-pyrrolidin-1-yl-piperidin-1 -yl | 463.6 | 464.1 | 4.702 | Method 2 |
| 9-16 | 4-methyl-[1,4]diazepan-1-yl | 423.5 | 424.1 | 2.361 | Method 4 |
| 9-17 | [1,4]oxazepan-4-yl | 410.5 | 411.1 | 2.051 | Method 4 |
| 9-18 | (R)-3-Dimethylamino-pyrrolidin-1-yl | 423.5 | 424.1 | 2.268 | Method 4 |
| 9-19 | (S)-3-Dimethylamino-pyrrolidin-1-yl | 423.5 | 424.1 | 2.279 | Method 4 |
| 9-20 | pyrrolidin-1-yl | 380.5 | 381.0 | 5.465 | Method 2 |
| 9-21 | 4,4-Difluoro-piperidin-1-yl | 430.5 | 431.0 | 6.191 | Method 2 |
| 9-22 | 4-thiazol-2-yl-piperazin-1-yl | 478.6 | 479.0 | 5.933 | Method 2 |
| 9-23 | 4-ethyl-piperazin-1-yl | 423.5 | 424.0 | 5.56 | Method 2 |
| 9-24 | (R)-3-Acetylamino-pyrrolidin-1-yl | 437.5 | 438.0 | 5.297 | Method 2 |
| 9-25 | (S)-3-Acetylamino-pyrrolidin-1-yl | 437.5 | 438.0 | 5.205 | Method 2 |
| 9-26 | piperazin-1-yl | 395.5 | 396.0 | 5.245 | Method 2 |
| 9-27 | 2,6-Dimethyl-morpholin-4-yl | 424.5 | 425.0 | 5.8 | Method 2 |
| 9-28 | 4-hydroxy-piperidin-1-yl | 410.5 | 411.0 | 5.19 | Method 2 |
| 9-29 | [1,4']Bipiperidinyl-1'-yl | 477.6 | 478.1 | 4.702 | Method 2 |
| 9-30 | 4-methoxy-piperidin-1-yl | 424.5 | 425.0 | 5.57 | Method 2 |
| 9-31 | 4-Acetyl-piperazin-1-yl | 437.5 | 438.0 | 5.297 | Method 2 |
| 9-32 | (R)-3-Diethylamino-pyrrolidin-1-yl | 451.6 | 452.1 | 5.047 | Method 2 |
| 9-33 | (S)-3-Diethylamino-pyrrolidin-1-yl | 451.6 | 452.1 | 5.036 | Method 2 |
| 9-34 | (S)-3-(ethyl-methyl-amino)-pyrrolidin-1-yl | 437.5 | 438.1 | 4.881 | Method 2 |
| 9-35 | (R)-3-(ethyl-methyl-amino)-pyrrolidin-1-yl | 437.5 | 438.2 | 4.752 | Method 2 |
| 9-36 | (S)-3-Amino-pyrrolidin-1-yl | 395.5 | 396.1 | 4.411 | Method 2 |
| 9-37 | (R)-3-Amino-pyrrolidin-1-yl | 395.5 | 396.1 | 4.358 | Method 2 |
| 9-38 | (R)-3-morpholin-4-yl-pyrrolidin-1-yl | 465.5 | 466.2 | 4.89 | Method 2 |
| 9-39 | (R)-3-piperidin-1-yl-pyrrolidin-1-yl | 463.6 | 464.2 | 4.887 | Method 2 |
| 9-40 | (R)-[1,3']Bipyrrolidinyl-1'-yl | 449.5 | 450.1 | 4.887 | Method 2 |
| 9-41 | (S)-3-morpholin-4-yl- pyrrolidin-1-yl | 465.5 | 466.2 | 4.91 | Method 2 |
| 9-42 | (S)-[1,3']Bipyrrolidinyl-1'-yl | 449.5 | 450.2 | 4.91 | Method 2 |
| 9-43 | (S)-3-piperidin-1-yl-pyrrolidin-1-yl | 463.6 | 464.2 | 4.96 | Method 2 |
| 9-44 | (R)-3-(2,2,2-trifluoro-acetamidyl)-pyrrolidin-1-yl | 491.5 | 492.2 | 5.947 | Method 2 |
| 9-45 | (R)-3-hydroxy-pyrrolidin-1-yl | 396.4 | 397.1 | 5.083 | Method 2 |
| 9-46 | (S)-3-hydroxy-pyrrolidin-1-yl | 396.4 | 397.1 | 5.125 | Method 2 |
| 9-47 | (3R,3'R)-3-fluoro-[1,3']bipyrrolidinyl-1'-yl | 467.5 | 468.2 | 5.017 | Method 2 |
| 9-48 | (R)-3-[(2-methoxy-ethyl)-methyl-amino]-pyrrolidin-1-yl | 467.7 | 468.1 | 4.925 | Method 2 |
| 9-49 | (3S,3'S)-3-fluoro-[1,3']bipyrrolidinyl-1'-yl | 467.5 | 468.1 | 4.972 | Method 2 |
| 9-50 | (3R,3'S)-3-fluoro-[1,3']bipyrrolidinyl-1'-yl | 467.5 | 468.1 | 4.992 | Method 2 |
| 9-51 | (S)-2,5,2',3',4',5'-hexahydro-[1,3']bipyrrolyl-1'-yl | 447.5 | 448.1 | 4.89 | Method 2 |
| 9-52 | (S)-3-[(2-methoxy-ethyl)-methyl-amino]-pyrrolidin-1-yl | 467.6 | 468.3 | 9.39 | Method 2 |
| 9-53 | (S)-3-fluoro-pyrrolidin-1-yl | 398.4 | 399.0 | 5.296 | Method 2 |
| 9-54 | (R)-3-fluoro-pyrrolidin-1-yl | 398.4 | 399.0 | 5.334 | Method 2 |
| 9-55 | piperidin-1-yl | 394.5 | 395.1 | 5.383 | Method 2 |
| 9-56 | (R)-3-(2-methoxy-ethylamino)-pyrrolidin-1-yl | 453.5 | 454.2 | 4.852 | Method 2 |
| 9-57 | 3-trifluoromethyl-piperidin-1-yl | 462.5 | 463.1 | 6.52 | Method 2 |
| 9-58 | 3-trifluoromethyl-5,6-dihydro-8H-[1,2,4]triazolo[4,3-a]pyrazin-7-yl | 501.5 | 502.1 | 8.84 | Method 5 |
| 9-59 | 4-(2-oxo-pyrrolidin-1-yl)-piperidin-1-yi | 477.6 | 478.1 | 7.76 | Method 5 |
| 9-60 | 4-Acetylamino-piperidin-1-yl | 451.5 | 452.1 | 7.26 | Method 5 |
| 9-61 | 4-carboxylic acid phenyl ester-piperazin-1-yl | 515.6 | 516.2 | 9.25 | Method 5 |
| 9-62 | 4-carboxylic acid benzylamide-piperazin-1-yl | 528.6 | 529.2 | 8.29 | Method 5 |
| 9-63 | 4-(3-methyl-benzoyl)-piperazin-1-yl | 513.6 | 514.3 | 8.81 | Method 5 |
| 9-64 | 4-carboxylic acid dimethylamide-piperazin-1-yl | 466.5 | 467.2 | 7.65 | Method 5 |
| 9-65 | (S)-3-(ethyl-(2-methoxy-ethyl)-amino]-pyrrolidin-1-yl | 481.6 | 482.1 | 4.956 | Method 2 |
| 9-66 | (S)-3-isopropoxy-pyrrolidin-1-yl | 438.5 | 439.1 | 5.903 | Method 2 |
| 9-67 | 3,9-Diaza-bicyclo[4.2.1]non-3-yl | 435.5 | 436.1 | 5.497 | Method 2 |
| 9-68 | 9-carboxylic acid tert-butyl ester 3,9-diaza-bicyclo[4.2.1]nonan-3-yl | 535.6 | 536.2 | 6.46 | Method 2 |
| 9-69 | 9-methyl-3,9-diaza-bicyclo[4.2.1]nonan-3-yl | 449.5 | 450.1 | 5.613 | Method 2 |
| 9-70 | 4-{[(2-methoxy-ethyl)-methyl-amino]-methyl}-piperidin-1-yl | 495.6 | 496.3 | 4.567 | Method 2 |
| 9-71 | 9-(2-methoxy-ethyl)-3,9-diaza-bicyclo[4.2.1]nonan-3-yl | 493.6 | 494.4 | 6.067 | Method 2 |
| 9-72 | 4-(methyl-carbamic acid tert-butyl ester)-piperidin-1-yl | 523.6 | 524.3 | 6.437 | Method 2 |
| 9-73 | 4-[(2-methoxy-ethyl)-methyl-amino]-piperidin-1-yl | 481.6 | 482.2, | 4.397 | Method 2 |
| 9-74 | 2-dimethylaminomethyl-morpholin-4-yl | 453.5 | 454.2 | 4.63 | Method 2 |
| 9-75 | (S)-3-Dimethylaminomethyl-piperidin-1-yl | 451.6 | 452.0 | 15.59 | Method 6 |
| 9-76 | (R)-3-Dimethylaminomethyl-piperidin-1-yl | 451.6 | 452.0 | 15.76 | Method 6 |
| 9-77 | (R)-3-Dimethylaminomethyl-pyrrolidin-1-yl | 437.5 | 438.0 | 15.1 | Method 6 |
| 9-78 | (S)-3-Dimethylaminomethyl-pyrrolidin-1-yl | 437.5 | 438.0 | 15.1 | Method 6 |
| 9-79 | 4-Dimethylaminomethyl-piperidin-1-yl | 451.6 | 452.0 | 15.86 | Method 6 |
| 9-80 | 1-methyl-piperidin-4-ylamino | 423.5 | 424.1 | 4.064 | Method 2 |
| 9-81 | (R)-pyrrolidin-3-ylamino | 395.5 | 396.1 | 4.238 | Method 2 |
| 9-82 | (R)-1-(tetrahydro-furan-2-yl)methyl-amino | 410.5 | 411.1 | 5.236 | Method 2 |
| 9-83 | (tetrahydro-pyran-4-ylmethyl)-amino | 424.5 | 425.1 | 5.137 | Method 2 |
| 9-84 | (S)-1-(tetrahydro-furan-2-yl)methyl]-amino | 410.5 | 411.1 | 5.34 | Method 2 |
| 9-85 | piperidin-4-ylamino | 409.5 | 410.1 | 4.13 | Method 2 |
| 9-86 | (S)-piperidin-3-ylamino | 409.5 | 410.1 | 4.16 | Method 2 |
| 9-87 | Azetidin-3-ylamino | 381.4 | 382.1 | 4.65 | Method 2 |
| 9-88 | 2-morpholin-4-yl- ethylamino | 439.5 | 440.1 | 4.38 | Method 2 |
| 9-89 | (R)-piperidin-3-ylamino | 409.5 | 410.1 | 4.08 | Method 2 |
| 9-90 | ((R)-1-pyrrolidin-2-ylmethyl)-amino | 409.5 | 410.2 | 4.38 | Method 2 |
| 9-91 | (S)-pyrrolidin-3-ylamino | 395.5 | 396.1 | 4.381 | Method 2 |
| 9-92 | (R)-pyrrolidin-3-ylamino | 395.5 | 396.1 | 4.238 | Method 2 |
| 9-93 | ((R)-1-pyrrolidin-3-ylmethyl)-amino | 409.5 | 410.4 | 4.343 | Method 2 |

### Intermediate 19B. N-[6-(3,5-Dimethyl-pyrazol-1-yl)-2-(5-methyl-furan-2-yl)-pyrimidin-4-yl]-acrylamide

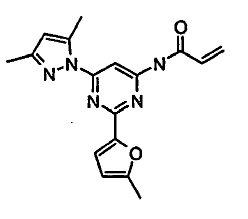

Intermediate 19B was prepared according to the procedures decribed in Intermediate 12B, except that 6-(3,5-Dimethyl-pyrazol-1-yl)-2-(5-methyl-furan-2-yl)-pyrimidin-4-ylamine was used instead of Intermediate 11.

The compounds of Table 9B were prepared by reacting Intermediate 19B with the appropriate amine.

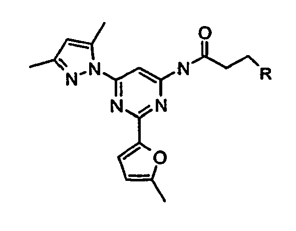

**Table 9B**

| **No.** | **R** | **MW** | **MS ION** | **Reten time (min)** | **HPLC GRADIENT** |
|---|---|---|---|---|---|
| 9-94 | (R)-3-Ethylamino-pyrrolidin-1-yl) | 437.5 | 438.0 | 4.512 | Method 2 |
| 9-95 | (S)-3-Dimethylamino-pyrrolidin-1-yl) | 437.5 | 438.0 | 4.566 | Method 2 |
| 9-96 | 4-methyl-piperazin-1-yl | 423.5 | 424.0 | 4.76 | Method 2 |
| 9-97 | morpholin-4-yl | 410.5 | 411.0 | 5.34 | Method 2 |
| 9-98 | 2,6-Dimethyl-morpholin-4-yl | 438.5 | 439.0 | 5.701 | Method 2 |
| 9-99 | 4-pyrrolidin-1-yl-piperidin-1-yl | 477.6 | 478.1 | 4.695 | Method 2 |
| 9-100 | [1,4]Diazepan-1-yl | 423.5 | 424.0 | 4.504 | Method 2 |
| 9-101 | 4-methyl-[1,4]diazepan-1-yl | 437.5 | 438.0 | 4.499 | Method 2 |
| 9-102 | 4-Acetyl-[1.4]diazepan-1-yl | 465.5 | 466.0 | 5.311 | Method 2 |
| 9-103 | (R)-3-Dimethylamino-pyrrolidin-1-yl | 437.5 | 438.1 | 4.516 | Method 2 |
| 9-104 | (S)-3-Dimethylamino-pyrrolidin-1-yl | 437.5 | 438.0 | 4.566 | Method 2 |
| 9-105 | [1,4]oxazepan-4-yl | 424.5 | 425.0 | 5.322 | Method 2 |
| 9-106 | [1,4']Bipiperidinyl-1'-yl | 491.6 | 492.1 | 4.641 | Method 2 |
| 9-107 | 4-methoxy-piperidin-1-yl | 438.5 | 439.0 | 5.499 | Method 2 |
| 9-108 | 4-thiazol-2-yl-piperazin-1-yl | 492.6 | 493.0 | 5.677 | Method 2 |
| 9-109 | 4-ethyl-piperazin-1-yl | 437.5 | 438.1 | 4.958 | Method 2 |
| 9-110 | 3-Diethylamino-pyrrolidin-1-yl | 465.6 | 466.1 | 4.833 | Method 2 |
| 9-111 | (3R, 5S)-3,5-dimethyl-piperazin-1-yl | 437.6 | 438.0 | 4.76 | Method 2 |
| 9-112 | Piperazin-1-yl | 409.5 | 410.0 | 4.68 | Method 2 |

### Intermediate 19C. 6-(3,5-Dimethyl-pyrazol-1-yl)-2-(5-methyl-furan-2-yl)-pyrimidin-4-ylamine

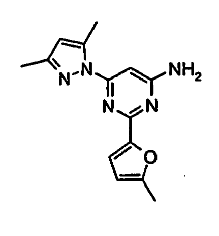

Intermediate 19C was prepared according to the procedures described in Intermediate 5, except that (i) 5-Methyl-furan-2-carboxamidine was used instead of Intermediate 1 and (ii) 3,5-dimethylpyrazol was used instead of pyrazol.

### Intermediate 19D. 4-Bromo-N-[6-(3,5-dimethyl-pyrazol-1-yl)-2-(5-methyl-furan-2-yl)-pyrimidin-4-yl]-butyramide

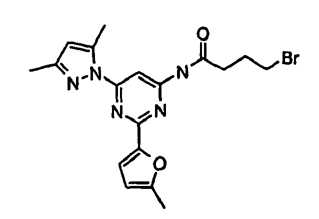

To 10 mL THF were added 3.0g (18mmol, 2eq) 4-bromobutyric acid, 2.5g (2.2eq) oxalyl chloride, followed by a few drops of DMF. The reaction mixture was stirred at r.t. for 1 hour. Solvent was removed by nitrogen flow and the residue was resuspended in 10 mL dichloromethane followed by of Intermediate 19C and 0.9 mL pyridine. The reaction mixture was stirred at r.t. for 2 hours. The reaction was quenched with 50mL saturated sodium bicarbonate, extracted twice with 50mL dichloromethane, the organic layers were combined, dried under sodium sulfate and concentrated to give Intermediate 19D. Intermediate 19D was used in the next step without further purification.

The compounds of Table 9C were prepared by reacting Intermediate 19D with the appropriate amine as follows: Intermediate 19D was dissolved in 1.5mL) DMF followed by 2eq of the amine of choice, heated at 80°C for two hours. The products were purified by MSQ5 LC-MS system, using 5-65% acetonitrile in water.

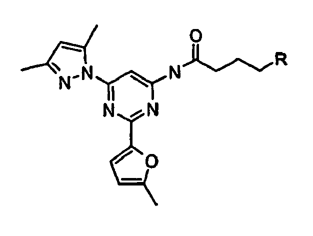

**Table 9C**

| **No.** | **R** | **MW** | **MS ION** | **Reten time (min)** | **HPLC GRADIENT** |
|---|---|---|---|---|---|
| 9-113 | Morpholin-4-yl | 424.5 | 425.0 | 5.22 | Method 2 |
| 9-114 | Pyrrolidin-1-yl | 408.5 | 409.0 | 5.43 | Method 2 |
| 9-115 | (S)-3-fluoro-pyrrolidin-1-yl | 426.5 | 427.0 | 5.44 | Method 2 |
| 9-116 | (R)-3-fluoro-pyrrolidin-1-yl | 426.5 | 427.0 | 5.44 | Method 2 |

### Intermediate 19E. 4-Chlorocarbonylmethyl-piperidine-1-carboxylic acid tert-butyl ester

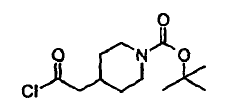

To 10 mL THF were added 540mg (2.2 mmol, 1 eq) 1-Boc-piperidine-4-ylacetic acid, 300mg (0.24mmol, 1.1 eq) oxalyl chloride and a few drops of DMF. The reaction mixture was stirred under nitrogen at r.t. for 1 hour and concentrated under vacuum to dryness.

### Compound 9-117. 4-{[6-(3,5-Dimethyl-pyrazol-1-yl)-2-(5-methyl-furan-2-yl)-pyrimidin-4-ylcarbamoyl]-methyl}-piperidine-1-carboxylic acid tert-butyl ester

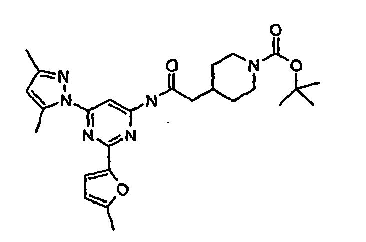

Intermediate 19E was dissolved in 10 mL dichloromethane, Intermediate 19C (300mg, 0.5eq) was added, followed by 150mg pyridine. The reaction was stirred at room temperature for four hours. The reaction was quenched by 50 mL saturated sodium bicarbonate, extracted twice with 50 mL dichloromethane, the organic layers were combined, dried under sodium sulfate, concentrated and purified by silica gel column using 40% ethyl acetate in hexane. Obtained 540 mg compound 9-117 as clear thick oil. Yield 98.0%.

### Compound 9-118. N-[6-(3,5-Dimethyl-pyrazol-1-yl)-2-(5-methyl-furan-2-yl)-pyrimidin-4-yl]-2-piperidin-4-yl-acetamide

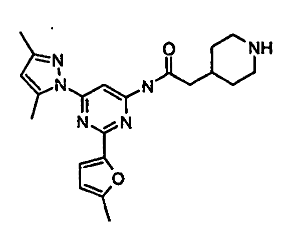

Compound 9-117 was dissolved in 10mL dichloromethane followed by addition of 10 mL TFA. The reaction was stirred at room temperature for 1 hour, solvent was removed by nitrogen flow and compound 9-118 was obtained as clear oil, used without further purification.

### Compound 9-119. N-[6-(3,5-Dimethyl-pyrazol-1-yl)-2-(5-methyl-furan-2-yl)-pyrimidin-4-yl]-2-(1-methyl-piperidin-4-yl)-acetamide

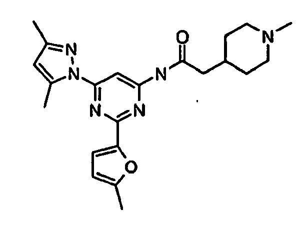

Compound 9-118 was dissolved in 10mL EtOH, followed by 2mL formaldehyde (30% in water), 10 drops of acetic acid, and 0.2mL borane-pyridine. The reaction was stirred at room temperature overnight. The reaction was quenched by 50mL 1 N HCl for 10 minutes, neutralized by saturated sodium bicarbonate, extracted twice with dichloromethane. The organic layers were combined, dried under sodium sulfate, concentrated and purified by silica gel column using 5% methanol in dichloromethane. Obtained 350mg compound 9-119 as clear oil. Yield: 78.5%. LCMS (APCI) *m*/*z* 409.2 (MH⁺). δ (300 MHz, CDCl₃): 1.33-1.38 (m, 4H), 1.77-1.81 (m, 2H), 1.91-1.95 (m, 2H), 2.27 (s, 3H), 2.28 (s, 3H), 2.31 (d, J=6Hz, 1H), 2.45 (s, 3H), 2.76 (s, 3H), 2.83-2.87 (m, 2H), 6.01 (s, 1H), 6.17 (d, J=3Hz, 1H), 7.16 (d, J=3Hz, 1H), 8.03(s, 1H), 8.49 (s, 1H). A portion of the product was converted to HCl salt. C22H28N6O2.2HCl.C2H6O (cal CHN 54.60, 6.45, 15.92; found 54.92, 6.58, 15.93).

### Compound 9-120. N-[6-(3,5-Dimethyl-pyrazol-1-yl)-2-(5-methyl-furan-2-yl)-pyrimidin-4-yl]-2-[1-(2-methoxy-ethyl)-piperidin-4-yl]-acetamide

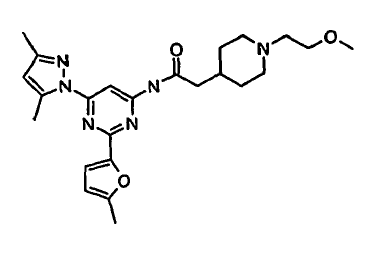

Compound 9-118 (150mg, 0.37mmol) was dissolved in 10 mL DMF followed by 70mg (1.2eq) 2-bromoethyl methyl ether and DIEA (2eq). The reaction mixture was stirred at 45 °C for 2 days. The reaction was quenched by 50 mL saturated sodium bicarbonate, extracted twice with 50 mL dichloromethane, the organic layers were combined, dried under sodium sulfate, concentrated and purified by silica gel column using 0%-10% methanol in dichloromethane. Obtained 62 mg compound 9-120 as clear thick oil. Yield 36.9%. LCMS (APCI) *m*/*z* 453.0 (MH⁺).

### Compound 9-121. N-[6-(3,5-Dimethyl-pyrazol-1-yl)-2-(5-methyl-furan-2-yl)-pyrlmidin-4-yl]-2-(2-pyrrolidin-1-yl-ethoxy)-acetamide

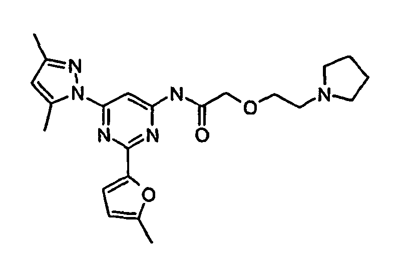

Compound 9-121 was prepared according to the procedures described in Compound 2-126, except that Intermediate 19A was used instead of Intermediate 12A.

### Intermediate 20A. N-[2-chloro-6-(1H-pyrazol-1-yl)pyrimidin-4-yl]acetamide

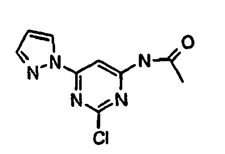

To a solution of *N*-[2,6-dichloropyrimidin-4-yl]acetamide (0.10 g, 0.5 mmol) in anhydrous DMF (1 mL) was added pyrazole (34 mg, 0.5 mmol) and cesium carbonate (0.16 g, 0.5 mmol). The mixture was heated at 80°C for 1 hour. The solution was poured into water (10 mL) and extracted with ethyl acetate (2x5 mL). The organic layer was washed with water (2x5 mL) and brine (5 mL), dried (Na₂SO₄) and the solvent removed under reduced pressure. The residue was purified by prep TLC plate with 3% methanol in methylene chloride, to give *N*-[2-chloro-6-(1*H*-pyrazol-1-yl)pyrimidin-4-yl]acetamide (30 mg, 26%).

### Intermediate 20B. 2-(1,3-Oxazol-2-yl)-6-(1H-pyrazol-1-yl)pyrimidin-4-amine

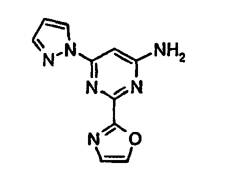

To a solution of oxazole (67 mL, 1.0 mmol) in anhydrous THF (2 mL) at -78°C was added nBuLi (1.6 M in hexane; 1.0 mL, 1.6 mmol) and stirred for 15 min. ZnCl₂ (0.5 M in THF; 6.6 mL, 3.3 mmol) was added and stirred for 1 hour when the temperature gradually rose to -20°C. Intermediate 20A (48 mg, 0.2 mmol) and tetrakis(triphenylphosphine)palladium(0) (46 mg, 0.04 mmol) were added and the mixture was heated at 80°C for 2 hours. The solution was poured into 1N HCl (10 mL) and extracted with ethyl acetate (2x10 mL). The organic layer was washed with water (2x5 mL) and brine (5 mL), dried (Na₂SO₄), and the solvent removed under reduced pressure. The residue was purified by prep TLC plate with 5% methanol in methylene chloride, to give 2-(1,3-oxazol-2-yl)-6-(1*H*-pyrazol-1-yl)pyrimidin-4-amine. LCMS (APCI) *m*/*z* 229.0 (MH⁺).

### Intermediate 20C. 2-Chloro-N-(2-oxazol-2-yl-6-pyrazol-1-yl-pyrimidin-4-yl)-acetamide

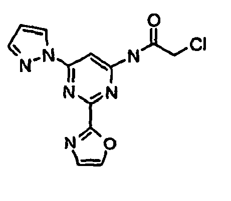

Intermediate 20C was prepared according to the procedures described in Intermediate 6, except that Intermediate 20B was used instead of Intermediate 5.

The compound of Table 10 was prepared by reacting Intermediate 20C with the appropriate amine.

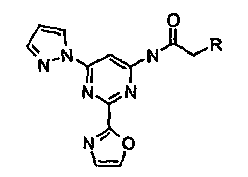

**Table 10**

| **No.** | **R** | **MW** | **MS ION** | **Reten time (min)** | **HPLC GRADIENT** |
|---|---|---|---|---|---|
| 10-1 | 4-Methyl-piperazin-1-yl | 368.4 | 369.0 | 3.075 | Method 2 |

### Intermediate 21. -2-Chloro-N-(6-pyrazol-1-yl-2-pyridin-2-yl-pyrimidin-4-yl) acetamide

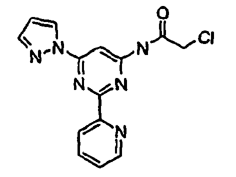

Intermediate 21 was prepared according to the procedures described in Intermediate 6, except that Pyridine-2-carboxamidine HCl was used instead of Intermediate 1.

The compounds of Table 11 were prepared by reacting Intermediate 21 with the appropriate amine.

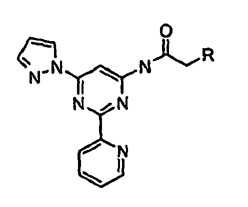

**Table 11**

| **No.** | **R** | **MW** | **MS ION** | **Reten time (min)** | **HPLC GRADIENT** |
|---|---|---|---|---|---|
| 11-1 | (R)-3-Dimethylamino-pyrrolidin-1-yl | 392.5 | 393.1 | 2.541 | Method 2 |
| 11-2 | (S)-3-Dimethylamino-pyrrolidin-1-yl | 392.5 | 393.1 | 2.552 | Method 2 |
| 11-3 | 2,6-Uimethyl-morpholin-4-yl | 393.4 | 394.1 | 3.234 | Method 2 |
| 11-4 | 4-pyrrolidin-1-yl-piperidin-1-yl | 432.5 | 433.1 | 2.494 | Method 2 |
| 11-5 | 4-Acetyl-piperazin-1-yl | 406.4 | 407.1 | 2.771 | Method 2 |
| 11-6 | 3,5-Dimethyl-piperazin-1-yl | 392.5 | 393.1 | 3.1 | Method 2 |
| 11-7 | 4-Methyl-piperazin-1-yl | 378.4 | 379.1 | 2.885 | Method 2 |
| 11-8 | Morpholin-4-yl | 365.4 | 366.0 | 2.817 | Method 2 |
| 11-9 | 4-Dimethylamino-piperidin-1-yl | 406.5 | 407.1 | 2.405 | Method 2 |
| 11-10 | 4-Methyl- [1,4]diazepan-1-yl | 392.5 | 393.1 | 2.831 | Method 2 |

### Intermediate 22. 2-Chloro-N-(6-pyrazol-1-yl-2-pyridin-2-yl-pyrimidin-4-yl)-acetamide

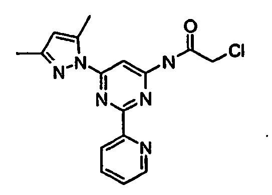

Intermediate 22 was prepared according to the procedures described in Intermediate 6, except that (i) Pyridine-2-carboxamidine was used instead of Intermediate 1 and (ii) 3,5-dimethylpyrazol was used instead of pyrazol.

The compounds of Table 12 were prepared by reacting Intermediate 22 with the appropriate amine.

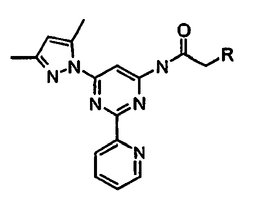

**Table 12**

| **No.** | **R** | **MW** | **MS ION** | **Reten time (min)** | **HPLC GRADIENT** |
|---|---|---|---|---|---|
| 12-1 | 3,5-Dimethyl-piperazin-1-yl | 420.5 | 421.1 | 3.99 | Method 2 |
| 12-2 | 4-pyrrolidin-1-yl-piperidin-1-yl | 460.6 | 461.2 | 3.413 | Method 2 |
| 12-3 | (S)-3-(ethyl-methyl-amino)-pyrrolidin-1-yl | 434.5 | 435.2 | 3.49 | Method 2 |
| 12-4 | (R)-3-amino-pyrrolidin-1-yl | 392.5 | 393.1 | 3.18 | Method 2 |
| 12-5 | (S)-3-amino-pyrrolidin-1-yl | 392.5 | 393.1 | 3.20 | Method 2 |
| 12-6 | 3-(2,2,2-trifluoro-acetamido)-pyrrolidin-1-yl | 488.5 | 489.1 | 9.01 | Method 5 |
| 12-7 | (3-trifluoromethyl-5,6-dihydro-8H-[1,2, 4]triazolo[4,3-a]pyrazin-7-yl) | 498.5 | 499.1 | 5.056 | Method 2 |
| 12-8 | morpholin-4-yl | 393.4 | 394.1 | 3.666 | Method 2 |
| 12-9 | [1,4]oxazepan-4-yl | 407.5 | 408.1 | 6.657 | Method 5 |
| 12-10 | 4-methyl-piperazin-1-yl | 406.5 | 407.1 | 3.746 | Method 2 |
| 12-11 | 4-methyl-[1,4]diazepam-1-yl | 420.5 | 421.3 | 6.725 | Method 5 |
| 12-12 | (R)-3-dimethylamino-pyrrolidin-1-yl | 420.5 | 421.1 | 3.365 | Method 2 |
| 12-13 | (S)-3-dimethylamino-pyrrolidin-1-yl | 420.5 | 421.1 | 3.372 | Method 2 |
| 12-14 | (R)-3-Dimethylaminomethyl-piperidin-1-yl | 448.6 | 449.1 | 9.35 | Method 5 |
| 12-15 | (S)-3-Dimethylaminomethyl-piperidin-1-yl | 448.6 | 449.1 | 3.14 | Method 2 |

### Intermediate 23A. 2,6-Bis(1H-pyrazol-1-yl)pyrimidin-4-amine

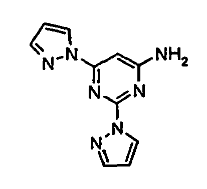

To a solution of *N*-[2,6-dichloropyrimidin-4-yl]acetamide (0.10 g, 0.5 mmol) in anhydrous DMF (1 mL) was added pyrazole (68 mg, 1.0 mmol) and cesium carbonate (0.32 g, 1.0 mmol). The mixture was heated at 120°C for 15 hours. The solution was poured into water (10 mL) and extracted with ethyl acetate (2x5 mL). The organic layer was washed with water (2x5 mL) and brine (5 mL), dried (Na₂SO₄), and the solvent removed under reduced pressure. The residue was purified by prep TLC plate with 3% methanol in methylene chloride, to give 2,6-bis(1*H*-pyrazol-1-yl)pyrimidin-4-amine. LCMS (APCI) *m*/*z* 228.0 (MH⁺).

### Intermediate 23B. 2-Chloro-N-(2,6-di-pyrazol-1-yl-pyrimidin-4-yl)-acetamide

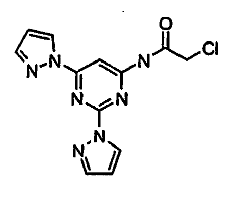

Intermediate 23B was prepared according to the procedures described in Intermediate 6, except that Intermediate 23A was used instead of Intermediate 5.

The compounds of Table 13 were prepared by reacting Intermediate 23B with the appropriate amine.

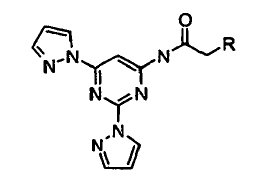

**Table 13**

| **No.** | **R** | **MW** | **MS ION** | **Reten time (min)** | **HPLC GRADIENT** |
|---|---|---|---|---|---|
| 13-1 | 4-methyl-4-methyl-piperazin-1-yl | 367.4 | 368.1 | 3.722 | Method 2 |
| 13-2 | 4-pyrrolidin-1-yl-piperidin-1-yl | 421.5 | 422.1 | 3.211 | Method 2 |
| 13-3 | 3-Dimethylamino-pyrrolldin-1-yl | 381.4 | 382.0 | 3.208 | Method 2 |

### Intermediate 24A. 2,6-Bis(1,3-thiazol-2-yl)-pyrimidin-4-amine

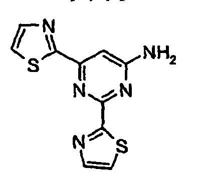

Intermediate 24A was obtained by reacting M-[2,6-dichloropyrimidin-4-yl]acetamide (0.10 g, 0.5 mmol) with thiazole (0.2 g, 2.5 mmol) according to the procedures described in Intermediate 20B. LCMS (APCI) *m*/*z* 262.0 (MH⁺).

### Intermediate 24B. N-(2,6-Bis-thiazol-2-yl-pyrimidin-4-yl)-2-chloro-acetamide

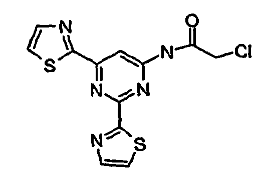

Intermediate 24B was prepared according to the procedures described in Intermediate 6, except that Intermediate 24A was used instead of Intermediate 5.

The compound of Table 14 was prepared by reacting Intermediate 24B with the appropriate amine.

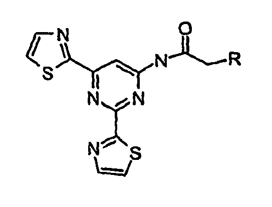

**Table 14**

| **No.** | **R** | **MW** | **MS ION** | **Reten time (min)** | **HPLC GRADIENT** |
|---|---|---|---|---|---|
| 14-1 | 4-methyl-piperazin-1-yl | 401.5 | 401.9 | 4.114 | Method 2 |

### Intermediate 25A. N-[6-(1H-pyrazol-1-yl)-2-({E)-styryl)pyrimidin-4-yl]acetamide

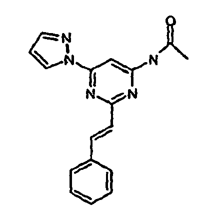

A mixture of Intermediate 20A (0.2 g, 0.84 mmol), phenylvinylboronic acid (0.25 g, 1.68 mmol) and sodium carbonate (0.54 g, 5.05 mmol) in dioxane/water (9/1, 10 mL) was degassed with bubbling N₂ for 15 min. Tetrakis(triphenylphosphine)palladium(0) (0.1 g, 0.08 mmol) was added and the mixture was heated at 90°C for 16 hours. The solution was poured into water (10 mL) and extracted with ethyl acetate (2x10 mL). The organic layer was washed with water (2x5 mL) and brine (5 mL), dried (Na₂SO₄), and the solvent removed under reduced pressure. The residue was purified by prep TLC plate with 3% methanol in methylene chloride, to give *N-*[6-(1*H*-pyrazol-1-yl)-2-((E)-styryl)pyrimidin-4-yl]acetamide.

### Intermediate 25B. N-[2-Formyl-6-(1H-pyrazol-1-yl)pyrimidin-4-yl]acetamide

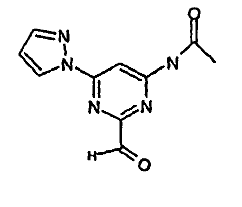

Intermediate 25A (0.2 g, 0.8 mmol) was dissolved in MeOH/DCM (4/1, 10 mL), cooled to -78°C, and O₃ was bubbled in for 5 min. After flushing the solution with N₂ for 10 min, dimethylsulfide (0.2 mL) was added and the reaction was allowed to warm up to RT. The solution was evaporated with N₂ purging to give crude aldehyde.

### Intermediate 25C. 2-(1,3-Oxazol-5-yl)-6-(1H-pyrazol-1-yl)pyrimidin-4-amine

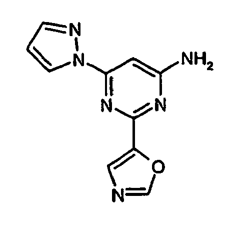

A mixture of Intermediate 25B (46 mg, 0.2 mmol), TOSMIC (80 mg, 0.4 mmol) and potassium carbonate (83 mg, 0.6 mmol) in MeOH (10 mL) was heated at 80°C for 16 hours. MeOH was evaporated and the residue was partitioned between ethyl acetate and water. The organic layer was washed with brine, dried (Na₂SO₄), and the solvent removed under reduced pressure. The residue was purified by prep TLC plate with 5% methanol in methylene chloride, to give 2-(1,3-oxazol-5-yl)-6-(1H pyrazol-1-yl)pyrimidin-4-yl]amine. LCMS (APCI) *m*/*z* 229.0 (MH⁺).

### Intermediate 25D. 2-Chloro-N-(2-oxazol-5-yl-6-pyrazol-1-yl-pyrimidin-4-yl)-acetamide

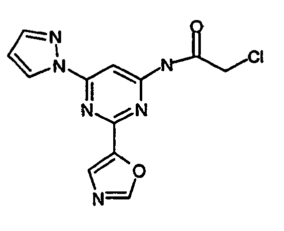

Intermediate 25D was prepared according to the procedures described in Intermediate 6, except that Intermediate 25D was used instead of Intermediate 5.

The compounds of Table 15 were prepared by reacting Intermediate 25D with the appropriate amine.

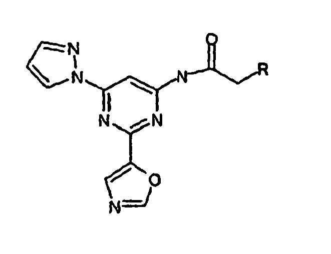

**Table 15**

| **No.** | **R** | **MW** | **MS ION** | **Reten time (min)** | **HPLC GRADIENT** |
|---|---|---|---|---|---|
| 15-1 | 4-Methyl-piperazin-1-yl | 368.4 | 369.0 | 3.174 | Method 2 |
| 15-2 | 3-Dimethytamino-pyrrolidin-1-yl | 382.4 | 383.0 | 2.704 | Method 2 |

### Intermediate 26A. 2-(4-Methyl-1,3-oxazol-5-yl)-6-(1H-pyrazol-1-yl)pyrimidin-4-amine

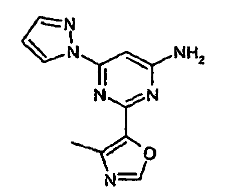

Intermediate 26A was obtained by reacting Intermediate 25B (46 mg, 0.2 mmol) with Me-TOSMIC (84 mg, 0.4 mmot) according to the procedures described in Intermediate 25C. LCMS (APCI) *m*/*z* 243.0 (MH⁺).

### Intermediate 26. 2-Chloro-N-[2-(4-methyl-oxazol-5-yl)-6-pyrazol-1-yl-pyrimidin-4-yl]-acetamide

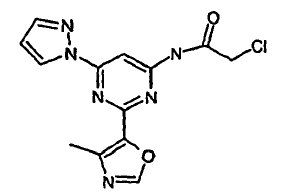

intermediate 26 was prepared according to the procedures described in Intermediate 6, except that Intermediate 26A was used instead of Intermediate 5.

The compound of Table 16 was prepared by reacting Intermediate 26 with the appropriate amine.

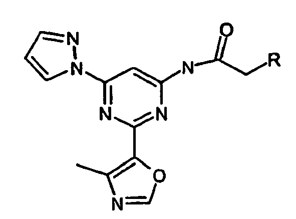

**Table 16**

| **No.** | **R** | **MW** | **MS ION** | **Reten time (min)** | **HPLC GRADIENT** |
|---|---|---|---|---|---|
| 16-1 | 4-methyl-piperazin-1-yl | 382.4 | 383.0 | 3.69 | Method 2 |

### Intermediate 27A. N-[6-Pyrazol-1-yl-2-(5-trimethylsilanyl-isoxazol-3-yl)-pyrimidin-4-yl]-acetamide

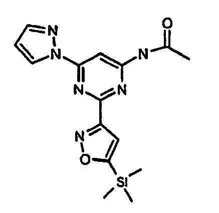

A mixture of Intermediate 25B (92 mg, 0.4 mmol), hydroxylamine hydrochloride (56 mg, 0.8 mmol) and sodium carbonate (85 mg, 0.8 mmol) in EtOH/water (2/1, 9 mL) was heated at 60°C for 1 hour. EtOH was removed and ethyl acetate added. The organic layer was washed with brine, dried (Na₂SO₄), and the solvent removed under reduced pressure to give crude oxime.

The oxime was dissolved in THF (5 mL) and treated with NCS (2 eq.) and pyridine (catalytic) at 60°C for 0.5 hour. Triethylamine and trimethylsilyl acetylene (2 eq. Each) were added, and the mixture was heated at 50°C for 2 hours. THF was removed and ethyl acetate added. The organic layer was washed with brine, dried (Na₂SO₄), and the solvent removed under reduced pressure. The residue was purified by prep TLC plate with 3% methanol in methylene chloride, to give N-[6-Pyrazol-1-yl-2-(5-trimethylsilanyl-isoxazol-3-yl)-pyrimidin-4-yl]-acetamide.

### Intermediate 27B. 2-(2-isoxazol-3-yl)-6-(1H-pyrazol-1-yl)pyrimidin-4-amine

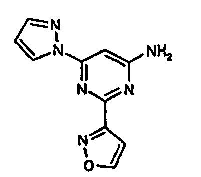

Intermediate 27A was dissolved in MeOH and KOH (1N aq.) was added. The mixture was stirred at RT for 15 hours. MeOH was evaporated and the residue was partitioned between ethyl acetate and water. The organic layer was washed with brine, dried (Na₂SO₄), and the solvent removed under reduced pressure. The residue was purified by prep TLC plate with 5% methanol in methylene chloride, to give 2-(2-isoxazol-3-yl)-6-(1H-pyrazol-1-yl)pyrimidin-4-amine. LCMS (APCI) *m*/*z* 229.0 (MH⁺).

### Intermediate 27C. 2-Chloro-N-(2-isoxazol-3-yi-6-pyrazol-1-yl-pyrimidin-4-yl)-acetamide

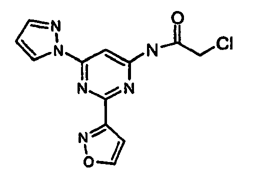

Intermediate 27C was prepared according to the procedures described in Intermediate 6, except that Intermediate 27B was used instead of Intermediate 5.

The compounds of Table 17 were prepared by reacting Intermediate 27C with the appropriate amine.

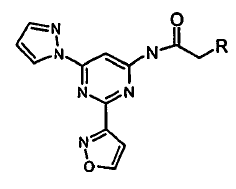

**Table 17**

| **No.** | **R** | **MW** | **MS ION** | **Reten time (min)** | **HPLC GRADIENT** |
|---|---|---|---|---|---|
| 17-1 | piperazin-1-yl | 368.4 | 369.0 | 3.543 | Method 2 |
| 17-2 | 3-Dimethylamino-pyrrolidin-1-yl | 382.4 | 383.0 | 3.044 | Method 2 |

### Intermediate 28. 2-Chloro-N-(6-pyrazol-1-yl-2-thiazol-2-yl-pyrlmidin-4-yl)-acetamide

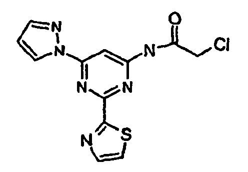

Intermediate 28 was prepared according to the procedures described in Intermediate 6, except that Thiazole-2-carboxamidine was used instead of Intermediate 1.

The compound of Table 18 was prepared by reacting Intermediate 28 with the appropriate amine.

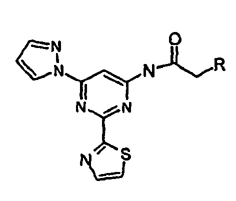

**Table 18**

| **No.** | **R** | **MW** | **MS ION** | **Reten time (min)** | **HPLC GRADIENT** |
|---|---|---|---|---|---|
| 18-1 | 4-Methyl-piperazin-1-yl | 384.5 | 385.0 | 3.916 | Method 2 |
| 18-2 | 4-pyrrolidin-1-yl-piperidin-1-yl | 438.5 | 439.0 | 3.399 | Method 2 |
| | 18-3 (S)-3-Dimethylamino- pyrrolidin-1-yl | 398.5 | 399.0 | 3.45 | Method 2 |
| 18-4 | (S)-3-Ethylamino-pyrrolidin-1-yl | 398.5 | 399.0 | 3.447 | Method 2 |

### Intermediate 28A. N-(6-Pyrazol-1-yl-2-thiazol-2-yl-pyrimidin-4-yl)-acrylamide

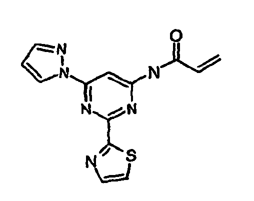

Intermediate 28A was prepared according to the procedures described in intermediate 6, except that 6-pyrazol-1-yl-2-thiazol-2-yl-pyrimidin-4-ylamine was used instead of Intermediate 11.

The compound of Table 18A was prepared by reacting Intermediate 28 with the appropriate amine.

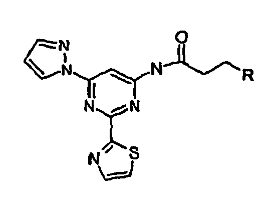

**Table 18A**

| **No.** | **R** | **MW** | **MS ION** | **Reten time (min)** | **HPLC GRADIENT** |
|---|---|---|---|---|---|
| 18-5 | (R)-3-Ethylamino-pyrrolidin-1-yl | 412.52 | 413.2 | 3.198 | Method 2 |

### Intermediate 29.2-Chloro-N-[6-(3,5-dimethyl-pyrazol-1-yl)-2-thiazol-2-yl-pyrimidin-4-yl]-acetamide

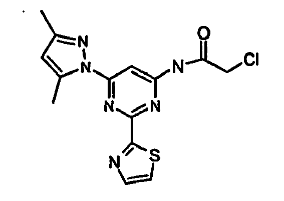

Intermediate 29 was prepared according to the procedures described in Intermediate 19, except that Thiazole-2-carboxamidine was used instead of 5-Methyl-furan-2-carboxamidine.

The compounds of Table 19 were prepared by reacting Intermediate 29 with the appropriate amine.

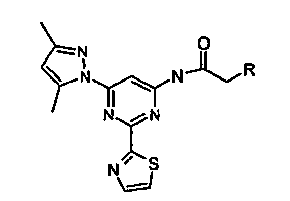

**Table 19**

| **No.** | **R** | **MW** | **MS ION** | **Reten time (min)** | **HPLC GRADIENT** |
|---|---|---|---|---|---|
| 19-1 | 4-Methyl-piperazin-1-yl | 412.5 | 413.0 | 4.78 | Method 2 |
| 19-2 | 4-pyrrolidin-1-yl-piperidin-1-yl | 466.6 | 467.0 | 4.172 | Method 2 |
| 19-3 | (S)-3-Dimethylamino-pyrrolidin-1-yl | 426.5 | 427.0 | 4.219 | Method 2 |
| 19-4 | (S)-3-Ethylamino-pyrrolidin-1-yl | 426.5 | 427.0 | 4.219 | Method 2 |
| 19-5 | 3-trifluoromethyl-5,6-dihydro-8H-[1,2,4]biazolo[4,3-a]pyrazin-7-yl | 504.5 | 505.1 | 6.627 | Method 2 |

### Intermediate 30. 6-(3,5-Dimethyl-pyrazol-1-yl)-2-furan-2-yl-pyrimidin-4-ylamine

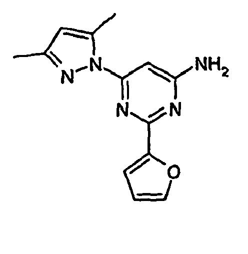

To a solution of Intermediate 4 (1.0 g, 5.1 mmol) in absolute EtOH (4.5 mL) was added anhydrous hydrazine (0.32 mL, 10.2 mmol). The mixture was heated at 90°C for 22 hours. The reaction was then cooled to room temperature and placed in an ice bath at 0°C while 2,4-pentanedione (1.05 mL, 10.2 mmol) was added slowly. The reaction mixture was heated at 90°C for 2 hours. Upon consumption of the hydrazine intermediate, the reaction was evaporated completely. The crude mixture was dissolved in CH₂Cl₂ (50 mL) and water (25 mL). The layers were separated and the aqueous layer was extracted with CH₂Cl₂ (4x25 mL). The combined organic layers were washed with brine (25 mL), dried over magnesium sulfate, filtered, and concentrated. The crude product was purified by flash chromatography using 1:1 EtOAc/ Hexanes to give the 0.94 g (3.68 mmol, 72%) of intermediate 30 as a white solid.

### Intermediate 31. 2-Chloro-N-[6-(3,5-dimethyl-pyrazol-1-yl)-2-furan-2-yl-pyrimidin-4-yl]-acetamide

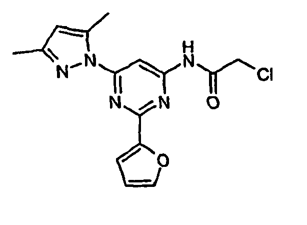

Intermediate 31 was prepared according to the procedures described in Intermediate 6, except that Intermediate 30 was used instead of Intermediate 5.

The compound in Table 20 was prepared by reacting Intermediate 31 with the appropriate amine.

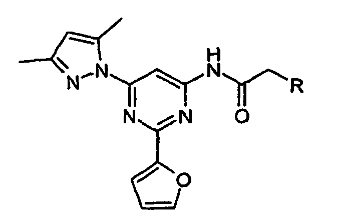

**Table 20**

| **No.** | **R** | **MW** | **MS ION** | **Reten time (min)** | **HPLC GRADIENT** |
|---|---|---|---|---|---|
| 20-1 | 4-Methyl-piperazine | 395.5 | 396.1 | 17.021 | Method 3 |

Free base product: NMR δ (300 MHz, CD₃OD): 2.27 (s, 3H), 2.35 (s, 3H), 2.54-2.75 (m, 8H), 2.77 (s, 3H), 3.29 (s, 2H), 6.13 (s, 1H), 6.65 (dd, *J*=1.8 and 3.6 Hz, 1H), 7.31 (dd, *J*=0.9 and 3.6 Hz, 1H), 7.76 (dd, *J*=0.6 and 1.5 Hz, 1H).

### Intermediate 32. 2-Chloro-N-[2-(5-methyl-furan-2-yl)-6-pyrazol-1-yl-pyrimidin-4-yl]-propionamide

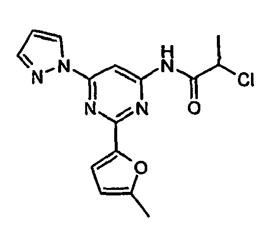

Intermediate 32 was prepared according to the procedures described in Intermediate 6, except that (i) 5-Methyl-furan-2-carboxamidine was used instead of Intermediate 1 and (ii) 2-chloropropionyl chloride was used instead of chloroacetyl chloride.

The compound in Table 21 was prepared by reacting Intermediate 32 with the appropriate amine.

**Table 21**

| **No.** | **R** | **MW** | **MS ION** | **Reten time (min)** | **HPLC GRADIENT** |
|---|---|---|---|---|---|
| 21-1 | 4-Methyl-piperazine | 395.5 | 396.1 | 4.727 | Method 2 |

### Compound 22-1. 1-Methyl-piperidine-4-carboxylic acid [6-(3,5-dimethyl-pyrazol-1-yl)-2-(5-methyl-furan-2-yl)-pyrimidin-4-yl]-amide

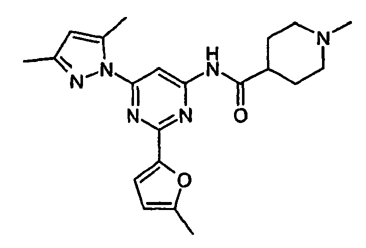

To a suspension of 1-methyl-piperidine-4-carboxylic acid HCl (0.30 g, 1.67 mmol) in anhydrous CH₂Cl₂ (3 mL) was added oxalyl chloride (0.19 mL, 2.18 mmol) followed by 2 catalytic drops of DMF. The reaction was stirred at room temperature for 1.5 hours and then evaporated to dryness. The crude acid chloride intermediate and intermediate 19C were resuspended in anhydrous THF (4 mL) and then pyridine was added and the reaction mixture stirred at room temperature for 18 hours. The reaction mixture was evaporated to dryness and then dissolved in CH₂Cl₂ (30 mL) and brine (15 mL). The layers were separated and the aqueous layer was extracted with CH₂Cl₂ (4x15 mL). The combined organic layers were then washed with brine (15 mL), dried over magnesium sulfate, flitered and concentrated. The crude product was purified by flash chromatography using 10% MeOH in CH₂Cl₂ to give 0.037 g (0.094 mmol, 16.8%) of the product as an off white solid. LCMS ret. time =5.373 by Method 2 (APCI) m/z 395.1 (MH⁺)

### Intermediate 33. 2-Chloro-N-[6-(3,5-dimethyl-pyrazol-1-yl)-2-(5-methyl-furan-2-yl)-pyrimidin-4-yl]-acetamide

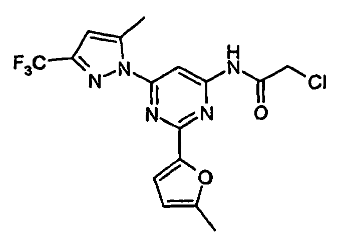

Intermediate 33 was prepared according to the procedures described in Intermediate 6, except that (i) 5-Methyl-furan-2-carboxamidine was used instead of Intermediate 1 and (ii) 3-methyl-5-(trifluoromethyl)pyrazole was used instead of pyrazole.

The compound in Table 22 was prepared by reacting Intermediate 33 with the appropriate amine.

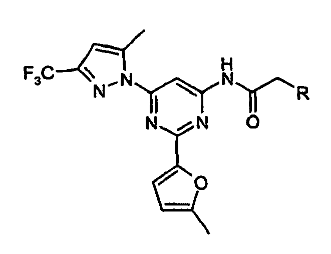

**Table 22**

| **No.** | **R** | **MW** | **MS ION** | **Reten time (min)** | **HPLC GRADIENT** |
|---|---|---|---|---|---|
| 22-1 | 4-Methyl-piperazine | 463.5 | 464.1 | 6.184 | Method 2 |

### Intermediate 34. (S)-pyrrolidine-3-carboxylic acid [6-(3,5-dimethyl-pyrazol-1-yl)-2-(5-methyl-furan-2-yl)-pyrimidin-4-yl]-amide

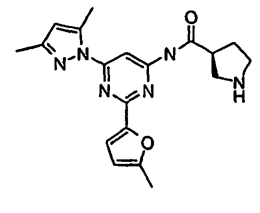

to a 100mL reaction Vial were added (S)-pyrrolidine-1,3-dicarboxylic acid 1-tert-butyl ester (1g, 1.5eq), 10m1 of dry THF, one drop of DMF and oxalyl chloride (0.42ml, 1.6eq). After 90 minutes at room temperature, solvents were evaporated and 20ml of DCM were added. Pyridine (0.36ml, 1.5eq) and Intermediate 19C (834mg, 1 eq) were added. The solution was stirred at room temperature overnight. The mixture was evaporated and the residue purified by liquid chromatography to give 1.48g of the Boc protected Intermediate 34. The product was dissolved in TFA/DCM (5/1 ml) and stirred for one hour at room temperature. Solvents were evaporated and the mixture used as is in the next step.

The compounds of Table 23 were prepared either by reductive-amination using the appropriate aldehyde with 1.4eq of borane-pyridine complex and a catalytic amount of acetic acid in ethanol or by alkylation with a bromoalkyl reagent in presence of N,N-diisopropylethylamine in DMF.

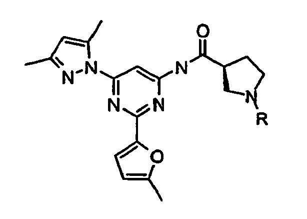

**Table 23**

| **No.** | **R** | **MW** | **MS ION** | **Reten time (min)** | **HPLC GRADIENT** |
|---|---|---|---|---|---|
| 23-1 | H | 366.4 | 367.1 | 5.23 | Method 2 |
| 23-2 | Me | 380.4 | 381.1 | 5.15 | Method 2 |
| 23-3 | 2-Methoxyethyl | 424.5 | 424.7 | 7.98 | Method 2 |

### Intermediate 35. (R)-pyrrolidine-3-carboxylic acid [6-(3,5-dimethyl-pyrazol-1-yl)-2-(5-methyl-furan-2-yl)-pyrimidin-4-yl]-amide

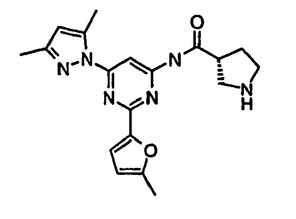

Intermediate 35 was prepared according to the procedures described in Intermediate 34, except that (R)-pyrrolidine-1,3-dicarboxylic acid 1-tert-butyl ester was used instead of (S)-pyrrolidine-1,3-dicarboxylic acid 1-tert-butyl ester.

The compounds of Table 24 were prepared either by reductive-amination using the appropriate aldehyde with 1.4eq of borane-pyridine complex and a catalytic amount of acetic acid in ethanol or by alkylation with a bromoalkyl reagent in presence of N,N-diisopropylethylamine in DMF.

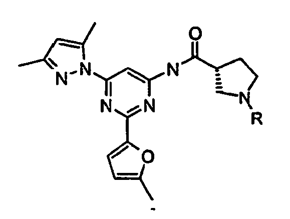

**Table 24**

| **No.** | **R** | **MW** | **MS ION** | **Reten time (min)** | **HPLC GRADIENT** |
|---|---|---|---|---|---|
| 24-1 | H | 366.4 | 367.2 | 7.17 | Method 2 |
| 24-2 | Me | 380.4 | 381.1 | 5.29 | Method 2 |
| 24-3 | 2-Methoxyethyl | 424.5 | 424.5 | 7.97 | Method 2 |
| 24-4 | 2,4,6-Trifluoro benzyl | 510.5 | 511.1 | 6.08 | Method |

### Intermediate 36. (R)-pyrrolidine-2-carboxylic acid [6-(3,5-dimethyl-pyrazol-1-yl)-2-(5-methyl-furan-2-yl)-pyrimidin-4-yl]-amide

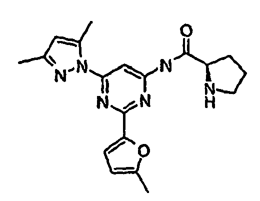

Intermediate 36 was prepared according to the procedures described in Intermediate 34, except (R)-pyrrolidine-1,2-dicarboxylic acid 1-benzyl ester was used instead of (S)-pyrrolidine-1,3-dicarboxylic acid 1-tert-butyl ester.

The compounds of Table 25 were prepared either by reductive-amination using the appropriate aldehyde with 1.4eq of borane-pyridine complex and a catalytic amount of acetic acid in ethanol or by alkylation with a bromoalkyl reagent in presence of N,N-diisopropylethylamine in DMF.

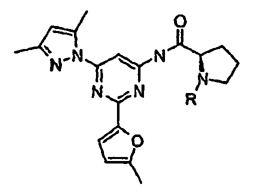

**Table 25**

| **No.** | **R** | **MW** | **MS ION** | **Reten time (min)** | **HPLC GRADIENT** |
|---|---|---|---|---|---|
| 25-1 | H | 366.4 | 367.1 | 5.21 | Method 2 |
| 25-2 | 2-methoxy-1-ethyl | 424.5 | 425.1 | 5.29 | Method 2 |

### Intermediate 37. (S)-Pyrrolidine-2-carboxylic acid [6-(3,5-dimethyl-pyrazol-1-yl)-2-(5-methyl-furan-2-yl)-pyrimidin-4-yl]-amide

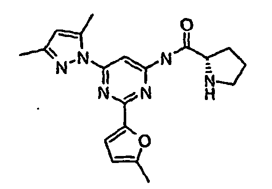

Intermediate 37 was prepared according to the procedures described in Intermediate 34, except that (1) (S)-pyrrolidine-1,2-dicarboxylic acid 1-benzyl ester was used instead of (S)-pyrrolidine-1,3-dicarboxylic acid 1-tert-butyl ester and (2) hydrogenation instead of TFA was used for deprotection.

The compounds of Table 26 were prepared either by reductive-amination using the appropriate aldehyde with 1.4eq of borane-pyridine complex and a catalytic amount of acetic acid in ethanol or by alkylation with a bromoalkyl reagent in presence of diisopropylamine in DMF.

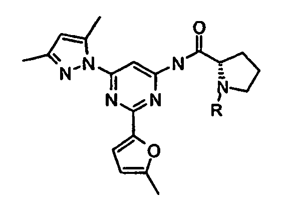

**Table 26**

| **No.** | **R** | **MW** | **MS ION** | **Reten time (min)** | **HPLC GRADIENT** |
|---|---|---|---|---|---|
| 26-1 | benzyloxycarbonyl | 500.5 | 501.1 | 9.11 | Method 2 |
| 26-2 | H | 366.4 | 367.1 | 5.20 | Method 2 |
| 26-3 | 2-methoxy-1-ethyl | 424.5 | 425.1 | 5.43 | Method 2 |

### Intermediate 38. N-[6-(3,5-dimethyl-pyrazol-1-yl)-2-thiazol-2-yl-pyrimidin-4-yl]-acylamide

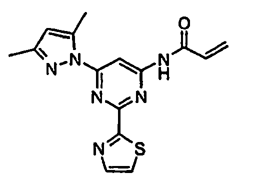

Intermediate 38 was prepared according to the procedures described in Intermediate 19B, except that thiazole-2-carboxamidine was used instead of 5-Methyl-furan-2-carboxamidine.

The compounds of Table 27 were prepared by reacting Intermediate 38 with the appropriate amine.

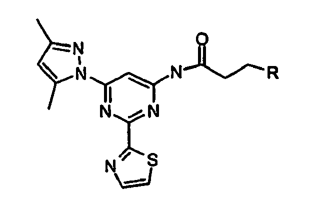

**Table 27**

| **No.** | **R** | **MW** | **MS ION** | **Reten time (min)** | **HPLC GRADIENT** |
|---|---|---|---|---|---|
| 27-1 | 4-Methyl-piperazin-1-yl | 426.6 | 427.0 | 4.39 | Method 2 |
| 27-2 | 4-pyrrolidin-1-yl-piperidin-1-yl | 480.6 | 481.1 | 4.30 | Method 2 |
| 27-3 | (R)-3-Dimethylamino-pyrrolidin-1-yl | 440.6 | 441.0 | 4.16 | Method 2 |
| 27-4 | (R)-3-Ethylamino- pyrrolidin-1-yl | 440.6 | 441.0 | 4.19 | Method 2 |
| 27-5 | [1,4]oxazepan-4-yl | 427.5 | 428.0 | 4.89 | Method 2 |

### Intermediate 39. N-[6-pyrazol-1-yl-2 thiazol-2-yl-pyrimidin-4-yl]-acylamide

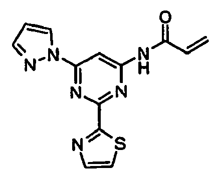

Intermediate 39 was prepared according to the procedures described in Intermediate 38, except that pyrazole was used instead of 3,5-dimethylpyrazole.

The compound of Table 28 was prepared by reacting Intermediate 39 with the appropriate amine.

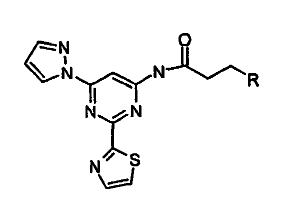

**Table 28**

| **No.** | **R** | **MW** | **MS ION** | **Reten time (min)** | **HPLC GRADIENT** |
|---|---|---|---|---|---|
| 28-1 | (R)-3-Ethylamino- pyrrolidin-1-yl | 412.5 | 413.2 | 3.20 | Method 2 |

### Intermediate 40. Morpholine-2-carboxylic acid [6-(3,5-dimethyl-pyrazol-1-yl)-2-(5-methyl-furan-2-yl)-pyrimidin-4-yl]-amide

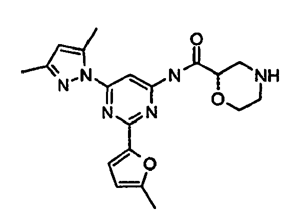

Intermediate 40 was prepared according to the procedures described in Intermediate 34, except that morpholine-2,4-dicarboxylic acid 4-tert-butyl ester was used instead of S-pyrrolidine-1,3-dicarboxylic acid 1-tert-butyl ester.

The compounds of Table 29 were prepared either by reductive-amination using the appropriate aldehyde with 1.4eq of borane-pyridine complex in THF or by acylation with an acyl chloride reagent in the presence of triethylamine in THF.

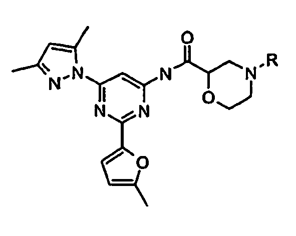

**Table 29**

| **No.** | **R** | **MW** | **MS ION** | **Reten time** (min) | **HPLC GRADIENT** |
|---|---|---|---|---|---|
| 29-1 | H | 382.4 | 383.1 | 5.07 | Method 2 |
| 29-2 | methyl | 396.4 | 397.1 | 5.28 | Method 2 |
| 29-3 | Pyrrolidine-1-carbonyl | 479.5 | 480.2 | 8.29 | Method 2 |

### Compound 30-1. Pyrrolidine-1-carboxylic acid [6-(3,5-dimethyl-pyrazol-1-yl)-2-(5-methyl-furan-2-yl)-pyrimidin-4-yl]-amide

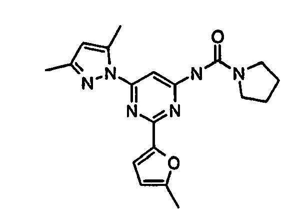

To a solution of Intermediate 19C (54 mg, 0.2 mmol) in THF (2 mL) was added NaH (10 mg, 1.25 eq) and stirred at RT for 10 min. Pyrrolidine-1-carbonyl chloride (53 mg, 2 eq) was added and the mixture was stirred for 2 hr. THF was removed and the residue was partitioned between water and ethyl acetate. The organic layer was dried over sodium sulfate, concentrated and purified by prep LCMS.

**Table 30A**

| **No.** | **Structure** | **MW** | **MS ION** | **Reten time (min)** | **HPLC GRADIENT** |
|---|---|---|---|---|---|
| 30-1 | 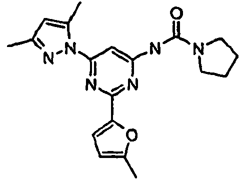 | 366.4 | 367.1 | 7.61 | Method 2 |

### Compound 30-2. Morpholine-4-carboxylic acid (6-(3,5-dimethyl-pyrazol-1-yl)-2-(5-methyl-furan-2-yl)-pyrimidin-4-yl]-amide

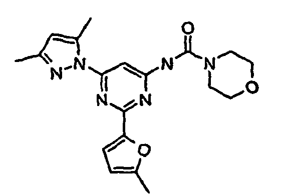

Compound 30-2 was prepared according to the procedure described In Compound 30-1, except that morpholine-4-carbonyl chloride was used instead of pyrrolidine-1-carbonyl chloride.

**Table 30B**

| **No.** | **Structure** | **MW** | **MS ION** | **Reten time** (min) | **HPLC GRADIENT** |
|---|---|---|---|---|---|
| 30-2 | 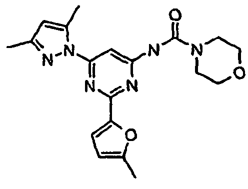 | 382.4 | 383.0 | 7.46 | Method 2 |

### Compound 30-3. 4-Methyl-piperazine-1-carboxylic acid [6-(3,5-dimethyl-pyrazol-1-yl)-2-(5-methyl-furan-2-yl)-pyrimidin-4-yl]-amide

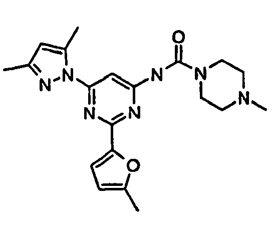

A slurry of Intermediate 19C (0.27 g, 1.0 mmol) and NaH (40 mg, 1 eq) in DMF/THF (1/1, 2 mL) was stirred until the evolution of H₂ had ceased (10 min). The resulting solution was slowly stirred into a solution of CDI (0.17 g, 1 eq) in DMF (1 mL). Alter 10 min, 1-methyl-piperazine (0.11 mL, 1 eq) was added and the mixture was stirred for 4 hr. The reaction was treated with glacial AcOH (0.13 mL) and concentrated under vacuum. Water (10 mL) was added and the resulting solid was filtered, washed with water and hexane, and purified by prep LCMS.

**Table 30C**

| **No.** | **Structure** | **MW** | **MS ION** | **Reten time (min)** | **HPLC GRADIENT** |
|---|---|---|---|---|---|
| 30-3 | 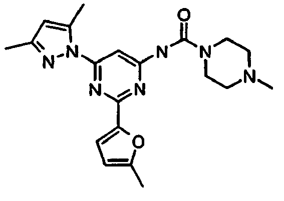 | 395.5 | 396.1 | 5.10 | Method 2 |

### Compound 30-4.1-[6-(3,5-Dimethyl-pyrazol-1-yl)-2-(5-methyl-furan-2-yl)-pyrimidin-4-yl]-3-(2-morpholin-4-yl-ethyl)-urea

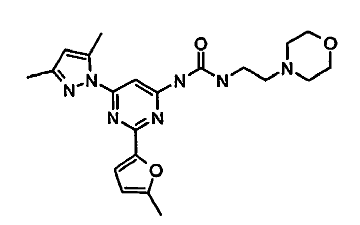

Compound 30-4 was prepared according to the procedure described in Compound 30-3, except that 2-morpholin-4-yl-ethylamine was used instead of 1-methyl-piperazine.

**Table 30D**

| **No.** | **Structure** | **MW** | **MS** | **Reten ION time (min)** | **HPLC GRADIENT** |
|---|---|---|---|---|---|
| 30-4 | 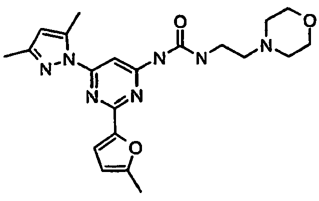 | 425.5 | 426.1 | 5.12 | Method 2 |

### Compound 30-5. 1-[6-(3,5-Dimethyl-pyrazol-1-yl)-2-(5-methyl-furan-2-yl)-pyrimidin-4-yl]-3-(3-piperidin-1-yl-propyl)-urea

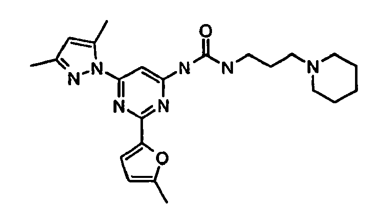

Compound 30-5 was prepared according to the procedure described in Compound 30-3, except that 3-piperidin-1-yl-propylamine was used instead of 1-methyl-piperazine.

**Table 30E**

| **No.** | **Structure** | **MW** | **MS ION** | **Reten time (min)** | **HPLC GRADIENT** |
|---|---|---|---|---|---|
| 30-5 | 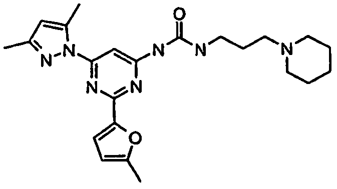 | 437.6 | 438.1 | 5.36 | Method 2 |

### Intermediate 41. N-[6-(3,5-Dimethyl-pyrazol-1-yl)-2-(5-methyl-furan-2-yl)-pyrimidin-4-yl]-2-pyrrolidin-3-yl-acetamide

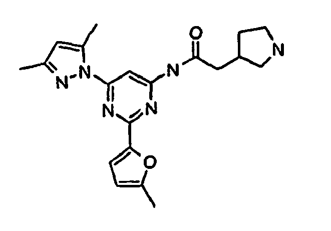

Intermediate 41 was prepared according to the procedures described in Intermediate 34, except that (R)- or (S)- 3-Carboxymethyl-pyrrolidine-1-carboxylic acid *tert*-butyl ester was used instead of (S)-pyrrolidine-1,3-dicarboxylic acid 1-tert-butyl ester.

The compounds of Table 31 were prepared either by reductive-amination using the appropriate aldehyde with 1.4eq of borane-pyridine complex in THF or by alkylation with an alkylhalide reagent in the presence of diisopropylethylamine in DMF.

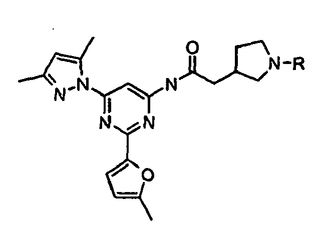

**Table 31**

| **No.** | **Chirality** | **R** | **MW** | **MS ION** | **Reten time (min)** | **HPLC GRADIENT** |
|---|---|---|---|---|---|---|
| 31-1 | S | H | 380.4 | 381.1 | 5.09 | Method 2 |
| 31-2 | S | methyl | 394.5 | 395.1 | 5.16 | Method 2 |
| 31-3 | S | dimethyl | 409.5 | 409.1 | 5.19 | Method 2 |
| 31-4 | S | 2-methoxy-1-ethyl | 438.5 | 439.1 | 5.41 | Method 2 |
| 31-5 | R | H | 380.4 | 381.1 | 5.41 | Method 2 |
| 31-6 | R | methyl | 394.5 | 395.1 | 5.19 | Method 2 |
| 31-7 | R | 2-methoxy-1-ethyl | 438.5 | 439.1 | 5.36 | Method 2 |

### Compound 32-1. 1-Methyl-piperidine-4-carboxylic acid [6-(3,5-dimethyl-pyrazol-1-yl)-2-(5-methyl-furan-2-yl)-pyrimidin-4-yl]-amide

Intermediate 42 was prepared according to the procedures described in Intermediate 34, except that 1-methylpiperidine 4-carboxylic acid was used instead of (S)-pyrrolidine-1,3-dicarboxylic acid 1-tert-butyl ester. The compound was purified by HPLC.

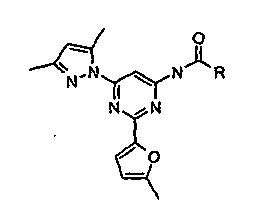

**Table 32**

| **No.** | **R** | **MW** | **MS ION** | **Reten time (min)** | **HPLC GRADIENT** |
|---|---|---|---|---|---|
| 32-1 | 1-Methyl-piperidine-4-yl | 394.5 | 395.1 | 5.419 | Method 2 |

The compound of Table 33 was prepared according to the procedure described for compound 9-106 except that R-1-BOC- piperidine 3-yl acetic acid was used instead of 1-BOC- piperidine 4-yl acetic acid.

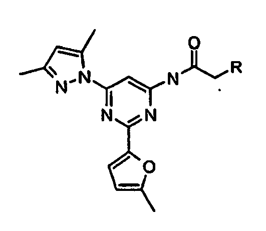

**Table 33**

| **No.** | **R** | **MW** | **MS ION** | **Reten time (min)** | **HPLC GRADIENT** |
|---|---|---|---|---|---|
| 33-1 | (R)-1-(2-methoxy-ethyl)-piperidin-3-yl | 452.5 | 453.1 | 5.595 | Method 2 |

The urea compounds of Table 34 were prepared according to the procedure described in Compound 30-3 using the appropriate amine.

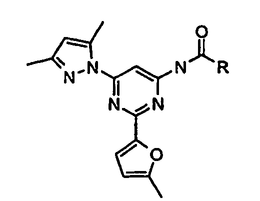

**Table 34**

| **No.** | **R** | **MW** | **MS ION** | **Reten time (min)** | **HPLC GRADIENT** |
|---|---|---|---|---|---|
| 34-1 | 3-pyrrolidin-1-yl-propylamine | 423.5 | 424.1 | 5.282 | Method 2 |
| 34-2 | 3-(4-methyl-piperazin-1-yl)-propylamine | 452.6 | 453.4 | 8.013 | Method 2 |
| 34-3 | 2-(1-methyl-pyrrolidin-2-yl)-ethylamine | 423.5 | 424.3 | 9.595 | Method 2 |
| 34-4 | 2-(1-methyl-piperidin-2-yl)-ethylamine | 437.5 | 538.2 | 10.32 | Method 2 |
| 34-5 | 2-piperidin-2-yl-ethylamine | 423.5 | 424.2 | 5.443 | Method 2 |
| 34-6 | 3-morpholin-4-yl-propylamine | 439.5 | 440.0 | 5.22 | Method 2 |

The carbamate compounds of Table 35 were prepared according to the procedure described in Compound 30-3 using the appropriate alcohol.

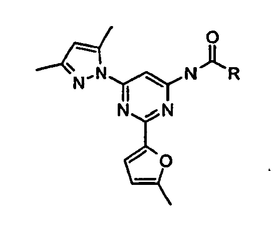

**Table 35**

| **No.** | **R** | **MW** | **MS ION** | **Reten time (min)** | **HPLC GRADIENT** |
|---|---|---|---|---|---|
| 35-1 | 2-(1-methyl-pyrrolidin-2-yl)-ethoxy | 424.5 | 425.0 | 5.65 | Method 2 |
| 35-2 | 2-azepan-1-yl-ethoxy | 438.5 | 439.0 | 5.81 | Method 2 |
| 35-3 | 3-piperidin-1-yl-propoxy | 438.5 | 439.0 | 5.73 | Method 2 |
| 35-4 | 3-(2-oxo-pyrrolidin-1-yl)-propoxy | 438.5 | 439.0 | 7.33 | Method 2 |
| 35-5 | 2-morpholin-4-yl-ethoxy | 426.5 | 427.0 | 5.34 | Method 2 |
| 35-6 | 2-piperidin-1-yl- ethoxy | 424.5 | 425.0 | 5.6 | Method 2 |
| 35-7 | 2-(2-oxo-pyrrolidin-1-yl)-ethoxy | 424.5 | 425.0 | 7.11 | Method 2 |
| 35-8 | 2-pyrrolidin-1-yl-ethoxy | 410.5 | 411.0 | 5.43 | Method 2 |

## Claims

1. A compound of formula (I) 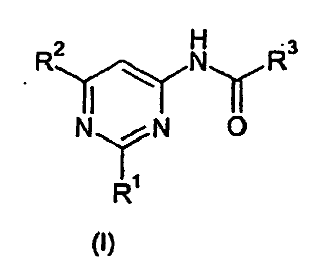 or a pharmaceutically acceptable salt, ester, solvate, or stereoisomer thereof, wherein:
each of R¹ and R² independently is an aryl or heteroaryl group optionally substituted by one or more substituents selected from the group of lower alkyl having 1 to 8 carbon atoms, halogen, cycloalkyl, hydroxy, lower alkoxy having 1 to 8 carbon atoms, -SH, NO₂, lower alkylthio having 1 to 8 carbon atoms, lower alkylamino having 1 to 8 carbon atoms, cyano, and amino, wherein the lower alkyl, cycloalkyl, lower alkoxy, lower alkylthio and lower alkylamino groups are optionally substituted by one or more substituents selected from the group of alkyl, alkenyl, alkynyl, aryl, heteroaryl, alkoxy, aryloxy, alkylthio, arylthio, cycloalkyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃;
R³ is - (CR⁴R⁵)ₙ-R⁶, - (CR⁴R⁵)ₙ-NR⁷R⁸, -O-(CR⁴R⁵)ₙ-R⁶ or is -(CR⁴R⁵)ₙ-O-R⁸;
each of R⁴ and R⁵ independently is at each occurrence selected from the group of hydrogen, lower alkyl having 1 to 8 carbon atoms , halogen, cycloalkyl, hydroxy, lower alkoxy having 1 to 8 carbon atoms, -SH, NO₂, lower alkylthio having 1 to 8 carbon atoms, lower alkylamino having 1 to 8 carbon atoms, cyano, and amino, wherein the lower alkyl, cycloalkyl, lower alkoxy, lower alkylthio and lower alkylamino groups are optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃;
R⁶ is a heterocycle having at least one nitrogen atom, wherein the heterocycle is optionally substituted by one or more members selected from the group of lower alkyl having 1 to 8 carbon atoms, alkoxy, cycloalkyl, aminoalkyl, aminodialkyl, alkylaminoalkyl, dialkylaminoalkyl, alkoxyalkyl, aryl, arylalkyl, heteroaryl heteroarylalkyl, heterocycle, heterocycylalkyl, amino, alkylamino, dialkylamino, cycloalkylamino, halogen, haloalkyl, ester, amide, acyl, carbamoyl, carbamoylalkyl, oxo, isoquinolinyl and imidoylamino, wherein said lower alkyl, alkoxy, cycloalkyl, aminoalkyl, alkylaminoalkyl, dialkylaminoalkyl, alkoxyalkyl, aryl, arylalkyl, heteroaryl heteroarylalkyl, heterocycle, heterocycylalkyl, amino, alkylamino, dialkylamino, cycloalkylamino, haloalkyl, ester, amide, acyl, carbamoyl, carbamoylalkyl, isoquinolinyl and imidoylamino groups are optionally substituted with lower alkyl having 1 to 8 carbon atoms, alkoxy, hydroxy, oxo or halogen;
R⁷ is hydrogen or lower alkyl having 1 to 8 carbon atoms optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃;
R⁸ is -(CR⁴R⁵)ₙ-R⁶; or
R⁷ and R⁸ together with the nitrogen atom to which they are attached form aheterocyclic ring optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂ NH₂, NHCH₃, NHCH₂CH₃, N(CH₃)₂, N(CH₂CH₃)₂, SH, SCH₃,OH, OCH₃, OCF₃, CH₃, and CF₃; and
n is independently at each occurrence 0, 1 , 2, 3 or 4;
with the proviso that when R¹ and R² are both heteroaryl, R⁶ is a non-aromatic heterocycle.

2. A compound according to claim 1, wherein: each of R¹ and R² is independently selected from the group of pyridinyl, furanyl, thiophenyl, thiazolyl, pyrazolyl, imidazolyl, oxazolyl, isoxazolyl and oxadiazolyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃.

3. A compound according to claim 2, wherein n is 1 or 2.

4. A compound according to claim 3, wherein:
R¹ is selected from the group of pyridinyl, furanyl, thiophenyl, thiazolyl, pyrazolyl, imidazolyl, oxazolyl and isoxazolyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, and CF₃.

5. A compound according to claim 4, wherein:
R² is selected from the group of pyridinyl, thiazolyl and pyrazolyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, and CF₃.

6. A compound according to claim 5, wherein:
R¹ is selected from the group of pyridinyl, furanyl, 5-methyl-furanyl, thiophenyl, thiazolyl, pyrazolyl, imidazolyl, oxazolyl, 4-methyl-oxazolyl and isoxazolyl; and
R² is selected from the group of pyridinyl, thiazolyl, pyrazolyl and 3,5-dimethylpyrazolyl.

7. A compound according to claim 6, wherein: R³ is -CR⁴R⁵)ₙ-R⁶ or -(CR⁴R⁵)ₙ-NR⁷R⁸;
each of R⁴ and R⁵ independently is at each occurrence hydrogen or lower alkyl having 1 to 8 carbon atoms;
R⁶ is a heterocyclic ring selected from the group of piperidinyl, piperazinyl, morpholinyl, thiomorpholinyl, pyrrolidinyl, isoquinolinyl, diazepanyl, dihydropyrrolyl, azepanyl, oxazepanyl, and pyrrolopyrazinyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, and CF₃; and R⁷ and R⁸ together with the nitrogen atom to which they are attached form a heterocyclic ring selected from the group of piperidinyl, piperazinyl, morpholinyl, thiomorpholinyl, pyrrolidinyl, isoquinolinyl, diazepanyl, dihydropyrrolyl, azepanyl, oxazepanyl, and pyrrolopyrazinyl optionally substituted by one or more substituents selected from the group of alkyl, alkenyl, alkynyl, aryl, heteroaryl, alkoxy, aryloxy, alkylthio, arylthio, cycloakyl, arylalkyl, amino, alkylamino, dialkylamino, amido, F, Cl, Br, I, CN, NO₂, NH₂, NHCH₃, NHCH₂CH₃, N(CH₃)₂, N(CH₂CH-₃)₂, SH, SCH₃,OH, OCH₃, OCF₃, CH₃, and CF₃.

8. A compound according to claim 7, wherein R⁶ is substituted with one or more members selected from the group of lower alkyl having 1 to 8 carbon atoms, alkoxy, amino, alkylamino, dialkylamino, piperidinyl, piperazinyl, pyrrolidinyl, morpholinyl, phenyl and benzyl.

9. A compound according to claim 7, wherein:
R¹ is furanyl, 5-methyl-furanyl, oxazolyl, 4-methyl-oxazolyl, pyridinyl, pyrazolyl, or isoxazolyl and R² is pyrazolyl.

10. A compound according to claim 7, wherein:
R¹ is furanyl, 5-methyl-furanyl, pyridinyl, thiophenyl or thiazolyl and R² is thiazolyl.

11. A compound according to claim 7, wherein:
R¹ is 5-methyl-furanyl, thiazolyl or pyridinyl and R² is 3,5- dimethylpyrazolyl.

12. A compound according to claim 7, wherein: R¹ is 5-methyl-furanyl or thiophenyl and R² is pyridinyl.

13. A compound according to claim 1, wherein:
R¹ is furanyl or 5-methyl-furanyl; and
R² is pyrazolyl, 3,5-dimethylpyrazolyl or thiazolyl.

14. A compound according to claim 13, wherein:
R³ is -(CR⁴R⁵)nR⁶; each of R⁴ and R⁵ is hydrogen; and n is 1 or 2.

15. A compound according to claim 14, wherein:
R⁶ is piperazinyl substituted with lower alkyl having 1 to 8 carbon atoms, pyrrolidinyl substsituted with alkylamino or dialkylamino, [1 ,4]-diazepanyl substituted with lower alkyl having 1 to 8 carbon atoms or [1,4]-oxazepanyl.

16. A compound according to claim 1 selected from the group of:
N-(2-Furan-2-yl-6-pyrazol-1-yl-pyrimidin-4-yl)-2-piperidin-1-yl-acetamide;
N-(2-Furan-2-yl-6-pyrazol-1-yl-pyrimidin-4-yl)-2-morpholin-4-yl-acetamide;
N-(2-Furan-2-yl-6-pyrazol-1-yl-pyrimidin-4-yl)-2-(4-methyl-piperazin-1-yl)-acetamide;
N-(2-Furan-2-yl-6-pyrazol-1-yl-pyrimidin-4-yl)-2-piperazin-1-yl-acetamide;
N-[2-(5-Methyl-furan-2-yl)-6-thiazol-2-yl-pyrimidin-4-yl]-2-morpholin-4-yl-acetamide;
N-[2-(5-Methyl-furan-2-yl)-6-thiazol-2-yl-pyrimidin-4-yl)-2-piperidin-1-yl-acetamide;
N-[2-(5-Methyl-furan-2-yl)-6-thiazol-2-yl-pyrimidin-4-yl]-2-pyrrolidin-1-yl-acetamide;
N-[2-(5-Methyl-furan-2-yl)-6-thiazol-2-yl-pyrimidin-4-yl]-2-(4-methyl-piperazin-1-yl)-acetamide;
2-(2,5-Dimethyl-piparazin-1-yl)-N-[2-(5-methyl-furan-2-yl)-6-thiazol-2-yl-pyrimidin4-yl] acetamide;
N-[2-(5-Methyl-furan-2-yl)-6-thiazol-2-yl-pyrimidin-4-yl]-2-piperazin-1-yl-acetamide;
N-[2-(5-Methyl-furan-2-yl)-6-thiazol-2-yl-pyrimidin-4-yl]-2-(4-phenyl-piperazin-1-yl)-acetamide;
2-[1,4]Diazepan-1-yl-N-[2-(5-methyl-furan-2-yl)-6-thiazol-2-yl-pyrimidin-4-yl]-acetamide;
N-(2-(5-Methyl-furan-2-yl)-6-thiazal-2-yl-pyrimidin4-yl]-2-(2-phenyl-piperidin-1-yl)-acetamide;
N-[2-(5-Methyl-furan-2 yl)-6-thiazol-2-yl-pyrimidin-4-yl]-2-(3-phenyl-piperidin-1-yl)-acetamide;
2-(4-Benzyl-piperazin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(2-methyl-piperidin-1-yl)-acetamide;
2-(2,5-Dihydro-pyrrol-1-yl)-N-[2-(5-methyl-furan-2-yl)-6-thiazol-2-yl-pyrimidin-4-yl]-acetamide;
2-(2,5-Dimethyl-2,5-dihydro-pyrrol-1-yl-N-[2-(5-methyl-furan-2-yl)-6-thiazol-2-yl-pyrimidin-4-yl]-acetamide;
2-(2,5-Dimethyl-pyrrolidin-1-yl)-N-[2-(5-ethyl-fufan-2-yl)-6-thiazol-2-yl-pyrimidin-4-yl]-acetamide;
2-(3,5-Dimethyl-piperazin-1-yl)-N-[2-(5-methyl-furan-2-yl)-6-thiazol-2-yl-pyrimidin-4-yl]-acetamide;
2-[4-(2-Methoxy-phenyl)-piperazin-1-yl]-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(3-methyl-piperidin-1-yl)-acetamide;
2-Azepan-1-yl-N-[2-(5-methyl-furan-2-yl)-6-thiazol-2-yl-pyrimidin-4-yl]-acetamide;
2-((S)-2-Methoxymethyl-pyrrolidin-1-yl)-N-[2-(5-methyl-furan-2-yl)-6-thiazol-2-yl-pyrimidin-4-yl]-acetamide;
2-(3.3-Dimethyl-piperidin-1-yl)-N-[2-(5-methyl-furan-2-yl)-6-thiazol-2-yl-pyrimidin4-yl]-acetamide;
2-(3,5-Dimethyl-piperidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(4-phenyl-piperidin-1-yl)-acetamide;
N-[2-(5-Methyl-furan-2-yl)-6-thiazol-2-yl-pyrimidin-4-yl]-2-(4-methyl-piperidin-1-yl)-acetamide;
2-(4-Benzy)-piperidin-1-yl)-N-[2-(5-niethyl-furan-2-yl)-6-thiazol-2-yl-pyrimidin-4-yl]-acetamide;
2-[1,4]Bipiperidinyl-1'-yl-N-[2-(5-methyl-furan-2-yl)-6-thiazol-2-yl-Pyrimidin-4-yl]-acetamide;
2-(3,4-Dihydro-1H-isoquinolin-2-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(octahydro-isoquinolin-2-yl)-acetamide;
N-[2-(5-Methyl-furan-2-yl)-6-thiazol-2-yl-pyrimidin-4-yl]-2-((S)-2-pyrrolidin-1-ylmethyl-pyrrolidin-1-yl)-acetamide;
2-[4-(3,4-Dimethyl-phenyl)-piperazin-1-yl]-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(4-pyrrolidin-1-yl-piperidin-1-yl)-acatemide;
2-[4-(3-Chloro-phenyl)-piperazin-1-yl]-N-[2-(5-methyl-furan-2-yl)-6-thiazol-2-yl-pyrimidin-4-yl]-acetamide;
2-(2,6-Dimethyl-morpholin-4-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(3-methyl-4-m-tolyl-piperazin-1-yl)-acetamide;
2-(4-Methyl-[1.4]diazepan-1-yl)-N-[2-(5-methyl-furan-2-yl)-6-thiazol-2 yl-pyrimidin-4-yl]-acetamide;
2-[4-(3-Methoxy-phenyl)-piperazin-1-yl]-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-[2-(2-piperidin-1-yl-ethyl)-piperidin-1-yl]-acetamide;
N-[2-(5-Methyl-furan-2-yl)-6-thiazol-2-yl-pyrimidin-4-yl]-2-[1,4]oxazepan-4-yl-acetamide;
2-(4,4-Difluoro-piperidin-1-yl)-N-[2-(5-methyl-furan-2-yl)-6-thiazol-2-yl-pyrimidin-4-yl]-acetamide:
2-(4-Acetyl-piperazin-1-yl)-N-[2-(5-methyl-furan-2-yl)thiazol-2-yl-pyrimidin-4-yl]-acetamide;
2-[(1-Benzyl-pyrrolidin-3-yl)-methyl-amino]-N-(2-(5-methyl-furan-2-yl)-6-thiazol-2-yl-pyrimidin-4-yl]-acetamide;
2-(4-Acetyl-[1,4]diazepan-1-yl)-N-[2-(5-methyl-furan-2-yl)-6-thiazol-2-yl-pyrimidin-4-yl]-acetamide,
2-(4-Benzyl-[1,4]diazepan-1-yl)-N-(2-(5-methyl-furan-2-yl)-6-thiazo-2-yl-pyrimidin-4-yl]-acetamide;
2-(3-Dimethylamino-pyrrolidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(3-trifluoromethyl-piperidin-1-yl)-acetamide;
2-(3-Diethylamino-pyrrolidin-1-yl)-N-[2-(5-metnyl-furan-2-yl)-6-thiazol-2-yl-pyrimidin-4-yl]-acetamide;
2-((R)-3-Dimethylamino-pyrrolidin-1-yl)-N-[2-(5-methyl-furan-2-yl)-6-thiazol-2-yl-pyrimidin-4-yl)-acetamide;
2-((S)-3-Dimethylamino-pyrrolidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(2-pyrrolidin-1-yl-ethylamino)-acetamide;
1-{[2-(5-Methyl-furan-2-yl)-6-thiazol-2-yl-pyrimidin-4-ylcarbamoyl]-methyl}-piperidine-3-carboxylic acid amide;
1-{[2-(5-Methyl-furan-2-yl)-6-thiazol-2-yl-pyrimidin-4-ylcarbamoyl]-methyl}-piperidine-4-carboxylic acid amide;
N-[2-(5-Methyl-furan-2-yl)-6-thiazol-2-yl-pyrimidin-4-yl]-2-((R)-3-methyl-piperazin-1-yl)-acetamide;
2-[4-(isopropylcarbamoyl-methyl)-piperazin-1-yl]-N-[2-(5-methyl-furan-2-yl)-6-thiazol-2-yl-pyrimidin-4-yl]-acetamide;
2-(4-Amino-piperidin-1-yl)-N-[2-(5-methyl-furan-2-yl)-6-thiazol-2-yl-pyrimidin-4-yl]-acetamide;
2-(4-Dimethylamino-piperidin-1-yl)-N-[2-(5-methyl-furan-2-yl)-6-thiazol-2-yl-pyrimidin-4-yl]-acetamide;
2-(4-Diethylamino-piperidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(4-morpholin4-yl-piperidin-1-yl)-acetamide;
2-(4-Isopropylamino-piperidin-1-yl)-N-[2-(5-methyl-furan-2-yl)-6-thiazol-2-yl-pyrimidin-4-yl]-acetamide;
2-(4-Cyclopentylamino-piperidin-1-yl)-N-[2-(5-methyl-furan-2-yl)-6-thiazol-2-yl-pyrimidin-4-yl]-acetamide;
2-(4-Dipropylamino-piperidin-1-yl)-N-[2-(5-methylt-furan-2-yl)-6-thiazol-2-yl-pyrimidin-4-yl]-acetamide;
2-(3-Amino-pyrrolidin-1-yl)-N-[2-(5-methyl-furan-2-yl)-6-thiazol-2-yl-pyrimidin-4-yl]-acetamide;
2-(3-Isopropylamino-pyrrolidin-1-yl)-N-[2-(5-Methyl-furan-2-yl)-6-thiazol-2-yl-pyrimidin-4-yl]-acetamide;
2-(3-Cyclopentylamino-pyrrolidin-1-yl)-N-[2 (5-methyl-furan-2-yl)-6-thiazol-2-yl-pyrimidin-4-yl]-acetamide;
2-(4-Acetylamino-piperidin-1-yl)-N-[2-(5-methyl-furan-2-yl)-6-thiazol-2-yl-pyrimidin-4-yl]-acetamide;
N-(1-{[2-(5-Methyl-furan-2-yl)-6-thiazol-2-yl-pyrimidin-4-ylcarbamoyl]-methyl}-piperidin-4-yl)-propionamide;
2-(3-Acetylamino-pyrrolidin-1-yl)-N-[2-(5-methyl-furan-2-yl)-6-thiazol-2-yl-Pyrimidin-4-yl-acetamide;
N-(1-{[2-(5-Methyl-furan-2 yl)-6-thiazol-2-yl-pyrimidin-4-ylcarbamoyl]-methyl}-pyrrolidin-3-yl)-propionamide;
N-[2-(5-Methyl-furan-2-yl)-6-thiazol-2-yl-pyrimidin-4-yl]-2-(4-thiazol-2-yl-piperazin-1-yl)-acetamide;
2-(Hexahydro-pyrrolo[1,2-a]pyrazin-2-yl)-N-[2-(5-methyl-furan-2 yl)-6-thiazol-2-yl-pyrimidin-4-yl]-acetamide;
N-[2-(5-Methyl-furan-2-yl)-6-thiazol-2-yl-pyrimidin-4-yl]-2-(3-piperidin-1-yl-pyrrolidin-1-yl)-acetamide;
N-(2-(5-Methyl-furan-2-yl)-6-thiaezol-2-yl-pyrimidin-4-yl]-2-(3-morpholin-4-yl-pyrrolidin-1-yl)-acetamide:
N-(2-(5-Methyl-furan-2-yl)-6-thiazol-2-yl-pyrimidin-4-yl]-2-(2-methyl-3-oxo-piperazin-1-yl)-acetamide;
1-{[2-(5-Methyl-furan-2-yl)-6-thiazol-2-yl-pyrimidin-4-ylcarbamoyl]-methyl}-piperidine-3-carboxylic acid ethyl ester;
1-{[2-(5-Methyl-furan-2-yl)-6-thiazol-2-yl-pyrimidin-4-ylcarbamoyl]-methyl}-piperidine-3-carboxylic acid (2-hydroxy-ethyl)-amide;
2-((S)-3-Ethylamino-pyrrolidin-1-yl)-N-[2-(5-methyl-furan-2-yl)-6-thiazol-2-yl-pyrimidin-4-yl-acelamide;
2-((R)-3-Ethylamino-pyrrolidin-1-yl)-N-[2-(5-methyl-furan-2-yl)-6-thiazol-2-yl-pyrimidin-4-yl]-acetamide;
2-(4-Ethyl-piperazin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-(2-piperidin-1-yl-ethylamino)-acetamide;
N-[2-(5-Methyl-furan-2-yl)-6-thiazol-2-yl-pyrimidin-4-yl]-2-[methyl-(1-methyl-piperidin-4-yl)-amino]-acetamide;
N-[2-(5-Methyl-furan-2-yl)-6-thiazol-2-yl-pyrimidin-4-yl]-2-[2-(1-methyl-pyrrolidin-2-yl)-ethylamino]-acetamide;
N-[2-(5-Methyl-furan-2-yl)-6-thiazol-2-yl-pyrimidin-4-yl]-2-(pyrrolidin-3-ylamino)-acetamide;
N-[2-(5-Methyl-furan-2-yl-6-thiazol-2-yl-pyrimidin-4-yl]-2-(3-pyrrolidin-1-yl-propylamino)-acetamide;
N-[2-(5-Methyl-furan-2-yl)-6-thiazol-2-yl-pyrimidin-4-yl]-2-(3-piperidin-1-yl-propylamino)-acetamide;
N-[2-(5-Methyl-furan-2-yl)-6-thiazol-2-yl-pyrimidin-4-yl]-2-(3-morpholin-4-yl-propylemino)-acetamide:
2-(4-Hydroxy-piperidin-1-yl)-N-[2-(5-methyl-furan-2-yl)-6-thiazol-2-yl-pyrimidin-4-yl]-acetamide;
2-(3-Hydroxy-piperidin-1-yl)-N-[2-(5-methyl-furan-2-yl}6-thiazol-2-yl-pyrimidin-4-yl]-acetamide;
2-((S)-3-Hydroxy-pyrrolidin-1-yl)-N-[2-(5-methyl-furan-2-yl)-6-thiazal-2-yl-pyrimidin-4-yl]-acetamide:
2-((S)3-Acetylamino-pyrrelidin-1-yl)-N-[2-(5-methyl-furan-2-yl)-6-thiazol-2-yl-pyrimidin-4-yl]-acetamide;
2-((R)-3-Acetylamino-pyrrolidin-1-yl)-N-[2-(5-methyl-furan-2-yl)-6-thiarzol-2-yl-pyrimidin-4-yl]-acetamide:
2-(3-Hydroxy-pyrrolidin-1-yl)-N-{2-(5-methyl-furan-2-yl)-6-thiazol-2-yl-pyrimidin-4-yl]-acetamide;
2-(4-Isopropyl-piperazin-1-yl)-N-[2-(5-Methyl-furan-2-yl)-6-thiazol-2-yl-pyrimidin-4-yl]-acetamide;
2-(4-Cyclopentyl-piperazin-1-yl)-N-[2-(5-methyl-furan-2-yl)-6-thiazol-2-yl-pyrimidin-4-yl]-acetamide;
2-(4-Cyclohexyl-piperazin-1-yl)-N-[2-(5-methyl-furan-2-yl)-6-thiazol-2-yl-pyrimidin-4-yl]-acetamide;
N-(2-(5-Methyl-furan-2-yl)-6-thiazol-2-yl-pyrimidin-4-yl]-2-(4-propionyl-piperazin-1-yl)-acetamide;
2-(4-Isobutyryl-piperazin-1-yl)-N-[2-(5-methyl-furan-2-yl)-6-thiazol-2-yl-pyrimidin-4-yl]-acetamide;
2-(4-Aminomethyl-piperidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-[(pyrrimidin-3-ylmethyl)-amino]-acetamide;
2-(3-Aminomethyl-piperidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-[(1-methyl-pyrrolidin-3-ylmethyl)-amino]-acetamide;
2-(3-Aminomethyl-pyrrolidin-1-yl)-N-[2-(5-methyl-furan-2-yl)-6-thiazol-2-yl-pyrimidin-4-yl]-acetamide;
2-{4-Methoxy-piperidin-1-yl)-N-[2-(5-methyl-furan-2-yl)-6-thiazol-2-yl-pyrimidin-4-yl]-acetamide;;
2-(3-Dimethylaminomethyl-piperidin-1-yl)-N-[2-(5-methyl-furan-2-yl)-6-thiazol-2-yl-pyrimidin-4-yl]-acetamide;
2-(4-Dimethylaminomethyl-piperidin-1-yl)-N-[2-(5-methyl-furan-2-yl)-6-thiazol-2-yl-pyrimidin-4-yl]-acetamlde;
N-[2-(5-Methyl-furan-2-yl)-6-thiazol-2-yl-pyrimidin-4-yl]-2-[methyl-(1-methyl-pyrrolidin-3-ylmethyl)-amino]-acetamide;
2-(3-Dimethylaminomethyl-pyrrolidin-1-yl)-N-[2-(5-methyl-furan-2-yl)-6-thiazol-2-yl-pyrimidin-4-yl]-acetamide;
N-[2-(5-Methyl-furan-2-yl)-6-thiazol-2-yl-pyrimidin-4-yl]-2-morpholin-4-yl-acetamide;
N-[2-(5-Methyl-furan-2-yl)-6-thiazol-2-yl-pyrimidin-4-yl]-2-(4-phenyl-piperidin-1-yl)-acetamide:
2-(2,5-Dimethyl-piperazin-1-yl)-N-[2-(5-methyl-furan-2-yl-6-thiazol-2-yl-pyrimidin-4-yl]-acetamide;
N-[2-(5-Methyl-furan-2-yl)-6-thiazol-2-ylt-pyrimidin-4-yl]-2-piperazin-1-yl-acetamide;
2-[1,4]Diazepan-1-yl-N-[2-(5-methyl-furan-2-yl)-6-thiazol-2-yl-pyrimidin-4-yl]-acetamide;
3-(((S)-3-Dimethylamino-pyrrolidin-1-yl)-N-[2-(5-methyl-furan-2-yl)-6-thiazol-2-yl-pyrimidin-4-yl]-propionamide;
3-(4-Acetyl-piperazin-1-yl)-N-[2-(5-methyl-furan-2-yl)-6-thiazol-2-yl-pyrimidin-4-yl]-propionamide;
N-[2-(5-Methyl-furan-1-yl)-6-thiazol-2-yl-pyrimidin-4-yl]-3-(4-methyl-piperazin-1-yl)-propionamide;
2-(5-Methyl-furan-2-yl)-6-(3-piperazin-1-yl-propionylamino)-N-vinyl-pyrimidine-4-carboximidothioic acid methyl ester,
N-[2-(5-Methyl-furan-2-yl)-6-thiazol-2-yl-pyrimidin-4-yl]-3-(4-pyrrolidin-1-yl-piperidin-1-yl)-propionamide;
N-[2-(5-Methyl-furan-2-yl)-6-thiazol-2-yl-pyrimidin-4-yl]-3-morpholin-4-yl-propionamide;
3-((R)-3-Dimethylamino-pyrrolidin-1-yl)-N-[2-(5-methyl-furan-2-yl)-6-thiazol-2-yl-pyrimidin-4-yl]-propionamide;
3-(4-Acetyl-[1,4]diazepan-1-yl)-N-[2-(5-methyl-furan-2-yl)-6-thiazol-2-yl-pyrimidin-4-yl]-propionamide;
N-[2-(5-Methyl-furan-2-yl)-6-thiazol-2-yl-pyrimidin-4-yl]-3-[1,4]oxazepan-4-yl-propionamide;
3-(4-Methyl-[1,4]diazepan-1-yl)-N-[2-(5-methyl-furan-2-yl)-6-thiazol-2-yl-pyrimidin-4-yl]-propionamide:
3-((R)-3-Ethylamino-pyrrolidin-1-yl)-N-[2-(5-methyl-furan-2-yl)-6-thiazol-2-yl-pyrimidin-4-yl]-propionamide;
3-(3,5-Dimethyl-piperazin-1-yl)-N-[2-(5-methyl-furan-2-yl)-6-thiazol-2-yl-pyrimidin-4-yl]-propionamide;
3-[1,4]Diazepen-1-yl-N-[2-(5-methyl-furan-2-yl)-6-thiazol-2-yl-pyrimidin-4-yl]-propionamide;
N-[2-(5-Methyl-furan-2-yl)-6-thiazol-2-yl-pyrimidin-4 yl]-2-(1-methyl-piperidin-4-yl)-acetamide; .
N-[2-(5-Methyl-furan-2-yl)-6-thiazol-2-yl-pyimidin-4-yl]-2-piperidin-4-yl-acetamide;
N-[2-(5-Methyl-furan-2-yl)-6-thiazol-2-yl-pyrimidin-4-yl]-2-(2-pyrrolidin-1-yl-ethoxy)-acetamide;
N-[6-(3,5-Dimethyl-pyrazol-1-yl)-2-(4-methoxy pyridin-3-yl)-pyrimidin-4-yl]-2-(4-methyl-piperazin-1-yl)-acetamide;
N-[6-(3,5-Dimethyl-pyrazol-1-yl)-2-(6-methoxy-pyridin-3-yl)-pyrimidin-4-yl]-2-(4-methyl-piperazin-1-yl)-acetamide;
N-[2-(3,4-Dimethoxy-phenyl)-6-(3,5-dimethyl-pyrazol-1-yl)-pyrimidin-4-yl]-2-(4-methyl-piperazin-1-yl)-acetamide;
N-[2-(4-Trifuoromethoxy-phenyl)-6-(3,5-dimethyl-pyrazol-1-yl)-pyrimidin-4-yl]-2-(4-methyl-piperazin-1-yl)-acetamide;
N-[2-(3-Fluoro-phenyl)-6-(3,5-dimethyl-pyrazol-1-yl)-pyrimidin-4-yl]-2-(4-methyl-piperazin-1-yl)-acetamide;
N-[2-(2-Fluoro-phenyl)-6-(3,5-dimethyl-pyrazol-1-yl)-pyrimidin-4-yl]-2-(4-methyl-piperazin-1-yl)-acetamide;
N-[2-(2-Fluoro-3-methoxy-phenyl)-6-(3,5-dimethyl-pyrazol-1-yl)-pyrimidin-4-yl]-2(4-methyl-piperazin-1-yl)-acetamide;
N-[2-(2,4-Dimethoxy-phenyl)-6-(3,5-dimethyl-pyrazol-1-yl)-pyrimidin-4-yl]-2-(4-methyl-piperazin-1-yl)acetamide;
N-[2-(2-Cyano-phenyl)-6(3,5-dimethyl-pyrazol-1-yl)-pyrimidin-4-yl]-2-(4-methyl-piperazin-1-yl)-acetamide;
N-[2-(2-Methoxy-phenyl)-6-(3,5-dimethyl-pyrazol-1-yl)-pyrimidin-4-yl]-2-(4-methyl-piperazin-1-yl)-acetamide;
N-[2-(4-Fluoro-2-methyl-phenyl)-6-(3,5-dimethyl-pyrazol-1-yl)-pyrimidin-4-yl]-2-(4-methyl-piperazin-1-yl)-acetamide;
N-[2-(3-Methoxy-phenyl)-6-(3,5-dimethyl-pyrazol-1-yl)-pyrimidin4-yl]-2-(4-methyl-piperazin-1-yl)-acetamide;
N-[2-(3,5-Dimethoxy-phenyl)-6-(3,5-dimethyl-pyrazol-1-yl)pyrimidin-4-yl]-2-(4-methyl-piparazin-1-yl)-acetamide;
N-[2-(3,5-Difluoro-phenyl)6-(3,5-dimethyl-pyrazol-1-yl)pyrimidin-4-yl]-2-(4-methyl-piperazin-1-yl)-acetamide;
N-[2-(3-Fluoro-4-methyl-phenyl)-6-(3,5-dimethyl-pyrazol-1-yl)-pyrimidin-4-yl]-2-(4-methyl-piperazin-1-yl)-acetamide;
N-[2-(3-Fluoro-2-methoxy-phenyl)-6-(3,5-dimethyl-pyrazol-1-yl)-pyrimidin-4-yl]-2-(4-methyl-piperazin-1-yl)acetamide;
N-[2-(3-Fluoro-4-methoxy-phenyl)-6-(3,5-dimethyl-pyrazol-1-yl)-pyrimidin-4-yl]-2-(4-methyl-piperazin-1-yl)-acetamide;
N-(2-(2,3-Difluoro-phenyl)-6-(3,5-dimethyl-pyrazol-1-yl)-pyrimidin-4-yl]-2-(4-methyl-piperazin-1-yl)-acetamide;
N-[2-(5-Methoxy-pyridin-3-yl)-6-(3,5-dimethyl-pyrazol-1-yl)-pyrimidin-4-yl]-2-(4-methyl-piperazin-1-yl)-acetamide;
N-[2-(3-Methoxy-phenyl)-6-pyrazol-1-yl-pyrimidin-4-yl]-2-(4-methyl-piperazin-1-yl)-acetamide;
N-[2-(3,4-Dimethoxy-phenyl)-6-pyrazol-1-yl-pyrimidin-4-yl]-2-(4-methyl-piperazin-1-yl)-acetamide;
N-[2-(3-Cyano-phenyl)-6-pyrazol-1-yl-pyrimidin-4-yl]-2-(4-methyl-piperazin-1-yl)-acetamide;
N-[2-(3-Flouro-4-Methoxy-phenyl)-6-pyrazol-1-yl-pyrimidin-4-yl]-2-(4-methyl-piperazin-1-yl)-acetamide;
N-[2-(2-Methoxy-phenyl)-6-pyrazol-1-yl-pyrimidin-4-yl]-2-(4-methyl-piperazin-1-yl)-acetamide;
N-[2-(4-Methoxy-phenyl-6-pyrazol-1-yl-pyrimidin-4-yl]-2-(4-methyl-piperazin-1-yl)-acetamide;
N-[2-(3-Trifluoromethyl-phenyl)-6-pyrazol-1-yl-pyrimidin-4-yl]-2-(4-methyl-piperazin-1-yl)-acetamide:
N-[2-(2,3-Difluoro-phenyl)-6-pyrazol-1-yl-pyrimidin-4-yl]-2-(4-methyl-piperazin-1-yl)-acetamide;
N-[2-(3-Trifluoromethoxy-phenyl)-6-pyrazol-1-yl-pyrimidin-4-yl]-2-(4-methyl-piperazin-1-yl)-acetamide;
N-[2-(2-Fluoro-3-methoxy-phenyl)-6-pyrazol-1-yl-pyrimidin-4-yl]-2-(4-methyl-piperazin-1-yl)-acetamide;
N-[2-(5-Methyl-furan-2-yl)-pyridin-2yl-pyrimidin-4-yl]-2-morpholin-4-yl-acetamide;
N-[2-(5-Methyl-furan-2-yl)-6-pyridin-2-yl-pyrimidin-4-yl]-2-piperidin-1-yl-acetamide;
2-[4-(Isopropylcarbanoyl-methyl)-piperazin-1-yl]-N-[2-(5-methyl-furan-2-yl)-6-pyridin-2-yl-pyrimidin-4-yl]-acetamide;
N-[2-(5-Methyl-furan-2-yl)-6-pyridin-2-yl-pyrimidin-4-yl]-2-(4-phenyl-piperidin-1-yl) acetamide;
2-[1,4]Bipiperidinyl-1-yl-N-[2-(5methyl-furan-2yl)-6-pyridin-2-yl-pyrimidin-4-yl]-acetamide;
2-(3,4-Dihydro-1H-isoquinolin-2-yl)-N-[2-(5-methyl-furan-2-yl)-6-pyridin-2-yl-pyrimidin-4-yl]-acetamide;
2-[4-(3-Methoxy-phenyl)-piperazin-1-yl]-N-[2-(5-methyl-furan-2-yl)-6-pyridin-2-yl-pyrimidin-4-yl]-acetamide;
N-[2-(5-Methyl-furan-2-yl)-6-pyridin-2-yl-pyrimidin-4-yl]-2-(4-methyl-piperazin-1-yl)-acetamide;
2-((R)-3-Dimethylamino-pyrrolidin-1-yl)-N-(2-pyridin-2-yl-6-thiazol-2-yl-pyrimidin-4-yl)-acetamide;
2-((S)3-Dimethylamino-pyrrolidin-1-yl)-N-(2-pyridin-2-yl-6-thiazol-2-yl-pyrimidin-4-yl)-acetamide;
2-(2,5-Dimethyl-piperazin-1-yl)-N-(2-pyridin-2-yl-6-thiazol-2-yl-pyrimidin-4-yl)-acetamide;
2-(3,5-Dimethyl-piperazin-1-yl)-N-(2-pyridin-2-yl-6-thiazol-2-yl-pyrimidin-4-yl)-acetamide:
2-(4-Phenyl-piperazin-1-yl)-N-(2-pyridin-2-yl-6-thiazol-2-yl-pyrimidin-4-yl)-acetamide;
2-[4-(2-Methoxy-phenyl)-piperazin-1-yl]-N-(2-pyridin-2-yl-6-thiazol-2-yl-pyrimidin-4-yl)-acetamide;
2-(4-Methyl-piperazin-1-yl)-N-(2-pyridin-2-yl-6-thiazol-2-yl-pyrimidin-4-yl)-acetamide;
2-(4-Benzyl-piperazin-1-yl)-N-(2-pyridin-2-yl-6-thiazol-2-yl-pyrimidin-4-yl)-acetamide;
2-Morpholin-4-yl-N-(2-pyridin-2-yl-6-thiazol-2-yl-pyrimidin-4-yl)-acetamide;
2-(2,6-Dimethyl-morpholin-4-yl)-N-(2-pyridin-2-yl-6-thiazol-2-yl-pyrimidin-4-yl)-acetamide;
2-Piperidin-1-yl-N-(2-pyridin-2-yl-6-thiazol-2-yl-pyrimidin-4-yl)-acetamide;
2-(2-Methyl-piperidin-1-yl)-N-(2-pyridin-2-yl-6-thiazol-2-yl-pyrimidin-4-yl)-acetamide:
2-(3-Methyl-piperidin-1-yl)N-(2-pyridin-2-yl-6-thiazol-2-yl-pyrimidin-4-yl)-acetamide;
2-(3,3-Dimethyl-piperidin-1-yl)-N-(2-pyridin-2-yl-6-thiazol-2-yl-pyrimidin-4-yl)-acetamide;
2-(3.5-Dimethyl-piperidin-1-yl)-N-(2-pyridin-2-yl-6-thiazol-2-yl-pyrimidin-4-yl)-acetamide;
2-(4-Methyl-piperidin-1-yl)-N-2-pyridin-2-yl-6thiazol-2-yl-pyrimidin-4-yl)-acetamide:
2-(4-Benzyl-piperidin-1-yl)-N-(2-pyridin-2-yl-6-thiazol-2-yl-pyrimidin-4-yl)-acetamide;
2-[1,4']Bipiperidinyl-1'-yl-N-(2-pyridin2-yl-6-thiazol-2-yl-pyrimidin-4-yl)-acetamide;
2-((S)-2-Methoxymethyl-pyrrolidin-1-yl)-N-(2-pyridin-2-yl-6-thiazol-2-yl-pyrimidin+yl)-acetamide;
2-(Octahydro-isoquinolin-2-yl)-N-(2-pyridin-2-yl-6-thiazol-2-yl-pyrimidin-4-yl)-acetamide;
N-(2-Pyridin-2-yl-6-thiazol-2-yl-pyrimidin-4-yl)-2-((S)-2-pyrrolidin-1-ylmethyl-pyrrolidin-1-yl)-acetamide:
2-[4-(3,4-Dimethyl-phenyl)-piperazin-1-yl]-N-(2-pyridin-2-yl-6-thiazol-2-yl-pyrimidin-4-yl)-acetamide:
N-(2-Pyridin-2-yl-6-thiazol-2-yl-pyrimidin-4-yl)-2-(4-pyrrolidin-1-yl-piperidin-1-yl)-acetamide;
2-[4-(3-Chloro-phenyl)piperazin-1-yl]-N-(2-pyridin-2-yl-6-thiazol-2-yl-pyrimidin-4-yl)-acetamide;
2-[4-(3-Methoxy-phenyl)-piperazin-1-yl]-N-(2-pyridin-2-yl-6-thiazol-2-yl-pyrimidin-4-yl)-acetamide;
2-[4-(4-Methoxy-phenyl)-piperazin-1-yl]-N-(2-pyridin-2-yl-6-thiazol-2-yl-pyrimidin-4-yl)-acetamide;
2-(4-Acetyl-piperazin-1-yl)-N-(2-pyridin-2-yl-6-thiazol-2-yl-pyrimidin-4-yl)-acetamide;
2-(4-Methyl-[1,4]diazepan-1-yl)-N-(2-pyridin-2-yl-6-thiazol-2-yl-pyrimidin-4-yl)acetamide;
2-[2-((S)-1-Methylpyrrolidin-2-ylmethyl)-piperidin-1-yl]-N-(2-pyridin-2-yl-6-thiazol-2-yl-pyrimidin-4-yl)-acetamide;
2-[2-(2-Piperidin-1-yl-ethyl)-piperidin-1-yl]-N-(2-pyridin-2-yl-6-thiazol-2-yl-pyrimidin-4-yl)-acetamide:
2-((R)-2-Methyl-piperanzin-1-yl)-N(2-pyridin-2-yl-6-thiazol-2-yl-pyrimidin-4-yl)acetamide;
2-(2,5-Dihydro-pyrrol-1-yl)-N-(2-pyridin-2-yl-6-thiazol-2-yl-pyrimidin-4-yl)-acetamide;
2-(4-Acetyl-[1,4]diazepan-1-yl)-N-(2-pyridin-2-yl-6-thiazol-2-yl-pyrimidin-4-yl)-acetamide;
2-(3-Dimethylamino-pyrrolidin-1-yl)-N-(2-pyridin-2-yl-6-thiazol-2-yl-pyrimidin-4-yl)-acetamide;
N-(2-Pyridin-2-yl-6-thiazol-2-yl-pyrimidin-4-yl)-2-(3-trifluoromethyl-piperidin-1-yl)-acetamide;
2-(3-Diethylamino-pyrrolidin-1-yl)-N-(2-pyridin-2-yl-6-thiazol-2-yl-pyrimidin-4-yl)-acetamide;
2-((R)-2-Methoxymethyl-pyrrolidin-1-yl)-N-(2-pyridin-2-yl-6-thiazol-2-yl-pyrimidin-4-yl)-acetamide;
2-(2,5-Dimethyl-2,5-dihydro-pyrrol-1yl)-N-(2-pyridin-2-yl-6-thiazol-2-yl-pyrimidin-4-yl)-acetamide;
N-(2-Pyridin-2-yl-6-thiazol-2-yl-pyrimidin-4-yl)-2-pyrrolidin-1-yl-acetamide;
2-(2,5-Dimethyl-pyrrolidin-1-yl)-N-(2-pyridin-2-yl-6-thiazol-2-yl-pyrimidin-4-yl)acetamide;
2-(3-Phenyl-piperidin-1-yl)-N-(2-pyridin-2-yl-6-thiazol-2-yl-pyrimidin-4-yl)acetamide;
2-(2-Phenyl-piperidin-1-yl)-N-(2-pyridin-2-yl-6-thiazol-2-yl-pyrimidin-4-yl)-acetamide;
2-[1,4]Oxazepan-4-yl-N-(2-pyridin-2-yl-6-thiazol-2-yl-pyrimidin-4-yl)-acetamide:
2-(4,4-Difluoro-piperidin-1-yl)-N-(2-pyridin-2-yl-6-thiazol-2-yl-pyrimidin-4-yl)-acetamide;
2-(4-Phenyl-piperidin-1-yl)-N-(2-pyridin-2-yl-6-thiazol-2-yl-pyrimidin-4-yl)-acetamide;
2-Piperazin-1-yl-N-(2-pyridin-2-yl-6-thiazol-2-yl-pyrimidin-4-yl)-acetamide;
2-[4-(Isopropylcarbamoyl-methyl)-piperazin-1-yl]-N-(2-pyridin-2-yl-6-thiazol-2-yl-pyrimidin-4-yl)-acetamide;
1-[(2-Pyridin-2-yl-6-thiazol-2-yl-pyrimidin-4-ylcarbamoyl)methyl]-piperidine-3-carboxylic acid amide;
2-[1,4]Diazepan-1-yl-N-(2-pyridin-2-yl-6-thiazol-2-yl-pyrimidin-4-yl)-acetamide;
2-[Methyl-(2-pyrrolidin-1-yl-ethyl)-amino]-N-(2-pyridin-2-yl-6-thiazol-2-yl-pyrimidin-4-yl)-acetamide;
2-(3,5-Dimethyl-piperazin-1-yl)-N-2-pyridin-2-yl-6-thiazol-2-yl-pyrimidin-4-yl)-acetamide;
2-(4-Acetyl-piperazin-1-yl)-N-(2-pyridin-2-yl-6-thiazol-2-yl-pyrimidin4-yl)-acetamide;
2-[4-(3-Chloro-phenyl)-piperazin-1-yl]-N-(2-pyridin-2-yl-6-thiazol-2-yl-pyrimidin-4-yl)-acetamide;
2-Morpholin-4-yl-N-(2-pyridin-2-yl-6-thiazol-2-yl-pyrimidin-4-yl)-acetamide;
2-Piperidin-1-yl-N-(2-pyridin-2-yl-6-thiazol-2-yl-pyrimidin-4-yl)-acetamide;
2-(4-Methyl-piperazin-1-yl)-N-(6-pyridin-2-yl-2-thiophen-2-yl-pyrimidin-4-yl)-acetamide;
2-Morpholin-4-yl-N-(6-pyridin-2-yl-2-thiophen-2-yl-pyrimidin-4-yl)-acetamide;
N-(6-Pyridin-2-yl-2-thiophen-2-yl-pyrimidin-4-yl)-2-pyrrolidin-1-yl-acetamide;
2-[Methyl-(1-methyl-piperidin-4-yl)-amino]-N-(6-pyridin-2-yl-2-thiophen-2-yl-pyrimidin-4-yl)-acetamide;
2-[1,4]Diazepan-1-yl-N-(6-pyridin-2-yl-2-thiophen-2-yl-pyrimidin-4-yl)-acetamide;
2-(4-Methyl-[1,4]diazepan-1-yl)-N-(6-pyridin-2-yl-2-thiophen-2-yl-pyrimidin-4-yl)-acetamide;
2-(4-Ethyl-[1,4]diazepan-1-yl)-N-(6-pyridin-2-yl-2-thiophen-2-yl-pyrimidin-4-yl)-acetamide;
2-(4-Propyl-[1,4]diazepan-1-yl)-N-(6-pyridin-2yl-2-thiophen-2-yl-pyrimidin-4-yl)-acetamide;
2-(4-Amino-piperidin-1-yl)-N-(6-pyridin-2-yl-2-thiophen-2-yl-pyrimidin-4-yl)-acetamide;
2-(4-Dimethylamino-piperidin-1-yl)-N-(6-pyridin-2-yl-2-thiophen-2-yl-pyrimidin-4-yl)-acetamide;
2-(4-Diethylamino-piperidin-1-yl)-N-(6-pyridin-2-yl-2-thiophen-2-yl-pyrimidin4-yl)-acetamide;
2-(4-Dipropylamino-piperidin-1-yl)-N-(6-pyridin-2-yl-2-thiophen-2-yl-pyrimidin-4-yl)-acetamide;
2-(4-Acetylamino-piperidin-1-yl)-N-(6-pyridin-2-yl-2-thiophen-2-yl-pyrimidin-4-yl)-acetamide;
N-{1-[(6-Pyridin-2-yl-2-thiophen-2-yl-pyrimidin-4-ylcarbamoyl)-methyl]-piperidin-4-yl}-propionamide;
N-(6-Pyridin-2-yl-2-thiophen-2-yl-pyrimidin-4-yl)-2-(4-pyrrolidin-1-yl-piperidin-1-yl)-acetamide;
2-(4-Morpholin-4-yl-piperidin-1-yl)-N-(6-pyridin-2-yl-2-thiophen-2-yl-pyrimidin-4-yl)-acetamide;
2-[1,4']Bipiperidinyl-1'-yl-N-(6-pyridin-2-yl-2-thiophen-2-yl-pyrimidin-4-yl)-acetamide;
2-[4-(2-Oxo-imidazolidin-1-yl)-piperidin-1-yl]-N-(6-pyridin-2-yl-2-thiophen-2-yl-pyrimidin-4-yl)-acetamide;
2-(4-Acetimidoylamino-piperidin-1-yl)-N-(6-pyridin-2-yl-2-thiophen-2-yl-pyrimidin-4-yl)-acetamide;
2-(4-Azetidin-1-yl-piperidin-1-yl)-N-(6-pyridin-2-yl-2-thiophen-2-yl-pyrimidin-4-yl)-acetamide;
N-(2-Furan-2-yl-6-thiazol-2-yl-pyrimidin-4-yl)-2-(4-methyl-piperazin-1-yl)-acetamide;
2-Piperidin-1-yl-N-(6-thiazol-2-yl-2-thiophen-2-yl-pyrimidin-4-yl)-acetemide;
2-(2-Pyrrolidin-1-ylmethyl-piperidin-1-yl)-N-(6-thiazol-2-yl-2-thiophen-2-yl-pyrimidin-4-yl)-acatemide;
2-[2-(2-Piperidin-1-yl-ethyl)-piperidin-1-yl]-N-(6-thiazol-2-yl-2-thiophen-2-yl-pyrimidin-4-yl)-acetamide;
2-(3-Methyl-piperidin-1-yl)-N-(6-thiazol-2-yl-2-thiophen-2-yl-pyrimidin-4-yl)-acetamide;
N-(6-Thiazol-2-yl-2-thiophen-2-yl-pyrimidin-4-yl)-2-(3-trifluoromethyl-piperidin-1-yl)-acetamide;
1-[(6-Thiazol-2-yl-2-thiophen-2-yl-pyrimidin-4-ylcarbamoyl)-methyl]-piperidine-3-carboxylic acid amide;
2-[1,4']Bipiperidinyl-1-yl-N-(6-thiazol-2-yl-2-thiophen-2-yl-pyrimidin-4-yl)-acetamide;
2-[1,4]Diazepan-1-yl-N-(6-thiazol-2-yl-2-thiophen-2-yl-pyrimidin-4-yl)-acetamide;
2-(4-Acetyl-[1,4]diazepan-1-yl)-N-(6-thiazol-2-yl-2-thiophen-2-yl-pyrimidin-4-yl)-acetamide;
2-(4-Pyrrolidin-1-yl-piperidin-1-yl)-N-(6-thiazol-2-yl-2-thiophen-2-yl-pyrimidin-4-yl)-acetamide;
2-Piperazin-1-yl-N-(thiazol-2-yl-2-thiophen-2-yl-pyrimidin-4-yl)-acetamide;
2-(2,5-Dimethyl-piperazin-1-yl)-N-(6-thiazol-2-yl-2-thiophen-2-yl-pyrimidin-4-yl)-acetamide;
2-(3,5-Dimethyl-piperazin-1-yl)-N-(6-thiazol-2-yl-2-thiophen-2-yl-pyrimidin-4-yl)-acetamide;
2-(4-Acetyl-piperazin-1-yl)-N-(6-{1-[(E)-methylimino]-ethyl}-2-thiophen-2-yl-pyrimidin-4-yl)-acetamide;
2-(4-Phenyl-piperazin-1-yl)-N-(6-thiazol-2-yl-2-thiophen-2-yl-pyrimidin-4-yl)-acetamide; N-Isopropyl-2-{4-[(6-thiazol-2-yl-2-thiophen-2-yl-pyrimidin-4-ylcarbamoyl)-methyl]-piperazin-1-yl}-acetamide;
2-Morpholin-4-yl-N-(6-thiazol-2-yl-2-thiophen-2-yl-pyrimidin-4-yl)-acetamide;
2-(2,6-Dimethyl-morpholin-4-yl)-N-(6-thiazol-2-yl-2-thiophen-2-yl-pyrimidin-4-yl)-acetamide;
N-(6-Thiazol-2-yl-2-thiophen-2-yl-pyrimidin-4-yl)-2-thiomorpholin-4-yl-acetamide;
2-Pyrrolidin-1-yl-N-(6-thiazol-2-yl-2-thiophen-2-yl-pyrimidin-4-yl)-acetamide;
2-(3-Dimethylamino-pyrrolidin-1-yl)-N-(6-thiazol-2-yl-2-thiophen-2-yl-pyrimidin-4-yl)-acetamide;
2-(3-Diethylamino-pyrrolidin-1-yl)-N-(6-thiazol-2-yl-2-thiophen-2-yl-pyrimidin-4-yl)-acetamide;
2-[2-(1-Methyl-pyrrolidin-2-yl)-ethylamino]-N-(6-thiazol-2-yl-2-thiophen-2-yl-pyrimidin-4-yl)-acetamide;
2-(3,5-Dimethyl-piperidin-1-yl)-N-(6-thiazol-2-yl-2-thiophen-2-yl-pyrimidin-4-yl)-acetamide;
2-(4,4-Difluoro-piperidin-1-yl)-N-(6-thiazol-2-yl-2-thiophen-2-yl-pyrimidin-4-yl)-acetamide;
2-(4-Phenyl-piperidin-1-yl)-N-(6-thiazol-2-yl-2-thiophen-2-yl-pyrimidin-4-yl)acetamide;
2-Azepan-1-yl-N-(6-thiazol-2-yl-2-thiophen-2-yl-pyrimidin-4-yl)-acetamide;
1-[(6-Thiazol-2-yl-2-thiophen-2-yl-pyrimidin-4-ylcarbamoyl)-methyl]-piperidine-4-carboxylic acid amide;
2-[4-(3-Methoxy-phenyl)-piperazin-1-yl]-N-(6-thiazol-2-yl-2-thiophen-2-yl-pyrimidin-4-yl)-acetamide;
2-((R)-3-Dimethylamino-pyrrolidin-1-yl)-N-(6-thiazol-2-yl-2-thiophen-2-yl-pyrimidin-4-yl)-acetamide;
2-((S)-3-Dimethylamino-pyrrolidin-1-yl)-N-(6-thiazol-2-yl-2-thiophen-2-yl-pyrimidin-4-yl)-acetamide;
2-[(1-Ethyl-pyrrolidin-2-ylmethyl)-amino]-N-(6-thiazol-2-yl-2-thiophen-2-yl-pyrimidin-4-yl)-acetamide;
2-((R)-3-Ethylamino-pyrrolidin-1-yl)-N-(6-thiazol-2-yl-2-thiophen-2-yl-pyrimidin-4-yl)-acetamide;
2-((S)-3-Ethylamino-pyrrolidin-1-yl)-N-(6-thiazol-2-yl-2-thiophen-2-yl-pyrimidin-4-yl)-acetamide;
2-(4-Methyl-piperazin-1-yl)-N-(6-thiazol-2-yl-2-thiophen-2-yl-pyrimidin-4-yl)-acetamide;
1-[(6-Thiazol-2-yl-2-thiophen-2-yl-pyrimidin-4-ylcarbamoyl)methyl)piperidine-3-carboxylic acid amide;
2-(4-Pyrrolidin-1-yl-piperidin-1-yl)-N-(6-thiazol-2-yl-2-thiophen-2-yl-pyrimidin-4-yl)-acetamide;
2-(3,5-Dimethyl-piperazin-1-yl)-1-N-(6-thiazol-2-yl-2-thiophen-2-yl-pyrimidin-4-yl)-acetamide;
2-[2-(1-Methyl-pyrrolidin-2-yl)-ethylamino]-N-(6-thiazol-2-yl-2-thiophen-2-yl-pyrimidin-4-yl)-acetamide;
N-[2-(5-Methyl-furan-2-yl)-6-pyrazol-1-yl-pyrimidin-4-yl]-2-(4-pyrrolidin-1-yl-piperidin-1-yl)-acetamide;
N-[2-(5-Methyl-furan-2-yl)-6-pyrazol-1-yl-pyrimidin-4-yl]-2-(4-methyl-piperazin-1-yl)-acetamide;
2-(4-Acetyl-piperazin-1-yl)-N-[2-(5-methyl-furan-2-yl)-6-pyrazol-1-yl-pyrimidin-4-yl)-acetamide;
2-((S)-3Ethylamino-pyrrolidin-1-yl)-N-[2-(5-methyl-furan-2-y)-6-pyrazol-1-yl-pyrimidin-4-yl)-acetamide;
2-(3,5-Dimethyl-piperazin-1-yl)-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4yl]-2-((R)-3-ethylamino-pyrrolidin-1-yl)-acetamide ;
N-[6-(3,5-Dimelhyl-pyrazol-1-yl)-2-(5-methyl-furan-2-yl)-pyrimidin-4-yl]-2-((S)-3-ethylamino-pyrrolidin-1-yl)-acetamide;
2-(3-Diethylamino-pyrrolidin-1-yl)-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-(pyrrolidin-3-ylamino)-acetamide;
N-[6-(3,5-Dimethyl-pyrazo-1-yl)-2-(5-methyl-furan-2-yl)-pyrimidin-4-yl]-2-(2-pyrrolidin-1-yl-ethylamino)-acetamide;
N-[6-(3,5-Dimethyl-pyrazol-1-yl)-2-(5-methyl-furan-2-yl)-pyrimidin-4-yl]-2-(3-pyrrolidinyl-propylamino)-acetamide;
N-[6-(3,5-Dimethyl-pyrazol-1-yl)-2-(5-methyl-furan-2-yl)-pyrimidin-4-yl]-2-[2-(1-methylpyrrolidin-2-yl)-ethylamino]-acetamide;
N-[6-(3,5-Dimethyl-pyrazol-1-yl)-2-(5-methyl-furan-2 yl)-pyrimidin-4-yl]-2-(2-piperidin-1-yl-ethylamino)-acetamide;
N-[6-(3,5-Dimethyl-pyrazol-2-yl)-2-(5-methyl-furan-2-yl)-pyrimidin-4-yl]-2-(3-piperidin-1-yl-propylamino)-acetamide;
N-[6-(3,5-Dimethyl-pyrazol-1-yl)-2-(5-methyl-furan-2-yl)-pyrimidin-4-yl]-2-(3-morpholin-4-yl-propylamino)-acetamide;
N-[6-(3,5-Dimethyl-pyrazol-1-yl)-2-(5-methyl-furan-2-yl)-pyrimidin-4-yl]-2-(4-methyl-piperazin-1-yl)-acetamide;
N-[6-(3,5-Dimethyl-pyrazol-1-yl)-2-(5-methyl-furan-2-yl)-pyrimidin-4-yl]-2-morpholin-4-yl-acetamide;
2-[1,4]Diazepan-1-yl-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)2-(5-methyl-furan-2-yl)-pyrimidin-4-yl]-2-(4-pyrrolidin-1-yl-piperidin-1-yl)-acetamide;
N-[6-(3,5-Dimethyl-pyrazol-1-yl)-2-(5-methyl-furan-2-yl)-pyrimidine-4-yl]-2-(4-methyl-[1,4]diazepan-1-yl)-acetamide;
N-[6-(3,5-Dimethyl-pyrazol-1-yl)-2-(5-methyl-furan-2-yl)-pyrimidin-4-yl]-2-[1,4]oxazepan-4-yl-acetamide;
2-((R)-Dimethylamino-pyrrolidin-1-yl)-N-[6-(3,5-dimethyl-pyrazol-1-yl)-2-(5-methylfuran-2-yl)-pyrimidin-4-yl]-acetamide;
2-((S)-3-Dimethylamino-pyrroldin-1-yl)-N-[6-(3,5-dimethyl-pyrazol-1-yl)-2-(5-methylfuran-2-yl)-pyrimidin-4-yl]-acetamide;
N-[6-(3,5-Dimethyl-pyrazol-1-yl)-2-(5-methyl-furan-2 yl)-pyrimidin-4-yl]-2-pyrrolidin-1-yl-acetamide;
2-(4,4-Difluoro-piperidin-1-yl)-N-[6-(3,5-dmethyl-pyrazol-1-yl)-2-(5-methyl-furan-2-yl)-pyrimidin4-yl]-acetamide; .
N-[6-(3,5-Dimethyl-pyrazol-1-yl)-2-(5-methyl-furan-2-yl)-pyrimidin-4-yl]-2-(4-thiazol-2-yl-piperazin-1-yl)-acetamide;
N-[6-(3,5-Dimethyl-pyrazol-1-yl)-2-(5-methyl-furan-2-yl)-pyrimidin-4-yl]-2-(4-ethyl-piperazin-1-yl)-acetamide;
2-((R)-3-Acetylamino-pyrrolidin-1-yl)-N-[6-(3,5-dimethyl-pyrazol-1-yl)-2-(5-methyl-furan-2-yl)-pyrimidin-4-yl]-acetamide;
2-((S)-3-Acetylamino-pyrrolidin-1-yl)-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-piperazin-1-yl-acetamide;
2-(2,6-Dimethyl-morpholin-4-yl)-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-(4-hydroxy-piperidin-1-yl)-acetamide;
2-[1,4']-Bipiperidinyl-1'-yl-N-[6-(3,5-Dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-(4-methoxy-piperidin-1-yl)-acetamide;
2-(4-Acetyl-piperazin-1-yl)-N-[6-(3,5-dimethyl-pyrazol-1-yl)-2-(5-methyl-furan-2-yl)-pyrimidin-4-yl]-acetamide;
2-((R)-3-Diethylamino-pyrrolidin-1-yl)-N-[6-(3,5-dimethyl-pyrazol-1-yl)-2-(5-methyl-furan-2-yl)-pyrimidin-4-yl]-acetamide;
2-((S)-3-Diethylamino-pyrrolidin-1-yl)-N-[6-(3,5dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-[(S)-3-(ethylmethyl-amino)-pyrrolidin-1-yl]-acetamide;
N-[6-(3,5-Dimethyl-pyrazol-1-yl)-2-(5-methyl-furan-2-yl)-pyrimidin-4-yl]-2-[(R)-3-(ethylmethyl-amino)-pyrrolidin-1-yl]-acetamide;
2-((S)-3-Amino-pyrrolidin-1-yl)-N-[6-(3,5-dimethyl-pyrazol-1-yl)-2-(5-methyl-furan-2-yl)-pyrimidin4-yl]-acetamide;
2-((R)-3-Amino-pyrrolidin-1-yl)-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-((R)-3-morphlin-4-yl-pyrrolidin-1-yl)-acetamide;
N-[6-(3,5-Dimethyl-pyrazol-1-yl)-2-(5-methyl-furan-2-yl)-pyrimidin-4-yl]-2-((R)-3-piperidin-1-yl-pyrrolidin-1-yl)-acetamide;
2-(R)-[1,3']-Bipyrrolidinyl-1'-yl-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-((S)-3-morpholin4-yl-pyrrolidin-1-yl)-acetamide;
2-(S)-[1,3']Bipyrrolidinyl-1'-yl-N-[6-(3,5-dimethyl-pyrazol-1-yl)-2-(5-methyl-furan-2-yl) pyrimidin-4-yl]-acetamide;
N-[6-(3,5-Dimethyl-pyrazol-1-yl)-2-(5-methyl-furan-2-yl)-pyrimidin-4-yl]-2-((S)-3-piperidin-1-yl-pyrrolidin-1-yl)-acetamide;
N-(1-{(6-(3,5-Dimethyl-pyrazol-1-yl)-2-(5-methyl-furan-2-yl)-pyrimidin-4-ylcarbamoyl]-methyl}-pyrrolidin-3-yl)-2,2,2-trifluoro-acetamide;
N-[6-(3,5-Dimethyl-pyrazol-1-yl)-2-(5-methyl-furan-2-yl)-pyrimidin-4-yl]-2-((R)-3-hydroxy-pyrrolidin-1-yl)-acetamide;
N-[6-(3,5-Dimethyl-pyrazol-1-yl)-2-(5-methyl-furan-2-yl)-pyrimidin-4-yl]-2-((S)-3-hydroxy-pyrrolidin-1-yl)-acetamide;
N-[6-(3,5-Dimethyl-pyrazol-1-yl)-2-(5-methyl-furan-2-yl)-pyrimidin-4-yl]-2-((3R,3'R)-3-fluoro-[1,3']bipyrrolidinyl-1'-yl)acetamide;
N-[6-(3,5-Dimethyl-pyrazol-1-yl)-2-(5-methyl-furan-2-yl)-pyrimidin-4-yl]-2-{(R)-3-[(2-methoxy-ethyl)-methyl-amino]-pyrrolidin-1-yl}-acetamide;
N-[6-(3,5-Dimethyl-pyrazol-1-yl)-2-(5-methyl-furan-2-yl)-pyrimidin-4-yl]-2-((3S,3'S)-3-fluoro-[1,3']bipyrrolidinyl-1'-yl)acetamide;
N-[6-(3,5-Dimethyl-pyrazol-1-yl)-2-(5-methyl-furan-2-yl)-pyrimidin-4-yl]-2-((3R,3'S)-3-fluoro-[1,3']bipyrrolidinyl-1'-yl)-acetamide;
N-[6-(3,5-Dimethyl-pyrazol-1-yl)-2-(5-methyl-furan-2-yl)-pyrimidin-4-yl]-2-(2,5,2',3',4',5'-hexahydro-[1,3']bipyrrolyl-1'-yl)-acetamide;
N-[6-(3,5-Dimethyl-pyrazol-1-yl)-2-(5-methyl-furan-2-yl)-pyrimidin-4-yl]-2-((S)-3-[(2-methoxy-ethyl)-methyl-amino]-pyrrolidin-1-yl)-acetamide;
N-[6-(3,5-Dimethyl-pyrazol-1-yl)-2-(5-methyl-furan-2-yl)-pyrimidin-4-yl]-2-((S)-3-fluoro-pyrrolidin-1-yl)-acetamide;
N-[6-(3,5-Dimethyl-pyrazol-1-yl)-2-(5-methyl-furan-2-yl)-pyrimidin-4-yl]-2-((R)-3-fluoro-pyrrolidin-1-yl)-acetamide;
N-[6-(3,5-Dimethyl-pyrazol-1-yl)-2-(5-methyl-furan-2 yl)-pyrimidin-4-yl]-2-piperidin-1-yl-acetamide;
N-[6-(3,5-Dimethyl-pyrazol-1-yl)-2-(5-methyl-furan-2-yl)-pyimidin-4-yl]-2-[(R)-3-(2-methoxy-ethylamino)-pyrrolidin-1-yl]-acetamide;
N-[6-(3,5-Dimethyl-pyrazol-1-yl)-2-(5-methyl-furan-2-yl)-pyrimidin-4-yl]-2-(3-trifluoromethyl-piperidin-1-yl)-acetamide;
N-[6-(3,5-Dimethyl-pyrazol-1-yl)-2-(5-methyl-furan-2-yl)-pyrimidin-4-yl]-2-(3-trifluoromethyl-5,6-dihydro-8H-[1,2,4]triazolo[4,3-a]pyrazin-7-yl)-acetamide;
N-[6-(3,5-Dimethyl-pyrazol-1-yl)-2-(5-methyl-furan-2-yl)-pyrimidin-4-yl]-2-[4-(2-oxopyrrolidin-1-yl)-piperidin-1-yl]-acetamide;
2-(4-Acetylamino-piperidin-1-yl)N-[6-(3,5-dimethyl-pyrazol-1-yl)-2-(5-methyl-furan-2-yl)-pyrimidin-4-yl]-acetamide;
4-{[6-(3,5-Dimethyl-pyrazol-1-yl)-2-(5-methyl-furan-2-yl)-pyrimidin-4-ylcarbamoyl]-methyl}-piperazine-1-carboxylic acid phenyl ester.
4-{[6-(3,5-Dimethyl-pyrazol-1-yl)-2-(5-methyl-furan-2-yl)-pyrimidin-4-ylcarbamoyl]-methyl}-piperazine-1-carboxylic acid benzylamide;
N-[6-(3,5-Dimethyl-pyrazol-1-yl)-2-(5-methyl-furan-2-yl)-pyrimidin-4-yl]-2-[4-(3-methylbenzoyl)-piperazin-1-yl]-acetamide;
4-{[6-(3,5-Dimethyl-pyrazol-1-yl)-2-(5-methyl-furan-2-yl)-pyrimidin-4-ylcarbamoyl]-methyl}-piperazine-1-carboxylic acid dimethylamide;
N-[6-(3,5-Dimethyl-pyrazol-1-yl)-2-(5-methyl-furan-2-yl)-pyrimidin-4-yl]-2-{(S)-3-[ethyl(2-methoxy-ethyl)-amino]-pyrrolidin-1-yl}-acetamide;
N-[6-(3,5-Dimethyl-pyrazol-1-yl)-2-(5-methyl-furan-2-yl)-pyrimidin-4-yl]-2-((S)-3-isopropoxy-pyrrolidin-1-yl)-acetamide;
2-(3,9-Diaza-bicyclo[4.2.1]non-3-yl)-N-[6-(3,5-dimethyl-pyrazol-1-yl)-2-(5-methyl-furan-2-yl)-pyrimidin-4-yl]-acetamide;
3-{[6-(3,5-Dimethyl-pyrazol-1-yl)-2-(5-methyl-furan-2-yl)-pyrimidin-4-ylcarbamoyl]-methyl}-3,9-diaza-bicyclo[4.2.1]nonane-9-carboxylic acid tert-butyl ester;
N-[6-(3,5-Dimethyl-pyrazol-1-yl)-2-(5-methyl-furan-2-yl)-pyrimidin-4-yl]-2-(9-methyl-3,9-diaza-bicyclo[4.2.1]non-3-yl)-acetamide;
N-[6(3,5-Dimethyl-pyrazol-1-yl)-2-(5-methyl-furan-2-yl)-pyrimidin-4-yl]-2-(4-{[(2-methoxy-ethyl)-methyl-amino]-methyl}-piperidin-1-yl)-acetamide;
N-[6-(3,5-Dimethyl-pyrazol-1-yl)-2-(5-methyl-furan-2-yl)-pyrimidin-4-yl]-2-[9-(2-methoxyethyl)-3,9-diaza-bicyclo[4.2.1]non-3-yl]-acetemide;
(1-{[6-(3,5-Dimethyl-pyrazol-1-yl)-2-(5-methyl-furan-2-yl)-pyrimidin-4-ylcarbamoyl]-methyl}-piperidin-4-yl)-methyl-carbamic acid tert-butyl ester;
N-[6-(3,5-Dimethyl-pyrazol-1-yl)-2-(5-methyl-furan-2-yl)-pyrimidin-4-yl]-2-{4-[(2-methoxyethyl)-methyl-amino]-piperidin-1-yl}-acetamide;
2-(2-Dimethylamino-morpholin-4-yl)-N-[6-(3,5-dimethyl-pyrazol-1-yl)-2-(5-methyl-furan-2-yl)-pyrimidin-4-yl]-acetamide;
2-((S)-3-Dimethylamino-piperidin-1-yl)-N-[6-(3,5-dimethyl-pyrazol-1-yl)-2-(5-methylfuran-2-yl)-pyrimidin-yl]acetamide;
2-((R)-3-Dimethylamino-piperidin-1-yl)-N-[6-(3,5-dimethyl-pyrazol-1-yl)-2-(5-methylfuran-2-yl)-pyrimidin-4-yl]-acetamide;
2-((R)-3-Dimethylamino-pyrrolidin-1-yl)-N-[6-(3,5-dimethyl-pyrazol-1-yl)-2-(5-methylfuran-2-yl)-pyrimidin-4-yl]-acetamide;
2-((S)-3-Dimethylaminomethyl-pyrrolidin-1-yl)-N-[6-(3,5-dimethyl-pyrazol-1-yl)-2-(5-methyl-furan-2-yl)-pyrimidin-4-yl]-acetamide;
2-(4-Dimethylaminomethyl-piperidin-1-yl)-N-[6-(3,5-dimethyl-pyrazol-1-yl)-2-(5-methylfuran-2-yl)-pyrimidin-4-yl]-acetamide;
N-[6-(3,5-Dimethyl-pyrazol-1-yl)-2-(5-methyl-furan-2-yl)-pyrimidin-4-yl]-2-(1-methyl-piperidin-4-ylamino)-acetamide;
N-[6-(3,5-Dimethyl-pyrazol-1-yl)-2-(5-methyl-furan-2-yl)-pyrimidin-4-yl]-2-((R)-pyrrolidin-3-ylamino)-acetamide;
N-[6-(3,5-Dimethyl-pyrazol-1-yl)-2-(5-methyl-furan-2-yl)-pyrimidin-4-yl]-2-{[(R)-1-(tetrahydro-furan-2-yl)methyl]-amino}-acetamide;
N-[6-(3,5-Dimethyl-pyrazol-1-yl)-2-(5-methyl-furan-2-yl)-pyrimidin-4-yl]-2-[(tetrahydropyran-4-ylmethyl)-amino]-acetamide;
N-[6-(3,5-Dimethyl-pyrazol-1-yl)-2-(5-methyl-furan-2-yl)-pyrimidin-4-yl]-2-{[(S)-1-(tetrahydro-furan-2-yl)methyl]-amino}-acetamide;
N-[6-(3,5-Dimethyl-pyrazol-1-yl)-2-(5-methyl-furan-2-yl)-pyrimidin-4-yl]-2-(piperidin-4-ylamino)acetamide;
N-[6-(3,5-Dimethyl-pyrazol-1-yl)-2-(5-methyl-furan-2-yl)-pyrimidin-4-yl]-2-((S)-piperidin-3-ylamino)-acetamide;
2-(Azetidin-3-ylamino)-N-[6-(3,5-dimethyl-pyrazol-1-yl)-2-(5-methyl-furan-2-yl)-pyrimidin-4-yl]-acetamide;
N-[6-(3,5-Dimethyl-pyrazol-1-yl)-2-(5-methyl-furan-2-yl)-pyrimidin-4-yl]-2-(2-morpholin-4-yl-ethylamino)-acetamide;
N-[6-(3,5-Dimethyl-pyrazol-1-yl)-2-(5-methyl-furan-2-yl)-pyrimidin-4-yl]-2-((R)-piperidin-3-ylamino)-acetamide;
N-[6-(3,5-Dimethyl-pyrazol-1-yl)-2-(5-methyl-furan-2-yl)-pyrimidin-4-yl]-2-[((R)-1-pyrrolidin-2-ylmethyl)-amino]-acetamide;
N-[6-(3,5-Dimethyl-pyrazol-1-yl)-2-(5-methyl-furan-2-yl)-pyrimidin-4-yl]-2-((S)-pyrrolidin-3-ylamino)-acetamide;
N-[6-(3,5-Dimethyl-pyrazol-1-yl)-2-(5-methyl-furan-2-yl)-pyrimidin-4-yl]-2-((R)-pyrrolidin-3-ylamino)-acetamide;
N-[6-(3,5-Dimethyl-pyrazol-1-yl)-2-(5-methyl-furan-2-yl)-pyrimidin-4-yl]-2-[((R)-1-pyrrolidin-3-ylmethyl)-amino]-acetamide;
N-[6-(3,5-Dimethyl-pyrazol-1-yl)-2-(5-methyl-furan-2-yl)-pyrimidin-4-yl]-3-((R)-3-ethylamino-pyrrolidin-1-yl)-propionamide;
3-((S)-3-Dimethylamino-pyrrolidin-1-yl)-N-[6-(3,5-dimethyl-pyrazol-1-yl)-2-(5-methylfuran-2-yl)-pyrimidin-4-yl]-propionamide;
N-[6-(3,5-Dimethyl-pyrazol-1-yl)-2-(5-methyl-furan-2-yl)-pyrimidin-4-yl]-3-(4-methyl-piperazin-1-yl)-propionamide;
N-[6-(3,5-Dimethyl-pyrazol-1-yl)-2-(5methyl-furan-2-yl)-pyrimidin-4-yl]-3-morpholin-4-yl-propionamide;
3-(2,6-Dimethyl-morpholin-4-yl)-N-[6-(3,5-dimethyl-pyrazol-1-yl)-2-(5-methyl-furan-2-yl)-pyrimidin-4-yl]-propionamid;
N-[6-(3,5-Dimethyl-pyrazol-1-yl)-2-(5-methyl-furan-2-yl)-pyrimidin-4-yl]3-(4-pyrrolidin-1-yl-piperidin-1-yl)-propionamid:
3-[1,4]Diazepan-1-yl-N-[6-(3,5-dimethyl-pyrazol-1-yl)-2-(5-methyl-furan-2-yl)-pyrimidin-4-yl]-propionamide;
N-[6-(3,5-Dimethy-pyraml-9-yl)-2-(5-methyl-furan-2-yl)-pyrimidin-4-yl]-3-(4-methyl-[1,4]diazepan-1-yl)-propionamide;
N-[6-(3,5-Dimethyl-pyrazol-1-yl)-2-(5-methyl-furan-2-yl)-pyrimidin-4-yl]-3-(4-methyl-[1,4]diazepan-1-yl)-propionamide;
3-((R)-3-Dimethylamino-pyrrolidin-1-yl)-N-[6-(3,5-dimethyl-pyrazol-1-yl)-2-(5-methylfuran-2-yl)-pyrimidin-4-yl]-propionamide;
N-[6-(3,5-Dimethyl-pyrazol-1-yl)-2-(5-methyl-furan-2-yl)-pyrimidin-4-yl]-3-((S)-3-ethylamino-pyrrolidin-1-yl)-propionamide;
N-[6-(3,5-Dimethyl-pyrazol-1-yl)-2-(5-methyl-furan-2-yl)-pyrimidin-4-yl]-3-{1,4}-oxazepan-4-yl-propionamide;
3-[1,4']Bipiperidinyl-1'-yl-N-[6-(3,5-dimethyl-pyrazol-1-yl)-2-(5-methyl-furan-2-yl)-pyrimidin-4-yl]-propionamide;
N-[6-(3,5-Dimethyl-pyrazol-1-yl)-2-(5-methyl-furan-2-yl)-pyrimidin-4-yl]-3-(4-methoxy-piperidin-1-yl)-propionamide;
N-[6-(3,5-Dimethyl-pyrazol-1-yl)-2-(5-methy-furan-2-yl)-pyrimidin-4-yl]-3-(4-thiazol-2-yl-piperazin-1-yl)-propionamide;
N-[6-(3,5-Dimethyl-pyrazol-1-yl)-2-(5-methyl-furan-2-yl)-pyrimidin-4-yl]-3-(4-ethyl-piperazin-1-yl)-propionamide;
3-(3-Diethylamino-pyrrolidin-1yl)-N-[6-(3,5-dimethyl-pyrazol-1-yl)-2-(5-methyl-furan-2-yl)-pyrimidin-4--yl]-propionamide;
3-((3R,5S)-3,5-Dimethyl-piperazin-1-yl)-N-[6-(3,5-dimethyl-pyrazol-1-yl)-2-(5-methylfuran-2-yl)-pyrimidin-4-yl]-propionamide;
N-[6-(3,5-Dimethyl-pyrazol-1-yl)-2-(5-methyl-furan-2-yl)-pyrimidin-4-yl]-3-piperazin-1-yl-proplonamide;
N-[6-(3,5-Dimethyl-pyrazol-1-yl)-2-(5-methyl-furan-2-yl)-pyrimidin-4-yl]-4-morpholin-4-yl-butyramide;
N-[6-(3,5-Dimethyl-pyrazol-1-yl)-2-(5-methyl-furan-2-yl)-pyrimidin-4-yl]-4-pyrrolidin-1-yl-butyramide:
N-[6-(3,5-Dimethyl-pyrazol-1-yl)-2-(5-methyl-furan-2-yl)-pyrimidin-4-yl]-4-((S)-3-fluoro-pyrrolidin-1-yl)-butyramide;
N-[6-(3,5-Dimethyl-pyrazol-1-yl)-2-(5-methyl-furen-2-yl)-pyrimidin-4-yl]-4-((R)-3-fluoro-pyrrolidin-1-yl)-butyramide;
4-{[6-(3,5-Dimethyl-pyrazol-1-yl)-2-(5-methyl-furan-2-yl)-pyrimidin-4-ylcarbamoyl]-methyl}-piperidine-1-carboxylic acid tert-butyl ester,
N-[6-(3,5-Dimethyl-pyrazol-1-yl)-2-(5-methyl-furan-2-yl)-pyrimidin-4-yl]-2-piperidin-4-yl-acetamide;
N-[6-(3,5-Dimethyl-pyrazol-1-yl)-2-(5-methyl-furan-2-yl)-pyrimidin-4-yl]-2-(1-methyl-piperidin-4-yl)-acetamide;
N-[6-(3,5-Dimethyl-pyrazol-1-yl)-2-(5-methyl-furan-2-yl)-pyrimidin-4-yl]-2-[1-(2-methoxyethyl)-piperidin-4-yl]-acetamide;
N-[6-(3,5-Dimethyl-pyrazol-1-yl)-2-(5-methyl-furan-2-yl)-pyrimidin-4-yl]-2-(2-pyrrolidin-1-yl-ethoxy)-acetamide;
2-(4-Methyl-piperazin-1-yl)-N-(2-oxazol-2-yl-6-pyrazol-1-yl-pyrimidin-4-yl)-acetamide;
2-((R)-3-Dimethylamino-pyrrolidin-1-yl)-N-(6-pyrazol-1-yl-2-pyridin-2-yl-pyrimidin-4-yl)-acetamide;
2-((S)-3-Dimethylamino-pyrrolidin-1-yl)-N-(6-pyrazol-1-yl-2-pyridin-2-yl-pyrimidin-4-yl)-acetamide;
2-(2,6-Dimethyl-morpholin-4-yl)-N-(6-pyrazol-1-yl-2-pyridin 2-yl-pyrimidin-4-yl)-acetamide;
N-(6-Pyrazol-1-yl-2-pyridin-2-yl-pyrimidin-4-yl)-2-(4-pyrrolidin-1-yl-piperidin-1-yl)-acetamide;
2-(4-Acetyl-piperazin-1-yl)-N-(6-pyrazol-1-yl-2-pyridin-2-yl-pyrimidin-4-yl)-acetamide;
2-(3,5-Dimethyl-piperazin-1-yl)-N-(6-pyrazol-1-yl-2-pyridin-2-yl-pyrimidin-4-yl)-acetamide;
2-(4-Methyl-piperazin-1-yl)-N-(6-pyrazol-1-yl-2-pyridin-2-yl-pyrimidin-4-yl)-acetamide;
2-Morpholin-4-yl-N-(6-pyrazol-1-yl-2-pyridin-2-yl-pyrimidin-4-yl)-acetamide;
2-(4-Dimethylamino-piperidin-1-yl)-N-(6-pyrazol-1-yl-2-pyridin-2-yl-pyrimidin-4-yl)-acetamide;
2-(4-Methyl-[1,4]diazepan-1-yl)-N-(6-pyrazol-1-yl-2-pyridin-2-yl-pyrimidin-4-yl)-acetamide;
2-(3,5-Dimethyl-piperazin-1-yl)-N-[6-(3,5-dimethyl-pyrazol-1-yl)-2-pyridin-2-yl-pyrimidin-4-yl]-acetamide;
N-[6-(3,5-Dimethyl-pyrazol-1-yl)-2-pyridin-2-yl-pyrimidin-4-yl]-2-(4-pyrrolidin-1-yl-piperidin-1-yl)-acetamide:
N-[6-(3,5-Dimethyl-pyrazol-1-yl)-2-pyridin-2-yl-pyrimidin-4-yl]-2-[(S)-3-(ethyl-methyl-amino)-pyrrolidin-1-yl]-acetamide;
2-((R)-3-Amino-pyrrolidin-1-yl)-N-[6-(3,5-dimethyl-pyrazol-1-yl)-2-pyridin-2-yl-pyrimidin-4-yl]-acetamide;
2-((S)-3-Amino-pyrrolidin-1-yl)-N-[6-(3,5-dimethyl-pyrazol-1-yl)-2-pyridin-2-yl-pyrimidin-4-yl]-acetamide;
N-((S)-1-{[6-(3,5-Dimethyl-pyrazol-1-yl)-2-pyridin-2-yl-pyrimidin-4-yl-carbamoyl]-methyl}-pyrrolidin-3-yl)-2,2,2-trifluoro-acetamide;
N-[6-(3,5-Dimethyl-pyrazol-1-yl)-2-pyridin-2-yl-pyrimidin-4-yl]-2-(3-trifluoromethyl-5,6-dihydro-8H-[1,2,4]-triazolo[4,3-a]pyrazin-7-yl-acetamide;
N-[6-(3,5-Dimethyl-pyrazol-1-yl)-2-pyridin-2-yl-pyrimidin-4-yl]-2-morpholin-4-yl-acetamide;
N-[6-(3,5-Dimethyl-pyrazol-1-yl)-2-pyridin-2-yl-pyrimidin-4-yl]-2-[1,4]oxazepan-4-yl-acetamide;
N-[6-(3,5-Dimethyl-pyrazol-1-yl)-2-pyridin-2-yl-pyrimidin-4-yl]-2-(4-methyl-piperazin-1-yl)-acetamide;
N-[6-(3,5-Dimethyl-pyrazol-1-yl)-2-pyridin-2-yl-pyrimidin-4-yl]-2-(4-methyl-[1,4]diazepan-1-yl)-acetamide;
2-((R)-3-Dimethylamino-pyrrolidin-1-yl)-N-[6-(3,5-dimethyl-pyazol-1-yl)-2-pyridin-2-yl-pyrimidin-4-yl]-acetamide;
2-((S)-3-Dimethylamino-pyrrolidin-1-yl)-N-[6-(3,5-dimethyl-pyrazol-1-yl)-2-pyridin-2-yl-pyrimidin-4-yl]-acetamide;
2-((R)-3-Dimethylaminomethyl-piperidin-1-yl)-N-[6-(3,5-dimethyl-pyrazol-1-yl)-2-pyridin-2-yl-pyrimidin-4-yl]-acetamide;
2-((S)3-Dimethylaminomethyl-piperidin-1-yl)-N-[6-(3,5-dimethyl-pyrazol-1-yl)-2-pyridin-2-yl-pyrimidin-4-yl]-acetamide;
N-(2,6-Di-pyrazol-1-yl-pyrimidin-4-yl)-2-(4-methyl-piperazin-1-yl)-acetamide;
N-(2,6-Di-pyrazol-1-yl-pyrimidin-4-yl)-2-(4-pyrrolidin-1-yl-piperidin-1-yl)-acetamide;
2-(3-Dimethylamino-pyrrolidin-1-yl)-N-(2,6-di-pyrazol-1-yl-pyrimidin-4-yl)-acetamide;
N-(2,6-Bis-thiazol-2-yl-pyrimidin-4-yl)-2-(4-methyl-piperazin-1-yl)-acetamide;
2-(4-Methyl-piperazin-1-yl)-N-(2-oxazol-5-yl-6-pyrazol-1-yl-pyrimidin-4-yl)-acetamide;
2-(3-Dimethylamino-pyrrolidin-1-yl)-N-(2-oxazol-5-yl-6-pyrazol-1-yl-pyrimidin-4-yl)-acetamide;
N-[2-(4-Methyl-oxazol-5-yl)-6-pyrazol-1-yl-pyrimidin-4-yl]-2-(4-methyl-piperazin-1-yl)-acetamide;
N-(2-Isoxazol-3-yl-6-pyrazol-1-yl-pyrimidin-4-yl)-2-(4-methyl-piperazin-1-yl)-acetamide;
2-(3-Dimethylamino-pyrrolidin-1-yl)-N-(2-isoxazol-3-yl-6-pyrazol-1-yl-pyrimidin-4-yl)-acetamide;
2-(4-Methyl-piperazin-1-yl)-N-(6-pyrazol-1-yl-2-thiazol-2-yl-pyrimidin-4-yl)-acetamide;
N-(6-Pyrazol-1-yl-2-thiazol-2-yl-pyrimidin-4-yl)-2-(4-pyrrolidin-1-yl-piperidin-1-yl)-acetamide;
2-((S)-3-Dimethylamino-pyrrolidin-1-yl)-N-(6-pyrazol-1-yl-2-thiazol-2-yl-pyrimidin-4-yl)-acetamide;
2-((S)-3-Ethylamino-pyrrolidin-1-yl)-N-(6-pyrazol-1-yl-2-thiazol-2-yl-pyrimidin-4-yl)-acetamide;
3-((R)-3-Ethylamino-pyrrolidin-1-yl)-N-(6-pyrazol-1-yl-2-thiazol-2-yl-pyrimidin-4-yl)-propionamide;
N-[6-(3,5-Dimethyl-pyrazol-1-yl)-2-thiazol-2-yl-pyrimidin-4-yl]-3-(4-methyl-piperazin-1-yl)-propionamide;
N-[6-(3,5-Dimethyl-pyrazol-1-yl)-2-thiazol-2-yl-pyrimidin-4-yl]-2-(4-pyrrolidin-1-yl-piperidin-1-yl)-acetamide;
2-((S)-3-Dimethylamino-pyrrolidin-1-yl)-N-[6-(3,5-dimethyl-pyrazol-1-yl)-2-thiazol-2-yl-pyrimidin-4-yl]-acetamide;
N-[6-(3,5-Dimethyl-pyrazol-1-yl)-2-thiazol-2-yl-pyrimidin-4-yl]-2-((S)-3-ethylamino-pyrrolidin-1-yl)-acetamide;
N-[6-(3,5-Dimethyl-pyrazol-1-yl)-2-thiazol-2-yl-pyrimidin-4-yl]-2-(3-trifluoromethyl-5,6-dihydro-8H-[1.2.4]triazolo[4.3-a]pyrazin-7-yl)-acetamide;
N-[6-(3,5-Dimethyl-pyrazol-1-yl)-2-furan-2-yl-pyrimidin-4-yl]-2-(4-methyl-piperazin-1-yl)-acetamide;
N-[6-(3,5-Dimethyl-pyrazol-1-yl)-2-furan-2-yl-pyrimidin-4-yl]-2-(4-methyl-piperazin-1-yl)-propionamide;
N-[2-(5-Methyl-furan-2-yl)-6-(5-methyl-3-trifluoromethyl-pyrazol-1-yl)-pyrimidin-4-yl]-2-(4-methyl-piperazin-1-yl)-acetamide;
(S)-Pyrrolidine-3-carboxylic acid [6-(3,5-dimethyl-pyrazol-1-yl)-2-(5-methyl-furan-2-yl)-pyrimidin-4-yl]-amide;
(S)-1-Methyl-pyrrolidine-3-carboxylic acid [6-(3,5-dimethyl-pyrazol-1-yl)-2-(5methylfuran-2-yl)-pyrimidin-4-yl]-amide;
(S)-1-(2-Methoxy-ethyl)-pyrrolidine-3-carboxylic acid [6-(3,5-dimethyl-pyrazol-1-yl)-2-(5-methyl-furan-2-yl)-pyrimidin-4-yl]-amide;
(R)-Pyrrolidine-3-carboxylic acid [6-(3,5-dimethyl-pyrazol-1-yl)-2-(5-methyl-furan-2-yl)-pyrimidin-4-yl]-amide;
(R)-1-Methyl-pyrrolidine-3-carboxylic acid [6-(3,5-dimethyl-pyrazol-1-yl)-2-(5-methylfuran-2-yl)-pyrimdin-4-yl]-amide;
(R)-1-(2-Methoxy-ethyl)-pyrrolidine-3-carboxylic acid [6-(3,5-dimethyl-pyrazol-1-yl)-2-(5-methyl-furan-2-yl)-pyrimidin-4-yl]-amide;
(R)-Pyrrolidine-2-carboxylic acid [6-(3,5-dimethyl-pyrazol-1-yl)-2-(5-methyl-furan-2-yl)-pyrimidin-4-yl]-amide;
(R)-1-(2-Methoxy-ethyl)-pyrrolidine-2-carboxylic acid [6-(3,5-dimethyl-pyrazol-1-yl)-2-(5-methyl-furan-2-yl)-pyrimidin-4-yl]-amide;
2-[6-(3,5-Dimethyl-pyrazol-1yl)-2-(5-methyl-furan-2-yl)-pyrimidin-4-ylcarbamoyl]-pyrrolidine-1-carboxylic acid benzyl ester,
(S)-Pyrrolidine-2-carboxylic acid [6-(3,5-dimethyl-pyrazol-1-yl)-2-(5-methyl-furan-2-yl)-pyrimidin-4-yl]-amide;
Pyrrolidine-2-carboxylic acid [6-(3,5-dimethyl-pyrazol-1-yl)-2-(5methyl-furan-2-yl)-pyrimidin-4-yl]-amide;
N-[6-(3,5-Dimethyl-pyrazol-1-yl)-2-thiazol-2-yl-pyrimidin-4-yl]-2-(4-methyJ-piperazin-1-yI)-acetamide;
N-[6-(3,5-Dimethyl-pyrazol-1-yl)-2-thiazol-2-yl-pyrimidin-4-yl]-2-(4-pyrrolidin-1-yl-piperidin-1-yl)-propionamide;
2-((R)-3-Dimethylamino-pyrrolidin-1-yl)-N-[6-(3,5-dimethyl-pyrazol-1-yl)-2-thiazol-2-yl-pyrimidin-4-yl]propionamide;
N-[6-(3,5-Dimethyl-pyrazol-1-yl)-2-thiazol-2-yl-pyrimidin-4-yl]-3-((R)-3-ethylamino-pyrrolidin-1-yl)-propionamide;
N-[6(3,5-Dimethyl-pyrazol-1-yl)-2-thiazol-2-yl-pyrimidin-4-yl]-3-[1,4]oxazepan-4-yl-propionamide;
3-((R)-3-Ethylamino-pyrrolidin-1-yl)-N-(6-pyrazol-1-yl-2-thiazol-2-yl-pyrimidin-4-yl)-propionamide;
Morpholine-2-carboxylic acid [6-(3,5-dimethyl-pyrazol-1-yl)-2-(5-methyl-furan-2yl)-pyrimidin-4-yl]-amide;
4-Methyl-morpholine-2-carboxylic acid [6-(3,5-dimethyl-pyrazol-1-yl)-2-(5-methyl-furan-2-yl)-pyrimidin-4-yl]-amide;
4-(Pyrrolidine-1-carbonyl)-morpholine-2-carboxylic acid [6-(3,5-dimethyl-pyrazol-1-yl)-2-(5-methyl-furan-2-yl)-pyrimidin-4-yl]-amide;
Pyrrolidine-1-carboxylic acid [6-(3,5-dimethyl-pyrazol-1-yl)-2-(5-methyl-furan-2-yl)-pyrimidin-4-yl]-amide;
Morpholine-4-carboxylic acid [6-(3,5-dimethyl-pyrazol-1-yl)-2-(5-methyl-furan-2-yl)-pyrimidin-4-yl]-amide;
4-Methyl-piperazine-1-carboxylic acid [6-(3,5-dimethyl-pyrazol-1-yl)-2-(5-methyl-furan-2-yl)-pyrimidin-4-yl]-amide;
1-[6-(3,5-Dimethyl-pyrazol-1-yl)-2-(5-methyl-furan-2-yl)-pyrimidin-4-yl]-3-(2-morpholin-4-yl-ethyl)-urea;
1-[6-(3,5-Dimethyl-pyrazol-1-yl)-2-(5-methyl-furan-2-yl)-pyrimidine-4-yl]-3-(3-piperidin-1-yl-propyl)-urea;
N-[6-(3,5-Dimethyl-pyrazol-1-yl)-2-(5-methyl-furan-2-yl)-pyrimidin-4-yl]-2-(S)-pyrrolidin-3-yl-acetamide;
N-[6-(3,5-Dimethyl-pyrazol-1-yl)-2-(5-methyl-furan-2-yl)-pyrimidin-4-yl]-2-((S)-1-methylpyrrolidin-3-yl)-acetamide;
(S)-3-{[6-(3,5-Dimethyl-pyrazol-1-yl)-2-(5-methyl-furan-2-yl)-pyrimidin-4-ylcarbamoyl]-methyl}-1,1-dimethyl-pyrrolidinium;
N-[6-(3,5-Dimethyl-pyrazol-1-yl)-2-(5-methyl-furan-2-yl)-pyrimidin-4-yl]-2-[(S)-1-(2-methoxy-ethyl)-pyrrolidin-3-yl]-acetamide;
N-[6-(3,5-Dimethyl-pyrazol-1-yl)-2-(5-methyl-furan-2-yl)-pyrimidin-4-yl]-2-(R)-pyrrolidin-3-yl-acetamide;
N-[6-(3,5-Dimethyl-pyrazol-1-yl)-2-(5-methyl-furan-2-yl)-pyrimidin-4-yl]-2-((R)-1-methylpyrrolidin-3-yl)-acetamide;
N-[6-(3,5-Dimethyl-pyrazol-1-yl)-2-(5-methyl-furan-2-yl)-pyrimidin-4-yl]-2-[(R)-1-(2-methoxy-ethyl)-pyrrolidin-3-yl]-acetamide;
1-Methyl-piperidine-4-carboxylic acid [6-(3,5-dimethyl-pyrazol-1-yl)-2-(5-methyl-furan-2-yl)-pyrimidin-4-yl]-amide;
N-[6-(3,5-Dimethyl-pyrazol-1-yl)-2-(5-methyl-furan-2-yl)-pyrimidin-4-yl]-2-[(R)-1-(2-methoxy-ethyl)-piperidin-3-yl]-acetamide;
1-[6-(3,5-Dimethyl-pyrazol-1-yl)-2-(5-methyl-furan-2-yl)-pyrimidin-4-yl]-3-(3-pyrrolidin-1-yl-propyl)-urea;
1-[6-(3,5-Dimethyl-pyrazol-1-yl)-2-(5-methyl-furan-2-yl)-pyrimidin-4-yl]-3-[3-(4-methyl-piperazin-1-yl)-propyl]-urea;
1-[6-(3,5-Dimethyl-pyrazol-1-yl)-2-(5-methyl-furan-2-yl)-pyrimidin-4-yl]-3-[2-(1-methylpyrrolidin-2-yl)-ethy]-urea;
1-[6-(3,5-Dimethyl-pyrazol-1-yl)-2-(5-methyl-furan-2-yl)-pyrimidin-4-yl]-3-[2-(1-methyl-piperidin-2-yl)-ethyl]-urea;
1-[6-(3,5-Dimethyl-pyrazol-1-yl)-2-(5-methyl-furan-2-yl)-pyrimidin-4-yl]-3-(2-piperidin-2-yl-ethyl)-urea;
1-[6-(3,5-Dimethyl-pyrazol-1-yl)-2-(5-methyl-furan-2-yl)-pyrimidin-yl]-3-(3-morpholin-4-yl-propyl)-urea;
N-[6-(3,5-Dimethyl-pyrazol-1-yl)2-(5-methyl-furan-2-yl)-pyrimidin-4-yl]-2-(2-methoxy-ethylamino)-acetamide;
[6-(3,5-Dimethyl-pyrazol-1-yl)-2-(5-methyl-furan-2-yl)-pyrimidin-4-yl]-carbamic acid 2-azepan-1-yl-ethyl ester;
[6-(3,5-Dimethyl-pyrazol-1-yl)-2-(5-methyl-furan-2-yl)-pyrimidin-4-yl]-carbamic acid 3-piperidin-1-yl-propyl ester,
[6-(3,5-Dimethyl-pyrazol-1-yl)-2-(5-methyl-furan-2-yl)-pyrimidin-4-yl]-carbamic acid 3-(2-oxo-pyrrolidin-1-yl)-propyl ester,
[6-(3,5-Dimethyl-pyrazol-1-yl)-2-(5-methyl-furan-2-yl)-pyrimidin-4-yl]-carbamic acid 2-morpholin-4-yl-ethyl ester;
[6-(3,5-Dimethyl-pyrazol-1-yl)-2-(5-methyl-furan-2-yl)-pyrimidin-4-yl]-carbamic acid 2-piperidin-1-yl-ethyl ester,
[6-(3,5-Dimethyl-pyrazol-1-yl)-2-(5-methyl-furan-2-yl)-pyrimidin-4-yl]-carbamic acid 2-(2-oxo-pyrrolidin-1-yl)-ethyl ester, and
[6-(3,5-Dimethyl-pyrazol-1-yl)-2-(5-methyl-furan-2-yl)-pyrimidin-4-yl]-carbamic acid 2-pyrrolidin-1-yl-ethyl-ester.

17. A compound according to claim 16, wherein the compound is selected from the group of
N-(2-Furan-2-yl-6-pyrazol-1-yl-pyrimidin-4-yl)-2-(4-methyl-piperazin-1-yl)-acetamide;
N-[2-(2-Fluoro-3-methoxy-phenyl)-6-(3,5-dimethyl-pyrazol-1-yl)-pyrimidin-4-yl]-2-(4-methyl-piperazin-1-yl)-acetamide;
N-(2-Furan-2-yl-6-thiazol-2-yl-pyrimidin-4-yl)-2-(4-methyl-piperazin-1-yl)-acetamide;
N-[6-(3,5-Dimethyl-pyrazol-1-yl)-2-(5-methyl-furan-2-yl)-pyrimidin-4-yl]-2-((S)-3-ethylamino-pyrrolidin-1-yl)-acetamide;
N-[6-(3,5-Dimethyl-pyrazol-1-yl)-2-(5-methyl-furan-2yl)-pyrimidin-4-yl]-2-(4-methyl-[1,4]diazepan-1-yl)-acetamide;
2-((S)-3-Dimethylamino-pyrrolidin-1-yl)-N-[6-(3,5-dimethyl-pyrazol-1-yl)-2-(5-methyl furan-2-yl)-pyrimidin-4-yl]-acetamide;
2-((R)-3-Dimethylamino-pyrrolidin-1-yl)-N-[6-(3,5-dimethyl-pyrazol-1-yl)-2-(5-methylfuran-2-yl)-pyrimidin-4-yl]-acetamide;
N-[6-(3,5-Dimethyl-pyrazol-1-yl)-2-(5-methyl-furan-2-yl)-pyrimidin-4-yl]-3-((R)-3-ethylamino-pyrrolidin-1-yl)-propionamide;
3-((S)-3-Dimethylamino-pyrrolidin-1-yl)-N-[6-(3,5-dimethyl-pyrazol-1-yl)-2-(5-methyl-furan-2-yl)-pyrimidin-4-yl]-propionamide;
N-[6-(3,5-Dimethyl-pyrazol-1-yl)-2-(5-methyl-furan-2-yl)-pyrimidin-4-yl]-3-[1,4]oxazepan-4-yl-propionamide;
N-[6-(3,5-Dimethyl-pyrazol-1-yl)-2-pyridin-2-yl-pyrimidin-4-yl]-2-(4-methyl-piperazin-1-yl)-acetamide
2-(4-Methyl-piperazin-1-yl)-N-(6-pyrazol-1-yl-2-thiazol-2-yl-pyrimidin-4-yl)-acetamide;
N-(6-Pyrazol-1-yl-2-thiazol-2-yl-pyrimidin-4-yl)-2-(4-pyrrolidin-1-yl-piperidin-1-yl)-acetamide;
2-((S)-3-Dimethylamino-pyrrolidin-1-yl)-N-(6-pyrazol-1-yl-2-thiazol-2-yl-pyrimidin-4-yl)-acetamide;
2-((S)-3-Ethylamino-pyrrolidin-1-yl)-N-(6-pyrazol-1-yl-2-thiazol-2-yl-pyrimidin-4-yl)-acetamide;
N-[6-(3,5-Dimethyl-pyrazol-1-yl)-2-thiazol-2-yl-pyrimidin-d-yl]-2-(4-methyl-piperazin-1-yl)-acetamide;
N-[6-(3,5-Dimethyl-pyrazol-1-yl)-2-thiazol-2-yl-pyrimidin-4-yl]-2-(4-pyrrolidin-1-yl-piperidin-1-yl)-acetamide;
2-((S)-3-Dimethylamino-pyrrolidin-1-yl)-N-[6-(3,5-dimethyl-pyrazol-1-yl)-2-thiazol-2-yl-pyrimidin-4-yl]-acetamide;
N-[6-(3,5-Dimethyl-pyrazol-1-yl)-2-thiazol-2-yl-pyrimidin-4-yl]-2-((S)-3-ethylamino-pyrrolidin-1-yl)-acetamide;
2-((R)-3-Dimethylamino-pyrrolidin-1-yl)-N-[6-(3,5-dimethyl-pyrazol-1-yl)-2-thiazol-2-yl-pyrimidin-4-yl]-propionamide; and
1-[6-(3,5-Dimethyl-pyrazol-1-yl)-2-(5-methyl-furan-2-yl)-pyrimidin-4-yl]-3-(3-morpholin-4-yl-propyl)-urea.

18. A pharmaceutical composition comprising a compound according to claim 1 and a pharmaceutically acceptable carrier or diluent.

19. A pharmaceutical composition comprising a compound according to claim 3 and a pharmaceutically acceptable carrier or diluent.

20. A pharmaceutical composition comprising a compound according to claim 7 and a pharmaceutically acceptable carrier or diluent.

21. A pharmaceutical composition comprising a compound according to claim 13 and a pharmaceutically acceptable carrier or diluent.

22. A pharmaceutical composition comprising a compound according to claim 15 and a pharmaceutically acceptable carrier or diluent.

23. A pharmaceutical composition comprising a compound according to claim 16 and a pharmaceutically acceptable carrier or diluent.

24. A pharmaceutical composition comprising a compound according to claim 17 and a pharmaceutically acceptable carrier or diluent.

25. Use of a compound according to claim 1 in manufacturing of a pharmaceutical composition for treating a subject having a condition susceptible to amelioration by antagonism of an adenosine receptor.

26. Use of claim 25, wherein the condition is ischemia, supraventricular arrhythmias, acute renal failure, myocardial reperfusion injury, autoimmune disease, inflammatory bowel diseases, asthma, diabetes mellitus, obesity, Parkinson disease, Huntington's disease, dystonia or dyskinesia.

27. Use of the compound according to claim 1 in manufacturing of a pharmaceutical composition for treating a subject having a condition susceptible to amelioration by antagonism of A2A adenosine receptor.

28. Use of claim 27 wherein the condition is ischemia, supraventricular arrhythmias, Parkinson disease, Huntington's disease, dystonia or dyskinesia.

## Patentansprüche

1. Eine Verbindung der Formel (I) 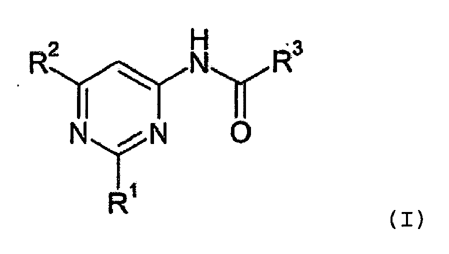 oder ein pharmazeutisch annehmbares Salz, Ester, Solvat oder Stereoisomer davon, wobei:
jedes R¹ und R² unabhängig eine Aryl- oder Heteroaryl-Gruppe ist, die optional durch einen oder mehrere Substituenten, die aus der Gruppe aus niederem Alkyl mit 1 bis 8 Kohlenstoffatomen, Halogen, Cycloalkyl, Hydroxy, niederem Alkoxy mit 1 bis 8 Kohlenstoffatomen, - SH, NO₂, niederem Alkylthio mit 1 bis 8 Kohlenstoffatomen, niederem Alkylamino mit 1 bis 8 Kohlenstoffatomen, Cyano und Amino besteht, ausgewählt werden, wobei die niederen Alkyl-, Cycloalkyl-, die niederen Alkoxy-, die niederen Alkylthio- und niederen Alkylamino-Gruppen optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂, NH₂, NHCH₃, NHCH₂CH₃ , N(CH₃)₂, N(CH₂CH₃)₂, SH, SCH₃ , OH, OCH₃ , OCF₃ , CH₃ und CF₃ ausgewählt werden, substituiert sind;
R³ - (CR⁴R⁵)ₙ-R⁶, - (C⁴R⁵)ₙ -NR⁷R⁸ , -O- (CR⁴R⁵)ₙ-R⁶ ist oder - (CR⁴R⁵)ₙ-O-R⁸ ist;
jedes R⁴ und R⁵ unabhängig bei jedem Auftreten aus der Gruppe aus Wasserstoff, niederem Alkyl mit 1 bis 8 Kohlenstoffatomen, Halogen, Cycloalkyl, Hydroxy, niederem Alkoxy mit 1 bis 8 Kohlenstoffen, -SH, NO₂, niederem Alkylthio mit 1 bis 8 Kohlenstoffatomen, niederem Alkylamino mit 1 bis 8 Kohlenstoffatomen, Cyano- und Amino ausgewählt wird, wobei die niederen Alkyl-, Cycloalkyl-, niederen Alkoxy-, niederen Alkylthio- und niederen Alkylamino-Gruppen optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂ NH₂, NHCH₃, NHCH₂CH₃, N(CH₃)₂, N(CH₂CH₃)₂, SH, SCH₃, OH, OCH₃, OCF₃, CH₃ und CF₃ ausgewählt werden, substituiert sind;
R⁶ ein Heterozyklus ist, der mindestens ein Stickstoffatom hat, wobei der Heterozyklus optional durch ein oder mehrere Elemente substituiert ist, die aus der Gruppe aus niederem Alkyl mit 1 bis 8 Kohlenstoffen, Alkoxy, Cycloalkyl, Aminoalkyl, Aminodialkyl, Alkylaminoalkyl, Dialkylaminoalkyl, Alkoxyalkyl, Aryl, Arylalkyl, Heteroaryl, Heteroarylalkyl, Heterozyklus, Heterocycylalkyl, Amino, Alkylamino, Dialkylamino, Cycloalkylamino, Halogen, Halogenalkyl, Ester, Amid, Acyl, Carbamoyl, Carbamoylalkyl, Oxo, Isochinolinyl und Imidoylamino ausgewählt werden, wobei die niederen Alkyl-, Alkoxy-, Cycloalkyl-, Aminoalkyl-, Alkylaminoalkyl-, Dialkylaminoalkyl-, Alkoxyalkyl-, Aryl-, Arylalkyl-, Heteroaryl-, Heteroarylalkyl-, Heterozyklus-, Heterocycylalkyl-, Amino-, Alkylamino-, Dialkylamino-, Cycloalkylamino-, Halogenalkyl-, Ester-, Amid-, Acyl-, Carbamoyl-, Carbamoylalkyl-, Isochinolinyl- und Imidoylamino-Gruppen optional mit niederem Alkyl mit 1 bis 8 Kohlenstoffen, Alkoxy, Hydroxy, Oxo oder Halogen substituiert sind;
R⁷ Wasserstoff oder niederes Alkyl mit 1 bis 8 Kohlenstoffen ist, das optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂ NH₂, NHCH₃, NHCH₂CH₃, N(CH₃)₂, N(CH₂CH₃)₂, SH, SCH₃, OH, OCH₃, OCF₃, CH₃ und CF₃ ausgewählt werden, substituiert ist;
R⁸ - (CR⁴CR⁵) ₙ-R⁶ ist; oder
R⁷ und R⁸ zusammen mit dem Stickstoffatom, an das sie gebunden sind, einen heterocyclischen Ring bilden, der optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂ NH₂, NHCH₃, NHCH₂CH₃, N (CH₃)₂, N (CH₂CH₃)₂, SH, SCH₃, OH, OCH₃, OCF₃, CH₃ und CF₃ ausgewählt werden, substituiert ist; und
n unabhängig bei jedem Auftreten 0, 1, 2, 3 oder 4 ist;
mit der Maßgabe, dass wenn R¹ und R² beide Heteroaryl sind, R⁶ ein nicht-aromatischer Heterozyklus ist.

2. Eine Verbindung gemäß Anspruch 1, wobei: jedes R¹ und R² unabhängig aus der Gruppe aus Pyridinyl, Furanyl, Thiophenyl, Thiazolyl, Pyrazolyl, Imidazolyl, Oxazolyl, Isoxazolyl und Oxadiazolyl, die optional durch einen oder mehrere Substituenten substituiert sind, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂ , NH₂ , NHCH₃, NHCH₂CH₃, N (CH₃)₂, N (CH₂CH₃)₂, SH, SCH₃, OH, OCH₃, OCF₃, CH₃ und CF₃ ausgewählt werden, ausgewählt wird.

3. Eine Verbindung gemäß Anspruch 2, wobei n 1 oder 2 ist.

4. Eine Verbindung gemäß Anspruch 3, wobei:
R¹ aus der Gruppe aus Pyridinyl, Furanyl, Thiophenyl, Thiazolyl, Pyrazolyl, Imidazolyl, Oxazolyl und Isoxazolyl ausgewählt wird, die optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂, NH₂, NHCH₃, NHCH₂CH₃ , N(CH₃)₂ , N(CH₂CH₃)₂, SH, SCH₃, OH, OCH₃, OCF₃, CH₃ und CF₃ ausgewählt werden, substituiert sind.

5. Eine Verbindung gemäß Anspruch 4, wobei:
R² aus der Gruppe aus Pyridinyl, Thiazolyl und Pyrazolyl ausgewählt wird, die optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂ NH₂, NHCH₃, NHCH₂ CH₃, N(CH₃)₂, N(CH₂CH₃)₂, SH, SCH₃, OH, OCH₃, OCF₃, CH₃ und CF₃ ausgewählt werden, substituiert sind.

6. Eine Verbindung gemäß Anspruch 5, wobei:
R¹ aus der Gruppe aus Pyridinyl, Furanyl, 5-Methylfuranyl, Thiophenyl, Thiazolyl, Pyrazolyl, Imidazolyl, Oxazolyl, 4-Methyloxazolyl und Isoxazolyl ausgewählt wird; und
R² aus der Gruppe aus Pyridinyl, Thiazolyl, Pyrazolyl und 3,5-Dimethylpyrazolyl ausgewählt wird.

7. Eine Verbindung gemäß Anspruch 6, wobei:
R³ -(CR⁴R⁵)ₙ-R⁶ oder -(CR⁴R⁵)ₙ-NR⁷R⁸ ist;
jedes von R⁴ und R⁵ unabhängig bei jedem Auftreten Wasserstoff oder niederes Alkyl mit 1 bis 8 Kohlenstoffen ist;
R⁶ ein heterocyclischer Ring ist, der aus der Gruppe aus Piperidinyl, Piperazinyl, Morpholinyl, Thiomorpholinyl, Pyrrolidinyl, Isochinolinyl, Diazepanyl, Dihydropyrrolyl, Azepanyl, Oxazepanyl und Pyrrolopyrazinyl ausgewählt wird, die optional durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂, NH₂ , NHCH₃ , NHCH₂CH₃ , N(CH₃)₂ , N(CH₂CH₃)₂, SH, SCH₃, OH, OCH₃, OCF₃, CH₃ und CF₃ ausgewählt werden, substituiert ist; und
R⁷ und R⁸ zusammen mit dem Stickstoffatom, an das sie gebunden sind, einen heterocyclischen Ring bilden, der aus der Gruppe aus Piperidinyl, Piperazinyl, Morpholinyl, Thiomorpholinyl, Pyrrolidinyl, Isochinolinyl, Diazepanyl, Dihydropyrrolyl, Azepanyl, Oxazepanyl und Pyrrolopyrazinyl ausgewählt wird, die durch einen oder mehrere Substituenten, die aus der Gruppe aus Alkyl, Alkenyl, Alkynyl, Aryl, Heteroaryl, Alkoxy, Aryloxy, Alkylthio, Arylthio, Cycloalkyl, Arylalkyl, Amino, Alkylamino, Dialkylamino, Amido, F, Cl, Br, I, CN, NO₂ NH₂, NHCH₃ , NHCH₂CH₃, N(CH₃)₂, N(CH₂CH₃)₂, SH, SCH₃, OH, OCH₃, OCF₃, CH₃ und CF₃ ausgewählt werden, substituiert ist.

8. Eine Verbindung gemäß Anspruch 7, wobei R⁶ mit einen oder mehreren Elementen, die aus der Gruppe aus niederem Alkyls mit 1 bis 8 Kohlenstoffen, Alkoxy, Amino, Alkylamino, Dialkylamino, Piperidinyl, Piperazinyl, Pyrrolidinyl, Morpholinyl, Phenyl und Benzyl ausgewählt werden, substituiert ist.

9. Eine Verbindung gemäß Anspruch 7, wobei:
R¹ Furanyl, 5-Methylfuranyl, Oxazolyl, 4-Methyloxazolyl, Pyridinyl, Pyrazolyl oder Isoxazolyl ist und R² Pyrazolyl ist.

10. Eine Verbindung gemäß Anspruch 7, wobei:
R¹ Furanyl, 5-Methylfuranyl, Pyridinyl, Thiophenyl oder Thiazolyl ist und R² Thiazolyl ist.

11. Eine Verbindung gemäß Anspruch 7, wobei:
R¹ ist 5-Methylfuranyl, Thiazolyl oder Pyridinyl ist und R² 3,5-Dimethylpyrazolyl ist.

12. Eine Verbindung gemäß Anspruch 7, wobei:
R¹ 5-Methylfuranyl oder Thiophenyl ist und R² Pyridinyl ist.

13. Eine Verbindung gemäß Anspruch 1, wobei:
R¹ Furanyl oder 5-Methylfuranyl ist; und
R² Pyrazolyl, 3,5-Dimethylpyrazolyl oder Thiazolyl ist.

14. Eine Verbindung gemäß Anspruch 13, wobei:
R³ - (CR⁴R⁵)ₙR⁶ ist; wobei jedes von R⁴ und R⁵ Wasserstoff ist; und n 1 oder 2 ist.

15. Eine Verbindung gemäß Anspruch 14, wobei:
R⁶ Piperazinyl, das mit niederem Alkyl mit 1 bis 8 Kohlenstoffatomen substituiert ist, Pyrrolidinyl, das mit Alkylamino oder Dialkylamino substituiert ist, [1,4]-Diazepanyl, das mit niederem Alkyl mit 1 bis 8 Kohlenstoffen substituiert ist, oder [1,4]-Oxazepanyl ist.

16. Eine Verbindung gemäß Anspruch 1, die aus der Gruppe aus:
N-(2-Furan-2-yl-6-pyrazol-1-yl-pyrimidin-4-yl)-2-piperidin-1-yl-acetamid;
N-(2-Furan-2-yl-6-pyrazol-1-yl-pyrimidin-4-yl)-2-morpholin-4-yl-acetamid;
N-(2-Furan-2-yl-6-pyrazol-1-yl-pyrimidin-4-yl)-2-(4-methyl-piperazin-1-yl)-acetamid;
N-(2-Furan-2-yl-6-pyrazol-1-yl-pyrimidin-4-yl)-2-piperazin-1-yl-acetamid;
N-[2-(5-Methyl-furan-2-yl)-6-thiazol-2-yl-pyrimidin-4-yl]-2-morpholin-4-yl-acetamid;
N-[2-(5-Methyl-furan-2-yl)-6-thiazol-2-yl-pyrimidin-4-yl]-2-piperidin-1-yl-acetamid;
N-[2-(5-Methyl-furan-2-yl)-6-thiazol-2-yl-pyrimidin-4-yl]-2-pyrrolidin-1-yl-acetamid;
N-[2-(5-Methyl-furan-2-yl)-6-thiazol-2-yl-pyrimidin-4-yl]-2-(4-methyl-piperazin-1-yl)-acetamid;
2-(2,5-Dimethyl-piperazin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-piperazin-1-yl-acetamid;
N-[2-(5-Methyl-furan-2-yl)-6-thiazol-2-yl-pyrimidin-4-yl]-2-(4-phenyl-piperazin-1-yl)-acetamid;
2-[1,4]Diazepan-1-yl-N-[2-(5-methyl-furan-2-yl)-6-thiazol-2-y1-pyrimidin-4-y1]-acetamid;
N-[2-(5-Methyl-furan-2-yl)-6-thiazol-2-yl-pyrimidin-4-yl]-2-(2-phenyl-piperidin-1-yl)-acetamid;
N-[2-(5-Methyl-furan-2-yl)-6-thiazol-2-yl-pyrimidin-4-yl]-2-(3-phenyl-piperidin-1-yl)-acetamid;
2-(4-Benzyl-piperazin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(2-methyl-piperidin-1-yl)-acetamid;
2-(2,5-Dihydro-pyrrol-1-yl)-N-[2-(5-methyl-furan-2-yl)-6-thiazol-2-yl-pyrimidin-4-yl]-acetamid;
2-(2,5-Dimethyl-2,5-dihydro-pyrrol-1-yl)-N-[2-(5-methyl-furan-2-yl)-6-thiazol-2-yl-pyrimidin-4-yl]-acetamid;
2-(2,5-Dimethyl-pyrrolidin-1-yl)-N-[2-(5-ethyl-furan-2-yl)-6-thiazol-2-yl-pyrimidin-4-yl]-acetamid;
2-(3,5-Dimethyl-piperazin-1-yl)-N-[2-(5-methyl-furan-2-yl)-6-thiazol-2-yl-pyrimidin-4-yl]-acetamid;
2-[4-(2-Methoxy-phenyl)-piperazin-1-yl]-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(3-methyl-piperidin-1-yl)-acetamid;
2-Azepan-1-yl-N-[2-(5-methyl-furan-2-yl)-6-thiazol-2-yl-pyrimidin-4-yl]-acetamid;
2-((S)-2-Methoxymethyl-pyrrolidin-1-yl)-N-[2-(5-methyl-furan-2-yl)-6-thiazol-2-yl-pyrimidin-4-yl]-acetamid;
2-(3,3-Dimethyl-piperidin-1-yl)-N-[2-(5-methyl-furan-2-yl)-6-thiazol-2-yl-pyrimidin-4-yl]-acetamid;
2-(3,5-Dimethyl-piperidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(4-phenyl-piperidin-1-yl)-acetamid;
N-[2-(5-Methyl-furan-2-yl)-6-thiazol-2-yl-pyrimidin-4-yl]-2-(4-methyl-piperidin-1-yl)-acetamid;
2-(4-Benzyl-piperidin-1-yl)-N-[2-(5-methyl-furan-2-yl)-6-thiazol-2-yl-pyrimidin-4-yl]-acetamid;
2-[1,4']Bipiperidinyl-1'-yl-N-[2-((5-methyl-furan-2-yl)-6-thiazol-2-yl-pyrimidin-4-yl]-acetamid;
2-(3,4-Dihydro-1H-isochinolin-2-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(octahydro-isochinolin-2-yl)-acetamid;
N-[2-(5-Methyl-furan-2-yl)-6-thiazol-2-yl-pyrimidin-4-yl]-2-((S)-2-pyrrolidin-1-ylmethyl-pyrrolidin-1-yl)-acetamid;
2-[4-(3,4-Dimethyl-phenyl)-piperazin-1-yl]-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(4-pyrrolidin-1-yl-piperidin-1-yl)-acetamid;
2-[4-(3-Chloro-phenyl)-piperazin-1-yl]-N-[2-(5-methyl-furan-2-yl)-6-thiazol-2-yl-pyrimidin-4-yl]-acetamid;
2-(2,6-Dimethyl-morpholin-4-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(3-methyl-4-m-tolyl-piperazin-1-yl)-acetamid;
2-(4-Methyl-[1,4]diazepan-1-yl)-N-[2-(5-methyl-furan-2-y))-6-thiazol-2-yl-pyrimidin-4-yl]-acetamid;
2-[4-(3-Methoxy-phenyl)-piperazin-1-yl]-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-[2-(2-piperidin-1-yl-ethyl)-piperidin-1-yl]-acetamid;
N-[2-(5-Methyl-furan-2-yl)-6-thiazol-2-yl-pyrimidin-4-yl]-2-[1,4]oxazepan-4-yl-acetamid;
2-(4,4-Difluoro-piperidin-1-yl)-N-[2-(5-methyl-furan-2-yl)-6-thiazol-2-yl-pyrimidin-4-yl]-acetamid;
2-(4-Acetyl-piperazin-1-yl)-N-[2-(5-methyl-furan-2-yl)-6-thiazol-2-yl-pyrimidin-4-yl]-acetamid;
2-[(1-Benzyl-pyrrolidin-3-yl)-methyl-amino]-N-[2-(5-methyl-furan-2-yl)-6-thiazol-2-yl-pyrimidin-4-yl]-acetamid;
2-(4-Acetyl-[1,4]diazepan-1-yl)-N-[2-(5-methyl-furan-2-yl)-6-thiazol-2-yl-pyrimidin-4-yl]-acetamid;
2-(4-Benzyl-[1,4]diazepan-1-yl)-N-[2-(5-methyl-furan-2-yl)-6-thiazol-2-yl-pyrimidin-4-yl]-acetamid;
2-(3-Dimethylamino-pyrrolidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(3-trifluoromethyl-piperdin-1-yl-acetamid;
2-(3-Diethylamino-pyrrolidin-1-yl)-N-[2-(5-methyl-furan-2-yl)-6-thiazol-2-yl-pyrimidin-4-yl]-acetamid;
2-((R)-3-Dimethylamino-pyrrolidin-1-yl)-N-[2-(5-methyl-furan-2-yl)-6-thiazol-2-yl-pyrimidin-4-yl]-acetamid;
2-((S)-3-Dimethylamino-pyrrolidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(2-pyrrolidin-1-yl-ethylamino)-acetamid;
1-{[2-(5-Methyl-furan-2-yl)-6-thiazol-2-yl-pyrimidin-4-ylcarbamoyl]-methyl}-piperidin-3-carbonsäureamid;
1-{[2-(5-Methyl-furan-2-yl)-6-thiazol-2-yl-pyrimidin-4-ylcarbamoyl]-methyl}-piperidin-4-carbonsäureamid;
N-[2-(5-Methyl-furan-2-yl)-6-thiazol-2-yl-pyrimidin-4-yl]-2-((R)-3-methyl-piperazin-1-yl)-acetamid;
2-[4-(Isopropylcarbamoyl-methyl)-piperazin-1-yl]-N-[2-(5-methyl-furan-2-yl)-6-thiazol-2-yl-pyrimidin-4-yl]-acetamid;
2-(4-Amino-piperidin-1-yl)-N-[2-(5-methyl-furan-2-yl)-6-thiazol-2-yl-pyrimidin-4-yl]-acetamid;
2-(4-Dimethylamino-piperidin-1-yl)-N-[2-(5-methyl-furan-2-yl)-6-thiazol-2-yl-pyrimidin-4-yl]-acetamid;
2-(4-Diethylamino-piperidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(4-morpholin-4-yl-piperidin-1-yl)-acetamid;
2-(4-Isopropylamino-piperidin-1-yl)-N-[2-(5-methyl-furan-2-yl)-6-thiazol-2-yl-pyrimidin-4-yl]-acetamid;
2-(4-Cyclopentylamino-piperidin-1-yl)-N-[2-(5-methyl-furan-2-yl)-6-thiazol-2-yl-pyrimidin-4-yl]-acetamid;
2-(4-Dipropylamino-piperidin-1-yl)-N-[2-(5-methyl-furan-2-yl)-6-thiazol-2-yl-pyrimidin-4-yl]-acetamid;
2-(3-Amino-pyrrolidin-1-yl)-N-[2-(5-methyl-furan-2-yl)-6-thiazol-2-yl-pyrimidin-4-yl]-acetamid;
2-(3-Isopropylamino-pyrrolidin-1-yl)-N-[2-(5-methyl-furan-2-yl)-6-thiazol-2-yl-pyrimidin-4-yl]-acetamid;
2-(3-Cyclopentylamino-pyrrolidin-1-yl)-N-[2-(5~methyl-furan-2-yl)-6-thiazol-2-yl-pyrimidin-4-yl]-acetamid;
2-(4-Acetylamino-piperidin-1-yl)-N-[2-(5-methyl-furan-2-yl)-6-thiazol-2-yl-pyrimidin-4-yl]-acetamid;
N-(1-{[2-(5-Methyl-furan-2-yl)-6-thiazol-2-yl-pyrimidin-4-ylcarbamoyl]-methyl}-piperidin-4-yl)-propionamid;
2-(3-Acetylamino-pyrrolidin-1-yl)-N-[2-(5-methyl-furan-2-yl)-6-thiazol-2-yl-pyrimidin-4-yl]-acetamid;
N-(1-{[2-(5-Methyl-furan-2-yl)-6-thiazol-2-yl-pyrimidin-4-ylcarbamoyl]-methyl}-pyrrolidin- 3-yl)-propionamid;
N-[2-(5-Methyl-furan-2-yl)-6-thiazol-2-yl-pyrimidin-4-yl]-2-(4-thiazol-2-yl-piperazin-1-yl)-acetamid;
2-(Hexahydro-pyrrolo[1,2-a]pyrazin-2-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(3-piperidin-1-yl-pyrrolidin-1-yl)-acetamid;
N-[2-(5-Methy)-furan-2-yl)-6-thiazol-2-yl-pyrimidin-4-yl]-2-(3-morpholin-4-yl-pyrrolidin-1-yl)-acetamid;
N-[2-(5-Methyl-furan-2-yl)-6-thiazol-2-yl-pyrimidin-4-yl]-2-(2-methyl-3-oxo-piperazin-1-yl)-acetamid;
1-{[2-(5-Methyl-furan-2-yl)-6-thiazol-2-yl-pyrimidin-4-ylcarbamoyl]-methyl}-piperidin-3-carbonsäureethylester;
1-{[2-(5-Methyl-furan-2-yl)-6-thiazol-2-yl-pyrimidin-4-ylcarbamoyl]-methyl}-piperidin-4-carbonsäure-(2-hydroxyethyl)-amid;
2-((S)-3-Ethylamino-pyrrolidin-1-yl)-N-[2-(5-methyl-furan-2-yl)-6-thiazol-2-yl-pyrimidin-4-yl]-acetamid;
2-((R)-3-Ethylamino-pyrrolidin-1-yl)-N-[2-(5-methyl-furan-2-yl)-6-thiazol-2-yl-pyrimidin-4-yl]-acetamid;
2-(4-Ethyl-piperazin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-(2-piperidin-1-yl-ethylamino)-acetamid;
N-[2-(5-Methyl-furan-2-yl)-6-thiazol-2-yl-pyrimidin-4-yl]-2-[methyl-(1-methyl-piperidin-4-yl)-amino]-acetamid;
N-[2-(5-Methyl-furan-2-yl)-6-thiazol-2-yl-pyrimidin-4-yl]-2-[2-(1-methyl-pyrrolidin-2-yl)-ethylamino]-acetamid;
N-[2-(5-Methyl-furan-2-yl)-6-thiazol-2-yl-pyrimidin-4-yl]-2-(pyrrolidin-3-ylamino)-acetamid;
N-[2-(5-Methyl-furan-2-yl)-6-thiazol-2-yl-pyrimidin-4-yl]-2-(3-pyrrolidin-1-yl-propylamino)-acetamid;
N-[2-(5-Methyl-furan-2-yl)-6-thiazol-2-yl-pyrimidin-4-yl]-2-(3-piperidin-1-yl-propylamino)-acetamid;
N-[2-(5-Methyl-furan-2-yl)-6-thiazol-2-yl-pyrimidin-4-yl]-2-(3-morpholin-4-yl-propylamino)-acetamid;
2-(4-Hydroxy-piperidin-1-yl)-N-[2-(5-methyl-furan-2-yl)-6-thiazol-2-yl-pyrimidin-4-yl]-acetamid;
2-(3-Hydroxy-piperidin-1-yl)-N-[2-(5-methyl-furan-2-yl)-6-thiazol-2-yl-pyrimidin-4-yl]-acetamid;
2-((S)-3-Hydroxy-pyrrolidin-1-yl)-N-[2-(5-methyl-furan-2-yl)-6-thiazol-2-yl-pyrimidin-4-yl]-acetamid;
2-((S)-3-Acetylamino-pyrrolidin-1-yl)-N-[2-(5-methyl-furan-2-yl)-6-thiazol-2-yl-pyrimidin-4-yl]-acetamid;
2-((R)-3-Acetylamino-pyrrolidin-1-yl)-N-[2-(5-methyl-furan-2-yl)-6-thiazol-2-yl-pyrimidin-4-yl]-acetamid;
2-(3-Hydroxy-pyrrolidin-1-yl)-N-[2-(5-methyl-furan-2-yl)-6-thiazol-2-yl-pyrimidin-4-yl]-acetamid;
2-(4-Isopropyl-piperazin-1-yl)-N-[2-(5-methyl-furan-2-yl)-6-thiazol-2-yl-pyrimidin-4-yl]-acetamid;
2-(4-Cyclopentyl-piperazin-1-yl)-N-[2-(5-methyl-furan-2-yl)-6-thiazol-2-yl-pyrimidin-4-yl]-acetamid;
2-(4-Cyclohexyl-piperazin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl-]-2-(4-propionyl-piperazin-1-yl)-acetamid;
2-(4-Isobutyryl-piperazin-1-yl)-N-[2-(5-methyl-furan-2-yl)-6-thiazol-2-yl-pyrimidin-4-yl]-acetamid;
2-(4-Aminomethyl-piperidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl-]-2-[(pyrrolidin-3-ylmethyl)-amino]-acetamid;
2-(3-Aminomethyl-piperidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-y)-pyrimidin-4-yl]-2-[(1-methyl-pyrrolidin-3-ylmethyl)-amino]-acetamid;
2-(3-Aminomethyl-pyrrolidin-1-yl)-N-[2-(5-methyl-furan-2-yl)-6-thiazol-2-yl-pyrimidin-4-yl]-acetamid;
2-(4-Methoxy-piperidin-1-yl)-N-[2-(5-methyl-furan-2-yl)-6-thiazol-2-yl-pyrimidin-4-yl]-acetamid;
2-(3-Dimethylaminomethyl-piperidin-1-yl)-N-[2-(5-methyl-furan-2-yl)-6-thiazol-2-yl-pyrimidin-4-yl]-acetamid;
2-(4-Dimethylaminomethyl-piperidin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-[methyl-(1-methyl-pyrrolidin-3-ylmethyl)-amino]-acetamid;
2-(3-Dimethylaminomethyl-pyrrolidin-1-yl)-N-[2-(5-methyl-furan-2-yl)-6-thiazo[-2-yl-pyrimidin-4-yl]-acetamid;
N-[2-(5-Methyl-furan-2-yl)-6-thiazol-2-yl-pyrimidin-4-yl]-2-morpholin-4-yl-acetamid;
N-[2-(5-Methyl-furan-2-yl)-6-thiazol-2-yl-pyrimidin-4-yl]-2-(4-phenyl-piperidin-1-yl)-acetamid;
2-(2,5-Dimethyl-piperazin-1-yl)-N-[2-(5-methyl-furan-2-yl)-6-thiazol-2-yl-pyrimidin-4-yl]-acetamid;
N-[2-(5-Methyl-furan-2-yl)-6-thiazol-2-yl-pyrimidin-4-yl]-2-piperazin-1-yl-acetamid;
2-[1,4]Diazepan-1-yl-N-[2-(5-methyl-furan-2-yl)-6-thiazol-2-yl-pyrimidin-4-yl]-acetamid;
3-((S)-3-Dimethylamino-pyrrolidin-1-yl)-N-[2-(5-methyl-furan-2-yl)-6-thiazol-2-yl-pyrimidin-4-yl]-propionamid;
3-(4-Acetyl-piperazin-1-yl)-N-[2-(5-methyl-furan-2-yl)-6-thiazol-2-yl-pyrimidin-4-yl]-propionamid;
N-[2-(5-Methyl-furan-2-yl)-6-thiazol-2-yl-pyrimidin-4-yl]-3-(4-methyl-piperazin-1-yl)-propionamid;
2₋(5-Methyl-furan-2-yl)-6-(3-piperazin-1-yl-propionylamino)-N-vinyl-pyrimidin-4-carboximidothiosäuremethylester;
N-[2-(5-Methyl-furan-2-yl)-6-thiazol-2-yl-pyrimidin-4-yl]-3-(4-pyrrolidin-1-yl-piperidin-1-yl)-propionamid;
N-[2-(5-Methyl-furan-2-yl)-6-thiazol-2-yl-pyrimidin-4-yl]-3-morpholin-4-yl-propionamid;
3-((R)-3-Dimethylamino-pyrrolidin-1-yl)-N-[2-(5-methyl-furan-2-yl)-6-thiazol-2-yl-pyrimidin-4-yl]-propionamid;
3-(4-Acetyl-[1,4]diazepan-1-yl)-N-[2-(5-methyl-furan-2-yl)-6-thiazol-2-yl-pyrimidin-4-yl]-propionamid;
N-[2-(5-Methyl-furan-2-yl)-6-thiazol-2-yl-pyrimidin-4-yl]-3-[1,4]oxazepan-4-yl-propionamid;
3-(4-Methyl-[1,4]diazepan-1-yl)-N-[2-(5-methyl-furan-2-yl)-6-thiazol-2-yl-pyrimidin-4-yl]-propionamid;
3-((R)-3-Ethylamino-pyrrolidin-1-yl)-N-[2-(5-methyl-furan-2-yl)-6-thiazol-2-yl-pyrimidin-4-yl]-propionamid;
3-(3,5-Dimethyl-piperazin-1-yl)-N-[2-(5-methyl-furan-2-yl)-6-thiazol-2-yl-pyrimidin-4-yl]-propionamid;
3-[1,4]Diazepan-1-yl-N-[2-(5-methyl-furan-2-yl)-6-thiazol-2-yl-pyrimidin-4-yl]-propionamid;
N-[2-(5-Methyl-furan-2-yl)-6-thiazol-2-yl-pyrimidin-4-yl]-2-(1-methyl-piperidin-4-yl)-acetamid;
N-[2-(5-Methyl-furan-2-yl)-6-thiazol-2-yl-pyrimidin-4-yl]-2-piperidin-4-yl-acetamid;
N-[2-(5-Methyl-furan-2-yl)-6-thiazol-2-yl-pyrimidin-4-yl]-2-(2-pyrrolidin-1-yl-ethoxy)-acetamid;
N-[6-(3,5-Dimethyl-pyrazol-1-yl)-2-(4-methoxy-pyridin-3-yl)-pyrimidin-4-yl]-2-(4-methyl-piperazin-1-yl)-acetamid;
N-[6-(3,5-Dimethyl-pyrazol-1-yl)-2-(6-methoxy-pyridin-3-yl)-pyrimidin-4-yl]-2-(4-methyl-piperazin-1-yl)-acetamid;
N-[2-(3,4-Dimethoxy-phenyl)-6-(3,5-dimethyl-pyrazol-1-yl)-pyrimidin-4-yl]-2-(4-methyl-piperazin-1-yl)-acetamid;
N-[2-(4-Trifuoromethoxy-phenyl)-6-(3,5-dimethyl-pyrazol-1-yl)-pyrimidin-4-yl]-2-(4-methyl-piperazin-1-yl)-acetamid;
N-[2-(3-Fluoro-phenyl)-6-(3,5-dimethyl-pyrazol-1-yl)-pyrimidin-4-yl]-2-(4-methyl-piperazin-1-yl)-acetamid;
N-[2-(2-Fluoro-phenyl)-6(3,5-dimethyl-pyrazol-1-yl)-pyrimidin-4-yl]-2-(4-methyl-piperazin-1-yl)-acetamid;
N-[2-(2-Fluoro-3-methoxy-phenyl)-6-(3,5-dimethyl-pyrazol-1-yl)-pyrimidin-4-yl]-2-(4-methyl-piperazin-1-yl)-acetamid;
N-[2-(2,4-Dimethoxy-phenyl)-6-(3,5-dimethyl-pyrazol-1-yl)-pyrimidin-4-yl]-2-(4-methyl-piperazin-1-yl)-acetamid;
N-[2-(2-Cyano-phenyl)-6-(3,5-dimethyl-pyrazol-1-yl)-pyrimidin-4-yl]-2-(4-methyl-piperazin-1-yl)-acetamid;
N-[2-(2-Methoxy-phenyl)-6-(3,5-dimethyl-pyrazol-1-yl)-pyrimidin-4-yl]-2-(4-methyl-piperazin-1-yl)-acetamid;
N-[2-(4-Fluoro-2-methyl-phenyl)-6-(3,5-dimethyl-pyrazol-1-yl)-pyrimidin-4-yl]-2-(4-methyl-piperazin-1-yl)-acetamid;
N-[2-(3-Methoxy-phenyl)-6-(3,5-dimethyl-pyrazol-1-yl)-pyrimidin-4-yl]-2-(4-methyl-piperazin-1-yl)-acetamid;
N-[2-(3,5-Dimethoxy-phenyl)-6-(3,5-dimethyl-pyrazol-1-yl)-pyrimidin-4-yl]-2-(4-methyl-piperazin-1-yl)-acetamid;
N-[2-(3,5-Difluoro-phenyl)-6-(3,5-dimethyl-pyrazol-1-yl)-pyrimidin-4-yl]-2-(4-methyl-piperazin-1-yl)-acetamid;
N-[2-(3-Fluoro-4-methyl-phenyl)-6-(3,5-dimethyl-pyrazol-1-yl)-pyrimidin-4-yl]-2-(4-methyl-piperazin-1-yl)-acetamid;
N-[2-(3-Fluoro-2-methoxy-phenyl)-6-(3,5-dimethyl-pyrazol-1-yl)-pyrimidin-4-yl]-2-(4-methyl-piperazin-1-yl)-acetamid;
N-[2-(3-Fluoro-4-methoxy-phenyl)-6-(3,5-dimethyl-pyrazol-1-yl)-pyrimidin-4-yl]-2-(4-methyl-piperazin-1-yl)-acetamid;
N-[2-(2,3-Difluoro-phenyl)-6-(3,5-dimethyl-pyrazol-1-yl)-pyrimidin-4-yl]-2-(4-methyl-piperazin-1-yl)-acetamid;
N-[2-(5-Methoxy-pyridin-3-yl)-6-(3,5-dimethyl-pyrazol-1-yl)-pyrimidin-4-yl]-2-(4-methyl-piperazin-1-yl)-acetamid N-[2-(3-Methoxy-phenyl)-6-pyrazol-1-yl-pyrimidin-4-yl]-2-(4-methyl-piperazin-1-yl)-acetamid;
N-[2-(3,4-Dimethoxy-phenyl)-6-pyrazol-1-yl-pyrimidin-4-yl]-2-(4-methyl-piperazin-1-yl)-acetamid;
N-[2-(3-Cyano-phenyl)-6-pyrazol-1-yl-pyrimidin-4-yl]-2-(4-methyl-piperazin-1-yl)-acetamid;
N-[2-(3-Flouro-4-Methoxy-phenyl)-6-pyrazol-1-yl-pyrimidin-4-yl]-2-(4-methyl-piperazin-1-yl)-acetamid;
N-[2-(2-Methoxy-phenyl)-6-pyrazol-1-yl-pyrimidin-4-yl]-2-(4-methyl-piperazin-1-yl)-acetamid;
N-[2-(4-Methoxy-phenyl)-6-pyrazol-1-yl-pyrimidin-4-yl]-2-(4-methyl-piperazin-1-yl)-acetamid;
N-[2-(3-Trifluoromethyl-phenyl)-6-pyrazol-1-yl-pyrimidin-4-yl]-2-(4-methyl-piperazin-1-yl)-acetamid;
N-[2-(2,3-Difluoro-phenyl)-6-pyrazol-1-yl-pyrimidin-4-yl]-2-(4-methyl-piperazin-1-yl)-acetamid;
N-[2-(3-Trifluoromethoxy-phenyl)-6-pyrazol-1-yl-pyrimidin-4-yl]-2-(4-methyl-piperazin-1-yl)-acetamid;
N-[2-(2-Fluoro-3-methoxy-phenyl)-6-pyrazol-1-yl-pyrimidin-4-yl]-2-(4-methyl-piperazin- 1-yl)-acetamid;
N-[2-(5-Methyl-furan-2-yl)-6-pyridin-2-yl-pyrimidin-4-yl]-2-morpholin-4-yl-acetamid;
N-[2-(5-Methyl-furan-2-yl)-6-pyridin-2-yl-pyrimidin-4-yl]-2-piperidin-1-yl)-acetamid;
2-[4-(Isopropylcarbamoyl-methyl)-piperazin-1-yl]-N-[2-(5-methyl-furan-2-yl)-6-pyridin-2-yl-pyrimidin-4-yl]-acetamid;
N-[2-(5-Methyl-furan-2-yl)-6-pyridin-2-yl-pyrimidin-4-yl]-2-(4-phenyl-piperidin-1-yl)-acetamid;
2-[1,4']Bipiperidinyl-1'-yl-N-[2-(5-methyl-furan-2-yl)-6-pyridin-2-yl-pyrimidin-4-yl]-acetamid;
2-(3,4-Dihydro-1H-isochinolin-2-yl)-N-[2-(5-methyl-furan-2-yl)-6-pyridin-2-yl-pyrimidin-4-yl]-acetamid;
2-[4-(3-Methoxy-phenyl)-piperazin-1-yl]-N-[2-(5-methyl-furan-2-yl)-6-pyridin-2-yl-pyrimidin-4-yl]-acetamid;
N-[2-(5-Methyl-furan-2-yl)-6-pyridin-2-yl-pyrimidin-4-yl]-2-(4-methyl-piperazin-1-yl)-acetamid;
2-((R)-3-Dimethylamino-pyrrolidin-1-yl)-N-(2-pyridin-2-yl-6-thiazol-2-yl-pyrimidin-4-yl)-acetamid;
2-((S)-3-Dimethylamino-pyrrolidin-1-yl)-N-(2-pyridin-2-yl-6-thiazol-2-yl-pyrimidin-4-yl)-acetamid;
2-(2,5-Dimethyl-piperazin-1-yl)-N-(2-pyridin-2-yl-6-thiazol-2-yl-pyrimidin-4-yl)-acetamid;
2-(3,5-Dimethyl-piperazin-1-yl)-N-(2-pyridin-2-yl-6-thiazol-2-yl-pyrimidin-4-yl)-acetamid;
2-(4-Phenyl-piperazin-1-yl)-N-(2-pyridin-2-yl-6-thiazol-2-yl-pyrimidin-4-yl)-acetamid;
2-[4-(2-Methoxy-phenyl)-piperazin-1-yl]-N-(2-pyridin-2-yl-6-thiazol-2-yl-pyrimidin-4-yl)-acetamid;
2-(4-Methyl-piperazin-1-yl)-N-(2-pyridin-2-yl-6-thiazol-2-yl-pyrimidin-4-yl)-acetamid;
2-(4-Benzyl-piperazin-1-yl)-N-(2-pyridin-2-yl-6-thiazol-2-yl-pyrimidin-4-yl)-acetamid;
2-Morpholin-4-yl-N-(2-pyridin-2-yl-6-thiazol-2-yl-pyrimidin-4-yl)-acetamid;
2-(2,6-Dimethyl-morpholin-4-yl)-N-(2-pyridin-2-yl-6-thiazol-2-yl-pyrimidin-4-yl)-acetamid;
2-Piperidin-1-yl-N-(2-pyridin-2-yl-6-thiazol-2-yl-pyrimidin-4-yl)-acetamid;
2-(2-Methyl-piperidin-1-yl)-N-(2-pyridin-2-yl-6-thiazol-2-yl-pyrimidin-4-yl)-acetamid;
2-(3-Methyl-piperidin-1-yl)-N-(2-pyridin-2-yl-6-thiazol-2-yl-pyrimidin-4-yl)-acetamid;
2-(3,3-Dimethyl-piperidin-1-yl)-N-(2-pyridin-2-yl-6-thiazol-2-yl-pyrimidin-4-yl)-acetamid;
2-(3,5-Dimethyl-piperidin-1-yl)-N-(2-pyridin-2-yl-6-thiazol-2-yl-pyrimidin-4-yl)-acetamid;
2-(4-Methyl-piperidin-1-yl)-N-(2-pyridin-2-yl-6-thiazol-2-yl-pyrimidin-4-yl)-acetamid;
2-(4-Benzyl-piperidin-1-yl)-N-(2-pyridin-2-yl-6-thiazol-2-yl-pyrimidin-4-yl)-acetamid;
2-[1,4']Bipiperidinyl-1'-yl-N-(2-pyridin-2-yl-6-thiazol-2-yl-pyrimidin-4-yl)-acetamid;
2-((S)-2-Methoxymethyl-pyrrolidin-1-yl)-N-(2-pyridin-2-yl-6-thiazol-2-yl-pyrimidin-4-yl)-acetamid;
2-(Octahydro-isochinolin-2-yl)-N-(2-pyridin-2-yl-6-thiazol-2-yl-pyrimidin-4-yl)-acetamid;
N-(2-Pyridin-2-yl-6-thiazol-2-yl-pyrimidin-4-yl)-2-((S)-2-pyrrolidin-1-ylmethyl-pyrrolidin-1-yl)-acetamid;
2-[4-(3,4-Dimethyl-phenyl)-piperazin-1-yl]-N-(2-pyridin-2-yl-6-thiazol-2-yl-pyrimidin-4-yl)-acetamid;
N-(2-Pyridin-2-yl-6-thiazol-2-yl-pyrimidin-4-yl)-2-(4-pyrrolidin-1-yl-piperidin-1-yl)-acetamid;
2-[4-(3-Chloro-phenyl)-piperazin-1-yl]-N-(2-pyridin-2-yl-6-thiazol-2-yl-pyrimidin-4-yl)-acetamid;
2-[4-(3-Methoxy-phenyl)-piperazin-1-yl]-N-(2-pyridin-2-yl-6-thiazol-2-yl-pyrimidin-4-yl)-acetamid;
2-[4-(4-Methoxy-phenyl)-piperazin-1-yl]-N-(2-pyridin-2-yl-6-thiazol-2-yl-pyrimidin-4-yl)-acetamid;
2-(4-Acetyl-piperazin-1-yl)-N-(2-pyridin-2-yl-6-thiazol-2-yl-pyrimidin-4-yl)-acetamid;
2-(4-Methyl-[1,4]diazepan-1-yl)-N-(2-pyridin-2-yl-6-thiazol-2-yl-pyrimidin-4-yl)-acetamid;
2-[2-((S)-1-Methyl-pyrrolidin-2-ylmethyl)-piperidin-1-yl]-N-(2-pyridin-2-yl-6-thiazol-2-yl-pyrimidin-4-yl)-acetamid;
2-[2-(2-Piperidin-1-yl-ethyl)-piperidin-1-yl]-N-(2-pyridin-2-yl-6-thiazol-2-yl-pyrimidin-4-yl)-acetamid;
2-((R)-2-Methyl-piperazin-1-yl)-N-(2-pyridin-2-yl-6-thiazol-2-yl-pyrimidin-4-yl)-acetamid;
2-(2,5-Dihydro-pyrrol-1-yl)-N-(2-pyridin-2-yl-6-thiazol-2-yl-pyrimidin-4-yl)-acetamid;
2-(4-Acetyl-[1,4]diazepan-1-yl)-N-(2-pyridin-2-yl-6-thiazol-2-yl-pyrimidin-4-yl)-acetamid;
2-(3-Dimethylamino-pyrrolidin-1-yl)-N-(2-pyridin-2-yl-6-thiazol-2-yl-pyrimidin-4-yl)-acetamid;
N-(2-Pyridin-2-yl-6-thiazol-2-yl-pyrimidin-4-yl)-2-(3-trifluoromethyl-piperidin-1-yl)-acetamid;
2-(3-Diethylamino-pyrrolidin-1-yl)-N-(2-pyridin-2-yl-6-thiazol-2-yl-pyrimidin-4-yl)-acetamid;
2-((R)-2-Methoxymethyl-pyrrolidin-1-yl)-N-(2-pyridin-2-yl-6-thiazol-2-yl-pyrimidin-4-yl)-acetamid;
2-(2,5-Dimethyl-2,5-dihydro-pyrroI-1-yl)-N-(2-pyridin-2-yl-6-thiazol-2-yl-pyrimidin-4-yl)-acetamid;
N-(2-Pyridin-2-yl-6-thiazol-2-yl-pyrimidin-4-yl)-2-pyrrolidin-1-yl-acetamid;
2-(2,5-Dimethyl-pyrrolidin-1-yl)-N-(2-pyridin-2-yl-6-thiazol-2-yl-pyrimidin-4-yl)-acetamid;
2-(3-Phenyl-piperidin-1-yl)-N-(2-pyridin-2-yl-6-thiazol-2-yl-pyrimidin-4-yl)-acetamid;
2-(2-Phenyl-piperidin-1-yl)-N-(2-pyridin-2-yl-6-thiazol-2-yl-pyrimidin-4-yl)-acetamid;
2-[1,4]Oxazepan-4-yl-N-(2-pyridin-2-yl-6-thiazol-2-yl-pyrimidin-4-yl)-acetamid;
2-(4,4-Difluoro-piperidin-1-yl)-N-(2-pyridin-2-yl-6-thiazol-2-yl-pyrimidin-4-yl)-acetamid;
2-(4-Phenyl-piperidin-1-yl)-N-(2-pyridin-2-yl-6-thiazol-2-yl-pyrimidin-4-yl)-acetamid;
2-Piperazin-1-yl-N-(2-pyridin-2-yl-6-thiazol-2-yl-pyrimidin-4-yl)-acetamid;
2-[4-(Isopropylcarbamoyl-methyl)-piperazin-1-yl]-N-(2-pyridin-2-yl-6-thiazol-2-yl-pyrimidin-4-yl)-acetamid;
1-[(2-Pyridin-2-yl-6-thiazol-2-yl-pyrimidin-4-ylcarbamoyl)-methyl]-piperidin-3-carbonsäureamid;
2-[1,4]Diazepan-1-yl-N-(2-pyridin-2-yl-6-thiazol-2-yl-pyrimidin-4-yl)-acetamid;
2-[Methyl-(2-pyrrolidin-1-yl-ethyl)-amino]-N-(2-pyridin-2-yl-6-thiazol-2-yl-pyrimidin-4-yl)-acetamid;
2-(3,5-Dimethyl-piperazin-1-yl)-N-(2-pyridin-2-yl-6-thiazol-2-yl-pyrimidin-4-yl)-acetamid; 2-(4-Acetyl-piperazin-1-yl)-N-(2-pyridin-2-yl-6-thiazol-2-yl-pyrimidin-4-yl)-acetamid;
2-[4-(3-Chloro-phenyl)-piperazin-1-yl]-N-(2-pyridin-2-yl-6-thiazol-2-yl-pyrimidin-4-yl)-acetamid;
2-Morpholin-4-yl-N-(2-pyridin-2-yl-6-thiazol-2-yl-pyrimidin-4-yl)-acetamid;
2-Piperidin-1-yl-N-(2-pyridin-2-yl-6-thiazol-2-yl-pyrimidin-4-yl)-acetamid;
2-(4-Methyl-piperazin-1-yl)-N-(6-pyridin-2-yl-2-thiophen-2-yl-pyrimidin-4-yl)-acetamid;
2-Morpholin-4-yl-N-(6-pyridin-2-yl-2-thiophen-2-yl-pyrimidin-4-yl)-acetamid;
N-(6-Pyridin-2-yl-2-thiophen-2-yl-pyrimidin-4-yl)-2-pyrrolidin-1-yl-acetamid;
2-[Methyl-(1-methyl-piperidin-4-yl)-amino]-N-(6-pyridin-2-yl-2-thiophen-2-yl-pyrimidin-4-yl)-acetamid;
2-[1,4]Diazepan-1-yl-N-(6-pyridin-2-yl-2-thiophen-2-yl-pyrimidin-4-yl)-acetamid;
2-(4-Methyl-[1,4]diazepan-1-yl)-N-(6-pyridin-2-yl-2-thiophen-2-yl-pyrimidin-4-yl)-acetamid;
2-(4-Ethyl-[1,4]diazepan-1-yl)-N-(6-pyridin-2-yl-2-thiophen-2-yl-pyrimidin-4-yl)-acetamid;
2-(4-Propyl-[1,4]diazepan-1-yl)-N-(6-pyridin-2-yl-2-thiophen-2-yl-pyrimidin-4-yl)-acetamid;
2-(4-Amino-piperidin-1-yl)-N-(6-pyridin-2-yl-2-thiophen-2-yl-pyrimidin-4-yl)-acetamide;
2-(4-Dimethylamino-piperidin-1-yl)-N-(6-pyridin-2-yl-2-thiophen-2-yl-pyrimidin-4-yl)-acetamid;
2-(4-Diethylamino-piperidin-1-yl)-N-(6-pyridin-2-yl-2-thiophen-2-yl-pyrimidin-4-yl)-acetamid;
2-(4-Dipropylamino-piperidin-1-yl)-N-(6-pyridin-2-yl-2-thiophen-2-yl-pyrimidin-4-yl)-acetamid;
2-(4-Acetylamino-piperidin-1-yl)-N-(6-pyridin-2-yl-2-thiophen-2-yl-pyrimidin-4-yl)-acetamid;
N-{1-[(6-Pyridin-2-yl-2-thiophen-2-yl-pyrimidin-4-ylcarbamoyl)~methyl]-piperidin-4-yl}-propionamid;
N-(6-Pyridin-2-yl-2-thiophen-2-yl-pyrimidin-4-yl)-2-(4-pyrrolidin-1-yl-piperidin-1-yl)-acetamid;
2-(4-Morpholin-4-yl-piperidin-1-yl)-N-(6-pyridin-2-yl-2-thiophen-2-yl-pyrimidin-4-yl)-acetamid;
2-[1,4']Bipiperidinyl-1-yl-N-(6-pyridin-2-yl-2-thiophen-2-yl-pyrimidin-4-yl)-acetamid;
2-[4-(2-Oxo-imidazolidin-1-yl)-piperidin-1-yl]-N-(6-pyridin-2-yl-2-thiophen-2-yl-pyrimidin-4-yl)-acetamid;
2-(4-Acetimidoylamino-piperidin-1-yl)-N-(6-pyridin-2-yl-2-thiophen-2-yl-pyrimidin-4-yl)-acetamid;
2-(4-Azetidin-1-yl-piperidin-1-yl)-N-(6-pyridin-2-yl-2-thiophen-2-yl-pyrimidin-4-yl)-acetamid;
N-(2-Furan-2-yl-6-thiazol-2-yl-pyrimidin-4-yl)-2-(4-methyl-piperazin-1-yl)-acetamid;
2-Piperidin-1-yl-N-(6-thiazol-2-yl-2-thiophen-2-yl-pyrimidin-4-yl)-acetamid;
2-(2-Pyrrolidin-1-ylmethyl-piperidin-1-yl)-N-(6-thiazol-2-yl-2-thiophen-2-yl-pyrimidin-4-yl)-acetamid;
2-[2-(2-Piperidin-1-yl-ethyl)-piperidin-1-yl]-N-(6-thiazol-2-yl-2-thiophen-2-yl-pyrimidin-4-yl)-acetamid;
2-(3-Methyl-piperidin-1-yl)-N-(6-thiazol-2-yl-2-thiophen-2-yl-pyrimidin-4-yl)-acetamid;
N-(6-Thiazol-2-yl-2-thiophen-2-yl-pyrimidin-4-yl)-2-(3-trifluoromethyl-piperidin-1-yl)-acetamid;
1-[(6-Thiazol-2-yl-2-thiophen-2-yl-pyrimidin-4-ylcarbamoyl)-methyl]-piperidin-3-carbonsäureamid;
2-[1,4']Bipiperidinyl-1-yl-N-(6-thiazol-2-yl-2-thiophen-2-yl-pyrimidin-4-yl)-acetamid;
2-[1,4]Diazepan-1-yl-N-(6-thiazol-2-yl-2-thiophen-2-yl-pyrimidin-4-yl)-acetamid;
2-(4-Acetyl-[1,4]diazepan-1-yl)-N-(6-thiazol-2-yl-2-thiophen-2-yl-pyrimidin-4-yl)-acetamid;
2-(4-Pyrrolidin-1-yl-piperidin-1-yl)-N-(6-thiazol-2-yl-2-thiophen-2-yl-pyrimidin-4-yl)-acetamid;
2-Piperazin-1-yl-N-(6-thiazol-2-yl-2-thiophen-2-yl-pyrimidin-4-yl)-acetamid;
2-(2,5-Dimethyl-piperazin-1-yl)-N-(6-thiazol-2-yl-2-thiophen-2-yl-pyrimidin-4-yl)-acetamid;
2-(3,5-Dimethyl-piperazin-1-yl)-N-(6-thiazol-2-yl-2-thiophen-2-yl-pyrimidin-4-yl)-acetamid;
2-(4-Acetyl-piperazin-1-yl)-N-(6-{1-[(E)-methylimino]-ethyl}-2-thiophen-2-yl-pyrimidin-4-yl)-acetamid;
2-(4-Phenyl-piperazin-1-yl)-N-(6-thiazol-2-yl-2-thiophen-2-yl-pyrimidin-4-yl)-acetamid;
N-Isopropyl-2-{4-[(6-thiazol-2-yl-2-thiophen-2-yl-pyrimidin-4-ylcarbamoyl)-methyl]-piperazin-1-yl}-acetamid;
2-Morpholin-4-yl-N-(6-thiazol-2-yl-2-thiophen-2-yl-pyrimidin-4-yl)-acetamid;
2-(2,6-Dimethyl-morpholin-4-yl)-N-(6-thiazol-2-yl-2-thiophen-2-yl-pyrimidin-4-yl)-acetamid;
N-(6-Thiazol-2-yl-2-thiophen-2-yl-pyrimidin-4-yl)-2-thiomorpholin-4-yl-acetamid;
2-Pyrrolidin-1-yl-N-(6-thiazol-2-yl-2-thiophen-2-yl-pyrimidin-4-yl)-acetamid;
2-(3-Dimethylamino-pyrrolidin-1-yl)-N-(6-thiazol-2-yl-2-thiophen-2-yl-pyrimidin-4-yl)-acetamid;
2-(3-Diethylamino-pyrrolidin-1-yl)-N-(6-thiazol-2-yl-2-thiophen-2-yl-pyrimidin-4-yl)-acetamid;
2-[2-(1-Methyl-pyrrolidin-2-yl)-ethylamino]-N-(6-thiazol-2-yl-2-thiophen-2-yl-pyrimidin-4-yl)-acetamid;
2-(3,5-Dimethyl-piperidin-1-yl)-N-(6-thiazol-2-yl-2-thiophen-2-yl-pyrimidin-4-yl)-acetamid;
2-(4,4-Difluoro-piperidin-1-yl)-N-(6-thiazol-2-yl-2-thiophen-2-yl-pyrimidin-4-yl)-acetamid;
2-(4-Phenyl-piperidin-1-yl)-N-(6-thiazol-2-yl-2-thiophen-2-yl-pyrimidin-4-yl)-acetamid;
2-Azepan-1-yl-N-(6-thiazol-2-yl-2-thiophen-2-yl-pyrimidin-4-yl)-acetamid;
1-[(6-Thiazol-2-yl-2-thiophen-2-yl-pyrimidin-4-ylcarbamoyl)-methyl]-piperidin-4-carbonsäureamid;
2-[4-(3-Methoxy-phenyl)-piperazin-1-yl]-N-(6-thiazol-2-yl-2-thiophen-2-yl-pyrimidin-4-yl)-acetamid;
2-((R)-3-Dimethylamino-pyrrolidin-1-yl)-N-(6-thiazol-2-yl-2-thiophen-2-yl-pyrimidin-4-yl)-acetamid;
2-((S)-3-Dimethylamino-pyrrolidin-1-yl)-N-(6-thiazol-2-yl-2-thiophen-2-yl-pyrimidin-4-yl)-acetamid;
2-[(1-Ethyl-pyrrolidin-2-ylmethyl)-amino]-N-(6-thiazol-2-y]-2-thiophen-2-yl-pyrimidin-4-yl)-acetamid;
2-((R)-3-Ethylamino-pyrrolidin-1-yl)-N-(6-thiazol-2-yl-2-thiophen-2-yl-pyrimidin-4-yl)-acetamid;
2-((S)-3-Ethylamino-pyrrolidin-1-yl)-N-(6-thiazol-2-yl-2-thiophen-2-yl-pyrimidin-4-yl)-acetamid;
2-(4-Methyl-piperazin-1-yl)-N-(6-thiazol-2-yl-2-thiophen-2-yl-pyrimidin-4-yl)-acetamid;
1-[(6-Thiazol-2-yl-2-thiophen-2-yl-pyrimidin-4-ylcarbamoyl)-methyl]-piperidin-3-carbonsäureamid;
2-(4-Pyrrolidin-1-yl-piperidin-1-yl)-N-(6-thiazol-2-yl-2-thiophen-2-yl-pyrimidin-4-yl)-acetamid;
2-(3,5-Dimethyl-piperazin-1-yl)-N-(6-thiazol-2-yl-2-thiophen-2-yl-pyrimidin-4-yl)-acetamid;
2-[2-(1-Methyl-pyrrolidin-2-yl)-ethylamino]-N-(6-thiazol-2-yl-2-thiophen-2-yl-pyrimidin-4-yl)-acetamid;
N-[2-(5-Methyl-furan-2-yl)-6-pyrazol-1-yl-pyrimidin-4-yl]-2-(4-pyrrolidin-1-yl-piperidin-1-yl)-acetamid;
N-[2-(5-Methyl-furan-2-yl)-6-pyrazol-1-yl-pyrimidin-4-yl]-2-(4-methyl-piperazin-1-yl)-acetamid;
2-(4-Acetyl-piperazin-1-yl)-N-[2-(5-methyl-furan-2-yl)-6-pyrazol-1-yl-pyrimidin-4-yl]-acetamid;
2-((S)-3-Ethylamino-pyrrolidin-1-yl)-N-[2-(5-methyl-furan-2-yl)-6-pyrazol-1-yl-pyrimidin-4-yl]-acetamid;
2-(3,5-Dimethyl-piperazin-1-yl)-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-((R)-3-ethylamino-pyrrolidin-1-yl)-acetamid
N*-*[6-(3,5-Dimethyl-pyrazol-1-yl)-2-(5-methyl-furan-2-yl)-pyrimidin-4-yl]-2-((S)-3-ethylamino-pyrrolidin-1-yl)-acetamid;
2-(3-Diethylamino-pyrrolidin-1-yl)-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(pyrrolidin-3-ylamino)-acetamid;
N-[6-(3,5-Dimethyl-pyrazol-1-yl)-2-(5-methyl-furan-2-yl)-pyrimidin-4-yl]-2-(2-pyrrolidin-1-yl-ethylamino)-acetamid;
N-[6-(3,5-Dimethyl-pyrazol-1-yl)-2-(5-methyl-furan-2-yl)-pyrimidin-4-yl]-2-(3-pyrrolidin-1-yl-propylamino)-acetamid;
N-[6-(3,5-Dimethyl-pyrazol-1-yl)-2-(5-methyl-furan-2-yl)-pyrimidin-4-yl]-2-[2-(1-methyl-pyrrolidin-2-yl)-ethylamino]-acetamid;
N-[6-(3,5-Dimethyl-pyrazol-1-yl)-2-(5-methyl-furan-2-yl)-pyrimidin-4-yl]-2-(2-piperidin-1-yl-ethylamino)-acetamid;
N-[6-{3,5-Dimethyl-pyrazol-1-yl)-2-(5-methyl-furan-2-yl)-pyrimidin-4-yl]-2-(3-piperidin-1-yl-propylamino)-acetamid;
N-[6-(3,5-Dimethyl-pyrazol-1-yl)-2-(5-methyl-furan-2-yl)-pyrimidin-4-yl]-2-(3-morpholin-4-yl-propylamino)-acetamid;
N-[6-(3,5-Dimethyl-pyrazol-1-yl)-2-(5-methyl-furan-2-yl)-pyrimidin-4-yl]-2-(4-methyl-piperazin-1-yl)-acetamid;
N-[6-(3,5-Dimethyl-pyrazol-1-yl)-2-(5-methyl-furan-2-yl)-pyrimidin-4-yl]-2-morpholin-4-yl-acetamid;
2-[1,4]Diazepan-1-yl-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(4-pyrrolidin-1-yl-piperidin-1-yl)-acetamid;
N-[6-(3,5-Dimethyl-pyrazol-1-yl)-2-(5-methyl-furan-2-yl)-pyrimidin-4-yl]-2-(4-methyl-[1,4]diazepan-1-yl)-acetamid;
N-[6-(3,5-Dimethyl-pyrazol-1-yl)-2-(5-methyl-furan-2-yl)-pyrimidin-4-yl]-2-[1,4]oxazepan-4-yl-acetamid;
2-((R)-3-Dimethylamino-pyrrolidin-1-yl)-N-[6-(3,5-dimethyl-pyrazol-1-yl)-2-(5-methyl-furan-2-yl)-pyrimidin-4-yl]-acetamid;
2-((S)-3-Dimethylamino-pyrrolidin-1-yl)-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-pyrrolidin-1-yl-acetamid;
2-(4,4-Difluoro-piperidin-1-yl)-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(4-thiazol-2-yl-piperazin-1-yl)-acetamid;
N-[6-(3,5-Dimethyl-pyrazol-1-yl)-2-(5-methyl-furan-2-yl)-pyrimidin-4-yl]-2-(4-ethyl-piperazin-1-yl)-acetamid;
2-((R)-3-Acetylamino-pyrrolidin-1-yl)-N-[6-(3,5-dimethylpyrazol-1-yl)-2-(5-methyl-furan-2-yl)-pyrimidin-4-yl]-acetamid;
2-((S)-3-Acetylamino-pyrrolidin-1-yl)-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-piperazin-1-yl-acetamid;
2-(2,6-Dimethyl-morpholin-4-yl)-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(4-hydroxy-piperidin-1-yl)-acetamid;
2-[1,4']Bipiperidinyl-1'-yl-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(4-methoxy-piperidin-1-yl)-acetamid;
2-(4-Acetyl-piperazin-1-yl)-N-[6-(3,5-dimethyl-pyrazol-1-yl)-2-(5-methyl-furan-2-yl)-pyrimidin-4-yl]-acetamid;
2-((R)-3-Diethylamino-pyrrolidin-1-yl)-N-[6-(3,5-dimethyl-pyrazol-1-yl)-2-(5-methyl-furan-2-yl)-pyrimidin-4-yl]-acetamid;
2-((S)-3-Diethylamino-pyrrolidin-1-yl)-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-[(S)-3-(ethyl-methyl-amino)-pyrrolidin-1-yl]-acetamid;
N-[6-(3,5-Dimethyl-pyrazol-1-yl)-2-(5-methyl-furan-2-yl)-pyrimidin-4-yl]-2-[(R)-3-(ethyl-methyl-amino)-pyrrolidin-1-yl]-acetamid;
2-((S)-3-Amino-pyrrolidin-1-yl)-N-[6-(3,5-dimethylpyrazol-1-yl)-2-(5-methyl-furan-2-yl)-pyrimidin-4-yl]-acetamid;
2-((R)-3-Amino-pyrrolidin-1-yl)-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-({R)-3-morpholin-4-yl-pyrrolidin-1-yl)-acetamid;
N-[6-(3,5-Dimethyl-pyrazol-1-yl)-2-(5-methyl-furan-2-yl)-pyrimidin-4-yl]-2-((R)-3-piperidin-1-yl-pyrrolidin-1-yl)-acetamid;
2-(R)-[1,3']Bipyrrolidinyl-1'-yl-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-((S)-3-morpholin-4-yl-pyrrolidin-1-yl)-acetamid;
2-(S)-[1,3']Bipyrrolidinyl-1'-yl-N-[6-(3,5-dimethylpyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-((S)-3-piperidin-1-yl-pyrrolidin-1-yl)-acetamid;
N-(1-{[6-(3,5-Dimethyl-pyrazol-1-yl)-2-(5-methyl-furan-2-yl)-pyrimidin-4-ylcarbamoyl]-methyl}-pyrrolidin-3-yl)-2,2,2-trifluoro-acetamid;
N-[6-(3,5-Dimethyl-pyrazol-1-yl)-2-(5-methyl-furan-2-yl)-pyrimidin-4-yl]-2-((R)-3-hydroxy-pyrrolidin-1-yl)-acetamid;
N-[6-(3,5-Dimethyl-pyrazol-1-yl)-2-(5-methyl-furan-2-yl)-pyrimidin-4-yl]-2-((S)-3-hydroxy-pyrrolidin-1-yl)-acetamid;
N-[6-(3,5-Dimethyl-pyrazol-1-yl)-2-(5-methyl-furan-2-yl)-pyrimidin-4-yl]-2-((3R,3'R)-3-fluoro-[1,3']bipyrrolidinyl-1'-yl)-acetamid;
N-[6-(3,5-Dimethyl-pyrazol-1-yl)-2-(5-methyl-furan-2-yl)-pyrimidin-4-yl]-2-{(R)-3-[(2-methoxy-ethyl)-methyl-amino]-pyrrolidin-1-yl}-acetamid;
N-[6-(3,5-Dimethyl-pyrazol-1-yl)-2-(5-methyl-furan-2-yl)-pyrimidin-4-yl]-2-((35,3'S)-3-fluoro-[1,3']bipyrrolidinyl-1'-yl)-acetamid;
N-[6-(3,5-Dimethyl-pyrazol-1-yl)-2-(5-methyl-furan-2-yl)-pyrimidin-4-yl]-2-((3R,3'S)-3-fluoro-[1,3']bipyrrolidinyl-1'-yl)-acetamid;
N-[6-(3,5-Dimethyl-pyrazol-1-yl)-2-(5-methyl-furan-2-yl)-pyrimidin-4-yl]-2-(2,5,2',3',4',5'-hexahydro-[1,3']bipyrrolidinyl-1'-yl}-acetamid;
N-[6-(3,5-Dimethyl-pyrazol-1-yl)-2-(5-methyl-furan-2-yl)-pyrimidin-4-yl]-2-{(S)-3-[(2-methoxy-ethyl)-methyl-amino]-pyrrolidin-1-yl}-acetamid;
N-[6-(3,5-Dimethyl-pyrazol-1-yl)-2-{5-methyl-furan-2-yl)-pyrimidin-4-yl]-2-((S)-3-fluoro-pyrrolidin-1-yl)-acetamid;
N-[6-(3,5-Dimethyl-pyrazol-1-yl)-2-{5-methyl-furan-2-yl)-pyrimidin-4-yl]-2-((R)-3-fluoro-pyrrolidin-1-yl)-acetamid;
N-[6-(3,5-Dimethyl-pyrazol-1-yl)-2-(5-methyl-furan-2-yl)-pyrimidin-4-yl]-2-piperidin-1-yl-acetamid;
N-[6-(3,5-Dimethyl-pyrazol-1-yl)-2-(5-methyl-furan-2-yl)-pyrimidin-4-yl]-2-[(R)-3-(2-methoxy-ethylamino)-pyrrolidin-1-yl]-acetamid;
N-[6-(3,5-Dimethyl-pyrazol-1-yl)-2-(5-methyl-furan-2-yl)-pyrimidin-4-yl]-2-(3-trifluoromethyl-piperidin-1-yl)-acetamid;
N-[6-(3,5-Dimethyl-pyrazol-1-yl)-2-(5-methyl-furan-2-yl)-pyrimidin-4-yl]-2-(3-trifluoromethyl-5,6-dihydro-8H-[1,2,4]triazolo[4,3-a]pyrazin-7-yl)-acetamid;
N-[6-(3,5-Dimethyl-pyrazol-1-yl)-2-(5-methyl-furan-2-yl)-pyrimidin-4-yl]-2-[4-(2-oxo-pyrrolidin-1-yl)-piperidin-1-yl]-acetamid;
2-(4-Acetylamino-piperidin-1-yl)-N-[6-(3,5-dimethylpyrazol-1-yl)-2-(5-methyl-furan-2-yl)-pyrimidin-4-yl]-acetamid;
4-{[6-(3,5-Dimethyl-pyrazol-1-yl)-2-(5-methyl-furan-2-yl)-pyrimidin-4-ylcarbamoyl]-methyl}-piperazin-1-carbonsäurephenylester;
4-{[6-(3,5-Dimethyl-pyrazol-1-yl)-2-(5-methyl-furan-2-yl)-pyrimidin-4-ylcarbamoyl]-methyl}-piperazin-1-carbonsäurebenzylamid;
N-[6-(3,5-Dimethyl-pyrazol-1-yl)-2-(5-methyl-furan-2-yl)-pyrimidin-4-yl]-2-[4-(3-methyl-benzoyl)-piperazin-1-yl]-acetamid;
4-{[6-(3,5-Dimethyl-pyrazol-1-yl)-2-(5-methyl-furan-2-yl)-pyrimidin-4-ylcarbamoyl]-methyl]-piperazin-1-carbonsäurediethylamid;
N-[6-(3,5-Dimethyl-pyrazol-1-yl)-2-(5-methyl-furan-2-yl)-pyrimidin-4-yl]-2-{(S)-3-[ethyl(2-methoxy-ethyl)-amino]-pyrrolidin-1-yl}-acetamid;
N-[6-(3,5-Dimethyl-pyrazol-1-yl)-2-(5-methyl-furan-2-yl)-pyrimidin-4-yl]-2-((S)-3-isopropoxy-pyrrolidin-1-yl)-acetamid;
2-(3,9-Diaza-bicyclo[4.2.1]non-3-yl)-N-[6-(3,5-dimethylpyrazol-1-yl)-2-(5-methyl-furan-2-yl)-pyrimidin-4-yl]-acetamid;
3-{[6-(3,5-Dimethyl-pyrazol-1-yl)-2-(5-methyl-furan-2-yl)-pyrimidin-4-ylcarbamoyl]-methyl}-3,9-diaza-bicyclo[4.2.1]nonan-9-carbonsäure-tert-butylester;
N-[6-(3,5-Dimethyl-pyrazol-1-yl)-2-(5-methyl-furan-2-yl)-pyrimidin-4-yl]-2-(9-methyl-3,9-diaza-bicyclo[4.2.1]non-3-yl)-acetamid;
N-[6-(3,5-Dimethyl-pyrazol-1-yl)-2-(5-methyl-furan-2-yl)-pyrimidin-4-yl]-2-(4-{[(2-methoxy-ethyl)-methyl-amino]-methyl}-piperidin-1-yl)-acetamid;
N-[6-(3,5-Dimethyl-pyrazol-1-yl)-2-(5-methyl-furan-2-yl)-pyrimidin-4-yl]-2-[9-(2-methoxy-ethyl)-3,9-diaza-bicyclo[4.2.1]non-3-yl]-acetamid;
(1-{[6-(3,5-Dimethyl-pyrazol-1-yl)-2-(5-methyl-furan-2-yl)-pyrimidin-4-ylcarbamoyl]-methyl}-piperidin-4-yl)-methyl-carbamsäure-tert-butylester;
N-[6-(3,5-Dimethyl-pyrazol-1-yl)-2-(5-methyl-furan-2-yl)-pyrimidin-4-yl]-2-(4-{[(2-methoxy-ethyl)-methyl-amino]-piperidin-1-yl)-acetamid;
2-(2-Dimethylamino-morpholin-4-yl)-N-[6-(3,5-dimethylpyrazol-1-yl)-2-(5-methyl-furan-2-yl)-pyrimidin-4-yl]-acetamid;
2-((S)-3-Dimethylamino-piperidin-1-yl)-N-[6-(3,5-dimethyl-pyrazol-1-yl)-2-(5-methyl-furan-2-yl)-pyrimidin-4-yl]-acetamid;
2-((R)-3-Dimethylamino-piperidin-1-yl)-N-[6-(3,5-dimethyl-pyrazol-1-yl)-2-(5-methyl-furan-2-yl)-pyrimidin-4-yl]-acetamid;
2-((R)-3-Dimethylamino-pyrrolidin-1-yl)-N-[6-(3,5-dimethyl-pyrazol-1-yl)-2-(5-methyl-furan-2-yl)-pyrimidin-4-yl]-acetamid;
2-((S)-3-Dimethylaminomethyl-pyrrolidin-1-yl)-N-[6-(3,5-dimethyl-pyrazol-1-yl)-2-(5-methyl-furan-2-yl)-pyrimidin-4-yl]-acetamid;
2-(4-Dimethylaminomethyl-piperidin-1-yl)-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(1-methyl-piperidin-4-ylamino)-acetamid;
N-[6-(3,5-Dimethyl-pyrazol-1-yl)-2-(5-methyl-furan-2-yl)-pyrimidin-4-yl]-2-((R)-pyrrolidin-3-ylamino)-acetamid;
N-[6-(3,5-Dimethyl-pyrazol-1-yl)-2-(5-methyl-furan-2-yl)-pyrimidin-4-yl]-2-{[(R)-1-(tetrahydro-furan-2-yl)methyl]-amino}-acetamid;
N-[6-(3,5-Dimethyl-pyrazol-1-yl)-2-(5-methyl-furan-2-yl)-pyrimidin-4-yl]-2-[(tetrahydro-pyran-4-ylmethyl)-amino]-acetamid;
N-[6-(3,5-Dimethyl-pyrazol-1-yl)-2-(5-methyl-furan-2-yl)-pyrimidin-4-yl]-2-{[(S)-1-(tetrahydro-furan-2-yl)methyl]-amino}-acetamid;
N-[6-(3,5-Dimethyl-pyrazol-1-yl)-2-(5-methyl-furan-2-yl)-pyrimidin-4-yl]-2-(piperidin-4-ylamino)-acetamid;
N-[6-(3,5-Dimethyl-pyrazol-1-yl)-2-(5-methyl-furan-2-yl)-pyrimidin-4-yl]-2-((S)-piperidin-3-ylamino)-acetamid;
2-(Azetidin-3-ylamino)-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-2-(2-morpholin-4-yl-ethylamino)-acetamid;
N-[6-(3,5-Dimethyl-pyrazol-1-yl)-2-(5-methyl-furan-2-yl)-pyrimidin-4-yl]-2-((R)-piperidin-3-ylamino)-acetamid;
N-[6-(3,5-Dimethyl-pyrazol-1-yl)-2-(5-methyl-furan-2-yl)-pyrimidin-4-yl]-2-[((R)-1 -pyrrolidin-2-ylmethyl)-amino]-acetamid;
N-[6-(3,5-Dimethyl-pyrazol-1-yl)-2-(5-methyl-furan-2-yl)-pyrimidin-4-yl]-2-((S)-pyrrolidin-3-ylamino)-acetamid;
N-[6-(3,5-Dimethyl-pyrazol-1-yl)-2-(5-methyl-furan-2-yl)-pyrimidin-4-yl]-2-((R)-pyrrolidin-3-ylamino)-acetamid;
N-[6-(3,5-Dimethy[-pyrazol-1-yl)-2-(5-methyl-furan-2-yl)-pyrimidin-4-yl]-2-[((R)-1-pyrrolidin-3-ylmethyl)-amino]-acetamid;
N-[6-(3,5-Dimethyl-pyrazol-1-yl)-2-(5-methyl-furan-2-yl)-pyrimidin-4-yl]-3-((R)-3-ethylamino-pyrrolidin-1-yl)-propionamid;
3-((S)-3-Dimethylamino-pyrrolidin-1-yl)-N-[6-(3,5-dimethyl-pyrazol-1-yl)-2-(5-methyl-furan-2-yl)-pyrimidin-4-yl]-propionamid;
N-[6-(3,5-Dimethyl-pyrazol-yl)-2-(5-methyl-furan-2-yl)-pyrimidin-4-yl]-3-(4-methyl-piperazin-1-yl)-propionamid;
N-[6-(3,5-Dimethyl-pyrazol-1-yl)-2-(5-methyl-furan-2-yl)-pyrimidin-4-yl]-3-morpholin-4-yl-propionamid;
3-(2,6-Dimethyl-morpholin-4-yl)-N-[6-(3,5-dimethylpyrazol-1-yl)-2-(5-methyl-furan-2-yl)-pyrimidin-4-yl]-propionamid;
N-[6-(3,5-Dimethyl-pyrazol-1-yl)-2-(5-methyl-furan-2-yl)-pyrimidin-4-yl]-3-(4-pyrrolidin-1-yl-piperidin-1-yl)-propionamid;
3-[1,4]Diazepan-1-yl-N-[6-(3,5-dimethyl-pyrazol-1-yl)-2-(5-methyl-furan-2-yl)-pyrimidin-4-yl]-propionamid;
N-[6-(3,5-Dimethyl-pyrazol-1-yl)-2-(5-methyl-furan-2-yl)-pyrimidin-4-yl]-3-(4-methyl-[1,4]diazepan-1-yl)-propionamid;
N-[6-(3,5-Dimethyl-pyrazol-1-yl)-2-(5-methyl-furan-2-yl)-pyrimidin-4-yl]-3-(4-methyl-[1,4]diazepan-1-yl)-propionamid;
3-((R)-3-Dimethylamino-pyrrolidin-1-yl)-N-[6-(3,5-dimethyl-pyrazol-1-y])-2-(5-methyl-furan-2-yl)-pyrimidin-4-yl]-propionamid;
N-[6-(3,5-Dimethyl-pyrazol-1-yl)-2-(5-methyl-furan-2-yl)-pyrimidin-4-yl]-3-((S)-3-ethylamino-pyrrolidin-1-yl)-propionamid;
N-[6-(3,5-Dimethyl-pyrazol-1-yl)-2-(5-methyl-furan-2-yl)-pyrimidin-4-yl]-3-[1,4]oxazepan-4-yl-propionamid;
3-[1,4']Bipiperidinyl-1'-yl-N-[6-(3,5-dimethyl-pyrazol-1-yl)-2-(5-methyl-furan-2-yl)-pyrimidin-4-yl]-propionamid;
N-[6-(3,5-Dimethyl-pyrazol-1-yl)-2-(5-methyl-furan-2-yl)-pyrimidin-4-yl]-3-(4-methoxy-piperidin-1-yl)-propionamid;
N-[6-(3,5-Dimethyl-pyrazol-1-yl)-2-(5-methyl-furan-2-yl)-pyrimidin-4-yl]-3-(4-thiazol-2-yl-piperazin-1-yl)-propionamid;
N-[6-(3,5-Dimethyl-pyrazol-1-yl)-2-(5-methyl-furan-2-yl)-pyrimidin-4-yl]-3-(4-ethyl-piperazin-1-yl)-propionamid;
3-(3-Diethylamino-pyrrolidin-1-yl)-N-[6-(3,5-dimethylpyrazol-1-yl)-2-(5-methyl-furan-2-yl)-pyrimidin-4-yl]-propionamid;
3-((3R,5S)-3,5-Dimethyl-piperazin-1-yl)-N-[6-(3,5-dimethyl-pyrazol-1-yl)-2-(5-methyl-furan-2-yl)-pyrimidin-4-yl]-propionamid;
N-[6-(3,5-Dimethyl-pyrazol-1-y))-2-(5-methyl-furan-2-yl)-pyrimidin-4-yl]-3-piperazin-1-yl-propionamid;
N-[6-(3,5-Dimethyl-pyrazol-1-yl)-2-(5-methyl-furan-2-yl)-pyrimidin-4-yl]-4-morpholin-4-yl-butyramid;
N-[6-(3,5-Dimethyl-pyrazol-1-yl)-2-(5-methyl-furan-2-yl)-pyrimidin-4-yl]-4-pyrrolidin-1-yl-butyramid;
N-[6-(3,5-Dimethyl-pyrazol-1-yl)-2-(5-methyl-furan-2-yl)-pyrimidin-4-yl]-4-((S)-3-fluoro-pyrrolidin-1-yl)-butyramid;
N-[6-(3,5-Dimethyl-pyrazol-1-yl)-2-(5-methyl-furan-2-yl)-pyrimidin-4-yl]-4-((R)-3-fluoro-pyrrolidin-1-yl)-butyramid;
4-{[6-(3,5-Dimethyl-pyrazol-1-yl)-2-(5-methyl-furan-2-yl)-pyrimidin-4-ylcarbamoyl]-methyl}-piperidin-1-carbonsäure-tert-butylester;
N-[6-(3,5-Dimethyl-pyrazol-1-yl)-2-(5-methyl-furan-2-yl)-pyrimidin-4-yl]-2-piperidin-4-yl-acetamid;
N-[6-(3,5-Dimethyl-pyrazol-1-yl)-2-(5-methyl-furan-2-yl)-pyrimidin-4-yl]-2-(1-methyl-piperidin-4-yl)-acetamid;
N-[6-(3,5-Dimethyl-pyrazol-1-yl)-2-(5-methyl-furan-2-yl)-pyrimidin-4-yl]-2-[1-(2-rnethoxy-ethyl)-piperidin-4-yl]-acetamid;
N-[6-(3,5-Dimethyl-pyrazol-1-yl)-2-(5-methyl-furan-2-yl)-pyrimidin-4-yl]-2-(2-pyrrolidin-1-yl-ethoxy)-acetamid;
2-(4-Methyl-piperazin-1-yl)-N-(2-oxazol-2-yl-6-pyrazol-1-yl-pyrimidin-4-yl)-acetamid;
2-((R)-3-Dimethylamino-pyrrolidin-1-yl)-N-(6-pyrazol-1-yl-2-pyridin-2-yl-pyrimidin-4-yl)-acetamid;
2-((S)-3-Dimethylamino-pyrrolidin-1-yl)-N-(6-pyrazol-1-yl-2-pyridin-2-yl-pyrimidin-4-yl)-acetamid;
2-(2,6-Dimethyl-morpholin-4-yl)-N-(6-pyrazol-1-yl-2-pyridin-2-yl-pyrimidin-4-yl)-acetamid;
N-(6-Pyrazol-1-yl-2-pyridin-2-yl-pyrimidin-4-yl)-2-(4-pyrrolidin-1-yl-piperidin-1-yl)-acetamid;
2-(4-Acetyl-piperazin-1-yl)-N-(6-pyrazol-1-yl-2-pyridin-2-yl-pyrimidin-4-yl)-acetamid;
2-(3,5-Dimethyl-piperazin-1-yl)-N-(6-pyrazol-1-yl-2-pyridin-2-yl-pyrimidin-4-yl)-acetamid;
2-(4-Methyl-piperazin-1-yl)-N-(6-pyrazol-1-yl-2-pyridin-2-yl-pyrimidin-4-yl)-acetamid;
2-Morpholin-4-yl-N-(6-pyrazol-1-yl-2-pyridin-2-yl-pyrimidin-4-yl)-acetamid;
2-(4-Dimethylamino-piperidin-1-yl)-N-(6-pyrazol-1-yl-2-pyridin-2-yl-pyrimidin-4-yl)-acetamid;
2-(4-Methyl-[1,4]diazepan-1-yl)-N-(6-pyrazol-1-yl-2-pyridin-2-yl-pyrimidin-4-yl)-acetamid;
2-(3,5-Dimethyl-piperazin-1-yl)-N-[6-(3,5-dimethylpyrazol-1-yl)-2-pyridin-2-yl-pyrimidin-4-yl]-acetamid;
N-[6-(3,5-Dimethyl-pyrazol-1-yl)-2-pyridin-2-yl-pyrimidin-4-yl]-2-(4-pyrrolidin-1-yl-piperidin-1-yl)-acetamid; N-[6-(3,5-Dimethyl-pyrazol-1-yl)-2-pyridin-2-yl-pyrimidin-4-yl]-2-[(S)-3-(ethyl-methyl-amino)-pyrrolidin-1-yl]-acetamid; 2-((R)-3-Amino-pyrrolidin-1-yl)-N-[6-(3,5-dimethylpyrazol-1-yl)-2-pyridin-2-yl-pyrimidin-4-yl]-acetamid; 2-((S)-3-Amino-pyrrolidin-1-yl)-N-[6-(3,5-dimethylpyrazol-1-yl)-2-pyridin-2-yl-pyrimidin-4-yl)-acetamid; N-((S)-1-{[6-(3,5-Dimethyl-pyrazol-1-yl)-2-pyridin-2-yl-pyrimidin-4-ylcarbamoyl]-methyl}-pyrrolidin-3-yl)-2,2,2-trifluoro-acetamid; N-[6-(3,5-Dimethyl-pyrazo)-1-yl)-2-pyridin-2-yl-pyrimidin-4-yl]-2-(3-trifluoromethyl-5,6-dihydro-8H-[1,2,4]triazolo[4,3-a]pyrazin-7-yl)-acetamid; N-[6-(3,5-Dimethyl-pyrazo)-1-yl)-2-pyridin-2-yl-pyrimidin-4-yl]-2-morpholin-4-yl-acetamid; N-[6-(3,5-Dimethyl-pyrazo)-1-yl)-2-pyridin-2-yl-pyrimidin-4-yl]-2-[1,4]oxazepan-4-yl-acetamid; N-[6-(3,5-Dimethyl-pyrazol-1-yl)-2-pyridin-2-yl-pyrimidin-4-yl)-2-(4-methyl-piperazin-1-yl)-acetamid; N-[6-(3,5-Dimethyl-pyrazol-1-yl)-2-pyridin-2-yl-pyrimidin-4-yl]-2-(4-methyl-[1,4]diazepan-1-yl)-acetamid; 2-((R)-3-Dimethylamino-pyrrolidin-1-yl)-N-[6-(3,5-dimethyl-pyrazol-1-yl)-2-pyridin-2-yl-pyrimidin-4-yl]-acetamid; 2-((S)-3-Dimethylamino-pyrrolidin-1-yl)-N-[6-(3,5-dimethyl-pyrazol-1-yl)-2-pyridin-2-yl-pyrimidin-4-yl]-acetamid; 2-((R)-3-Dimethylaminomethyl-piperidin-1-yl)-N-[6-(3,5-dimethyl-pyrazol-1-yl)-2-pyridin-2-yl-pyrimidin-4-yl]-acetamid;
2-((S)-3-Dimethylaminomethyl-piperidin-1-yl)-N-[6-(3,5-dimethyl-pyrazol-1-yl)-2-pyridin-2-yl-pyrimidin-4-yl]-acetamid; N-(2,6-Di-pyrazol-1-yl-pyrimidin-4-yl)-2-(4-methylpiperazin-1-yl)-acetamid; N-(2,6-Di-pyrazol-1-yl-pyrimidin-4-yl)-2-(4-pyrrolidin-1-yl-piperidin-1-yl)-acetamid; 2-(3-Dimethylamino-pyrrolidin-1-yl)-N-(2,6-di-pyrazol-1-yl-pyrimidin-4-yl)-acetamid; N-(2,6-Bis-thiazol-2-yl-pyrimidin-4-yl)-2-(4-methylpiperazin-1-yl)-acetamid; 2-(4-Methyl-piperazin-1-yl)-N-(2-oxazol-5-yl-6-pyrazol-1-yl-pyrimidin-4-yl)-acetamid; 2-(3-Dimethylamino-pyrrolidin-1-yl)-N-(2-oxazol-5-yl-6-pyrazol-1-yl-pyrimidin-4-yl)-acetamid; N-[2-(4-Methyl-oxazol-5-yl)-6-pyrazol-1-yl-pyrimidin-4-yl]-2-(4-methyl-piperazin-1-yl)-acetamid; N-(2-Isoxazol-3-yl-6-pyrazol-1-yl-pyrimidin-4-yl)-2-(4-methyl-piperazin-1-yl)-acetamid; 2-(3-Dimethylamino-pyrrolidin-1-yl)-N-(2-isoxazol-3-yl-6-pyrazol-1-yl-pyrimidin-4-yl)-acetamid; 2-(4-Methyl-piperazin-1-yl)-N-(6-pyrazol-1-yl-2-thiazol-2-yl-pyrimidin-4-yl)-acetamid; N-(6-Pyrazol-1-yl-2-thiazol-2-yl-pyrimidin-4-yl)-2-(4-pyrrolidin-1-yl-piperidin-1-yl)-acetamid; 2-((S)-3-Dimethylamino-pyrrolidin-1-yl)-N-(6-pyrazol-1-yl-2-thiazol-2-yl-pyrimidin-4-yl)-acetamid; 2-((S)-3-Ethylamino-pyrrolidin-1-yl)-N-(6-pyrazol-1-yl-2-thiazol-2-yl-pyrimidin-4-yl)-acetamid; 3-((R)-3-Ethylamino-pyrrolidin-1-yl)-N-(6-pyrazol-1-yl-2-thiazol-2-yl-pyrimidin-4-yl)-propionamid; N-[6-(3,5-Dimethyl-pyrazol-1-yl)-2-thiazol-2-yl-pyrimidin-4-yl]-3-(4-methyl-piperazin-1-yl)-propionamid;
N-[6-(3,5-Dimethyl-pyrazol-1-yl)-2-thiazol-2-yl-pyrimidin-4-yl]-2-(4-pyrrolidin-1-yl-piperidin-1-yl)-acetamid; 2-((S)-3-Dimethylamino-pyrrolidin-1-yl)-N-[6-(3,5-dimethyl-pyrazol-1-yl)-2-thiazol-2-yl-pyrimidin-4-yl]-acetamid; N-[6-(3,5-Dimethyl-pyrazol-1-yl)-2-thiazol-2-yl-pyrimidin-4-yl]-2-((S)-3-ethylamino-pyrrolidin-1-yl)-acetamid; N-[6-(3,5-Dimethyl-pyrazol-1-yl)-2-thiazol-2-yl-pyrimidin-4-yl]-2-(3-trifluoromethyl-5,6-dihydro-8H-[1,2,4]triazolo[4,3-a]pyrazin-7-yl)-acetamid; N-[6-(3,5-Dimethyl-pyrazol-1-yl)-2-furan-2-yl-pyrimidin-4-yl]-2-(4-methyl-piperazin-1-yl)-acetamid; N-[6-(3,5-Dimethyl-pyrazol-1-yl)-2-furan-2-yl-pyrimidin-4-yl]-2-(4-methyl-piperazin-1-yl)-propionamid; N-[2-(5-Methyl-furan-2-yl)-6-(5-methyl-3-trifluoromethyl-pyrazol-1-yl)-pyrimidin-4-yl]-2-(4-methyl-piperazin-1-yl)-acetamid; (S)-Pyrrolidin-3-carbonsäure[6-(3,5-dimethyl-pyrazol-1-yl)-2-(5-methyl-furan-2-yl)-pyrimidin-4-yl]-amid; (S)-1-Methyl-pyrrolidin-3-carbonsäure[6-(3,5-dimethylpyrazol-1-yl)-2-(5-methyl-furan-2-yl)-pyrimidin-4-yl]-amid; (S)-1-(2-Methoxy-ethyl)-pyrrolidin-3-carbonsäure[6-(3,5-dimethyl-pyrazol-1-yl)-2-(5-methyl-furan-2-yl)-pyrimidin-4-yl]-amid; (R)-Pyrrolidin-3-carbonsäure[6-(3,5-dimethyl-pyrazol-1-yl)-2-(5-methyl-furan-2-yl)-pyrimidin-4-yl]-amid; (R)-1-Methyl-pyrrolidin-3-carbonsäure[6-(3,5-dimethylpyrazol-1-yl)-2-(5-methyl-furan-2-yl)-pyrimidin-4-yl]-amid; (R)-1-(2-Methoxy-ethyl)-pyrrolidin-3-carbonsäure[6-(3,5-dimethyl-pyrazol-1-yl)-2-(5-methyl-furan-2-yl)-pyrimidin-4-yl]-amid; (R)-Pyrrolidin-2-carbonsäure[6-(3,5-dimethyl-pyrazol-1-yl)-2-(5-methyl-furan-2-yl)-pyrimidin-4-yl]-amid; (R)-1-(2-Methoxy-ethyl)-pyrrolidin-2-carbonsäure[6-(3,5-dimethyl-pyrazol-1-yl)-2-(5-methyl-furan-2-yl)-pyrimidin-4-yl]-amid; 2-[6-(3,5-Dimethyl-pyrazol-1-yl)-2-(5-methyl-furan-2-yl)-pyrimidin-4-ylcarbamoyl]-pyrrolidin-1-carbonsäure-benzylester; (S)-Pyrrolidin-2-carbonsäure[6-(3,5-dimethyl-pyrazol-1-yl)-2-(5-methyl-furan-2-yl)-pyrimidin-4-yl]-amid; Pyrrolidin-2-carbonsäure[6-(3,5-dimethyl-pyrazol-1-yl)-2-(5-methyl-furan-2-yl)-pyrimidin-4-yl]-amid; N-[6-(3,5-Dimethyl-pyrazol-1-yl)-2-thiazol-2-yl-pyrimidin-4-yl]-2-(4-methyl-piperazin-1-yl)-acetamid; N-[6-(3,5-Dimethyl-pyrazol-1-yl)-2-thiazol-2-yl-pyrimidin-4-yl]-2-(4-pyrrolidin-1-yl-piperidin-1-yl)-propionamid; 2-((R)-3-Dimethylamino-pyrrolidin-1-yl)-N-[6-(3,5-dimethyl-pyrazol-1-yl)-2-thiazol-2-yl-pyrimidin-4-yl]-propionamid; N-[6-(3,5-Dimethyl-pyrazol-1-yl)-2-thiazol-2-yl-pyrimidin-4-yl]-3-((R)-3-ethylamino-pyrrolidin-1-yl)-propionamid; N-[6-(3,5-Dimethyl-pyrazol-1-yl)-2-thiazol-2-yl-pyrimidin-4-yl]-3-[1,4]oxazepan-4-yl-propionamid; 3-((R)-3-Ethylamino-pyrrolidin-1-yl)-N-(6-pyrazol-1-yl-2-thiazol-2-yl-pyrimidin-4-yl)-propionamid; Morpholin-2-carbonsäure[6-(3,5-dimethyl-pyrazol-1-yl)-2-(5-methyl-furan-2-yl)-pyrimidin-4-yl]-amid; 4-Methyl-morpholin-2-carbonsäure[6-(3,5-dimethyl-pyrazol-1-yl)-2-(5-methyl-furan-2-yl)-pyrimidin-4-yl-3-amid;
4-(Pyrrolidin-1-carbonyl)-morpholin-2-carbonsäure[6-(3,5-dimethyl-pyrazol-1-yl)-2-(5-methyl-furan-2-yl)-pyrimidin-4-yl]-amid; Pyrrolidin-1-carbonsäure[6-(3,5-dimethyl-pyrazol-1-yl)-2-(5-methyl-furan-2-yl)-pyrimidin-4-yl]-amid; Morpholin-4-carbonsäure[6-(3,5-dimethyl-pyrazol-1-yl)-2-(5-methyl-furan-2-yl)-pyrimidin-4-yl]-amid; 4-Methyl-piperazin-1-carbonsäure[6-(3,5-dimethyl-pyrazol-1-yl)-2-(5-methyl-furan-2-yl)-pyrimidin-4-yl]-amid; 1-[6-(3,5-Dimethyl-pyrazol-1-yl)-2-(5-methyl-furan-2-yl)-pyrimidin-4-yl]-3-(2-morpholin-4-yl-ethyl)-harnstoff; 1-[6-(3,5-Dimethyl-pyrazol-1-yl)-2-(5-methyl-furan-2-yl)-pyrimidin-4-yl]-3-(3-piperidin-1-yl-propyl)-harnstoff; N-[6-(3,5-Dimethyl-pyrazol-1-yl)-2-(5-methyl-furan-2-yl)-pyrimidin-4-yl]-2-(S)-pyrrolidin-3-yl-acetamid; N-[6-(3,5-Dimethyl-pyrazol-1-yl)-2-(5-methyl-furan-2-yl)-pyrimidin-4-yl]-2-((S)-1-methyl-pyrrolidin-3-yl)-acetamid; (S)-3-{[6-(3,5-Dimethyl-pyrazol-1-yl)-2-(5-methyl-furan-2-yl)-pyrimidin-4-ylcarbamoyl]-methyl}-1,1-dimethyl-pyrrolidinium; N-[6-(3,5-Dimethyl-pyrazol-1-yl)-2-(5-methyl-furan-2-yl)-pyrimidin-4-yl]-2-[(S)-1-(2-methoxy-ethyl)-pyrrolidin-3-yl]-acetamid; N-[6-(3,5-Dimethyl-pyrazol-1-yl)-2-(5-methyl-furan-2-yl)-pyrimidin-4-yl]-2-(R)-pyrrolidin-3-yl-acetamid; N-[6-(3,5-Dimethyl-pyrazol-1-yl)-2-(5-methyl-furan-2-yl)-pyrimidin-4-yl]-2-((R)-1-methyl-pyrrolidin-3-yl)-acetamid; N-[6-(3,5-Dimethyl-pyrazol-1-yl)-2-(5-methyl-furan-2-yl)-pyrimidin-4-yl]-2-[(R)-1-(2-methoxy-ethyl)-pyrrolidin-3-yl]-acetamid; 1-Methyl-piperidin-4-carbonsäure[6-(3,5-dimethyl-pyrazol-1-yl)-2-(5-methyl-furan-2-yl)-pyrimidin-4-yl]-amid;
N-[6-(3,5-Dimethyl-pyrazol-1-yl)-2-(5-methyl-furan-2-yl)-pyrimidin-4-yl]-2-[(R)-1-(2-methoxy-ethyl)-piperidin-3-yl]-acetamid;
1-[6-(3,5-Dimethyl-pyrazol-1-yl)-2-(5-methyl-furan-2-yl)-pyrimidin-4-yl]-3-(3-pyrrolidin-1-yl-propyl)-harnstoff;
1-[6-(3,5-Dimethyl-pyrazol-1-yl)-2-(5-methyl-furan-2-yl)-pyrimidin-4-yl]-3-[3-(4-methyl-piperazin-1-yl)-propyl]-harnstoff;
1-[6-(3,5-Dimethyl-pyrazol-1-yl)-2-(5-methyl-furan-2-yl)-pyrimidin-4-yl]-3-[2-(1-methyl-pyrrolidin-2-yl)-ethyl]-harnstoff;
1-[6-(3,5-Dimethyl-pyrazol-1-yl)-2-(5-methyl-furan-2-yl)-pyrimidin-4-yl]-3-[2-(1-methyl-piperidin-2-yl)-ethyl]-harnstoff;
1-[6-(3,5-Dimethyl-pyrazol-1-yl)-2-(5-methyl-furan-2-yl)-pyrimidin-4-yl]-3-(2-piperidin-2-yl-ethyl)-harnstoff;
1-[6-(3,5-Dimethyl-pyrazol-1-yl)-2-(5-methyl-furan-2-yl)-pyrimidin-4-yl]-3-(3-morpholin-4-yl-ethyl)-harnstoff;
N-[6-(3,5-Dimethyl-pyrazol-1-yl)-2-(5-methyl-furan-2-y])-pyrimidin-4-yl]-2-(2-methoxy-ethylamino)-acetamid;
[6-(3,5-Dimethyl-pyrazol-1-yl)-2-(5-methyl-furan-2-yl)-pyrimidin-4-yl]-carbamsäure-2-azepan-1-yl-ethylester;
[6-(3,5-Dimethyl-pyrazol-1-yl)-2-(5-methyl-furan-2-yl)-pyrimidin-4-yl]-carbamsäure-3-piperidin-1-yl-propylester;
[6-(3,5-Dimethyl-pyrazol-1-yl)-2-(5-methyl-furan-2-yl)-pyrimidin-4-yl]-carbamsäure-3-(2-oxo-pyrrolidin-1-yl)-propylester;
[6-(3,5-Dimethyl-pyrazol-1-yl)-2-(5-methyl-furan-2-yl)-pyrimidin-4-yl]-carbamsäure-2-morpholin-4-yl-ethylester;
[6-(3,5-Dimethyl-pyrazol-1-yl)-2-(5-methyl-furan-2-yl)-pyrimidin-4-yl]-carbamsäure-2-piperidin-1-yl-ethylester;
[6-(3,5-Dimethyl-pyrazol-1-yl)-2-(5-methyl-furan-2-yl)-pyrimidin-4-yl]-carbamsäure-2-(2-oxo-pyrrolidin-1-yl)-ethylester; und
[6-(3,5-Dimethyl-pyrazol-1-yl)-2-(5-methyl-furan-2-yl)-pyrimidin-4-yl]-carbamsäure-2-pyrrolidin-1-yl-ethylester ausgewählt wird.

17. Eine Verbindung gemäß Anspruch 16, wobei die Verbindung aus der Gruppe aus:
N-(2-Furan-2-yl-6-pyrazol-1-yl-pyrimidin-4-yl)-2-(4-methyl-piperazin-1-yl)-acetamid;
N-[2-(2-Fluoro-3-methoxy-phenyl)-6-(3,5-dimethyl-pyrazol-1-yl)-pyrimidin-4-yl]-2-(4-methyl-piperazin-1-yl)-acetamid;
N-(2-Furan-2-yl-6-thiazol-2-yl-pyrimidin-4-yl)-2-(4-methyl-piperazin-1-yl)-acetamid;
N-[6-(3,5-Dimethyl-pyrazol-1-yl)-2-(5-methyl-furan-2-yl)-pyrimidin-4-yl]-2-((S)-3-ethylamino-pyrrolidin-1-yl)-acetamid;
N-[6-(3,5-Dimethyl-pyrazol-1-yl)-2-(5-methyl-furan-2-yl)-pyrimidin-4-yl]-2-(4-methyl-[1,4]-diazepan-1-yl)-acetamid;
2-((S)-3-Dimethylamino-pyrrolidin-1-yl)-N-[6-(3,5-dimethyl-pyrazol-1-yl)-2-(5-methyl-furan-2-yl)-pyrimidin-4-yl]-acetamid;
2-((R)-3-Dimethylamino-pyrrolidin-1-yl)-N-[6-(3,5-dimethyl-pyrazol-1-yl)-2-(5-methyl-furan-2-yl)-pyrimidin-4-yl]-acetamid;
N-[6-(3,5-Dimethyl-pyrazol-1-yl)-2-(5-methyl-furan-2-yl)-pyrimidin-4-yl]-3-((R)-3-ethylamino-pyrrolidin-1-yl)-propionamid;
3-((S)-3-Dimethylamino-pyrrolidin-1-yl)-N-[6-(3,5-dimethyl-pyrazol-1-yl)-2-(5-methyl-furan-2-yl)-pyrimidin-4-yl]-propionamid
N-[6-(3,5-Dimethyl-pyrazol-1-yl)-2-(5-methyl-furan-2-yl)-pyrimidin-4-yl]-[1,4]-oxazepan-4-yl-propionamid;
N-[6-(3,5-Dimethyl-pyrazol-1-yl)-2-pyridin-2-yl-pyrimidin-4-yl]-2-(4-methyl-piperazin-1-yl)-acetamid;
2-(4-Methyl-piperazin-1-yl)-N-(6-pyrazol-1-yl-2-thiazol-2-yl-pyrimidin-4-yl)-acetamid;
N-(6-Pyrazol-1-yl-2-thiazol-2-yl-pyrimidin-4-yl)-2-(4-pyrrolidin-1-yl-piperidin-1-yl)-acetamid;
2-((S)-3-Dimethylamino-pyrrolidin-1-yl)-N-(6-pyrazol-1-yl-2-thiazol-2-yl-pyrimidin-4-yl)-acetamid;
2-((S)-3-Ethylamino-pyrrolidin-1-yl)-N-(6-pyrazol-1-yl-2-thiazol-2-yl-pyrimidin-4-yl)-acetamid;
N-[6-(3,5-Dimethyl-pyrazol-1-yl)-2-thiazol-2-yl-pyrimidin-4-yl]-2-(4-methyl-piperazin-1-yl)-acetamid;
N-[6-(3,5-Dimethyl-pyrazol-1-yl)-2-thiazol-2-yl-pyrimidin-4-yl]-2-(4-pyrrolidin-1-yl-piperidin-1-yl)-acetamid;
2-((S)-3-Dimethylamino-pyrrolidin-1-yl)-N-[6-(3,5-dimethyl-pyrazol-1-yl)-2-thiazol-2-yl-pyrimidin-4-yl]-acetamid;
N-[6-(3,5-Dimethyl-pyrazol-1-yl)-2-thiazol-2-yl-pyrimidin-4-yl]-2-((S)-3-ethylamino-pyrrolidin-1-yl)-acetamid;
2-((R)-3-Dimethylamino-pyrrolidin-1-yl)-N-[6-(3,5-dimethyl-pyrazol-1-yl)-2-thiazol-2-yl-pyrimidin-4-yl]-propionamid; und
1-[6-(3,5-Dimethyl-pyrazol-1-yl)-2-(5-methyl-furan-2-yl)-pyrimidin-4-yl]-3-(3-mörpholin-4-yl-propyl)-harnstoff ausgewählt wird.

18. Eine pharmazeutische Zusammensetzung, die eine Verbindung gemäß Anspruch 1 und einen pharmazeutische geeigneten Träger oder Verdünnungsmittel umfasst.

19. Eine pharmazeutische Zusammensetzung, die eine Verbindung gemäß Anspruch 3 und einen pharmazeutische geeigneten Träger oder Verdünnungsmittel umfasst.

20. Eine pharmazeutische Zusammensetzung, die eine Verbindung gemäß Anspruch 7 und einen pharmazeutische geeigneten Träger oder Verdünnungsmittel umfasst.

21. Eine pharmazeutische Zusammensetzung, die eine Verbindung gemäß Anspruch 13 und einen pharmazeutische geeigneten Träger oder Verdünnungsmittel umfasst.

22. Eine pharmazeutische Zusammensetzung, die eine Verbindung gemäß Anspruch 15 und einen pharmazeutische geeigneten Träger oder Verdünnungsmittel umfasst.

23. Eine pharmazeutische Zusammensetzung, die eine Verbindung gemäß Anspruch 16 und einen pharmazeutische geeigneten Träger oder Verdünnungsmittel umfasst.

24. Eine pharmazeutische Zusammensetzung, die eine Verbindung gemäß Anspruch 17 und einen pharmazeutische geeigneten Träger oder Verdünnungsmittel umfasst.

25. Verwendung einer Verbindung gemäß Anspruch 1 für die Herstellung einer pharmazeutischen Zusammensetzung zur Behandlung eines Subjekts mit einer Bedingung, die einer Verbesserung durch Antagonismus eines Adenosin-Rezeptors empfänglich ist.

26. Verwendung nach Anspruch 25, wobei die Bedingung Ischämie, supraventrikuläre Arrhythmie, akutes Nierenversagen, myokardialer Reperfusionsschaden, Autoimmunkrankheit, Reizdarmsyndrom, Asthma, Diabetes mellitus, Fettleibigkeit, Parkinson-Krankheit, Huntington-Krankheit, Dystonie oder Dyskinesie ist.

27. Verwendung der Verbindung gemäß Anspruch 1 für die Herstellung einer pharmazeutischen Zusammensetzung zur Behandlung eines Subjekts mit einer Bedingung, die einer Verbesserung durch Antagonismus eines A2A-Adenosinrezeptors empfänglich ist.

28. Verwendung gemäß Anspruch 27, wobei die Bedingung Ischämie, supraventrikuläre Arrhythmie, Parkinson-Krankheit, Huntington-Krankheit, Dystonie oder Dyskinesie ist.

## Revendications

1. Composé de formule (I) 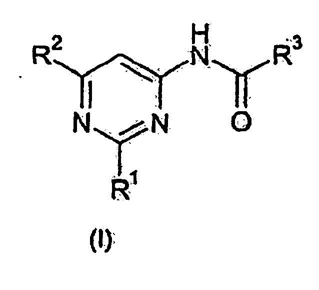 ou un sel, ester, solvate, ou stéréoisomère pharmaceutiquement acceptable de celui-ci, où :
chacun parmi R¹ et R² est indépendamment un groupe aryle ou hétéroaryle éventuellement substitué par un ou plusieurs substituants choisis parmi le groupe alkyle inférieur ayant 1 à 8 atomes de carbone, halogène, cycloalkyle, hydroxy, alcoxy inférieur ayant 1 à 8 atomes de carbone, -SH, NO₂, alkylthio inférieur ayant 1 à 8 atomes de carbone, alkylamino inférieur ayant 1 à 8 atomes de carbone, cyano, et amino, où les groupes alkyle inférieur, cycloalkyle, alcoxy inférieur, alkylthio inférieur et alkylamino inférieur sont éventuellement substitués par un ou plusieurs substituants choisis parmi le groupe consistant en alkyle, alcényle, alcynyle, aryle, hétéroaryle, alcoxy, aryloxy, alkylthio, arylthio, cycloalkyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃,OH, OCH₃, OCF₃, CH₃, et CF₃;
R³ est -(CR⁴R⁵)ₙ-R⁶, -(CR⁴R⁵)ₙ-NR⁷R⁸, -O-(CR⁴R⁵) ₙ-R⁶ ou est -(CR⁴R⁵)ₙ-O-R⁸;
chacun parmi R⁴ et R⁵ est à chaque fois indépendamment choisi parmi le groupe consistant en hydrogène, alkyle inférieur ayant 1 à 8 atomes de carbone, halogène, cycloalkyle, hydroxy, alcoxy inférieur ayant 1 à 8 atomes de carbone, -SH, NO₂, alkylthio inférieur ayant 1 à 8 atomes de carbone, alkylamino inférieur ayant 1 à 8 atomes de carbone, cyano, et amino, où les groupes alkyle inférieur, cycloalkyle, alcoxy inférieur, alkylthio inférieur et alkylamino inférieur sont éventuellement substitués par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃;
R⁶ est un hétérocycle ayant au moins un atome d'azote, où l'hétérocycle est éventuellement substitué par un ou plusieurs éléments choisis parmi le groupe consistant en alkyle inférieur ayant 1 à 8 atomes de carbone, alcoxy, cycloalkyle, aminoalkyle, aminodialkyle, alkylaminoalkyle, dialkylaminoalkyle, alkoxyalkyle, aryle, arylalkyle, hétéroaryle hétéroarylalkyle, hétérocycle, hétérocycylalkyle, amino, alkylamino, dialkylamino, cycloalkylamino, halogène, haloalkyle, ester, amide, acyle, carbamoyle, carbamoylalkyle, oxo, isoquinolinyle et imidoylamino, où lesdits groupes alkyle inférieur, alcoxy, cycloalkyle, aminoalkyle, alkylaminoalkyle, dialkylaminoalkyle, alcoxyalkyle, aryle, arylalkyle, hétéroaryle hétéroarylalkyle, hétérocycle, hétérocycylalkyle, amino, alkylamino, dialkylamino, cycloalkylamino, haloalkyle, ester, amide, acyle, carbamoyle, carbamoylalkyle, isoquinolinyle et imidoylamino sont éventuellement substitués par un alkyle inférieur ayant 1 à 8 atomes de carbone, alcoxy, hydroxy, oxo ou halogène ;
R⁷ est un hydrogène ou un alkyle inférieur ayant 1 à 8 atomes de carbone éventuellement substitué par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃;
R⁸ est - (CR⁴R⁵)ₙ-R⁶; ou
R⁷ et R⁸ conjointement avec l'atome d'azote auquel ils sont attachés forment un noyau hétérocyclique éventuellement substitué par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃; et
n est indépendamment à chaque fois 0, 1 , 2, 3 ou 4;
à condition que lorsque R¹ et R² sont tout les deux un hétéroaryle, R⁶ est un hétérocycle non-aromatique.

2. Composée selon la revendication 1, où : chacun parmi R¹ et R² est indépendamment choisi parmi le groupe consistant en pyridinyle, furanyle, thiophényle, thiazolyle, pyrazolyle, imidazolyle, oxazolyle, isoxazolyle et oxadiazolyle éventuellement substitués par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃.

3. Composé selon la revendication 2, où n est 1 ou 2.

4. Composé selon la revendication 3, où :
R¹ est choisi parmi le groupe consistant en pyridinyle, furanyle, thiophényle, thiazolyle, pyrazolyle, imidazolyle, oxazolyle et isoxazolyle éventuellement substitués par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃.

5. Composé selon la revendication 4, où :
R² est choisi parmi le groupe consistant en pyridinyle, thiazolyle et pyrazolyle éventuellement substitués par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃.

6. Composé selon la revendication 5, où :
R¹ est choisi parmi le groupe consistant en pyridinyle, furanyle, 5-méthyl-furanyle, thiophényle, thiazolyle, pyrazolyle, imidazolyle, oxazolyle, 4-méthyl-oxazolyle et isoxazolyle; et
R² est choisi parmi le groupe consistant en pyridinyle, thiazolyle, pyrazolyle et 3,5-diméthylpyrazolyle.

7. Composé selon la revendication 6, où : R³ est -CR⁴R⁵)ₙ-R⁶ ou
-(CR⁴R⁵)ₙ-NR⁷R⁸;
chacun parmi R⁴ et R⁵ est indépendamment à chaque fois un hydrogène ou un alkyle inférieur ayant 1 à 8 atomes de carbone ;
R⁶ est un noyau hétérocyclique choisi parmi le groupe consistant en pipéridinyle, pipérazinyle, morpholinyle, thiomorpholinyle, pyrrolidinyle, isoquinolinyle, diazépanyle, dihydropyrrolyle, azépanyle, oxazépanyle, et pyrrolopyrazinyle éventuellement substitués par un ou plusieurs substituants choisis parmi le groupe consistant en alkyle, alcényle, alcynyle, aryle, hétéroaryle, alcoxy aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF₃, CH₃, et CF₃; et R⁷ et R⁸ conjointement avec l'atome d'azote auquel ils sont attachés forment un noyau hétérocyclique choisi parmi le groupe consistant en pipéridinyle, pipérazinyle, morpholinyle, thiomorpholinyle, pyrrolidinyle, isoquinolinyle, diazépanyle, dihydropyrroiyle, azépanyle, oxazépanyle, et pyrrolopyrazinyle éventuellement substitués par un ou plusieurs substituants choisis parmi le groupe consistant en alkyle, alcényle, alcynyle, aryle, hétéroaryle, alcoxy, aryloxy, alkylthio, arylthio, cycloakyle, arylalkyle, amino, alkylamino, dialkylamino, amido, F, Cl, Br, I, CN, NO₂, NH₂, NHCH₃, NHCH₂CH₃, N(CH₃)₂, N(CH₂CH₃)₂, SH, SCH₃, OH, OCH₃, OCF3, CH₃, et CF₃.

8. Composé selon la revendication 7, où R⁶ est substitué avec un ou plusieurs éléments choisis parmi le groupe consistant en alkyle inférieur ayant 1 à 8 atomes de carbone, alcoxy, amino, alkylamino, dialkylamino, pipéridinyle, pipérazinyle, pyrrolidinyle, morpholinyle, phényle et benzyle.

9. Composé selon la revendication 7, où :
R¹ est un furanyle, 5-méthyl-furanyle, oxazolyle, 4-méthyl-oxazolyle, pyridinyle, pyrazolyle, ou isoxazolyle et R² est un pyrazolyle.

10. Composé selon la revendication 7, où :
R¹ est un furanyle, 5-méthyl-furanyle, pyridinyle, thiophényle ou thiazolyle et R² est un thiazolyle.

11. Composé selon la revendication 7, où :
R¹ est un 5-méthyl-furanyle, thiazolyle ou pyridinyle et R² est un 3,5-diméthylpyrazolyle.

12. Composé selon la revendication 7, où : R¹ est un 5-méthyl-furanyle ou thiophényle et R² est un pyridinyle.

13. Composé selon la revendication 1, où :
R¹ est un furanyle ou 5-méthyl-furanyle; et
R² est un pyrazolyle, 3,5-diméthylpyrazolyle ou thiazolyle.

14. Composé selon la revendication 13, où :
R³ est -(CR⁴R⁵)ₙR⁶; chacun parmi R⁴ et R⁵ est un hydrogène ; et n est 1 ou 2.

15. Composé selon la revendication 14, où :
R⁶ est un pipérazinyle substitué avec un alkyle inférieur ayant 1 à 8 atomes de carbone, pyrrolidinyle substitué avec un alkylamino ou dialkylamino, [1,4]-diazépanyle substitué avec un alkyle inférieur ayant 1 à 8 atomes de carbone ou [1,4]-oxazépanyle.

16. Composé selon la revendication 1 choisis parmi le groupe consistant en :
N-(2-Furan-2-yl-6-pyrazol-1-yl-pyrimidin-4-yl)-2-pipéridin-1-yl -acétamide;
N-(2-Furan-2-yl-6-pyrazol-1-yl-pyrimidin-4-yl)-2-morpholin-4-yl -acétamide;
N-(2-Furan-2-yl-6-pyrazol-1-yl-pyrimidin-4-yl)-2-(4-méthyl-pipérazin-1-yl)-acétamide;
N-(2-Furan-2-yl-6-pyrazol-1-yl-pyrimidin-4-yl)-2-pipérazin-1-yl- acétamide;
N-[2-(5-Méthyl-furan-2-yl)-6-thiazol-2-yl-pyrimidin-4-yl]-2-morpholin-4-yl-acétamide;
N-[2-(5-Méthyl-furan-2-yl)-6-thiazol-2-yl-pyrimidin-4-yl]-2-pipéridin-1-yl-acétamide;
N-[2-(5-Méthyl-furan-2-yl)-6-thiazol-2-yl-pyrimidin-4-yl]-2-pylrrolidin-1-yl-acétamide;
N-[2-(5-Méthyl-furan-2-yl)-6-thiazol-2-yl-pyrimidin-4-yl]-2-(4-méthyl-pipérazin-1-yl) -acétamide;
2-(2,5-Diméthyl-pipérazin-1-yl)-N-[2-(5-méthyl-furan-2-yl)-6-thiazol-2-yl-pyrimidin-4-yl] -acétamide;
N-[2-(5-Méthyl-furan-2-yl)-8-thiazol-2-yl-pyrimidin-4-yl]-2-pipérazin-1-yl-acétamide;
N-{2-(5-Méthyl-furan-2-yl)-6-thiazol-2-yl-pyrimidin-4-yl]-2-(4-phényl-pipérazin-1-yl) -acétamide;
2-[1,4]Diazépan-1-yl-2-(5-méthyl-furan-2-yl)-6-thiazol-2-yl-pyrimidin-4-yl]-acétamide;
N-{2-(5-Méthyl-furan-2-yl)-6-thiazol-2-yl-pyrimidin-4-yl]-2-(2-phényl-pipéridin-1-yl) -acétamide;
N-[2-(5-Méthyl-furan-2-yl)-6-thiazol-2-yl-pyrimidin-4-yl]-2-(3-phény-pipéridin-1-yl) -acétamide;
2-(4-Benzyl-pipérazin-1-yl)-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-{2-méthyl-pipéridin-1-yl) -acétamide;
2-(2,5-Dihydro-pyrrol-1-yl)-N-[2-(5-méthyl-furan-2-yl)-6-thiazol-2-yl-pyrimidin-4-yl] -acétamide;
2-{2,5-Diméthyl-2,5-dihydro-pyrrol-1-yl)-N-[2-(5-méthyl-furan-2-yl)-6-thiazol-2-yl-pyrimidin-4-yl] -acétamide;
2-(2,5-Diméthyl-pyrrolidin-1-yl)-N-[2-(5-éthyl-furan-2-yl)-6-thiazol-2-yl-pyrimidin-4-yl] -acétamide;
2-(3,5-Diméthyl-pipérazin-1-yl)-N-[2-(5-méthyl-furan-2-yl)-6-thiazol-2-yl-pyrimidin-4-yl] -acétamide;
2-[4-(2-Méthoxy-phényl)-pipérazin-1-yl]-N-[2-(5-méthyl-furan-2-yl)-6-thiazol-2-yl-pyrimidin-4-yl] -acétamide;
N-[2-(5-Méthyl-furan-2-yl)-5-thiazol-2-yl-pyrimidin-4-yl]-2-(3-méthyl-pipéridin-1-yl) -acétamide;
2-Azépan-1-yl-N-[2-(5-méthyl-furan-2-yl)-6-thiazol-2-yl-pyrimidin-4-yl] acétamide;
2-((S)-2-Méthoxyméthyl-pyrrolidin-1-yl)-N-[2-(5-méthyl-furan-2-y 1)-6-thiazol-2-yl-pyrimidin-4-yl] -acétamide;
2-(3,3-Diméthyl-pipéridin-1-yl)-N-[2-(5-méthyl-furan-2-yl)-6-thiazol-2-yl-pyrimidin-4-yl]- acétamide;
2-(3,5-Diméthyl-pipéridin-1-yl)-N-[2-(5-méthyl-furan-2-yl)-6-thiazol-2-yl-pyrimidin-4-yl] -acétamide;
N-(2-(5-Méthyl-furan-2-yl)-6-(thiazol-2-yl-pyrimidin-4-yl]-2-(4-phényl-pipéridin-1-yl) -acétamide;
N-[2-(5-Méthyl-furan-2-yl}-6-thiazol-2-y)-pyrimidin-4-yl]-2-(4-méthyl-pipéridin-1-yl) -acétamide;
2,4-Benzyl-pipéridin-1-yl)-N-[2-(5-méthyl-furan-2-yl)-6-thiazol-2-yl-pyrimidin-4-yl] -acétamide;
2-[1,4']Bipipéridinyl-1'-yl-N-[2,5-méthyl-furan-2-yl)-6-thiazol-2-yl-pyrimidin-4-yl] -acétamide;
2-(3,4-Dihydro-1H-isoquinolin-2-yl)-N-[2-(5-méthyl-furan-2-yl)-6-thiazol-2-yl-pyrimidin-4-yl] -acétamide;
N[2-(5-Méthyl-furan-2-yl)-thiazol-2-yl)-pyrimidin-4-yl]-2-(octahydro-isoquinolin-2-yl) -acétamide;
N[2(5-Méthyl-furan-2-yl)-8-thiazol-2-yl-pyrimidin-4-yl]-2-((S)-2-pyrrolidin-1-ylméthyl-pyrrolidin-1-yl) -acétamide;
2-[4-(3,4-Diméthyl-phényl)-pipérazin-1-yl]-N-{2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-(4-pyrrolidin-1-yl-pipéridin-1-yl) -acétamide;
2-[4-(3-chloro-phényl)-pipérazin-1-yl]-N-[2-(5-méthyl-furan-2-yl)-6-thiazol-2-yl-pyrimidin-4-yl] -acétamide;
2-(2,6-Diméthyl-morpholin-4-yl)-N-[2-(5-méthyl-furan-2-yl)-6-thiazol-2-yl-pyrimidin-4-yl]- acétamide;
N-[2-(5-Méthyl-furan-2-yl)-6-thiazol-2-yl-pyrimidin-4-yl)-2-(3-méthyl-4-m-tolyl-pipérazin-1-yl) -acétamide;
2-{4-Méthyl-[1,4]diazépan-1-yl)-N-{2-(5-méthyl-furan-2-yl)-6-thiazol-2-yl-pyrimidin-4-yl -acétamide;
2-[4-(3-Méthoxy-phényl-pipérazin-1-yl]-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-[2-(2-pipéridin-1-yl-éthyl)-pipéridin-1-yl] -acétamide;
N-[2(5-Méthyl-furan-2-yl)-6-thiazol-2-yl-pyrimidin-4-yl]-2-[1,4]oxazépan-4-yl - acétamide;
2-(4,4-Difluoro-pipéridin-1-yl)-N-[2-(5-méthyl-furan-2-yl)-6-thiazol-2-yl-pyrimidin-4-yl] -acétamide;
2-(4-Acétyl-pipérazin-1-yl)-N-[2-(5-méthyl-furan-2-yl)-6-thiazol-2-yl-pyrimidin-4-yl] -acétamide;
2-[(1-Benzyl-pyrrolidin-3-yl)-méthyl-amino]-N-[2-(5-méthyl-furan-2-yl)-6-thiazol-2-yl-pyrimidin-4-yl] -acétamide;
2-(4-Acétyl-[1,4]diazépan-1-yl)-N-[2-(5-méthyl-furan-2-yl)-6-thiazol-2-yl-pyrimidin-4-yl -acétamide;
2-(4-Benzyl-[1,4]diazépan-1-yl)-N-(2-(5-méthyl-furan-2-yl)-6-thiazol-2-yl-pyrimidin-4-yl] -acétamide;
2-(3-Diméthylamino-pyrrolidin-1-yl)-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-(3-trifluorométhyl-pipéridin-1-yl) -acétamide;
2-(3-Diéthylamino-pyrrolldin-1-yl)-N-[2-(5-méthyl-furan-2-yl)-6-thiazol-2-yl-pyrimidin-4-yl] -acétamide;
2-((R)-3-Diméthylamino-pyrrolidin-1-yl)-N-[2-(5-méthyl-furan-2-yl)-6-thiazol-2-yl-pyrimidin-4-yl] -acétamide;
2-((S)-3-Diméthylamino-pyrrolidin-1-yl)-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-(2-pyrrolidin-1-yl-éthylamino) -acétamide;
Acide 1-{[2-(5-Méthyl-furan-2-yl)-6-thiazol-2-yl-pyrimidin-4-ylcarbamoyl]-méthyl} -pipéridine-3-carboxylique amide;
Acide 1-{[2-(5-Méthyl-furan-2-yl)-6-thiazol-2-yl-pyrimidin-4-ylcarbamoyl]-méthyl}-pipéridin-4-carboxylique amide;
N-[2-(5-Méthyl-furan-2-yl)-thiazol-2-yl-pyrimidin-4-yl]-2-((R)-3-méthyl-pipérazin-1-yl) -acétamide;
2-[4-(isopropy)carbamoyl-méthyl)-pipérazin-1-yl]-N-[2-(5-méthyl-furan-2-yl)-6-thiazol-2-yl-pyrimidin-4-yl] -acétamide;
2-(4-Amino-pipéridin-1-yl)-N-[2-(5-méthyl-furan-2-yl)-6-thiazol-2-yl-pyrimidin-4-yl] -acétamide;
2-(4-Diméthylamino-pipéridin-1-yl)-N-[2-(5-méthyl-furan-2-yl)-6-thiazol-2-yl-pyrimidin-4-yl] -acétamide;
2-(4-Diéthylamino-pipéridin-1-yl)-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-(4-morpholin-4-yl-pipéridin-1-yl) -acétamide;
2-(4-isopropylamino-pipéridin-1-yl)-N-[2-(5-méthyl-furan-2-yl)-6-thiazol-2-yl-pyrimidin-4-yl] -acétamide;
2-(4-Cyclopentylamino-pipéridin-1-yl)-N-[2-(5-méthyl-furan-2-yl)-6-thiazol-2-yl-pyrimidin-4-yl] -acétamide;
2-(4-Dipropylamino-pipéridin-1-yl)-N-[2-(5-méthyl-furan-2-yl)-6-thiazol-2-yl-pyrimidin-4-yl] -acétamide;
2-(3-Amino-pyrrolidin-1-yl)-N-[2-(5-méthyl-furan-2-yl)-6-thiazol-2-yl-pyrimidin-4-yl] -acétamide;
2-(3-isopropylamino-pyrrolidin-1-yl}-N-[2-(5-méthyl-furan-2-yl)-6-thiazol-2-yl-pyrimidin-4-yl] -acétamide;
2-(3-Cyclopentylamino-pyrrolidin-1-yl)-N-{2-(5-méthyl-furan-2-yl)-6-thiazol-2-yl-pyrimidin-4-yl] -acétamide;
2-(4-Acétylamino-pipéridin-1-yl)-N-[2-(5-méthyl-furan-2-yl)-thiazol-2-yl-pyrimidin-4-yl] -acétamide;
N-(1-{[2-(5-Méthyl-furan-2-yl)-thiazol-2-ylpyrimidin-4-ylcarbamoyl]-méthyl}-pipéridin-4-yl)- propionamide;
2-{3-Acétylamino-pyrrolidin-1-yl)-N-[2-(5-méthyl-furan-2-yl)-6-thiazol-2-yl-pyrimidin-4-yl]-acétamide;
N-(1-[2-(5-Méthyl-furan-2-yl)-6-thiazol-2-yl-pyrimidin-4-ylcarbamoyl]-méthyl}-pyrrolidin-3-yl)-propionamide;
N-[2-(5-Méthyl-furan-2-yl)-6-thiazol-2-yl-pyrimidin-4-yl]-2-(4-thiazol-2-yl-pipérazin-1-yl)-acétamide;
2-(Hexahydro-pyrrolo[1,2-a]pyrazin-2-yl)-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl]-2-(3-pipéridin-1-yl-pyrrolidin-1-yl)-acétamide;
N-[2-(6-Méthyl-furan-2-yl)-6-thiazol-2-yl-pyrimidin-4-yl)-2-(3-morpholin-4-yl-pyrrolidin-1-yl) -acétamide;
N-[2-(5-Méthyl-furan-2-yl)-6-thiazol-2-yl-pyrimidin-4-yl)-2-(2-méthyl-3-oxo-pipérazin-1-yl) -acétamide;
Acide 1-{[2-(5-Méthyl-furan-2-yl)-6-thiazol-2-yl-pyrimidin-4-ylcarbamoyl]-méthyl}- pipéridine-3-carboxylique éthyl ester;
Acide 1-[2-(5-Méthyl-furan-2-yl)-6-thiazol-2-yl-pyrimidin-4-ylcarbamoyl]-méthyl}-pipéridine-3-carboxylique (2-hydroxy-éthyl)-amide;
2-((S)-3-Éthylamino-pyrrolidin-1-yl)-N-[2-(5-méthyl-furan-2-yl)-6-thiazol-2-yl-pyrimidin-4-yl] -acétamide;
2-((R)-3-Éthylamino-pyrrolidin-1-yl)-N-[2-(5-méthyl-furan-2-yl)-6-thiazol-2-yl-pyrimidin-4-yl] -acétamide;
2-(4-Éthyl-pipérazin-1-yl)-N-[2-(5-méthyl-furan-2-yl)-6-thiazol-2-yl-pyrimidin-4-yl]- acétamide;
N-[2-(5-Méthyl-furan-2-yl)-6-thiazol-2-yl-pyrimidin-4-yl]-2-(2-pipéridin-1-yl-éthylamino)- acétamide;
N-[2-(5-Méthyl-furan-2-yl)-6-thiazol-2-yl-pyrimidin-4-yl]-2-[méthyl-(1 -méthyl-pipéridin-4-yl)-amino] -acétamide;
N-[2-(5-Méthyl-furan-2-yl)-6-thiazol-2-yl-pyrimidin-4-yl]-2-(2-(1-méthyl-pyrrolidin-2-yl)-éthylamino-acétamide;
N-[2-(5-Méthyl-furan-2-yl)-6-thiazol-2-yl-pyrimidin-4-yl)-2-(pyrrolidin-3-ylamino)-acétamide;
N-[2-(5-Méthyl-furan-2-yl)-6-thiazol-2-yl-pyrimidin-4-yl]-2-(3-pyrrolidin-1-yl-propylamino)-acétamide;
N-[2-(5-Méthyl-furan-2-yl)-6-thiazol-2-yl-pyrimidin-4-yl]-2-(3-pipéridin-1-y}-propylamino)-acétamide;
N-[2-(5-Méthyl-furan-2-yl)-6-thiazol-2-yl-pyrimidin-4-yl]-2-(3-morpholin-4-yl-propylamino) -acétamide;
2-(4-Hydroxy-pipéridin-1-yl)-N-[2-(5-méthyl-furan-2-yl)-6-thiazol-2-yl-pyrimidin-4-yl]-acétamide;
2-(3-Hydroxy-pipéridin-1-yl)-N-{2-(5-méthyl-furan-2-yl)-6-thiazol-2-yl-pyrimidin-4-yl]-acétamide;
2-((S)-3-Hydroxy-pyrrolidin-1-yl)-N-[2-(5-méthyl-furan-2-yl)-6-thiazol-2-yl-pyrimidin-4-yl]-acétamide;
2-((S)-3-Acétylamino-pyrrolidin-1-yl)-N-[2-(5-méthyl-furan-2-yl)-6-thiazol-2-yl-pyrimidin-4-yl] -acétamide;
2-((R)-3-Acétylamino-pyrrolidin-1-yl)-N-[2-{5-méthyl-furan-2-yl)-6-thiazol-2-yl-pyrimidin-4-yl] -acétamide;
2-(3-Hydroxy-pyrrolidin-1-yl)-N-[2-(5-méthyl-furan-2-yl)-6-thiazol-2-yl-pyrimidin-4-yl]-acétamide;
2-(4-isopropyl-pipérazin-1-yl)-N-[2-(5-méthyl-furan-2-yl)-6-thiazol-2-yl-pyrimidin-4-yl]-acétamide;
2-(4-Cyclopentyl-pipérazin-1-yl)-N-[2-(5-méthyl-furan-2-yl)-6-thiazol-2-yl-pyrimidin-4-yl]-acétamide;
2-{4-Cyclohexyl-pipérazin-1--yl)-N-[2-(5-méthyl-furan-2-yl)-6-thiazol-2-yl-pyrimidin-4-yl]-acétamide;
N-[2-{5-Méthyl-furan-2-yl)-6-thiazol-2-yl-pyrimidin-4-yl)-2-(4-propionyl-pipérazin-1-yl)-acétamide;
2-(4-isobutyryl-pipérazin-1-yl)-N-[2-(5-méthyl-furan-2-yl)-6-thiazol-2-yl-pyrimidin-4-yl]-acétamide;
2-(4-Aminométhyl-pipéridin-1-yl)-N-[2-(5-(méthyl-furan-2-yl)-6-thiazol-2-yl-pyrimidin-4-yl]-acétamide;
N-[2-(5-Méthyl-furan-2-yl)-6-thiazol-2-yl-pyrimidin-4-yl]-2-[(pyrrolidin-3-ylméthyl)-amino]-acétamide;
2-(3-Aminométhyl-pipéridin-1-yl)-N-(2-(5-méthyl-furan-2-yl)-6-thiazol-2-yl-pyrimidin-4-yl)-acétamide;
N-[2-(5-Méthyl-furan-2-yl)-6-thiazol-2-yl-pyrimidin-4-yl]-2-[(1-méthyl-pyrrolidin-3-ylméthyl)-amino] -acétamide;
2-(3-Aminométhylpyrrolidin-1-yl)-N-[2-(5-méthyl-furan-2-yl)-6-thiazol-2-yl-pyrimidin-4-yl] -acétamide;
2-(4-Méthoxy-pipéridin-1-yl)-N-[2-(5-méthyl-furan-2-yl)-6-thiazol-2-yl-pyrimidin-4-yl]-acétamide;
2-(3-Diméthylaminométhyl-pipéridin-1-yl)-N-[2-(5-(méthyl-furan-2-yl)-6-thiazol-2-yl-pyrimidin-4-yl] -acétamide;
2-(4-Diméthylaminométhyl-pipéridin-1-yl)-N-[2-(5-méthyl-furan-2-yl)-6-thiazol-2-yl-pyrimidin-4-yl] -acétamide;
N-[2-(5-Méthyl-furan-2-yl)-6-thiazol-2-yl-pyrimidin-4-yl)]-2-[méthyl-(1-méthyl-pyrrolidin-3-ylméthyl)-amino] -acétamide;
2-(3-Diméthylaminométhyl-pyrrolidin-1-yl)-N-[2-(5-méthyl-furan-2-yl)-6-thiazol-2-yl-pyrimidin-4-yl]-acétamide;
N-[2-(5-Méthyl-furan-2-yl)-6-thiazol-2-yl-pyrimidin-4-yl]-2-morpholin-4-yl-acétamide;
N-(2-(5-Méthyl-furan-2-yl)-6-thiazol-2-yl-pyrimidin-4-yl]-2-(4-phényl-pipéridin-1-yl)-acétamide;
2-(2,5-Diméthyl-pipérazin-1-yl)-N-[2-(5-méthyl-furan-2-yl)-6-thiazol-2-yl-pyrimidin-4-yl]-acétamide;
N-[2-(5-Méthyl-furan-2-yl)-6-thiazol-2-yl-pyrimidin-4-yl]-2-pipérazin-1-yl-acétamide;
2-[1,4]Diazépan-1-yl-N-[2-(5-méthyl-furan-2-yl)-6-thiazol-2-yl-pyrimidin-4-yl]-acétamide;
3-((S)-3-Diméthylamino-pyrrolidin-1-yl)-N-[2-(5-méthyl-furan-2-yl)-6-thiazol-2-yl-pyrtmidin-4-yl)-propionamide;
3-{4-Acétyl-pipérazin-1-yl)-N-[2-(5-méthyl-furan-2-yl]-6-thiazol-2-yl-pyrimidin-4-yl)-propionamide;
N-[2-(5-Méthyl-furan-4-yl)-6-thiazol--2-yl-pyrimidin-4-yl]-3-(4-méthyl-pipérazin-1-yl)-propionamide;
Acide 2-(5-Méthyl-furan-2-yl)-6-(3-pipérazin-1-yl-propionylamino)-N-vinyl-pyrimidine-4-carboximidothioïque méthyl ester;
N- [2-(5-Méthyl-furan-2-yl)-6-thiazol-2-yl-pyrimidin-4-yl]-3-(4-pyrrolidin-1-yl-pipéridin-1-yl)- propionamide;
N-[2-(5-Méthyl-furan-2-yl)-6-thiazol-2-yl-pyrimidin-4-yl]-3-morpholin-4-yl-propionamide;
3-((R)-3-Dimérthylamino-pyrrolidin-1-yl)-N-[2-(5-méthyl-furan-2-yl)-6-thiazol-2-yl-pyrimidin-4-yl]-propionamide;
3-(4-Acétyl-[1,4]diazépan-1-yl)-2-(5-méthyl-furan-2-yl)-6-thiazol-2-yl-pyrimidin-4-yl)-propionamide;
N-[2-(5-Méthyl-furan-2-yl)-6-thiazol-2-yl-pyrimidin-4-yl]-3-[1,4] oxazépan-4-yl-propionamide;
3-(4-Méthyl-[1,4]diazépan-1-yl)-N-[2-(5-méthyl-furan-2-yl)-6-thiazol-2-yl-pyrimidin-4-yl]- propionamide;
3-((R)-3-Éthylamino-pyrrolidin-1-yl)-N-[2-(5-méthyl-furan-2-yl)-6-thiazol-2-yl-pyrimidin-4-yl]-propionamide;
3-(3,5-Diméthyl-pipérazin-1-yl)-N-[2-(5-méthyl-furan-2-yl)-6-thiazol-2-yl-pyrimidin-4-yl]-propionamide;
3-[1,4]Diazépan-1-yl-N-[2-(5-méthyl-furan-2-yl)-6-thiazol-2-yl-pyrimidin-4-yl]-proptonamide;
N-[2-(5-Méthyl-furan-2-yl)-6-thiazol-2-yl-pyrimidin-4-yl)-2-(1-méthyl-pipéridin-4-yl]-acétamide;
N-[2-(5-Méthyl-furan-2-yl)-6-thiazol-2-yl-pyrimidin-4-yl]-2-pipéridin-4-yl-acétamide;
N-[2-(5-Méthyl-furan-2-yl)-6-thiazol-2-yl-pyrimidin-4-yl]-2-(2-pyrrolidin-1-yl-éthoxy)-acétamide;
N-[6-(3,5-Diméthyl-pyrazol-1-yl)-2-(4-méthoxy-pyridin-3-yl)-pyrimidin-4-yl]-2-(4-méthyl-pipérazin-1-yl) -acétamide;
N-[6-(3,5-Diméthyl-pyrazol-1-yl)-2-(6-méthoxy-pyridin-3-yl)-pyrimidin-4-yl]-2-(4-méthyl-pipérazin-1-yl) -acétamide;
N-[2-(3,4-Diméthoxy-phényl)-6-(3,5-diméthyl-pyrazol-1-yl)-pyrimidin-4-yl]-2-(4-méthyl-pipérazin-1-yl) -acétamide;
N-[2-(4-Trifuorométhoxy-phényl)-6-(3,5-diméthyl-pyrazol-1-yl)-pyrimidin-4-yl]-2-(4-méthyl-pipérazin-1-yl) -acétamide;
N-[2-(3-Fluoro-phényl)-6-(3,5-diméthyl-pyrazol-1-yl)-pyrimidin-4-yl]-2-(4-méthyl-pipérazin-1-yl) -acétamide;
N-[2-(2-Fluoro-phényl)-6-(3,5-diméthyl-pyrazol-1-yl)-pyrimidin-4-yl]-2-(4-méthyl-pipérazin-1-yl) -acétamide;
N-(2-(2-Fluoro-3-méthoxy-phényl)-6-(3,5-diméthyl-pyrazol-1-yl)-pyrimidin-4-yl]-2-{4-méthyl-pipérazin-1-yl) -acétamide;
N-[2-(2,4-Diméthoxy-phényl)-6-(3,5-diméthyl-pyrazol-1-yl)-pyrimidin-4-yl]-2-(4-méthyl-pipérazin-1-yl) -acétamide;
N-[2-(2-Cyano-phényl)-6-(3,5-diméthyl-pyrazol-1-yl)-pyrimidin-4-yl]-2(4-méthyl-pipérazin-1-yl) -acétamide;
N-[2-(2-Méthoxy-phényl)-6-(3,5-diméthy)-pyrazol-1-yl)-pyrimidin-4-yl]-2-(4-méthyl-pipérazin-1-yl) -acétamide;
N-[2-(4-Fluoro-2-méthyl-phényl)-6-{3,5-diméthyl-pyrazol-1-yl)-pyrimidin-4-yl]-2-(4-méthyl-pipérazin-1-yl) -acétamide;
N-[2-(3-Méthoxy-phényl)-6-(3,5-diméthyl-pyrazol-1-yl}-pyrimidin-4-yl]-2-(4-méthyl-pipérazin-1-yl) -acétamide;
N-[2-(3,5-Diméthoxy-phényl)-6-(3,5-diméthyl-pyrazol-1-yl)-pyrimidin-4-yl]-2-(4-méthyl-pipérazin-1 -yl) -acétamide;
N-[2-(3,5-Difluoro-phényl)-6-(3,5-diméthyl-pyrazol-1-yl)-pyrimidin-4-yl]-2-(4-méthyl-pipérazin-1-yl) -acétamide;
N-[2-(3-Fluoro-4-méthyl-phényl)-6-(3,5-diméthyl-pyrazol-1-yl)-pyrimidin-4-yl]-2-(4-méthyl-pipérazin-1-yl)-acétamide;
N-[2-(3-Fluoro-2-méthoxy-phényl)-6-(3,5-diméthyl-pyrazol-1 -yl)-pyrimidin-4-yl]-2-(4-méthyl-pipérazin-1-yl) -acétamide;
N-[2-(3-Fluoro-4-méthoxy-phényl)-6-(3,5-diméthyl-pyrazol-1-yl)-pyrimidin-4-yl]-2-(4-méthyl-pipérazin-1-yl) -acétamide;
N-[2-(2,3-Difluoro-phényl)-6-(3,5-diméthyl-pyrazol-1-yl)-pyrimidin-4-yl]-2-(4-méthyl-pipérazin-1-yl) -acétamide;
N-[2-(5-Méthoxy-pyridin-3-yl)-6-{3,5-diméthyl-pyrazol-1-yl)-pyrimidin-4-yl]-2-(4-méthyl-pipérazin-1-yl) -acétamide;
N-[2-(3-Méthoxy-phényl)-6-pyrazol-1-yl-pyrimidin-4-yl]-2-(4-méthyl-pipérazin-1-yl)-acétamide;
N-[2-(3,4-Diméthoxy-phényl)-6-pyrazol-1-yl-pyrimidin-4-yl]-2-(4-méthyl-pipérazin-1-yl)-acétamide;
N-[2-(3-Cyano-phényl)-6-pyrazol-1-yl-pyrimidin-4-yl]-2-(4-méthyl-pipérazin-1 -yl)-acétamide;
N-[2-(3-Fluoro-4-Méthoxy-phényl)-6-pyrazol-1-yl-pyrimidin-4-yl]-2-(4-méthyl-pipérazin-1-yl) -acétamide;
N-[2-(2-Méthoxy-phényl)-6-pyrazol-1-yl-pyrimidin-4-yl]-2-(4-méthyl-pipérazin-1-yl)-acétamide;
N-[2-(4-Méthoxy-phényl)-6-pyrazol-1-yl-pyrimidin-4-yl]-2-(4-méthyl-pipérazin-1-yl)- acétamide;
N-(2-(3-Trifluorométhyl-phényl)-6-pyrazol-1-yl-pyrimidin-4-yl]-2-(4-méthyl-pipérazin-1-yl)- acétamide;
N-[2-(2,3-Difluoro-phényl)-6-pyrazol-1-yl-pyrimidin-4-yl]-2-(4-méthyl-pipéradin-1-yl)- acétamide;
N-[2-(3-Trifluorométhoxy-phényl)-6-pyrazol-1-yl-pyrimidin-4-yl]-2-(4-méthyl-pipérazin-1-yl) -acétamide;
N-[2-(2-Fluoro-3-méthoxy-phényl)-6-pyrazol-1-yl-pyrimidin-4-yl]-2-(4-méthyl-pipérazin-1-yl) -acétamide;
N-[2-(5-Méthyl-furan-2-yl)-6-pyridin-2-yl-pyrimidin-4-yl]-2-morpholin-4-yl - acétamide;
N-[2-(5-Méthyl-furan-2-yl)-6-pyridin-2-yl-pyrimidin-4-yl]-2-pipéridin-1-yl-acétamide;
2-[4-(isoprapylcarbamoyl-méthyl)-pipérazin-1-yl]-N-[2-(5-méthyl-furan-2-yl)-6-pyridin-2-yl-pyrimidin-4-yl] -acétamide;
N-[2-(5-Méthyl-furan-2-yl)-6-pyridin-2-yl-pyrimidin-4-yl]-2-(4-phényl-pipéridin-1-yl)-acétamide;
2-[1,4']Bipipéridinyl-1'-yl-N-[2-(5-méthyl-furan-2-yl)-6-pyridin-2-yl-pyrimidin-4-yl]-acétamide;
2-(3,4-Dihydro-H-isoquinolin-2-yl)-N-[2-(5-méthyl-furan-2-yl)-6-pyridin-2-yl-pyrimidin-4-yl] -acétamide;
2-[4-(3-Méthoxy-phényl)-pipérazin-1-yl)-N-[2-(5-méthyl-furan-2-yl)-6-pyridin-2-yl-pyrimidin-4-yl] -acétamide;
N-[2-(5-Méthyl-furan-2-yl)-6-pyridin-2-yl-pyrimidin-4-yl]-2-{4-méthyl-pipérazin-1-yl)-acétamide;
2-((R)-3-Diméthylamino-pyrrolidino-1-yl)-N-(2-pyrrolidin-2-yl-6-thiazol-2-yl-pyrimidin-4-yl)-acétamide;
2-((S)-3-Diméthylamino-pyrrolidin-1-yl)-N-(2-pyridin-2-yl-6-thiazol-2-yl-pyrimidin-4-yl)-acétamide;
2-(2,5-Diméthyl-pipérazin-1-yl)-N-(2-pyridin-2-yl-6-thiazol-2-yl-pyrimidin-4-yl)-acétamide; ,
2-(3,5-Diméthyl-pipérazin-1-yl)-N-(2-pyridin-2-yl-6-thiazol-2-yl-pyrimidin-4-yl) -acétamide;
2-(4-Phényl-pipérazin-1-yl)-N-(2-pyridin-2-yl-6-thiazol-2-yl-pyrimidin-4-yl) - acétamide;
2-[4-(2-Méthoxy-phényl)-pipérazin-1-yl]-N-(2-pyridin-2-yl-6-thiazol-2-yl-pyrimidin-4-yl)-acétamide;
2-(4-Méthyl-pipérazin-1-yl)-N-{2-pyridin-2-yl-6-thiazol-2-yl-pyrimidin-4-yl) - acétamide;
2-(4-Benzyl-pipérazin-1-yl)-N-(2-pyridin-2-yl-6-thiazol-2-yl-pyrimidin-4-yl) - acétamide;
2-Morpholin-4-yl-N-(2-pyridin-2-yl-6-thiazol-2-yl-pyrimidin-4-yl)-acétamide;
2-(2,6-Diméthyl-morpholin-4-yl)-N-(2-pyridin-2-yl)-6-thiazol-2-yl-pyrimidin-4-yl)-acétamide;
2-pipéridin-1-yl-N-(2-pyridin-2-yl-6-thiazol-2-yl-pyrimidin-4-yl)-acétamide;
2-(2-Méthyl-pipéridin-1-yl)-N-(2-pyridin-2-yl-6-thiazol-2-yl-pyrimidin-4-yl)-acétamide;
2-(3-Méthyl-pipéridin-1-yl)-N-(2-pyridin-2-yl-6-thiazol-2-yl-pyrimidin-4-yl)-acétamide;
2-(3,3-Diméthyl-pipéridin-1-yl)-N-{2-pyridin-2-yl-6-thiazol-2-yl-pyrimidin-4-yl)-acétamide;
2-(3,5-Diméthyl-pipéridin-1-yl)-N-(2-pyridin-2-yl-6-thiazol-2-yl-pyrimidin-4-yl) -acétamide;
2-(4-Méthyl-pipéridin-1-yl)-N-(2-pyridin-2-yl-6-thiazol-2-yl-pyrimidin-4-yl) - acétamide;
2-(4-Benzyl-pipéridin-1-yl)-N-(2-pyridin-2-yl-6-thiazol-2-yl-pyrimidin-4-yl) - acétamide;
2-[1,4']Bipipéridinyl-1'-yl-N-(2-pyridin-2-yl-6-thiazol-2-yl-pyrimidin-4-yl) - acétamide;
2-((S)-2-Méthoxyméthyl-pyrrolidin-1-yl)-N-(2-pyridin-2-yl-6-thiazol-2-yl-pyrimidin-4-yl)-acétamide;
2-(Octahydro-isoquinolin-2-yl)-N-(2-pyridin-2-yl-6-thiazol-2-yl-pyrimidin-4-yl)-acétamide;
N-(2-Pyridin-2-yl-6-thiazol-2-yl-pyrimidin-4-yl)-2-((S)-2-pyrrolidin-1-ylméthyl-pyrrolidin-1-yl)-acétamide;
2-[4-(3,4-Diméthyl-phényl)-pipérazin-1-yl]-N-(2-pyridin-2-yl-6-thiazol-2-yl-pyrimidin-4-yl)-acétamide;
N-(2-Pyridin-2-yl-6-thiazol-2-yl-pyrimidin-4-yl)-2-(4-pyrrolidin-1-yl- pipéridin-1-yl)-acétamide;
2-[4-(3-Chloro-phényl)-pipérazin-1-yl]-N-(2-pyridin-2-yl-6-thiazol-2-yl-pyrimidin-4-yl)-acétamide;
2-[4-(3-Méthoxy-phényl)-pipérazin-1-yl]-N-(2-pyridin-2-yl-6-thiazol-2-yl-pyrimidin-4-yl)-acétamide;
2-[4-(4-Méthoxy-phényl)-pipérazin-1-yl]-N-(2-pyridin-2-yl-6-thiazol-2-yl-pyrimidin-4-yl)-acétamide;
2-(4-Acétyl-pipérazin-1-yl)-N-(2-pyridin-2-yl-6-thiazol-2-yl-pyrimidin-4-yl) - acétamide;
2-(4-Méthyl-[1,4]diazépan-1-yl)-N-(2-pyridin-2-yl-6-thiazol-2-yl-pyrimidin-4-yl) -acétamide;
2-[2-((S)-1-Méthyl-pyrrolidin-2-ylméthyl)-pipéridin-1-yl]-N-(2-pyridin-2-yl-6-thiazol-2-yl-pyrimidin-4-yl) -acétamide;
2-[2-(2-pipéridin-1-yl-éthyl)-pipéridin-1-yl]-N-(2-pyridin-2-yl-6-thiazol-2-yl)-pyrimidin-4-yl)-acétamide;
2-((R)-2-Méthyl-pipérazin-1-yl)-N-(2-pyridin-2-yl-6-thiazol-2-yl-pyrimidin-4-yl).acétamide;
2-(2,5-Dihydro-pyrrol-1-yl)-N-(2-pyridin-2-yl-6-thiazol-2-yl-pyrimidin-4-yl) - acétamide;
2-(4-Acétyl-[1,4]diazépan-1-yl)-N-(2-pyridin-2-yl-6-thiazol-2-yl-pyrimidin-4-yl) -acétamide;
2-(3-Diméthylamino-pyrrolidin-1-yl)-N-(2-pyridin-2-yl-6-thiazol-2-yl-pyrimidin-4-yl)-acétamide;
N-(2-Pyridin-2-yl-6-thiazol-2-yl-pyrimidin-4-yl)-2-(3-trifluorométhyl-pipéridin-1-yl)-acétamide;
2-(3-Diéthylamino-pyrrolidin-1-yl)-N-(2-pyridin-2-yl-6-thiazol-2-yl-pyrimidin-4-yl) -acétamide;
2-((R)-2-Méthoxyméthyl-pyrrolidin-1-yl)-N-(2-pyridin-2-yl-6-thiazol-2-yl-pyrimidin-4-yl) -acétamide;
2-(2,5-Diméthyl-2,5-dihydro-pyrrolidin-1-yl)-N-(2-pyridin-2-yl-6-thiazol-2-yl-pyrimidin-4-yl) -acétamide;
N-(2-Pyridin-2-yl-6-thiazol-2-yl-pyrimidin-4-yl)-2-pyrrolidin-1-yl -acétamide;
2-(2,5-Diméthyl-pyrrolidin-1-yl)-N-(2-pyridin-2-yl-6-thiazol-2-yl-pyrimidin-4-yl)-acétamide;
2-(3-Phényl-pipéridin-1-yl)-N-(2-pyridin-2-yl-6-thiazol-2-yl-pyrimidin-4-yl)-acétamide;
2-(2-Phényl-pipéridin-1-yl)-N-(2-pyridin-2-yl-6-thiazol-2-yl-pyrimidin-4-yl) acétamide;
2-[1,4]Oxazépan-4-yl-N-(2-pyridin-2-yl-6-thiazol-2-yl-pyrimidin-4-yl} - acétamide;
2-(4,4-Difluoro-pipéridin-1-yl)-N-(2-pyridin-2-yl-6-thiazol-2-yl-pyrimidin-4-yl) -acétamide;
2-(4-Phényl-pipéridin-1-yl)-N-(2-pyridin-2-yl-6-thiazol-2-yl-pyrimidin-4-yl) acétamide;
2-Pipérazin-1-yl-N-(2-pyridin-2-yl-6-thiazol-2-yl-pyrimidin-4-yl)-acétamide;
2-[4-(isopropylcarbamoyl-méthyl)-pipérazin-1-yl]-N-(2-pyridin-2-yl-6-thiazol-2-yl-pyrimidin-4-yl) -acétamide;
acide 1-[(2-Pyridin-2-yl-6-thiazol-2-yl-pyrimidin-4-ylcarbamoyl)-méthyl]-pipéridine-3-carboxylique amide;
2-[1,4]Diazépan-1-yl-N-(2-pyridin-2-yl-6-thiazol-2-yl-pyrimidin-4-yl) - acétamide;
2-[Méthyl-(2-pyrrolidin-1-yl-éthyl)-amino]-N-(2-pyridin-2-yl-6-thiazol-2-yl-pyrimidin-4-yl) -acétamide;
2-(3,5-Diméthyl-pipérazin-1-yl)-N-(2-pyridin-2-yl-6-thiazol-2-yl-pyrimidin-4-yl) -acétamide;
2-(4-Acétyl-pipérazin-1-yl)-N-(2-pyridin-2-yl-6-thiazol-2-yl-pyrimidin-4-yl} - acétamide;
2-[4-(3-Chloro-phényl)-pipérazin-1-yl]-N-(2-pyridin-2-yl-6-thiazol-2-yl-pyrimidin-4-yl)-acétamide;
2-Morpholin-4-yl-N-(2-pyridin-2-yl-6-thiazol-2-yl-pyrimidin-4-yl)-acétamide;
2-pipéridin-1-yl-N-(2-pyridin-2-yl-6-thiazol-2-yl-pyrimidin-4-yl)-acétamide;
2-{4-Méthyl-pipérazin-1-yl)-N-(6-pyridin-2-yl-2-thiophèn-2-yl-pyrimidin-4-yl} -acétamide;
2-Morpholin-4-yl-N-(6-pyridin-2-yl-2-thiophèn-2-yl-pyrimidin-4-yl) - acétamide;
N-(6-Pyridin-2-yl-2-thiophèn-2-yl-pyrimidin-4-yl)-2-pyrrolidin-1-yl -acétamide;
2-[Méthyl-1-méthyl-pipéridin-4-yl)-amino]-N-(6-pyridin-2-yl-2-thiophèn-2-yl-pyrimidin-4-yl) -acétamide;
2-[1,4]Diazépan-1-yl-N-(5-pyridin-2-yl-2-thiophèn-2-yl-pyrimidin-4-yl) - acétamide;
2-(4-Méthyl-[1,4]diazépan-1-yl )-N-(6-pyridin-2-yl-2-thiophèn-2-yl-pyrimidin-4-yl) -acétamide;
2-(4-Éthyl-[1,4]diazépan-1-yl)-N-(6-pyridin-2-yl-2-thiophèn-2-yl-pyrimidin-4-yl)-acétamide;
2-(4-Propyl-[1,4] diazépan-1-yl)-N-(6-pyridin-2-yl-2-thiophèn-2-yl-pyrimidin-4-yl}-acétamide;
2-(4-Amino-pipéridin-1-yl)-N-(6-pyridin-2-yl-2-thiophèn-2-yl-pyrimidin-4-yl)-acétamide;
2-(4-Diméthylamlno-pipéridin-1-yl)-N-(6-pyridin-2-yl-2-thiophèn-2-yl-pyrimidin-4-yl)-acétamide;
2-(4-Diéthylamino-pipéridin-1-yl)-N-(6-pyridin-2-yl-2-thiophèn-2-yl-pyrimidin-4-yl)-acétamide;
2-(4-Dipropylamino-pipéridin-1-yl)-N-(6-pyridin-2-yl-2-thiophèn-2-yl-pyrimidin-4-yl)-acétamide;
2-(4-Acétylamino-pipéridin-1-yl)-N-(6-pyridin-2-yl-2-thiophèn-2-yl-pyrimidin-4-yl)-acétamide;
N-{1-[(6-Pyridin-2-yl-thiophèn-2-yl-pyrimidin-4-ylcarbamoyl)-méthyl]-pipéridin-4-yl)-propionamide;
N-(6-pyridin-2-yl-2-thiophèn-2-yl-pyrimidin-4-yl)-2-(4-pyrrolidin-1-yl-pipéridin-1-yl)-acétamide;
2-(4-Morpholin-4-yl-pipéridin-1-yl)-N-(6-pyridin-2-yl-2-thiophèn-2-yl-pyrimidin-4-yl)-acétamide;
2-[1,4']Bipipéridinyl-1'-yl-N-(6-pyridin-2-yl-2-thiophèn-2-yl-pyrimidin-4-yl)-acétamide;
2-[4-{2-Oxo-imidazolidin-1-yl)-pipéridin-1-yl]-N-(6-pyridin-2-yl-2-thiophèn-2-yl-pyrimidin-4-yl) -acétamide;
2-(4-Acetimidoylamino-pipéridin-1-yl)-N-(6-pyridin-2-yl-2-thiophèn-2-yl-pyrimidin-4-yl)-acétamide;
2-(4-Azétidin-1-yl-pipéridin-1-yl)-N-(6-pyridin-2-yl-2-thiophèn-2-yl-pyrimidin-4-yl)-acétamide;
N-(2-Furan-2-yl-6-thiazol-2-yl-pyrimidin-4-yl)-2-(4-méthyl-pipérazin-1-yl) - acétamide;
2-pipéridin-1-yl-N-(6-thiazol-2-yl-2-thiophèn-2-yl-pyrimidin-4-yl) -acétamide;
2-(2-Pyrrolidin-1-ylméthyl-pipéridin-1-yl)-N-(6-thiazol-2-yl-thiophèn-2-yl-pyrimidin-4-yl)-acétamide;
2-[2-(2-pipéridin-1-yl-éthyl)-pipéridin-1-yl]-N-(6-thiazol-2-yl-2-thiophèn-2-pyrimidin-4-yl) -acétamide;
2-(3-Méthyl-pipéridin-1-yl)-N-(6-thiazol-2-yl-2-thiophèn-2-yl-pyrimidin-4-yl) - acétamide;
N-(6-Thiazol-2-yl-2-thiophèn-2-yl-pyrimidin-4-yl)-2-(3-trifluorométhyl-pipéridin-1-yl)-acétamide;
Acide 1-[(6-Thiazol-2-yl-2-thiophèn-2-yl-pyrimidin-4-ylcarbamoyl)-méthyl)-pipéridine-3-carboxylique amide;
2-[1,4']Bipipéridinyl-1'-yl-N-(6-thiazol-2-yl-2-thiophèn-2-yl-pyrimidin-4-yl)-acétamide;
2-[1,4]Diazépan-1-yl-N-(6-thiazol-2-yl-2-thiophèn-2-yl-pyrimidin-4-yl)-acétamide;
2-(4-Acétyl-[1,4]diazépan-1-yl)-N-(6-thiazol-2-yl-2-thiophèn-2-yl-pyrimidin-4-yl)-acétamide;
2-(4-Pyrrolidin-1-yl-pipéridin-1-yl)-N-(6-thiazol-2-yl-2-thiophèn-2-yl-pyrimidin-4 -yl) -acétamide;
2-pipérazin-1 -N-(6-thiazol-2-yl-2-thiophèn-2-yl-pyrimidin-4-yl)-acétamide;
2-(2,5-Diméthyl-pipérazin-1-yl)-N-(6-thiazol-2-yl-2-thiophèn-2-yl -pyrimidin-4 -yl) -acétamide;
2-(3,5-Diméthyl-pipérazin-1-yl)-N-(6-thiazol-2-yl-2-thiophèn-2 -yl -pyrimidin-4 -yl) -acétamide;
2-(4-Acétyl-pipérazin-1-yl)-N-(6-{1-[(E)-méthylimino]-éthyl}-2-thiophèn-2-yl-pyrimidin-4-yl)-acétamide;
2-(4-Phényl-pipérazin-1-yl)-N-(6-thiazol-2-yl-2-thiophèn-2-yl-pyrimidin-4 -yl) - acétamide;
N-Isopropyl-2-{4-[(6-thiazol-2-yl-2-thiophèn-2-yl-pyrimidin-4-ylcarbamoyl)-méthyl]-pipérazin-1-yl}-acétamide;
2-Morpholin-4-yl-N-(6-thiazol-2-yl-2-thiophèn-2-yl-pyrimidin-4-yl)-acétamide;
2-(2,6-Diméthyl-morpholin-4-yl)-N-(6-thiazol-2-yl-2-thiophèn-2-yl-pyrimidin-4 -yl) -acétamide;
N- (6 -Thiazol -2 -yl -2 -thiophèn-2-yl -pyrimidin-4 -yl)-2 -thiomorpholin-4-yl-acétamide;
2-Pyrrolidin-1-yl-N-(6-thiazol-2-yl-2-thiophèn-2-yl-pyrimidin-4-yl)-acétamide;
2-(3-Diméthylamino-pyrrolidin-1-yl)-N-(6-thiazol-2-yl-2-thiophèn-2-yl-pyrimidin-4-yl)-acétamide;
2-(3-Diéthylamino-pyrrolidin-1-yl)-N-(6-thiazol-2-yl-2-thiophèn-2-yl-pyrimidin-4-yl)-acétamide;
2-[2-(1-Méthyl-pyrrolidin-2-yl)-éthylamino]-N-(6-thiazol-2-yl-2-thiophèn-2-yl-pyrimidin-4-yl)-acétamide;
2-(3,5-Diméthyl-pipéridin-1-yl)-N-(6-thiazol-2-yl-2-thiophèn-2-yl-pyrimidin-4-yl)-acétamide;
2-(4,4-Difluoro-pipéridin-1-yl)-N-(6-thiazol-2-yl-2-thiophèn-2-yl-pyrimidin-4-yl)-acétamide;
2-(4-Phényl-pipéridin-1-yl)-N-(6-thiazol-2-yl-2-thiophèn-2-yl-pyrimidin-4-yl)-acétamide;
2-Azépan-1-yl-N-(6-thiazol-2-yl-2-thiophèn-2-yl-pyrimidin-4-yl)-acétamide;
Acide 1-[(6-Thiazol-2-yl-2-thiophèn-2-yl-pyrimidin-4-ylcarbamoyl)-méthyl]-pipéridine-4-carboxylique amide;
2-[4-(3-Méthoxy-phényl)-pipérazin-1-yl]-N-(6-thiazol-2-yl-2-thiophèn-2-yl-pyrimidin-4-yl)-acétamide;
2-((R)-3-Diméthylamino-pyrrolidin-1-yl)-N-(6-thiazol-2-yl-2-thiophèn-2-yl-pyrimidin-4-yl)-acétamide;
2-((S)-3-Diméthylamino-pyrrolidin-1-yl)-N-(6-thiazol-2-yl-2-thiophèn-2-yl-pyrimidin-4-yl)-acétamide;
2-[(1-Éthyl-pyrrolidin-2-ylméthyl)-amino]-N-(6-thiazol-2-yl-2-thiophèn-2-yl-pyrimidin-4-yl)-acétamide;
2-((R)-3-Éthylamino-pyrrolidin-1-yl)-N-(6-thiazol-2-yl-2-thiophèn-2-yl-pyrimidin-4-yl)-acétamide;
2-((S)-3-Éthylamino-pyrrolidin-1-yl)-N-(6-thiazol-2-yl-2-thiophèn-2-yl-pyrimidin-4-yl)-acétamide;
2-(4-Méthyl-pipérazin-1-yl)-N-(6-thiazol-2-yl-2-thiophèn-2-yl-pyrimidin-4-yl)-acétamide;
Acide 1-[(6-thiazol-2-yl-2-thiophèn-2-yl-pyrimidin-4-ylcar-bamoyl)-méthyl]-pipéridine-3-carboxylicque amide;
2-(4-Pyrrolidin- 1 -yl-pipéridin-1-yl)-N-(6-thiazol-2-yl-2-thiophèn-2-yl-pyrimidin-4-yl)-acétamide;
2-(3,5-Diméthyl-pipérazin-1-yl)-N-(6-thiazol-2-yl-2-thiophèn-2-yl-pyrimidin-4-yl)-acétamide;
2-[2-(1-Méthyl-pyrrolidin-2-yl)-éthylamino]-N-(6-thia-zol-2-yl-2-thiophèn-2-yl-pyrimidin-4-yl)-acétamide;
N-[2-(5-Méthyl-furan-2-yl)-6-pyrazol-1-yl-pyrimidin-4-yl]-2-(4-pyrrolidin-1 - yl-pipéridin-1-yl)-acétamide;
N-[2-(5-Méthyl-furan-2-yl)-6-pyrazol-1-yl-pyrimidin-4-yl]-2-(4-méthyl-pipérazin-1 -yl)-acétamide;
2-(4-Acétyl-pipérazin-1-yl)-N-[2-(5-méthyl-furan-2-yl)-6-pyrazol-1-yl-pyrimidin-4-yl] -acétamide;
2-((S)-3-Éthylamino-pyrrolidin-1-yl)-N-[2-(5-méthyl-furan-2-yl)-6-pyrazol-1-yl-pyrimidin-4-yl]-acétamide;
2-(3,5-Diméthyl-pipérazin-1-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-((R)-3-éthylamino-pyrrolidin-1-yl)-acétamide;
N-[6-(3,5-Diméthyl-pyrazol-1-yl)-2-(5-méthyl-furan-2-yl)-pyrimidin-4-yl]-2-((S)-3-éthylamino-pyrrolidin-1-yl)-acétamide;
2-(3-Diéthylamino-pyrrolidin-1-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-(pyrrolidin-3-ylamino)-acétamide;
N-[6-(3,5-Diméthyl-pyrazol-1-yl)-2-(5-méthyl-furan-2-yl)-pyrimidin-4-yl] -2-(2-pyrrolidin-1-yl-éthylamino)-acétamide;
N-[6-(3,5-Diméthyl-pyrazol-1-yl)-2-(5-méthyl-furan-2-yl)-pyrimidin-4-yl]-2-(3-pyrrolidin-1-yl-propylamino)-acétamide;
N-[6-(3,5-Diméthyl-pyrazol-1-yl)-2-(5-méthyl-furan-2-yl)-pyrimidin-4-yl]-2-[2-(1-méthyl-pyrrolidin-2-yl)-éthylamino] -acétamide;
N-[6-(3,5-Diméthyl-pyrazol-1-yl)-2-(5-méthyl-furan-2-yl)-pyrimidin-4-yl]-2-(2-pipéridin-1 -yl-éthylamino)-acétamide;
N-[6-(3,5-Diméthyl-pyrazol-1-yl)-2-(5-méthyl-furan-2-yl)-pyrimidin-4-yl]-2-(3-pipéridin-1 -yl-propylamino)-acétamide;
N-[6-(3,5-Diméthyl-pyrazol-1-yl)-2-(5-méthyl-furan-2-yl)-pyrimidin-4-yl]-2-(3-morpholin-4-yl-propylamino)-acétamide;
N-[6-(3,5-Diméthyl-pyrazol-1-yl)-2-(5-méthyl-furan-2-yl)-pyrimidin-4-yl]-2-(4-méthyl-pipérazin-1-yl)-acétamide;
N-[6-(3,5-Diméthyl-pyrazol-1-yl)-2-(5-méthyl-furan-2-yl)-pyrimidin-4-yl]-2-morpholin-4-yl-acétamide;
2-[1,4]Diazépan-1-yl-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-(4-pyrrolidin-1-yl-pipéridin-1-yl)-acétamide;
N-[6-(3,5-Diméthyl-pyrazol-1-yl)-2-(5-méthyl-furan-2-yl)-pyrimidin-4-yl]-2-(4-méthyl-[1,4]diazépan-1-yl)-acétamide;
N-[6-(3,5-Diméthyl-pyrazol-1-yl)-2-(5-méthyl-furan-2-yl)-pyrimidin-4-yl]-2-[1,4] oxazépan-4-yl-acétamide;
2-((R)-3-Diméthylamino-pyrrolidin-1-yl)-N-[6-(3,5-diméthyl-pyrazol-1-yl)-2-(5-méthyl-furan-2-yl)-pyrimidin-4-yl] -acétamide;
2-((S)-3-Diméthylamino-pyrrolidin-1-yl)-N-[6-(3,5-diméthyl-pyrazol-1-yl)-2-(S-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-pyrrolidin-1-yl-acétamide;
2-(4,4-Difluoro-pipéridin-1-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-(4-thiazol-2-yl-pipérazin-1-yl)-acétamide;
N-[6-(3,5-Diméthyl-pyrazol-1-yl)-2-(5-méthyl-furan-2-yl)-pyrimidin-4-yl] -2-(4-éthyl-pipérazin-1-yl)-acétamide;
2-((R)-3-Acétylamino-pyrrolidin-1-yl)-N-[6-(3,5-diméthyl-pyrazol-1-yl)-2-(5-méthyl-furan-2-yl)-pyrimidin-4-yl]-acétamide;
2-((S)-3-Acétylamino-pyrrolidin-1-yl)-N-[6-(3,5-diméthyl-pyrazol-1-yl)-2-(5-méthyl-furan-2-yl)-pyrimidin-4-yl] -acétamide;
N-[6-(3,5-Diméthyl-pyrazol-1-yl)-2-(5-méthyl-furan-2-yl)-pyrimidin-4-yl]-2-pipérazin-1-yl-acétamide;
2-(2,6-Diméthyl-morpholin-4-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-(4-hydroxy-pipéridin-1-yl)-acétamide;
2-[1,4']Bipipéridinyl-r-yl-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-(4-méthoxy-pipéridin-1-yl)-acétamide;
2-(4-Acétyl-pipérazin-1-yl)-N-[6-(3,5-diméthyl-pyrazol-1-yl)-2-(5-méthyl-furan-2-yl)-pyrimidin-4-yl]-acétamide;
2-((R)-3-Diéthylamino-pyrrolidin-1-yl)-N-[6-(3,5-diméthyl-pyrazol-1-yl)-2-(5-méthyl-furan-2-yl)-pyrimidin-4-yl]-acétamide;
2-((S)-3-Diéthylamino-pyrrolidin-1-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-[(S)-3-(éthyl-méthyl-amino)-pyrrolidin-1-yl] -acétamide;
N-[6-(3,5-Diméthyl-pyrazol-1-yl)-2-(5-méthyl-furan-2-yl)-pyrimidin-4-yl]-2-[(R)-3-(éthyl-méthyl-amino)-pyrrolidin-1-yl] -acétamide;
2-((S)-3-Amino-pyrrolidin-1-yl)-N-[6-(3,5-diméthyl-pyrazol-1-yl)-2-(5-méthyl-furan-2-yl)-pyrimidin-4-yl]-acétamide;
2-((R)-3-Amino-pyrrolidin-1-yl)-N-[6-(3,5-diméthyl-pyrazol-1-yl)-2-(5-méthyl-furan-2-yl)pyrimidin-4-yl]-acétamide;
N-[6-(3,5-Diméthyl-pyrazol-1-yl)-2-(5-méthyl-furan-2-yl)-pyrimidin-4-yl]-2-((R)-3-morpholin-4-yl-pyrrolidin-1-yl)-acétamide;
N-[6-(3,5-Diméthyl-pyrazol-1-yl)-2-(5-méthyl-furan-2-yl)-pyrimidin-4-yl]-2-((R)-3-pipéridin-1-yl-pyrrolidin-1-yl)-acétamide;
2-(R)-[1,3']Bipyrrolidinyl-l'-yl-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-((S)-3-morpholin-4-yl-pyrrolidin-1-yl)-acétamide;
2-(S)-[1,3']Bipyrrolidinyl-r-yl-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-((S)-3-pipéridin-1-yl-pyrrolidin-1-yl)-acétamide;
N-(1-{[6-(3,5-Diméthyl-pyrazol-1-yl)-2-(5-méthyl-furan-2-yl)-pyrimidin-4-ylcarbamoyl]-méthyl}-pyrrolidin-3-yl)-2,2,2-trifluoro-acétamide;
N-[6-(3,5-Diméthyl-pyrazol-1-yl)-2-(5-méthyl-furan-2-yl)-pyrimidin-4-yl]-2-((R)-3-hydroxy-pyrrolidin-1-yl)-acétamide;
N-[6-(3,5-Diméthyl-pyrazol-1-yl)-2-(5-méthyl-furan-2-yl)-pyrimidin-4-yl]-2-((S)-3-hydroxy-pyrrolidin-1-yl)-acétamide;
N-[6-(3,5-Diméthyl-pyrazol-1-yl)-2-(5-méthyl-furan-2-yl)-pyrimidin-4-yl]-2-((3R,3'R)-3-fluoro-[1,3']bipyrrolidinyl-1-yl)-acétamide;
N-[6-(3,5-Diméthyl-pyrazol-1-yl)-2-(5-méthyl-furan-2-yl)-pyrimidin-4-yl]-2-{(R)-3-[(2-méthoxy-éthyl)-méthyl-amino] -pyrrolidin-1'-yl} -acétamide;
N-[6-(3,5-Diméthyl-pyrazol-1-yl)-2-(5-méthyl-furan-2-yl)-pyrimidin-4-yl]-2-((3S,3'S)-3-fluoro-[1,3']bipyrrolidinyl-1'-yl)-acétamide;
N-[6-(3,5-Diméthyl-pyrazol-1-yl)-2-(5-méthyl-furan-2-yl)-pyrimidin-4-yl]-2-((3R,3'S)-3-fluoro-[1,3']bipyrrolidinyl-1'-yl)-acétamide;
N-[6-(3,5-Diméthyl-pyrazol-1-yl)-2-(5-méthyl-furan-2-yl)-pyrimidin-4-yl]-2-(2,5,2',3',4',5'-hexahydro-[1,3']bipyrrolidinyl-1'-yl) -acétamide;
N-[6-(3,5-Diméthyl-pyrazol-1-yl)-2-(5-méthyl-furan-2-yl)-pyrimidin-4-yl]-2-{(S)-3-[(2-méthoxy-éthyl)-méthyl-amino]-pyrrolidin-1-yl}-acétamide;
N-[6-(3,5-Diméthyl-pyrazol-1-yl)-2-(5-méthyl-furan-2-yl)-pyrimidin-4-yl]-2-((S)-3-fluoro-pyrrolidin-1-yl)-acétamide;
N-[6-(3,5-Diméthyl-pyrazol-1-yl)-2-(5-méthyl-furan-2-yl)-pyrimidin-4-yl]-2-((R)-3-fluoro-pyrrolidin-1-yl)-acétamide;
N-[6-(3,5-Diméthyl-pyrazol-1-yl)-2-(5-méthyl-furan-2-yl)-pyrimidin-4-yl]-2-pipéridin-1-yl-acétamide;
N-[6-(3,5-Diméthyl-pyrazol-1-yl)-2-(5-méthyl-furan-2-yl)-pyrimidin-4-yl]-2-[(R)-3-(2-méthoxy-éthylamino)-pyrrolidin-1-yl]-acétamide;
N-[6-(3,5-Diméthyl-pyrazol-1-yl)-2-(5-méthyl-furan-2-yl)-pyrimidin-4-yl]-2-(3-trifluorométhyl-pipéridin-1-yl)-acétamide;
N-[6-(3,5-Diméthyl-pyrazol-1-yl)-2-(5-méthyl-furan-2-yl)-pyrimidin-4-yl]-2-(3-trifluorométhyl-5,6-dihydro-8H-[1,2,4]triazolo[4,3-a]pyrazin-7-yl)-acétamide;
5 N-[6-(3,5-Diméthyl-pyrazol-1-yl)-2-(5-méthyl-furan-2-yl)-pyrimidin-4-yl]-2-[4-(2-oxo-pyrrolidin-1-yl)-pipéridin-1-yl]-acétamide;
2-(4-Acétylamino-pipéridin-1-yl)-N-[6-(3,5-diméthyl-pyrazol-1-yl)-2-(5-méthyl-furan-2-yl)-pyrimidin-4-yl]-acétamide;
Acide 4-{[6-(3,5-Diméthyl-pyrazol-1-yl)-2-(5-méthyl-furan-2-yl)-pyrimidin-4-ylcarbamoyl]-méthyl}-pipérazine-1-carboxylique phényl ester;
Acide 4-{[6-(3,5-Diméthyl-pyrazol-1-yl)-2-(5-méthyl-furan-2-yl)-pyrimidin-4-ylcarbamoyl]-méthyl}-pipérazine-1-carboxylique benzylamide;
N-[6-(3,5-Diméthyl-pyrazol-1-yl)-2-(5-méthyl-furan-2-yl)-pyrimidin-4-yl]-2-[4-(3-méthyl-benzoyl)-pipérazin-1-yl]-acétamide;
Acide 4-{[6-(3,5-Diméthyl-pyrazol-1-yl)-2-(5-méthyl-furan-2-yl)-pyrimidin-4-ylcarbamoyl]-méthyl}-pipérazine-1-carboxylique diméthylamide;
N-[6-(3,5-Diméthyl-pyrazol-1-yl)-2-(5-méthyl-furan-2-yl)-pyrimidin-4-yl]-2-{(S)-3-[éthyl(2-méthoxy-éthyl)-amino]-pyrrolidin-1-yl-acétamide;
N-[6-(3,5-Diméthyl-pyrazol-1-yl)-2-(5-méthyl-furan-2-yl)-pyrimidin-4-yl]-2-((S)-3-isopropoxy-pyrrolidin-1-yl)-acétamide;
2-(3,9-Diaza-bicyclo[4.2.1]non-3-yl)-N-[6-(3,5-diméthyl-pyrazol-1-yl)-2-(5-méthyl-furan-2-yl)-pyrimidin-4-yl]-acétamide;
Acide. 3-{[6-(3,5-Diméthyl-pyrazol-1-yl)-2-(5-méthyl-furan-2-yl)-pyrimidin-4-ylcarbamoyl]-méthyl}-3,9-diaza-bicyclo [4.2.1]nonane-9-carboxylique tert-butyl ester;
N-[6-(3,5-Diméthyl-pyrazol-1-yl)-2-(5-méthyl-furan-2-yl)-pyrimidin-4-y]-2-(9-méthyl-3,9-diaza-bicyclo[4.2.1] non-3-yl)-acétamide;
N-[6-(3,5-Diméthyl-pyrazol-1-yl)-2-(5-méthyl-furan-2-yl)-pyrimidin-4-yl]-2-(4-{[(2-méthoxy-éthyl)-méthyl-amino]-méthyl}-pipéridin-1-yl)-acétamide;
N-[6-(3,5-Diméthyl-pyrazol-1-yl)-2-(5-méthyl-furan-2-yl)-pyrimidin-4-yl]-2-[9-(2 -méthoxy-éthyl)-3,9-diaza-bicyclo [4.2.1]non-3 -yl] -acétamide;
Acide (1-{[6-(3,5-Diméthyl-pyrazol-1-yl)-2-(5-méthyl-furan-2-yl)-pyrimidin-4-ylcarbamoyl]-méthyl}-pipéridin-4-yl)-methyl-carbamique tert-butyl ester;
N-[6-(3,5-Diméthyl-pyrazol-1-yl)-2-(5-méthyl-furan-2-yl) -pyrimidin-4 -y 1] -2 - {4-[(2-méthoxy-éthyl)-méthyl-amino]-pipéridin-1-yl}-acétamide;
2-(2-Diméthylamino-morpholin-4-yl)-N-[6-(3,5-diméthyl-pyrazol-1-yl)-2-(5-méthyl-furan-2-yl)-pyrimidin-4-yl]-acétamide;
2-((S)-3-Diméthylamino-pipéridin-1-yl)-N-[6-(3,5-diméthyl-pyrazol-1-yl)-2-(5-méthyl-furan-2-yl)-pyrimidin-4-yl]-acétamide;
2-((R)-3-Diméthylamino-pipéridin-1-yl)-N-[6-(3,5-diméthyl-pyrazol-1-yl)-2-(5-méthyl-furan-2-yl)-pyrimidin-4-yl]-acétamide;
2-((R)-3-Diméthylamino-pyrrolidin-1-yl)-N-[6-(3,5-diméthyl-pyrazol-1-yl)-2-(5-méthyl-furan-2-yl)-pyrimidin-4-yl]-acétamide;
2-((S)-3-Diméthylaminométhyl-pyrrolidin-1-yl)-N-[6-(3,5-diméthyl-pyrazol-1-yl)-2-(5-méthyl-furan-2-yl)-pyrimidin-4-yl]-acétamide;
2-(4-Diméthylaminométhyl-pipéridin-1-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-kran-2-yl)-pyrimidin-4-yl]-2-(1-méthyl-pipéridin-4-ylamino)-acétamide;
N-[6-(3,5-Diméthyl-pyrazol-1-yl)-2-(5'-méthyl-furan-2-yl)-pynmidin-4-yl]-2-((R)-pyrrolidin-3-ylamino)-acétamide;
N-[6-(3,5-Diméthyl-pyrazol-1-yl)-2-(5-méthyl-furan-2-yl)-pyrimidin-4-yl] -2-{ [(R)-1-(tétrahydro-furan-2-yl)méthyl] -amino } -acétamide;
N-[6-(3,5-Diméthyl-pyrazol-l-yl)-2-(5-méthyl-furan-2-yl) -pyrimidin-4 -yl]-2 - [(tétrahydro-pyran-4-ylméthyl)-amino] -acétamide;
N-[6-(3,5-Diméthyl-pyrazol-1-yl)-2-(5-méthyl-furan-2-yl)-pyrimidin-4-yl]-2-{ [(S)-1-(tétrahydro-furan-2-yl)méthyl] -amino} -acétamide;
N-[6-(3,5-Diméthyl-pyrazol-1-yl)-2-(5-méthyl-furan-2-yl)-pyrimidin-4-yl]-2-(pipéridin-4-ylamino)-acétamide;
N-[6-(3,5-Diméthyl-pyrazol-1-yl)-2-(5-méthyl-furan-2-yl)-pyrimidin-4-yl]-2-((S)-pipéridin-3-ylamino)-acétamide;
2-(Azetidin-3-ylamino)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-2-(2-morpholin-4-yl-éthylamino)-acétamide;
N-[6-(3,5-Diméthyl-pyrazol-1-yl)-2-(5-méthyl-furan-2-yl)-pyrimidin-4-yl]-2-((R)-pipéridin-3-ylamino)-acétamide;
N-[6-(3,5-Diméthyl-pyrazol-1-yl)-2-(5-méthyl-furan-2-yl) -pyrimidin-4 -yl] -2 - [((R)-1 -pyrrolidin-2-ylméthyl) -amino] -acétamide;
N-[6-(3,5-Diméthyl-pyrazol-1-yl)-2-(5-méthyl-furan-2-yl)-pyrimidin-4-yl]-2-((S)-pyrrolidin-3-ylamino)-acétamide;
N-[6-(3,5-Diméthyl-pyrazol-1-yl)-2-(5-méthyl-furan-2-yl)-pyrimidin-4-yl]-2-((R)-pyrrolidin-3-ylamino)-acétamide;
N-[6-(3,5-Diméthyl-pyrazol-1-yl)-2-(5-méthyl-furan-2-yl) -pyrimidin-4 -yl] -2 - [((R)-1 -pyrrolidin-3-ylméthyl) -amino] -acétamide;
N-[6-(3,5-Diméthyl-pyrazol-1-yl)-2-(5-méthyl-furan-2-yl)-pyrimidin-4-yl]-3-((R)-3-éthylamino-pyrrolidin-1-yl)-propionamide;
3-((S)-3-Diméthylamino-pyrrolidin-1-yl)-N-[6-(3,5-diméthyl-pyrazol-1-yl)-2-(5-méthyl-mran-2-yl)-pyrimidin-4-yl] -propionamide;
N-[6-(3,5-Diméthyl-pyrazol-1-yl)-2-(5-méthyl-furan-2-yl)-pyrimidin-4-yl] -3 - (4-méthyl-pipérazin-1-yl)-propionamide;
N-[6-(3,5-Diméthyl-pyrazol-1-yl)-2-(5-méthyl-furan-2-yl)-pyrimidin-4-yl]-3-morpholin-4-yl-propionamide;
3-(2,6-Diméthyl-morpholin-4-yl)-N-[6-(3,5-diméthyl-pyrazol-1-yl)-2-(5-méthyl-furan-2-yl)-pyrimidin-4-yl]-propionamid;
N-[6-(3,5-Diméthyl-pyrazol-1-yl)-2-(5-méthyl-furan-2-yl)-pyrimidin-4-yl]-3-(4-pyrrolidin-1-yl-pipéridin-1-yl)-propionamide;
3-[1,4]Diazépan-1-yl-N-[6-(3,5-diméthyl-pyrazol-1-yl)-2-(5-méthyl-furan-2-yl)-pyrimidin-4-yl]-propionamide;
N-[6-(3,5-Diméthyl-pyrazol-1-yl)-2-(5-méthyl-furan-2-yl)-pyrimidin-4-yl]-3-(4-méthyl-[1 ,4]diazépan-1-yl)-propionamide;
N-[6-(3,5-Diméthyl-pyrazol-1-yl)-2-(5-méthyl-furan-2-yl)-pyrimidin-4-yl]-3-(4-méthyl-[1,4]diazépan-1-yl)-propionamide;
3-((R)-3-Diméthylamino-pyrrolidin-1-yl)-N-[6-(3,5-diméthyl-pyrazol-1-yl)-2-(5-méthyl-furan-2-yl)-pyrimidin-4-yl]-propionamide;
N-[6-(3,5-Diméthyl-pyrazol-1-yl)-2-(5-méthyl-furan-2-yl)-pyrimidin-4-yl]-3-((S)-3-éthylamino-pyrrolidin-1-yl)-propionamide;
N-[6-(3,5-Diméthyl-pyrazol-1-yl)-2-(5-méthyl-furan-2-yl)-pyrimidin-4-yl]-3-[1,4] oxazépan-4-yl-propionamide;
3-[1,4']Bipipéridinyl-1'-yl-N-[6-(3,5-diméthyl-pyrazol-1-yl)-2-(5-méthyl-furan-2-yl)-pyrimidin-4-yl]-propionamide;
N-[6-(3,5-Diméthyl-pyrazol-1-yl)-2-(5-méthyl-furan-2-yl)-pyrimidin-4-yl] -3 - (4-méthoxy-pipéridin-1-yl)-propionamide;
N-[6-(3,5-Diméthyl-pyrazol-1-yl)-2-(5-méthyl-furan-2-yl)-pyrimidin-4-yl]-3-(4-thiazol-2-yl-pipérazin-1-yl)-propionamide;
N-[6-(3,5-Diméthyl-pyrazol-1-yl)-2-(5-méthyl-furan-2-yl)-pyrimidin-4-yl] -3 - (4-éthyl-pipérazin-1-yl)-propionamide;
3-(3-Diéthylamino-pyrrolidin-1-yl)-N-[6-(3,5-diméthyl-pyrazol-1-yl)-2-(5-méthyl-furan-2-yl)-pyrimidin-4-yl]-propionamide;
3-((3R,5S)-3,5-Diméthyl-pipérazin-l-yl)-N-[6-(3,5-diméthyl-pyrazol-l-yl)-2-(5-méthyl-furan-2-yl)-pyrimidin-4-yl]-propionamide;
N-[6-(3,5-Diméthyl-pyrazol-1-yl)-2-(5-méthyl-furan-2-yl)-pyrimidin-4-yl] -3 - pipérazin-1-yl-propionamide;
N-[6-(3,5-Diméthyl-pyrazol-1-yl)-2-(5-méthyl-furan-2-yl)-pyrimidin-4-yl]-4-morpholin-4-yl-butyramide;
N-[6-(3,5-Diméthyl-pyrazol-1-yl)-2-(5-méthyl-furan-2-yl)-pyrimidin-4-yl]-4-pyrrolidin-1-yl-butyramide;
N-[6-(3,5-Diméthyl-pyrazol-1-yl)-2-(5-méthyl-furan-2-yl)-pyrimidin-4-yl]-4-((S)-3-fluoro-pyrrolidin-1-yl)-butyramide;
N-[6-(3,5-Diméthyl-pyrazol-1-yl)-2-(5-méthyl-furan-2-yl)-pyrimidin-4-yl]-4-((R)-3-fluoro-pyrrolidin-1-yl)-butyramide;
Acide 4-{[6-(3,5-Diméthyl-pyrazol-1-yl)-2-(5-méthyl-furan-2-yl)-pyrimidin-4-ylcarbamoyl]-méthyl}-pipéridine-1-carboxylique tert-butyl ester;
N-[6-(3,5-Diméthyl-pyrazol-1-yl)-2-(5-méthyl-furan-2-yl)-pyrimidin-4-yl]-2-pipéridin-4-yl-acétamide;
N-[6-(3,5-Diméthyl-pyrazol-1-yl)-2-(5-méthyl-furan-2-yl)-pyrimidin-4-yl]-2-(1-méthyl-pipéridin-4-yl)-acétamide;
N-(6-(3,5-Diméthyl-pyrazol-1-yl)-2-(5-méthyl-furan-2-yl)-pyrimidin-4-yl]-2-[1-(2-méthoxy-éthyl)-pipéridin-4-yl] -acétamide;
N-[6-(3,5-Diméthyl-pyrazol-1-yl)-2-(5-méthyl-furan-2-yl)-pyrimidin-4-yl] -2-(2-pyrrolidin-1-yl-éthoxy) -acétamide;
2-(4-Méthyl-pipérazin-1-yl)-N-(2-oxazol-2-yl-6-pyrazol-1-yl-pyrimi din-4-yl)-acétamide;
2-((R)-3-Diméthylamino-pyrrolidin-1-yl)-N-(6-pyrazol-1-yl-2-pyridin-2-yl-pyrimidin-4-yl)-acétamide;
2-((S)-3-Diméthylamino-pyrrolidin-1-yl)-N-(6-pyrazol-1-yl-2-pyridin-2-yl-pyrimidin-4-yl)-acétamide;
2-(2,6-Diméthyl-morpholin-4-yl)-N-(6-pyrazol-1-yl-2-pyridin-2-yl-pyrimidin-4-yl)-acétamide;
N-(6-Pyrazol-1-yl-2-pyridin-2-yl-pyrimidin-4-yl)-2-(4-pyrrolidin-1-yl-pipéridin-1-yl)-acétamide;
2-(4-Acétyl-pipérazin-1-yl)-N-(6-pyrazol-1-yl-2-pyridin-2-yl-pyrimidin-4-yl)-acétamide;
2-(3,5-Diméthyl-pipérazin-1-yl)-N-(6-pyrazol-1-yl-2-py-ridin-2-yl-pyridin-4 -yl) -acétamide;
2-(4-Méthyl-pipérazin-1-yl)-N-(6-pyrazol-1-yl-2-pyri-din-2-yl-pyrimidin-4 -yl) - acétamide; 2-Morpholin-4-yl-N-(6-pyrazol-1-yl-2-pyridin-2-yl-pyrimidin-4-yl)-acétamide;
2-(4-Diméthylamino-pipéridin-1-yl)-N-(6-pyrazol-1-yl-2-pyridin-2-yl-pyrimidin-4-yl) -acétamide;
2-(4-Méthyl-[1,4]diazépan-1-yl)-N-(6-pyrazol-1-yl-2-py-ridin-2-yl-pyrimidin-4-yl)-acétamide;
2-(3,5-Diméthyl-pipérazin-1-yl)-N-[6-(3,5-diméthyl-pyrazol-1-yl)-2-pyridin-2-yl-pyrimidin-4-yl]-acétamide;
N-[6-(3,5-Diméthyl-pyrazol-1-yl)-2-pyridin-2-yl-pyrimidin-4-yl]-2-(4-pyrrolidin-1-yl-pipéridin-1-yl)-acétamide;
N-[6-(3,5-Diméthyl-pyrazol-1-yl)-2-pyridin-2-yl-pyrimidin-4-yl]-2-[(S)-3-(éthyl-méthylamino)-pyrrolidin-1-yl]-acétamide;
2-((R)-3-Amino-pyrrolidin-1-yl)-N-[6-(3,5-diméthyl-pyrazol-1-yl)-2-pyridin-2-yl-pyrimidin-4-yl]-acétamide;
2-((S)-3-Amino-pyrrolidin-1-yl)-N-[6-(3,5-diméthyl-pyrazol-1-yl)-2-pyridin-2-yl-pyrimidin-4-yl]-acétamide;
N-((S)-1- {[6-(3,5-Diméthyl-pyrazol-1-yl)-2-pyridin-2-yl-pyrimidin-4-ylcarbamoyl]-méthyl}-pyrrolidin-3-yl)-2,2, 2-trifluoro-acétamide;
N-[6-(3,5-Diméthyl-pyrazol-1-yl)-2-pyridin-2-yl-pyrimidin-4-yl]-2-(3-trifluorométhyl-5,6-dihydro-8H-[1,2,4]triazolo[4,3-a]pyrazin-7-yl)-acétamide;
N-[6-(3,5-Diméthyl-pyrazol-1-yl)-2-pyridin-2-yl-pyrimidin-4-yl]-2-morpholin-4-yl-acétamide;
N-[6-(3,5-Diméthyl-pyrazol-1-yl)-2-pyridin-2-yl-pyrimidin-4-yl]-2-[1,4]oxazépan-4-yl-acétamide;
N-[6-(3,5-Diméthyl-pyrazol-1-yl)-2-pyridin-2-yl-pyrimidin-4-yl]-2-(4-méthyl-pipérazin-1-yl)-acétamide;
N-[6-(3,5-Diméthyl-pyrazol-1-yl)-2-pyridin-2-yl-pyrimidin-4-yl]-2-(4-méthyl-(1,4]diazépan-1-yl)-acétamide;
2-((R)-3-Diméthylamino-pyrrolidin-1-yl)-N-[6-(3,5-diméthyl-pyrazol-1-yl)-2-pyridin-2-yl-pyrimidin-4-yl]-acétamide;
2-((S)-3-Diméthylamino-pyrrolidin-1-yl)-N-[6-(3,5-diméthyl-pyrazol-1-yl)-2-pyridin-2-yl-pyrimidin-4-yl]-acétamide;
2-((R)-3 -Diméthylaminométhyl-pipéridin-1-yl) -N- [6-(3, 5-diméthyl-pyrazol-1-yl)-2-pyridin-2-yl-pyrimidin-4-yl]-acétamide;
2-((S)-3-Diméthylamino méthyl-pipéridin-1-yl)-N-[6-(3, 5-diméthyl-pyrazol-1 - yl)-2-pyridin-2-yl-pyrimidin-4-yl] -acétamide;
N-(2,6-Di-pyrazol-1-yl-pyrimidin-4-yl)-2-(4-méthyl-pipérazin-1-yl)-acétamide;
N-(2,6-Di-pyrazol-1-yl-pyrimidin-4-yl)-2-(4-pyrrolidin-1-yl-pipéridin-1-yl)-acétamide;
2 - (3 -Diméthylamino-pyrrolidin-1-yl) -N- (2,6-di -pyrazol -1 -yl-pyrimidin-4-yl)-acétamide;
N-(2,6-Bis-thiazol-2-yl-pyrimidin-4-yl)-2-(4-méthyl-pipérazin-1-yl)-acétamide;
2-(4-Méthyl-pipérazin-1-yl)-N-(2-oxazol-5-yl-6-pyrazol-1-yl-pyrimidin-4-yl)-acétamide;
2-(3-Diméthylamino-pyrrolidin-1-yl)-N-(2-oxazol-5-yl-6-pyrazol-1-yl-pyrimidin-4-yl)-acétamide;
N-[2-(4-Méthyl-oxazol-5-yl)-6-pyrazol-1-yl-pyrimidin-4-yl]-2-(4-méthyl-pipérazin-1-yl)-acétamide;
N-(2-Isoxazol-3-yl-6-pyrazol-1-yl-pyrimidin-4-yl)-2-(4-méthyl-pipérazin-1-yl)-acétamide;
2-(3-Diméthylamino-pyrrolidin-1-yl)-N-(2-isoxazol-3-yl-6-pyrazol-1-yl-pyrimidin-4-yl)-acétamide;
2-(4-Méthyl-pipérazin-1-yl)-N-(6-pyrazol-1-yl-2-thiazol-2-yl-pyrimidin-4-yl)-acétamide;
N-(6-Pyrazol-1-yl-2-thiazol-2-yl-pyrimidin-4-yl)-2-(4-pyrrolidin-1-yl-pipéridin-1-yl)-acétamide;
2-((S)-3-Diméthylamino-pyrrolidin-1-yl)-N-(6-pyrazol-1-yl-2-thiazol-2-yl-pyrimidin-4-yl)-acétamide;
2-((S)-3-Éthylamino-pyrrolidin-1-yl)-N-(6-pyrazol-1-yl-2-thiazol-2-yl-pyrimidin-4-yl)-acétamide;
3-((R)-3-Éthylamino-pyrrolidin-1-yl)-N-(6-pyrazol-1-yl-2-thiazol-2-yl-pyrimidin-4-yl)-propionamide;
N-[6-(3,5-Diméthyl-pyrazol-1-yl)-2-thiazol-2-yl-pyrimidin-4-yl]-3-(4-méthyl-pipérazin-1-yl)-propionamide;
N-[6-(3,5-Diméthyl-pyrazol-1-yl)-2-thiazol-2-yl-pyrimidin-4-yl]-2-(4-pyrrolidin-1 -yl-pipéridin-1-yl)-acétamide;
2-((S)-3-Diméthylamino-pyrrolidin-1-yl)-N-[6-(3,5-diméthyl-pyrazol-1-yl)-2-thiazol-2-yl-pyrimidin-4-yl]-acétamide;
N-[6-(3,5-Diméthyl-pyrazol-1-yl)-2-thiazol-2-yl-pyrimidin-4-yl] -2 - ((S) -3 - ethy lamino -pyrrolidin-1-yl) -acétamide;
N-[6-(3,5-Diméthyl-pyrazol-1-yl)-2-thiazol-2-yl-pyrimidin-4-yl]-2-(3-trifluorométhyl-5,6-dihydro-8H-[1,2,4]triazolo[4,3-a]pyrazin-7-yl)-acétamide;
N-[6-(3,5-Diméthyl-pyrazol-1-yl)-2-furan-2-yl-pyrimidin-4-yl]-2-(4-méthyl-pipérazin-1-yl)-acétamide;
N-[6-(3,5-Diméthyl-pyrazol-1-yl)-2-furan-2-yl-pyrimidin-4-yl]-2-(4-méthyl-pipérazin-1-yl)-propionamide;
N-[2-(5-Méthyl-furan-2-yl)-6-(5-méthyl-3-trifluorométhyl-pyrazol-1-yl)-pyrimidin-4-yl]-2-(4-méthyl-pipérazin-1-yl)-acétamide;
Acide (S)-Pyrrolidine-3-carboxylique [6-(3,5-diméthyl-pyrazol-1-yl)-2-(5-méthyl-furan-2-yl)-pyrimidin-4-yl]-amide;
Acide (S)-1-Méthyl-pyrrolidine-3-carboxylique [6-(3,5-diméthyl-pyrazol-1-yl)-2-(5-méthyl-furan-2-yl)-pyrimidin-4-yl]-amide;
Acide (S)-1-(2-Méthoxy-éthyl)-pyrrolidine-3-carboxylique [6-(3,5-diméthyl-pyrazol-1-yl)-2-(5-méthyl-furan-2-yl)-pyrimidin-4-yl] -amide;
Acide (R)-Pyrrolidine-3-carboxylique [6-(3,5-diméthyl-pyrazol-1-yl)-2-(5-méthyl-furan-2-yl)-pyrimidin-4-yl]-amide;
Acide (R)-1-Méthyl-pyrrolidine-3-carboxylique [6-(3,5-diméthyl-pyrazol-1-yl)-2-(5-méthyl-furan-2-yl)-pyrimidin-4-yl]-amide;
Acide (R)-1-(2-Méthoxy-éthyl)-pyrrolidine-3-carboxylique [6-(3,5-diméthyl-pyrazol-1-yl)-2-(5-méthyl-furan-2-yl)-pyrimidin-4-yl] -amide;
Acide (R)-Pyrrolidine-2-carboxylique [6-(3,5-diméthyl-pyrazol-1-yl)-2-(5-méthyl-furan-2-yl)-pyrimidin-4-yl]-amide;
Acide (R)-1-(2-Méthoxy-éthyl)-pyrrolidine-2-carboxylique [6-(3,5-diméthyl-pyrazol-1-yl)-2-(5-méthyl-furan-2-yl)-pyrimidin-4-yl] -amide;
Acide 2-[6-(3,5-Diméthyl-pyrazol-1-yl)-2-(5-méthyl-furan-2-yl)-pyrimidin-4-ylcarbamoyl]-pyrrolidine-1-carboxylique benzyl ester;
Acide (S)-Pyrrolidine-2-carboxylique [6-(3,5-diméthyl-pyrazol-1-yl)-2-(5-méthyl-furan-2-yl)-pyrimidin-4-yl]-amide;
Acide Pyrrolidine-2-carboxylique [6-(3,5-diméthyl-pyrazol-1-yl)-2-(5-méthyl-furan-2-yl)-pyrimidin-4-yl]-amide;
N-[6-(3,5-Diméthyl-pyrazol-1-yl)-2-thiazol-2-yl-pyrimidin-4-yl]-2-(4-méthyl-pipérazin-1-yl)-acétamide;
N-[6-(3,5-Diméthyl-pyrazol-1-yl)-2-thiazol-2-yl-pyrimidin-4-yl]-2-(4-pyrrolidin-1-yl-pipéridin-1-yl)-propionamide;
2-((R)-3-Diméthylamino-pyrrolidin-1-yl)-N-[6-(3,5-diméthyl-pyrazol-1-yl)-2-thiazol-2-yl-pyrimidin-4-yl] -propionamide;
N-[6-(3,5-Diméthyl-pyrazol-1-yl)-2-thiazol-2-yl-pyrimidin-4-yl]-3-((R)-3-éthylamino-pyrrolidin-1-yl)-propionamide;
N-[6-(3,5-Diméthyl-pyrazol-1-yl)-2-thiazol-2-yl-pyrimidin-4-yl]-3-[1,4]oxazépan-4-yl-propionamide;
3-((R)-3-Éthylamino-pyrrolidin-1-yl)-N-(6-pyrazol-1-yl-2-thiazol-2-yl-pyrimidin-4-yl)-propionamide;
Acide Morpholine-2-carboxylique [6-(3,5-diméthyl-pyrazol-1-yl)-2-(5-méthyl-furan-2-yl)-pyrimidin-4-yl]-amide;
Acide 4-Méthyl-morpholine-2-carboxylique [6-(3,5-diméthyl-pyrazol-1-yl)-2-(5-méthyl-furan-2-yl)-pyrimidin-4-yl]-amide;
Acide 4-(Pyrrolidine-1-carbonyl)-morpholine-2-carboxylique [6-(3,5-diméthyl-pyrazol-1-yl)-2-(5-méthyl-furan-2-yl)-pyrimidin-4-yl] -amide;
Acide Pyrrolidine-1-carboxylique [6-(3,5-diméthyl-pyrazol-1-yl)-2-(5-méthyl-furan-2-yl)-pyrimidin-4-yl]-amide;
Acide Morpholine-4-carboxylique [6-(3,5-diméthyl-pyrazol-1-yl)-2-(5-méthyl-furan-2-yl)-pyrimidin-4-yl]-amide;
Acide 4-Méthyl-pipérazine-1-carboxylique [6-(3,5-diméthyl-pyrazol-1-yl)-2-(5-méthyl-furan-2-yl)-pyrimidin-4-yl]-amide;
1-[6-(3,5-Diméthyl-pyrazol-1-yl)-2-(5-méthyl-furan-2-yl)-pyrimidin-4-yl]-3-(2-morpholin-4-yl-éthyl)-urée;
1-[6-(3,5-Diméthyl-pyrazol-1-yl)-2-(5-méthyl-furan-2-yl)-pyrimidin-4-yl]-3-(3-pipéridin-1-yl-propyl)-urée;
N-[6-(3,5-Diméthyl-pyrazol-l-yl)-2-(5-méthyl-furan-2-yl)-pyrimidin-4-yt]-2-(S)-pyrrolidin-3-yl-acétamide;
N-[6-(3,5-Diméthyl-pyrazol-1-yl)-2-(5-méthyl-furan-2-yl)-pyrimidin-4-yl]-2-((S)-1-méthyl-pyrrolidin-3-yl)-acétamide;
(S)-3-{[6-(3,5-Diméthyl-pyrazol-1-yl)-2-(5-méthyl-furan-2-yl)-pyrimidin-4-ylcarbamoyl]-méthyl} -1,1-diméthyl-pyrrolidinium;
N-[6-(3,5-Diméthyl-pyrazol-l-yl)-2-(5-méthyl-furan-2-yl)-pyrimidin-4-yl] -2-[(S)-1-(2-méthoxy-éthyl)-pyrrolidin-3-yl]-acétamide;
N-[6-(3,5-Diméthyl-pyrazol-1-yl)-2-(5-méthyl-furan-2-yl)-pyrimidin-4-yl]-2-(R)-pyrrolidin-3-yl-acétamide; N-[6-(3,5-Diméthyl-pyrazol-1-yl)-2-(5-méthyl-furan-2-yl)-pyrimidin-4-yl]-2-((R)-1-méthyl-pyrrolidin-3-yl)-acétamide;
N-[6-(3,5-Diméthyl-pyrazol-1-yl)-2-(5-méthyl-furan-2-yl)-pyrimidin-4-yl]-2-[(R)-1-(2-méthoxy-éthyl)-pyrrolidin-3-yl] -acétamide;
Acide 1-Méthyl-pipéridine-4-carboxylique [6-(3,5-diméthyl-pyrazol-1-yl)-2-(5-méthyl-furan-2-yl)-pyrimidin-4-yl]-amide;
N-[6-(3,5-Diméthyl-pyrazol-1-yl)-2-(S-méthyl-furan-2-yl)-pyrimidin-4-yl]-2-[(R)-1-(2-méthoxy-éthyl)-pipéridin-3-yl]-acétamide;
1-[6-(3,5-Diméthyl-pyrazol-1-yl)-2-(5-méthyl-furan-2-yl)-pyrimidin-4-yl]-3-(3-pyrrolidin-1-yl-propyl)-urée;
1-[6-(3,5-Diméthyl-pyrazol-1-yl)-2-(5-méthyl-furan-2-yl)-pyrimidin-4-yl]-3-[3-(4-méthyl-pipérazin-l-yl)-propyl]-urée;
1-[6-(3,5-Diméthyl-pyrazol-1-yl)-2-(5-méthyl-furan-2-yl)-pyrimidin-4-yl]-3-[2-(1-méthyl-pyrrolidin-2-yl)-éthyl]-urée;
1-[6-(3,5-Diméthyl-pyrazol-1-yl)-2-(5-méthyl-furan-2-yl)-pyrimidin-4-yl]-3-[2-(1-méthyl-pipéridin-2-yl)-éthyl]-urée;
1 -[6-(3,5-Diméthyl-pyrazol-1-yl)-2-(5-méthyl-kran-2-yl)-pyrimidin-4-yl]-3-(2-pipéridin-2-yl-éthyl)-urée;
1-[6-(3,5-Diméthyl-pyrazol-1-yl)-2-(5-méthyl-furan-2-yl)-pyrimidin-4-yl]-3-(3-morpholin-4-yl-propyl)-urée;
N-[6-(3,5-Diméthyl-pyrazol-1-yl)-2-(5-méthyl-furan-2-yl)-pyrimidin-4-yl]-2-(2-méthoxy-éthylamino)-acétamide;
Acide [6-(3,5-Diméthyl-pyrazol-1-yl)-2-(5-méthyl-furan-2-yl)-pyridin-4-yl]-carbamique 2-azépan-1-yl-éthyl ester;
Acide [6-(3,5-Diméthyl-pyrazol-1-yl)-2-(5-méthyl-furan-2-yl)-pyrimidin-4-yl]-carbamique -pipéridin-1-yl-propyl ester;
Acide [6-(3,5-Diméthyl-pyrazol-1-yl)-2-(5-méthyl-furan-2-yl)-pyrimidin-4-yl]-carbamique 3-(2-oxo-pyrrolidin-1-yl)-propyl ester;
Acide [6-(3,5-Diméthyl-pyrazol-1-yl)-2-(5-méthyl-furan-2-yl)-pyrimidin-4-yl]-carbamique 2-morpholin-4-yl-éthyl ester;
Acide [6-(3,5-Diméthyl-pyrazol-1-yl)-2-(5-méthyl-furan-2-yl)-pyrimidin-4-yl]-carbamique 2-pipéridin-1-yl-éthyl ester;
Acide [6-(3,5-Diméthyl-pyrazol-l-yl)-2-(5-méthyl-furan-2-yl)-pyrimidin-4-yl]-carbamique 2-(2-oxo-pyrrolidin-1-yl)-éthyl ester; et
Acide [6-(3,5-Diméthyl-pyrazol-1-yl)-2-(5-méthyl-furan-2-yl)-pyrimidin-4-yl]-carbamique 2-pyrrolidin-1-yl-éthyl ester.

17. Composé selon la revendication 16, où le composé est choisi parmi le groupe consistant en
N-(2-Furan-2-yl-6-pyrazol-1-yl-pyrimidin-4-yl)-2-(4-méthyl-pipérazin-1-yl)-acétamide;
N-[2-(2-Fluoro-3-méthoxy-phényl)-6-(3,5-diméthyl-pyrazol-1-yl)-pyrimidin-4-yl]-2-(4-méthyl-pipérazin-1-yl)-acétamide;
N-(2-Furan-2-yl-6-thiazol-2-yl-pyrimidin-4-yl)-2-(4-méthyl-pipérazin-1-yl)-acétamide;
N-[6-(3,5-Diméthyl-pyrazol-1-yl)-2-(5-méthyl-furan-2-yl)-pyrimidin-4-yl]-2-((S)-3-éthylamino-pyrrolidin-1-yl)-acétamide;
N-[6-(3,5-Diméthyl-pyrazol-1-yl)-2-(5-méthyl-furan-2-yl)-pyrimidin-4-yl]-2-(4-méthyl-[1,4]diazépan-1-yl)-acétamide;
2-((S)-3-Diméthylamino-pyrrolidin-1-yl)-N-[6-(3,5-diméthyl-pyrazol-1-yl)-2-(5-méthyl-furan-2-yl)-pyrimidin-4-yl]-acétamide;
2-((R)-3-Diméthylamino-pyrrolidin-1-yl)-N-[6-(3,5-diméthyl-pyrazol-1-yl)-2-(5-méthyl-furan-2-yl)-pyrimidin-4-yl]-acétamide;
N-[6-(3,5-Diméthyl-pyrazol-1-yl)-2-(5-méthyl-furan-2-yl)-pyrimidin-4-yl]-3-((R)-3-éthylamino-pyrrolidin-1-yl)-propionamide;
3-((S)-3-Diméthylamino-pyrrolidin-1-yl)-N-[6-(3,5-diméthyl-pyrazol-1-yl)-2-(5-méthyl-furan-2-yl)-pyrimidin-4-yl]-propionamide;
N-[6-(3,5-Diméthyl-pyrazol-1-yl)-2-(5-méthyl-furan-2-yl)-pyrimidin-4-yl]-3-[1,4]oxazépan-4-yl-propionamide;
N-[6-(3,5-Diméthyl-pyrazol-1-yl)-2-pyridin-2-yl-pyrimidin-4-yl]-2-(4-méthyl-pipérazin-1-yl)-acétamide
2-(4-Méthyl-pipérazin-1-yl)-N-(6-pyrazol-1-yl-2-thiazol-2-yl-pyrimidin-4-yl)-acétamide;
N-(6-Pyrazol-1-yl-2-thiazol-2-yl-pyrimidin-4-yl)-2-(4-pyrrolidin-1-yl-pipéridin-1-yl)-acétamide;
2-((S)-3-Diméthylamino-pyrrolidin-1-yl)-N-(6-pyrazol-1-yl-2-thiazol-2-yl-pyrimidin-4-yl)-acétamide;
2-((S)-3-Éthylamino-pyrrolidin-1-yl)-N-(6-pyrazol-1-yl-2-thiazol-2-yl-pyrimidin-4-yl)-acétamide;
N-[6-(3,5-Diméthyl-pyrazol-1-yl)-2-thiazol-2-yl-pyrimidin-4-yl]-2-(4-méthyl-pipérazin-1-yl)-acétamide;
N-[6-(3,5-Diméthyl-pyrazol-1-yl)-2-thiazol-2-yl-pyrimidin-4-yl]-2-(4-pyrrolidin-1-yl-pipéridin-1-yl)-acétamide;
2-((S)-3-Diméthylamino-pyrrolidin-1-yl)-N-[6-(3,5-diméthyl-pyrazol-1-yl)-2-thiazol-2-yl-pyrimidin-4-yl] -acétamide;
N-[6-(3,5-Diméthyl-pyrazol-1-yl)-2-thiazol-2-yl-pyrimidin-4-yl]-2-((S)-3-éthylamino-pyrrolidin-1-yl)-acétamide;
2-((R)-3-Diméthylamino-pyrrolidin-1-yl)-N-[6-(3,5-diméthyl-pyrazol-l-yl)-2-thiazol-2-yl-pyrimidin-4-yl] -propionamide; et
1-[6-(3,5-Diméthyl-pyrazol-1-yl)-2-(5-méthyl-furan-2-yl)-pyrimidin-4-yl]-3-(3-morpholin-4-yl-propyl)-urée.

18. Composition pharmaceutique comprenant un composé selon la revendication 1 et un support ou diluant pharmaceutiquement acceptable.

19. Composition pharmaceutique comprenant un composé selon la revendication 3 et un support ou diluant pharmaceutiquement acceptable.

20. Composition pharmaceutique comprenant un composé selon la revendication 7 et un support ou diluant pharmaceutiquement acceptable.

21. Composition pharmaceutique comprenant un composé selon la revendication 13 et un support ou diluant pharmaceutiquement acceptable.

22. Composition pharmaceutique comprenant un composé selon la revendication 15 et un support ou diluant pharmaceutiquement acceptable.

23. Composition pharmaceutique comprenant un composé selon la revendication 16 et un support ou diluant pharmaceutiquement acceptable.

24. Composition pharmaceutique comprenant un composé selon la revendication 17 et un support ou diluant pharmaceutiquement acceptable.

25. Utilisation d'un composé selon la revendication 1 dans la fabrication d'une composition pharmaceutique pour le traitement d'un sujet ayant un état susceptible d'amélioration par un antagonisme avec un récepteur de l'adénosine.

26. Utilisation selon la revendication 25, où l'état est une ischémie, des arythmies supraventriculaires, une insuffisance rénale aiguë, une lésion de reperfusion myocardique, une maladie auto-immune, des affections abdominales inflammatoires, l'asthme, des diabètes sucrés, l'obésité, la maladie de Parkinson, la maladie de Huntington, la dystonie ou la dyskinésie.

27. Utilisation du composé selon la revendication 1 dans la fabrication d'une composition pharmaceutique pour le traitement d'un sujet ayant un état susceptible d'amélioration par un antagonisme avec un récepteur de l'adénosine A2A.

28. Utilisation selon la revendication 27, où l'état est une ischémie, des arythmies supraventriculaires, la maladie de Parkinson, la maladie de Huntington, la dystonie ou la dyskinésie.
